# EUROPEAN PATENT SPECIFICATION

(11) **EP 3 202 415 B1**
(45) Date of publication and mention of the grant of the patent: **08.12.2021**
(21) Application number: 17161149.4
(22) Date of filing: 30.06.2011
(51) Int. Cl.: A61K 38/17, A61P 25/28

(54) **FUSION PROTEIN COMPRISING A C1ORF32 POLYPEPTIDE AND ONE OR MORE DOMAINS OF AN IMMUNOGLOBULIN HEAVY CHAIN CONSTANT REGION FOR TREATMENT OF MULTIPLE SCLEROSIS, RHEUMATOID ARTHRITIS AND OTHER AUTOIMMUNE DISORDERS.**
FUSIONSPROTEIN BEINHALTEND C1ORF32 UND EINE ODER MEHRERE DOMÄNEN EINER IMMUNOGLOBULIN HEAVY CHAIN KONSTANTEN REGION ZUR BEHANDLUNG VON MULTIPLER SKLEROSE, RHEUMATOIDER ARTHRITIS UND ANDEREN AUTOIMMUNERKRANKUNGEN
ROTÉINE DE FUSION COMPRENANT LA C1ORF32 ET UN OU PLUSIEURS DOMAINES DE LA RÉGION CONSTANTE DE LA CHAINE CONSTANTE D'IMMUNUOGLOBULINE POUR LE TRAITEMENT DE LA SCLÉROSE EN PLAQUES, LA POLYARTHRITE RHUMATOÏDE ET D'AUTRES MALADIES AUTO-IMMUNES

(30) Priority: 30.06.2010 US 360011 P
(43) Date of publication of application: 09.08.2017
(62) Divisional of application: 11748719.9
(73) Proprietor: Compugen Ltd., 5885849 Holon (IL)
(72) Inventor: HECHT, Iris, Tel Aviv (IL); ROTMAN, Galit, 46725 Herzliya (IL); LEVINE, Zurit, 46725 Herzliya (IL); MILLER, Stephen D., Chicago, IL Illinois 60611 (US); PODOJIL, Joseph R., Chicago, IL Illinois 60611 (US)
(74) Representative: Fuchs Patentanwälte Partnerschaft mbB

(56) References cited:
- WO-A2-2009/032845
- Anonymous: "CGEN-15001 An Example of "Discovery on Demand"", , 27 July 2010 (2010-07-27), pages 1-25, XP055029933, Retrieved from the Internet: URL:http://cgen.com/Data/Uploads/Images/CG EN-15001, an example of discovery.pdf [retrieved on 2012-06-14]
- anonymous: "New membrane protein for the treatment of autoimmune diseases", , 3 February 2010 (2010-02-03), pages 1-3, XP002677793, Retrieved from the Internet: URL:http://themspodcast.com/research/new-m embrane-protein-for-the-treatment-of-autoi mmune-diseases/ [retrieved on 2012-06-14]
- anonymous: "Compugen Announces Positive Therapeutic Effect of CGEN-15001 in Animal Model of Rheumatoid Arthritis", , 14 December 2010 (2010-12-14), pages 1-3, XP002677794, Retrieved from the Internet: URL:http://www.drugs.com/clinical_trials/c ompugen-announces-positive-therapeutic-cge n-15001-animal-model-rheumatoid-arthritis- 10899.html [retrieved on 2012-06-14]
- GONZALEZ-REY E ET AL: "Human adipose-derived mesenchymal stem cells reduce inflammatory and T cell responses and induce regulatory T cells in vitro in rheumatoid arthritis", ANNALS OF THE RHEUMATIC DISEASES, BRITISH MEDICAL ASSOCIATION, LONDON, GB , vol. 69, no. 1 1 January 2010 (2010-01-01), pages 241-248, XP008152922, ISSN: 0003-4967, DOI: 10.1136/ARD.2008.101881 Retrieved from the Internet: URL:http://ard.bmjjournals.com/ [retrieved on 2009-01-05]
- GONZALEZ-REY E ET AL: "Vasoactive intestinal peptide induces CD4+,CD25+ T regulatory cells with therapeutic effect in collagen-induced arthritis", ARTHRITIS & RHEUMATISM, JOHN WILEY & SONS, INC, US, vol. 54, no. 3, 1 January 2006 (2006-01-01), pages 864-876, XP002441869, ISSN: 0004-3591, DOI: 10.1002/ART.21652
- Elvin A. Kabat ET AL: "Sequences of Proteins of Immunological Interest" In: "Sequences of Proteins of Immunological Interest", 1 January 1991 (1991-01-01), XP055374199, page FP1-2,iv,661,662,671,680, * pages 662,671 *
- Sandrine Béranger ET AL: "IMGT Scientific chart: Correspondence between the IM GT unique numbering for C-DOM AIN, the IM GT exon numbering, the Eu and Kabat numberings: Human IGHG", , 17 May 2001 (2001-05-17), XP055297333, Retrieved from the Internet: URL:http://www.imgt.org/IMGTScientificChar t/Numbering/Hu_IGHGnber.html [retrieved on 2016-08-24]
- Elodie Foulquier: "IMGT Scientific chart - IMGT color menu", , 19 April 2001 (2001-04-19), pages 1-9, XP055374236, Retrieved from the Internet: URL:http://www.imgt.org/IMGTScientificChar t/RepresentationRules/colormenu.php [retrieved on 2017-05-18]
- E V Koonin ET AL: "Chapter 2 Evolutionary Concept in Genetics and Genomics" In: "Sequence - Evolution - Function: Computional Approaches in Comparative Genomics", 1 January 2003 (2003-01-01), Kluwer Academic, Boston, USA, XP055374527, pages 1-23, * paragraph [02.1] *
- RAGHAVA GPS ET AL: "Quantification of the variation in percentage identity for protein sequence alignments", BMC BIOINFORMATICS, BIOMED CENTRAL, LONDON, GB, vol. 7, no. 1, 19 September 2006 (2006-09-19), page 415, XP021013928, ISSN: 1471-2105, DOI: 10.1186/1471-2105-7-415
- NEEDLEMAN S B ET AL: "A general method applicable to the search for similarities in the amino acid sequence of two proteins", JOURNAL OF MOLECULAR BIOLOGY, ACADEMIC PRESS, UNITED KINGDOM, vol. 48, no. 3, 28 March 1970 (1970-03-28) , pages 443-453, XP024011703, ISSN: 0022-2836, DOI: 10.1016/0022-2836(70)90057-4 [retrieved on 1970-03-28]
- Schulz, Heidi: "Towards a comprehensive description of the human retinal transcriptome: identification and characterization of differentially expressed genes", INTERNET CITATION, 22 December 2003 (2003-12-22), pages E1-E5, XP002614183, Retrieved from the Internet: URL:http://www.opus-bayern.de/uni-wuerzbur g/frontdoor.php?source_opus=727 [retrieved on 2010-12-14]
- HEIDI SCHULZ: "Towards a Comprehensive Descriptionof the Human Retinal Transcriptome: Identification and Characterization of Differentially Expressed Genes", INTERNET CITATION, 22 December 2003 (2003-12-22), pages E1-E169, XP002614182, Retrieved from the Internet: URL:http://www.opus-bayern.de/uni-wuerzbur g/volltexte/2003/727/pdf/Thesis.pdf [retrieved on 2010-12-14]
- Marija Dokmanovic-Chouinard ET AL: "Positional Cloning of "Lisch-like", a Candidate Modifier of Susceptibility to Type 2 Diabetes in Mice", PLoS Genetics, vol. 4, no. 7, 25 July 2008 (2008-07-25), page e1000137, XP055360371, DOI: 10.1371/journal.pgen.1000137

## Description

### FIELD OF THE INVENTION

This invention relates to fusion proteins for use in the treatment of an immune related disorder selected from multiple sclerosis, rheumatoid arthritis, type I diabetes, psoriasis, systemic lupus erythematosus, inflammatory bowel disease, uveitis, or Sjogren's syndrome.

### BACKGROUND OF THE INVENTION

Induction of immune tolerance has long been considered the "holy grail" for autoimmune disease therapy. The immune system has the reciprocal tasks to protect the host against invading pathogens, but simultaneously to prevent damage resulting from unwanted reactions to self antigens. The latter part is known as immune tolerance and performed by a complex set of interactive and complementary pathways, which regulate immune responses. T cells have the ability to react to a variety of antigens, both self and nonself. Therefore, there are many mechanisms that exist naturally to eliminate potentially self-reactive responses - this is known as natural tolerance. The main mechanism for eliminating potential autoreactive T cells occurs in the thymus and is known as central tolerance. Some potentially autoreactive T cells escape central tolerance and, therefore, peripheral tolerance mechanisms also exist. Despite these mechanisms, some self-reactive T cells may 'escape' and be present in the repertoire; it is believed that their activation may lead to autoimmune disease.

Studies on therapeutic tolerance have attempted to induce and amplify potent physiological mechanisms of tolerance in order to eliminate or neutralize self-reactive T cells and prevent or treat autoimmune diseases. One way to induce tolerance is by manipulation of the interaction between costimulatory ligands and receptors on antigen presenting cells (APCs) and lymphocytes.

CTLA-4 is the most extensively studied costimulatory molecule which downregulates immune responses. The attributes of immunosuppressive qualities and capacity to induce tolerance have made its recognition as a potential immuno-therapeutic agent for autoimmune mediated inflammatory disorders. Abatacept (commercial name: Orencia) is a fusion protein composed of the ECD (extracellular domain) of CTLA-4 fused to the Fc fragment of hIgG1. Abatacept is believed to induce costimulation blockade, which has been approved for treating patients with rheumatoid arthritis, by effectively interfering with the inflammatory cascade.

Induction of disease control with the current therapies, followed by progressive withdrawal in parallel with re-establishing immune tolerance, may be an attractive approach in the future of autoimmune therapies. Furthermore, due to their immune specificity, in the absence of global immunosuppression, such therapies should be safe for chronic use.

Multiple sclerosis (MS) is a chronic, inflammatory, demyelinating disorder of the central nervous system (CNS), which involves autoimmune responses to myelin antigens. It is characterized by lesions within the CNS and demyelination is a key feature of these lesions. Autoreactive T cells are thought to initiate an autoimmune response directed against components of CNS myelin. The main targets of the autoimmune reactions are thought to be myelin basic protein (MBP), proteolipid protein (PLP) and myelin oligodendrocyte glycoprotein (MOG). Experimental autoimmune encephalomyelitis (EAE), an animal model of MS induced by immunization with myelin components in adjuvant, shows comparable neuronal pathology. Wihout wishing to be limited by a single hypothesis, studies in EAE have provided convincing evidence that T cells specific for self-antigens mediate pathology in these diseases.

T helper type 1 (Th1) cells are induced by IL-12 and produce IFN-γ, while T helper type 2 (Th2) cells secrete IL-4, IL-5 and IL-13. Th1 cells can mediate proinflammatory or cell-mediated immune responses, whereas Th2 cells mainly promote certain types of humoral immunity. Some immune related diseases, such as autoimmune reactions, inflammation, chronic infection and sepsis, are characterized by a dysregulation of the proversus anti-inflammatory tendencies of the immune system, as well as an imbanlance in the Th1 versus Th2 cytokine balance. During inflammation, induction of a shift in the balance from Th1 to Th2 protects the organism from systemic 'overshooting' with Th1/proinflammatory cytokines, by reducing the inflammatory tendencies of the immune system. Immunomodulatory therapies that are associated with a Th1 to Th2 immune shift have protective effects in Th1-mediated autoimmune diseases, such as multiple sclerosis and rheumatoid arthritis. For example, Laquinimod, which has demonstrated efficacy in animal models of several autoimmune diseases including MS, shows immunomodulatory effects through Th1/Th2 shift, and does not lead to immunosuppression. Glatiramer acetate (Copaxone) also induces Th1/Th2 shift with decreased secretion of proinflammatory cytokines, and increased secretion of antiinflammatory cytokines. Furtheremore, glatiramer acetate -specific Th2 cells are able to migrate across the blood-brain barrier and cause in situ bystander suppression of autoaggressive Th1 T cells.

Certain immune cells and immune cell signal transduction pathways are promising targets for new agents for treating immune disorders. For example Th1, Th17, Th2 and regulatory T cells (Tregs) play important roles in modulating autoimmunity and inflammation. Mounting evidence from numerous studies shows the importance of these immune cells in disorders such as rheumatoid arthritis, inflammatory bowel disease, multiple sclerosis, psoriasis, lupus erythematosus, type 1 diabetes and uveitis. Most existing therapies target only one pathway at a time.

WO 2009/032845 A2 relates to the discovery of certain proteins that are differentially expressed in specific tissues and their use as therapeutic and diagnostic targets. C1ORF32 is disclosed as one of these proteins. WO 2009/032845 A2 discloses the C1ORF32_T8_P8_V1_ECD_mFc (also referred to herein as C1ORF32_ECD_mFc) amino acid sequence (428aa) (herein: SEQ ID NO:8, in WO 2009/032845 A2: SEQ ID NO:105) Induction of immune tolerance in individuals with immune related disorder is not disclosed.

### BRIEF SUMMARY OF THE INVENTION

The background art fails to provide therapies that target multiple cells and pathways involved in autoimmunity and inflammation, such as Th1, Th17, Th22, Th2, Tregs, or other cells that secrete, or influence other cells that secrete, inflammatory molecules such as cytokines, metalloproteases, chemokines and other molecules.

The present invention is of fusion proteins for use in the treatment of immune related diseases selected from multiple sclerosis, rheumatoid arthritis, type I diabetes, psoriasis, systemic lupus erythematosus, inflammatory bowel disease, uveitis, or Sjogren's syndrome, the fusion protein having a first fusion partner and a second fusion partner, wherein
- the first fusion partner comprises a C1ORF32 polypeptide selected from the group consisting of polypeptide comprising a sequence of amino acid residues having at least 95% sequence identity with amino acid residues 21-186 of H19011_1_P8 (SEQ ID NO: 4), corresponding to amino acid sequence depicted in SEQ ID NO: 14, or residues 21-186 of H19011_1_P8_V1 (SEQ ID NO:5), corresponding to amino acid sequence depicted in SEQ ID NO:35, or residues 21-169 of H19011_1_P9 (SEQ ID NO:6), corresponding to amino acid sequence depicted in SEQ ID NO: 15, or residues 21-169 of H19011_1_P9_V1 (SEQ ID NO:34), corresponding to amino acid sequence depicted in SEQ ID NO:36, or residues 1-184 of the sequence H19011_1_P8 (SEQ ID NO:4), corresponding to amino acid sequence depicted in SEQ ID NO:37, or residues 1-184 of the sequence H19011_1_P8_V1 (SEQ ID NO:5), corresponding to amino acid sequence depicted in SEQ ID NO: 19, or residues 1-169 of H19011_1_P9 (SEQ ID NO:6), corresponding to amino acid sequence depicted in SEQ ID NO:28, or residues 1-169 of H19011_1_P9_V1 (SEQ ID NO:34), corresponding to amino acid sequence depicted in SEQ ID NO:30
- the second fusion partner is composed of a heterologous sequence and contains one or more domains of an immunoglobulin heavy chain constant region, and
- the fusion partners are fused together directly or indirectly via a peptide linker sequence or a chemical linker,
   characterized in that the fusion protein induces immune tolerance in individuals with the immune related disorder.

As used herein the term "immune related diseases" refers to any of the below listed types and subtypes of the following diseases: multiple sclerosis, rheumatoid arthritis, type I diabetes, psoriasis, systemic lupus erythematosus, inflammatory bowel disease, uveitis, or Sjogren's syndrome.

As used herein, "multiple sclerosis" comprises one or more of multiple sclerosis, benign multiple sclerosis, relapsing remitting multiple sclerosis, secondary progressive multiple sclerosis, primary progressive multiple sclerosis, progressive relapsing multiple sclerosis, chronic progressive multiple sclerosis, transitional/progressive multiple sclerosis, rapidly worsening multiple sclerosis, clinically-definite multiple sclerosis, malignant multiple sclerosis, also known as Marburg's Variant, and acute multiple sclerosis. Optionally, "conditions relating to multiple sclerosis" include, e.g., Devic's disease, also known as Neuromyelitis Optica; acute disseminated encephalomyelitis, acute demyelinating optic neuritis, demyelinative transverse myelitis, Miller-Fisher syndrome, encephalomyelradiculoneuropathy, acute demyelinative polyneuropathy, tumefactive multiple sclerosis and Balo's concentric sclerosis.

As used herein, "rheumatoid arthritis" comprises one or more of rheumatoid arthritis, gout and pseudo-gout, juvenile idiopathic arthritis, juvenile rheumatoid arthritis, Still's disease, ankylosing spondylitis, rheumatoid vasculitis. Optionally, conditions relating to rheumatoid arthritis include, e.g., osteoarthritis, sarcoidosis, Henoch-Schonlein purpura, Psoriatic arthritis, Reactive arthritis, Spondyloarthropathy, septic arthritis, Haemochromatosis, Hepatitis, vasculitis, Wegener's granulomatosis, Lyme disease, Familial Mediterranean fever, Hyperimmunoglobulinemia D with recurrent fever, TNF receptor associated periodic syndrome, and Enteropathic arthritis associated with inflammatory bowel disease.

As used herein, "Uveitis" comprises one or more of uveitis, anterior uveitis (or iridocyclitis), intermediate uveitis (pars planitis), posterior uveitis (or chorioretinitis) and the panuveitic form.

As used herein, "inflammatory bowel disease" comprises one or more of inflammatory bowel disease Crohn's disease, ulcerative colitis (UC), Collagenous colitis, Lymphocytic colitis, Ischaemic colitis, Diversion colitis, Behçet's disease, Indeterminate colitis.

As used herein, "psoriasis" comprises one or more of psoriasis, Nonpustular Psoriasis including Psoriasis vulgaris and Psoriatic erythroderma (erythrodermic psoriasis), Pustular psoriasis including Generalized pustular psoriasis (pustular psoriasis of von Zumbusch), Pustulosis palmaris et plantaris (persistent palmoplantar pustulosis, pustular psoriasis of the Barber type, pustular psoriasis of the extremities), Annular pustular psoriasis, Acrodermatitis continua, Impetigo herpetiformis. Optionally, conditions relating to psoriasis include, e.g., drug-induced psoriasis, Inverse psoriasis, Napkin psoriasis, Seborrheic-like psoriasis, Guttate psoriasis, Nail psoriasis, Psoriatic arthritis.

As used herein, "type 1 diabetes" comprises one or more of type 1 diabetes, insulin-dependent diabetes mellitus, idiopathic diabetes, juvenile type 1diabetes, maturity onset diabetes of the young, latent autoimmune diabetes in adults, gestational diabetes. Conditions relating to type 1 diabetes include, neuropathy including polyneuropathy, mononeuropathy, peripheral neuropathy and autonomicneuropathy; eye complications: glaucoma, cataracts, retinopathy.

As used herein, "Sjogren's syndrome" comprises one or more of Sjogren's syndrome, Primary Sjogren's syndrome and Secondary Sjogren's syndrome, as well as conditions relating to Sjogren's syndrome including connective tissue disease, such as rheumatoid arthritis, systemic lupus erythematosus, or scleroderma. Other complications include pneumonia, polmunary fibrosis, interstitial nephritis, inflammation of the tissue around the kidney's filters, glomerulonephritis, renal tubular acidosis, carpal tunnel syndrome, peripheral neuropathy, cranial neuropathy, primary biliary cirrhosis (PBC), cirrhosis, Inflammation in the esophagus, stomach, pancreas, and liver (including hepatitis), Polymyositis, Raynaud's phenomenon, Vasculitis, Autoimmune thyroid problems, lymphoma.

As used herein, "systemic lupus erythematosus", comprises one or more of systemic lupus erythematosus, discoid lupus, lupus arthritis, lupus pneumonitis, lupus nephritis. Conditions relating to systemic lupus erythematosus include osteoarticular tuberculosis, antiphospholipid antibody syndrome, inflammation of various parts of the heart, such as pericarditis, myocarditis, and endocarditis, Lung and pleura inflammation, pleuritis, pleural effusion, chronic diffuse interstitial lung disease, pulmonary hypertension, pulmonary emboli, pulmonary hemorrhage, and shrinking lung syndrome, lupus headache, Guillain-Barre syndrome, aseptic meningitis, demyelinating syndrome, mononeuropathy, mononeuritis multiplex, myasthenia gravis, myelopathy, cranial neuropathy, polyneuropathy, vasculitis.

In one embodiment, the C1ORF32 polypeptide may optionally be fused to one or more domains of an Ig heavy chain constant region having an amino acid sequence corresponding to the hinge, CH2 and CH3 regions of a human immunoglobulin Cγ1, Cγ2, Cy3 or Cy4 chains or to the hinge, CH2 and CH3 regions of a murine immunoglobulin Cy2a chain.

According to at least some embodiments of the present invention, there is provided a pharmaceutical composition comprising the C1OR-F32 fusion protein, and a pharmaceutically acceptable diluent or carrier, for use in the treatment of immune related disorder selected from multiple sclerosis, rheumatoid arthritis, type I diabetes, psoriasis, systemic lupus erythematosus, inflammatory bowel disease, uveitis, or Sjogren's syndrome.

In one embodiment but without wishing to be limited by a single hypothesis, C1ORF32 polypeptides or fusion proteins or pharmaceutical composition containing same, enhance the suppressive activity of Tregs on the immune system. Tregs can suppress differentiation, proliferation, activity, and/or cytokine production and/or secretion by Th1, Th17, Th22, and/or other cells that secrete, or cause other cells to secrete, inflammatory molecules. In one embodiment the C1ORF32 fusion proteins or pharmaceutical composition containing same, enhance the suppressive activity of Tregs on naive T cells to inhibit or reduce naive T cells from differentiating into Th1, Th17, Th22 cells and thereby reduce the number of Th1, Th17, Th22, and/or other cells that secrete, or cause other cells to secrete, inflammatory molecules, including, but not limited to, IL-1beta, TNF-alpha, TGF-beta, IFN-gamma, IL-17, IL-6, IL-23, IL-22, IL-21, and MMPs in a subject.

In one embodiment, C1ORF32 fusion proteins or pharmaceutical composition containing same, enhance the activity of Th2 immune responses or to increase the number of Th2 cells. Th2 cells can modulate the differentiation, proliferation, activity, and/or cytokine production and/or secretion by Th1, Th17, Th22, and/or other cells that secrete, or cause other cells to secrete, inflammatory molecules, resulting in inhibition of Th1 and/or Th17 responses, and in immune modulation via a Th1/Th2 shift. In one embodiment the C1ORF32 fusion proteins or pharmaceutical composition containing same, enhance the immunomodulatory activity of Th2 on naive T cells to inhibit or reduce naive T cells from differentiating into Th1, Th17, Th22 cells and thereby reduce the number of Th1, Th17, Th22, and/or other cells that secrete, or cause other cells to secrete, inflammatory molecules, including, but not limited to, IL-1beta, TNF-alpha, TGF-beta, IFN-gamma, IL-17, IL-6, IL-23, IL-22, IL-21, and MMPs in a subject. In one embodiment the C1ORF32 fusion proteins or pharmaceutical composition containing same, promote or enhance production of IL-4, IL-5 or IL-10 by Th2 cells. The composition is used for treatment of immune related disorders selected from multiple sclerosis, rheumatoid arthritis, type I diabetes, psoriasis, systemic lupus erythematosus, inflammatory bowel disease, uveitis, or Sjogren's syndrome.

The polypeptide comprises a sequence of amino acid residues having at least 95% sequence identity with amino acid residues 21-186 of H19011_1_P8 (SEQ ID NO:4), corresponding to amino acid sequence depicted in SEQ ID NO:14, or residues 21-186 of H19011_1_P8_V1 (SEQ ID NO:5), corresponding to amino acid sequence depicted in SEQ ID NO:35, or residues 21-169 of H19011_1_P9 (SEQ ID NO:6), corresponding to amino acid sequence depicted in SEQ ID NO:15, or residues 21-169 of H19011_1_P9_V1 (SEQ ID NO:34), corresponding to amino acid sequence depicted in SEQ ID NO:36, or residues 1-184 of the sequence H19011_1_P8 (SEQ ID NO:4), corresponding to amino acid sequence depicted in SEQ ID NO:37, or residues 1-184 of the sequence H19011_1_P8_V1 (SEQ ID NO:5), corresponding to amino acid sequence depicted in SEQ ID NO:19, or residues 1-169 of H19011_1_P9 (SEQ ID NO:6), corresponding to amino acid sequence depicted in SEQ ID NO:28, or residues 1-169 of H19011_1_P9_V1 (SEQ ID NO:34), corresponding to amino acid sequence depicted in SEQ ID NO:30, for use in the treatment of immune related disorder selected from multiple sclerosis, rheumatoid arthritis, type I diabetes, psoriasis, systemic lupus erythematosus, inflammatory bowel disease, uveitis, or Sjogren's syndrome. Optionally the polypeptide is attached to a detectable or therapeutic moiety.

C1ORF32 fusion proteins can be administered in combination with one or more additional therapeutic agents, including, but not limited to, antibodies against other lymphocyte surface markers (e.g., CD40, alpha-4 integrin) or against cytokines, other fusion proteins, e.g. CTLA4-Ig (Orencia^{®}, belatacept), TNFR-Ig (Enbrel^{®}), TNF- alpha blockers such as Remicade, Cimzia and Humira, CD73-Ig, cyclophosphamide (CTX) (i.e. Endoxan^{®}, Cytoxan^{®}, Neosar^{®}, Procytox^{®}, Revimmune^{™}), methotrexate (MTX) (i.e. Rheumatrex^{®}, Trexall^{®}), belimumab (i.e. Benlysta^{®}), Tysabri or other immunosuppressive drugs, antiproliferatives, cytotoxic agents, or other compounds that may assist in immunosuppression.

In one embodiment, the additional therapeutic agent targets a different pathway involved in immune activation. In a further embodiment, the additional therapeutic agent is a CTLA-4 fusion protein, such as CTLA-4 Ig (abatacept). In a further embodiment, the additional therapeutic agent is a CTLA4-Ig fusion protein known as belatacept that contains two amino acid substitutions (L104E and A29Y) that markedly increases its avidity to CD86 in vivo.

In another embodiment, the second therapeutic agent is cyclophosphamide (CTX). In a further embodiment, C1ORF32 polypeptides, fragments or fusion proteins thereof and CTX are coadministered in an effective amount to treat a chronic autoimmune disease or disorder such as Systemic lupus erythematosus (SLE).

In another embodiment, the second therapeutic agent is methotrexate (MTX). In a further embodiment, C10RF32 polypeptides, fragments or fusion proteins thereof and MTX are coadministered in an effective amount to treat a chronic autoimmune disease or disorder such as Rheumatoid arthritis (RA).In another embodiment, the second therapeutic agent increases the amount of adenosine in the serum.

In a further embodiment, the second therapeutic is CD73-Ig, recombinant CD73, or another agent (e.g. a cytokine or monoclonal antibody or small molecule) that increases the expression of CD73. In another embodiment the second therapeutic agent is Interferon- beta.

In another embodiment, the second therapeutic is Tysabri or another therapeutic for MS. In a further embodiment, C1ORF32 polypeptides, fragments or fusion proteins thereof is cycled with Tysabri or used during a drug holiday in order to allow less frequent dosing with the second therapeutic and reduce the risk of side effects such as PML and to prevent resistance to the second therapeutic.

In another embodiment, the second therapeutic agent is a small molecule that inhibits or reduces differentiation, proliferation, activity, and/or cytokine production and/or secretion by Th1, Th17, Th22, and/or other cells that secrete, or cause other cells to secrete, inflammatory molecules. In another embodiment, the second therapeutic agent is a small molecule that interacts with Tregs, enhances Treg activity, promotes or enhances IL-10 secretion by Tregs, increases the number of Tregs, increases the suppressive capacity of Tregs, or combinations thereof. In one embodiment, the small molecule is retinoic acid or a derivative thereof. In another embodiment, the second therapeutic agent is a small molecule that interacts with Th2 cells, enhances Th2 activity, promotes or enhances IL-10, IL-4 or IL-5 production by Th2 cells, increases the number of Th2 cells, increases the immunomodulatory capacity of Th2 cells, or combinations thereof.

Also disclosed, but not claimed, is a method for treating immune related disorder, comprising administering to a subject in need thereof a pharmaceutical composition comprising: a soluble molecule having the extracellular domain of C1ORF32 polypeptide, or a fragment or a variant or a homolog thereof; or a fusion protein or a conjugate thereof; or polypeptide, comprising amino acid residues 21-186 of H19011_1_P8 (SEQ ID NO:4), corresponding to amino acid sequence depicted in SEQ ID NO:14, or residues 21-186 of H19011_1_P8_V1 (SEQ ID NO:5), corresponding to amino acid sequence depicted in SEQ ID NO:35, or residues 21-169 of H19011_1_P9 (SEQ ID NO:6), corresponding to amino acid sequence depicted in SEQ ID NO:15, or residues 21-169 of H19011_1_P9_V1 (SEQ ID NO:34), corresponding to amino acid sequence depicted in SEQ ID NO:36, or residues 1-184 of the sequence H19011_1_P8 (SEQ ID NO:4), corresponding to amino acid sequence depicted in SEQ ID NO:37, or residues 1-184 of the sequence H19011_1_P8_V1 (SEQ ID NO:5), corresponding to amino acid sequence depicted in SEQ ID NO:19, or residues 1-169 of H19011_1_P9 (SEQ ID NO:6), corresponding to amino acid sequence depicted in SEQ ID NO:28, or residues 1-169 of H19011_1_P9_V1 (SEQ ID NO:34), corresponding to amino acid sequence depicted in SEQ ID NO:30, or residues 21-167 of the sequence H19011_1_P8_V1 (SEQ ID NO:5), or a fragment, or a variant, or a homolog thereof.

Also disclosed, but not claimed, is a method for prevention of damage to the myelin coat of neural cells in the central nervous system in MS patients comprising administering to a subject in need thereof a pharmaceutical composition comprising: a soluble molecule having the extracellular domain of C1ORF32 polypeptide, or a fragment, variant, a homolog, a fusion protein or a conjugate thereof; or a polypeptide, comprising amino acid residues 21-186 of H19011_1_P8 (SEQ ID NO:4), corresponding to amino acid sequence depicted in SEQ ID NO:14, or residues 21-186 of H19011_1_P8_V1 (SEQ ID NO:5), corresponding to amino acid sequence depicted in SEQ ID NO:35, or residues 21-169 of H19011_1_P9 (SEQ ID NO:6), corresponding to amino acid sequence depicted in SEQ ID NO:15, or residues 21-169 of H19011_1_P9_V1 (SEQ ID NO:34), corresponding to amino acid sequence depicted in SEQ ID NO:36, or residues 1-184 of the sequence H19011_1_P8 (SEQ ID NO:4), corresponding to amino acid sequence depicted in SEQ ID NO:37, or residues 1-184 of the sequence H19011_1_P8_V1 (SEQ ID NO:5), corresponding to amino acid sequence depicted in SEQ ID NO:19, or residues 1-169 of H19011_1_P9 (SEQ ID NO:6), corresponding to amino acid sequence depicted in SEQ ID NO:28, or residues 1-169 of H19011_1_P9_V1 (SEQ ID NO:34), corresponding to amino acid sequence depicted in SEQ ID NO:30, or a fragment or a variant or a homolog thereof; optionally provided as a pharmaceutical composition.

Also disclosed, but not claimed, is a method for treating immune related disorder, wherein the treatment does not cause a global immunosuppression of the immune system in the subject, comprising administering to a subject in need thereof a pharmaceutical composition comprising: a soluble molecule having the extracellular domain of C1ORF32 polypeptide, fragment, variant, homolog, fusion protein or conjugate thereof; or polypeptide, comprising amino acid residues 21-186 of H19011_1_P8 (SEQ ID NO:4), corresponding to amino acid sequence depicted in SEQ ID NO:14, or residues 21-186 of H19011_1_P8_V1 (SEQ ID NO:5), corresponding to amino acid sequence depicted in SEQ ID NO:35, or residues 21-169 of H19011_1_P9 (SEQ ID NO:6), corresponding to amino acid sequence depicted in SEQ ID NO:15, or residues 21-169 of H19011_1_P9_V1 (SEQ ID NO:34), corresponding to amino acid sequence depicted in SEQ ID NO:36, or residues 1-184 of the sequence H19011_1_P8 (SEQ ID NO:4), corresponding to amino acid sequence depicted in SEQ ID NO:37, or residues 1-184 of the sequence H19011_1_P8_V1 (SEQ ID NO:5), corresponding to amino acid sequence depicted in SEQ ID NO:19, or residues 1-169 of H19011_1_P9 (SEQ ID NO:6), corresponding to amino acid sequence depicted in SEQ ID NO:28, or residues 1-169 of H19011_1_P9_V1 (SEQ ID NO:34), corresponding to amino acid sequence depicted in SEQ ID NO:30, or a fragment or a variant or a homolog thereof; optionally provided as a pharmaceutical composition thereof.

Optionally treating comprises one or more of preventing, curing, managing, reversing, attenuating, alleviating, minimizing, suppressing, managing, or halting the deleterious effects of the above-described diseases.

Optionally, managing comprises reducing the severity of the disease, reducing the frequency of episodes of the disease, reducing the duration of such episodes, or reducing the severity of such episodes or a combination thereof.

The fusion proteins, or pharmaceutical composition comprising same, for use in the treatment of an immune related disorder selected from multiple sclerosis, rheumatoid arthritis, type I diabetes, psoriasis, systemic lupus erythematosus, inflammatory bowel disease, uveitis, or Sjogren's syndrome can be used to treat patients who do not respond to TNF blockers.

### BRIEF DESCRIPTION OF THE FIGURES

Figure 1A shows alignment of H19011_1_P8 (SEQ ID NO:4) protein to known proteins Q71H61_HUMAN and NP_955383 (SEQ ID NO:3).
Figure 1B shows alignment of H19011_1_P9 (SEQ ID NO:6) protein to known proteins Q71H61_HUMAN and NP_955383 (SEQ ID NO:3).
Figure 2 shows the C1ORF32_T8_P8_V1_ECD_mFc (also referred to herein as C1ORF32_ECD_mFc) DNA sequence (1287bp) (SEQ ID NO:7). The ECD sequence is marked in bold faced, TEV cleavage site sequence is underlined, mFc sequence is unbold Italic and Signal Peptide sequence is bold Italic.
Figure 3 shows the C1ORF32_T8_P8_V1_ECD_mFc (also referred to herein as C1ORF32_ECD_mFc) amino acid sequence (428aa) (SEQ ID NO:8). The ECD sequence is marked in bold faced, TEV cleavage site sequence is underlined and is surrounded by a GS linker on the N -Ter of the TEV sequence and a SG on the C-Ter end of the TEV sequence, mFc sequence is unbold Italic and Signal Peptide sequence is bold Italic.

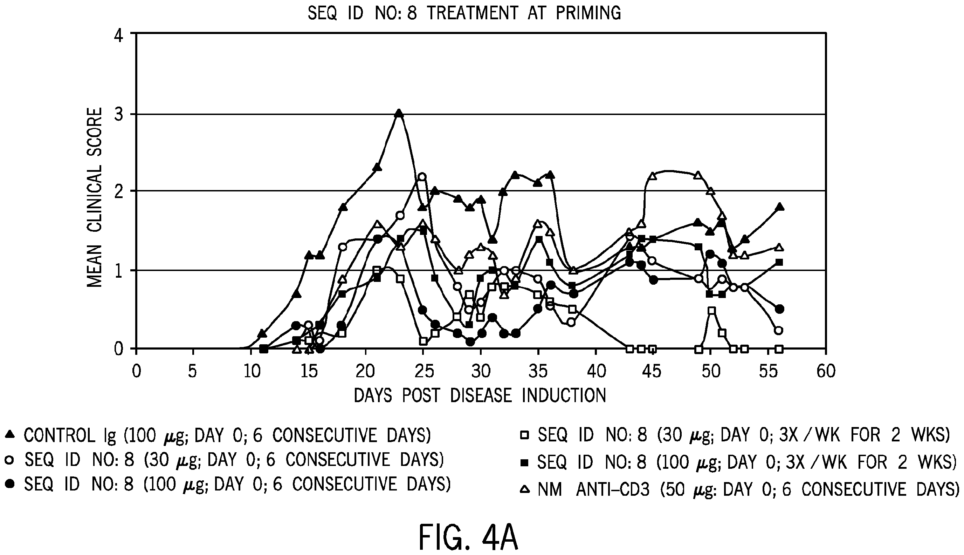

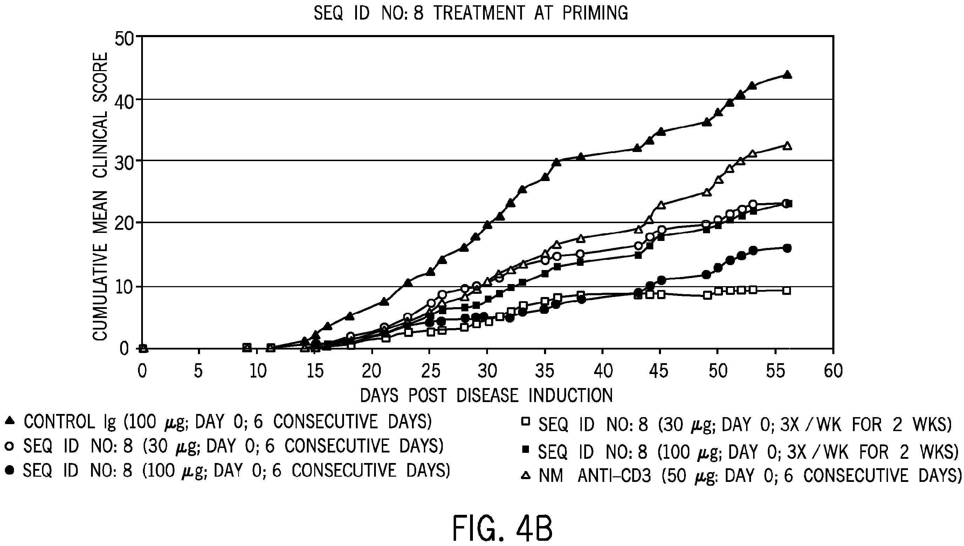

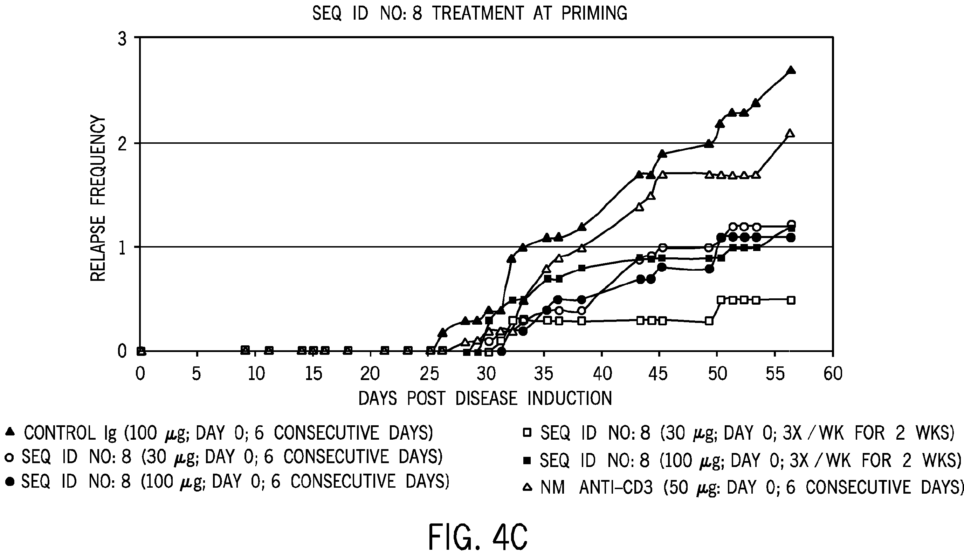

Figure 4 shows the effect of six administrations of C1ORF32-P8-V1-ECD-mFc (SEQ ID NO:8) given in a preventive mode starting from day of disease induction at two doses (30 and 100 microg/mouse) and two frequencies (daily or 3 times per week) on clinical symptoms in the mouse R-EAE model, demonstrated as Mean Clinical Score (Figure 4A), Cumulative Clinical Score (Figure 4B) and as Relapse Frequency (Figure 4C). In this study the effect of C1ORF32-P8-V1-ECD-mFc (SEQ ID NO:8) was studied in comparison to Ig control (100microg/mouse) and NM-anti -CD3 (50microg/mouse) that were administered on 6 consecutive days.

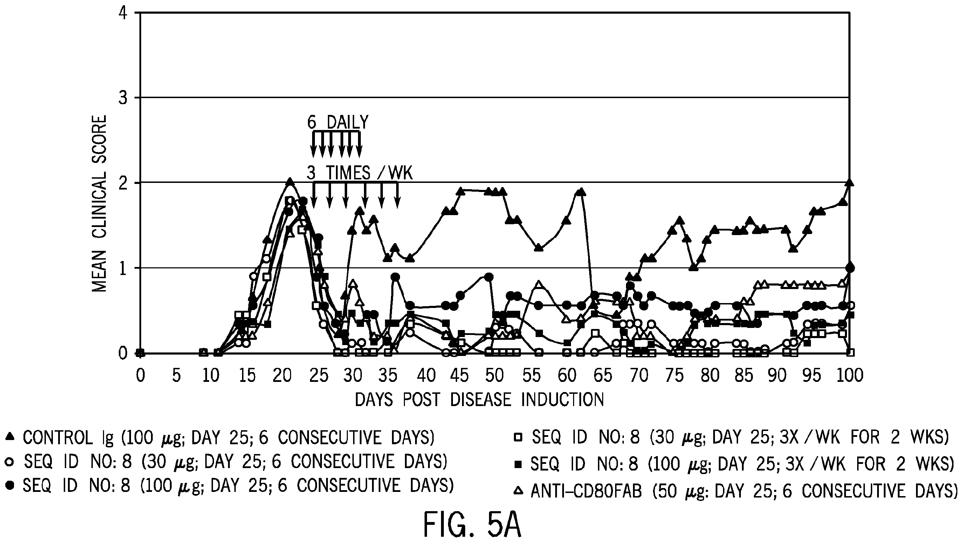

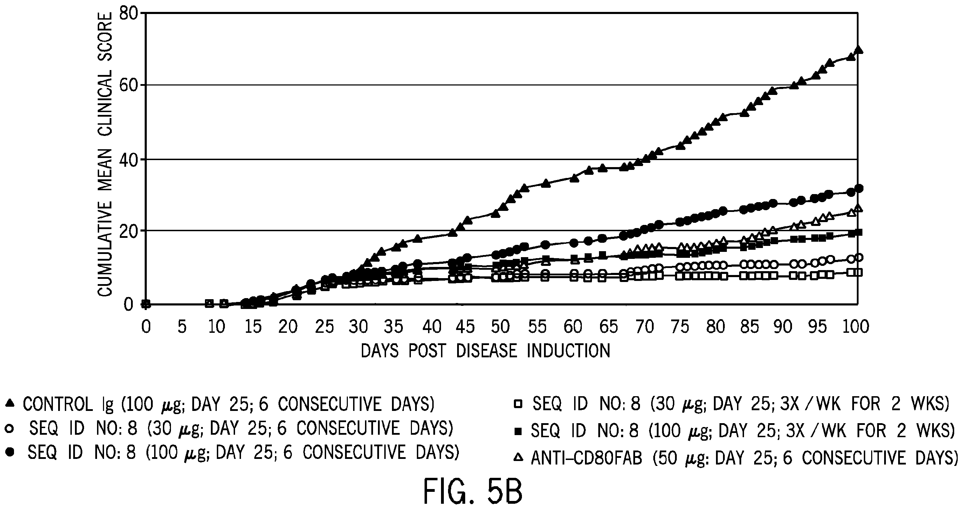

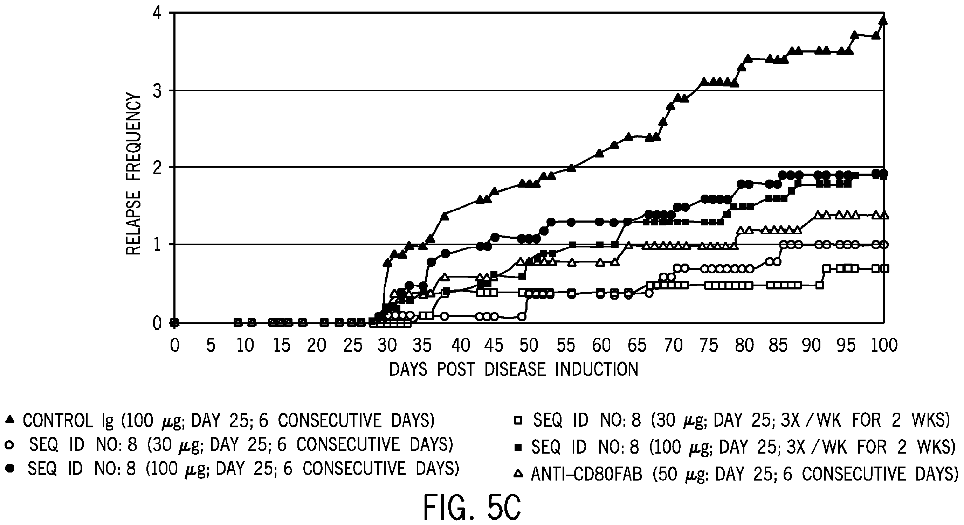

Figure 5 shows the effect of six administrations of C1ORF32-P8-V1-ECD-mFc(SEQ ID NO:8) given in a therapeutic mode at the onset of disease remission (on day 25), at two doses (30 and 100 microg/mouse) and two frequencies (daily or 3 times per week) on clinical symptoms in the PLP139-151-induced R-EAE model in SJL mice demonstrated as Mean Clinical Score (Figure 5A), Cumulative Clinical Score (Figure 5B) and as Relapse Frequency (Figure 5C). In this study the effect of C1ORF32-P8-V1-ECD-mFc (SEQ ID NO:8) was studied in comparison to Ig control (100microg/mouse) and anti ―CD80FaB that were administered on 6 consecutive days.
Figure 6 shows the effect of C1ORF32-ECD-mFc (SEQ ID NO:8) treatment in comparison to control Ig and B7-H4-Ig on proliferation of T cells derived from SJL or BALB/c mice.
Figure 7 shows the in vitro effect of C10RF32-ECD-mFc (SEQ ID NO:8) presented as soluble or bound to either plate or beads, on the proliferation and cytokine secretion of Naive CD4⁺ T cells isolated from SJL mice.
Figure 8 shows the in vitro effect of C10RF32-ECD-mFc (SEQ ID NO:8) presented as soluble or bound to either plate or beads, on the proliferation and cytokine secretion of Naive CD4⁺ T cells isolated from BALB/c mice.
Figure 9 shows the effect of C1ORF32-ECD-mFc (SEQ ID NO:8) on T cell activation and differentiation under Th0, Th1, Th2 and Th17- promoting conditions, when presented as bead-bound together with anti-CD3 and anti-CD28, as well as when added as a soluble protein to irradiated APC isolated from DO.11 mice+OVA₃₂₃₋₃₃₉.
Figure 10 shows the effect of C10RF32-ECD-mFc (SEQ ID NO:8) produced in HEK-293 in the mouse R-EAE model. C1ORF32-ECD-mFc (SEQ ID NO:8) was administred in a preventive mode via i.p. injection on days 0-5 post disease induction.
Figure 11 shows a comparison of the in-vitro activity of C1ORF32-ECD-mFc (SEQ ID NO:8) produced in CHO and in HEK-293, on CD4+ T cell proliferation and cytokine production under Th0, Th1, Th2 and Th17 deriving conditions.

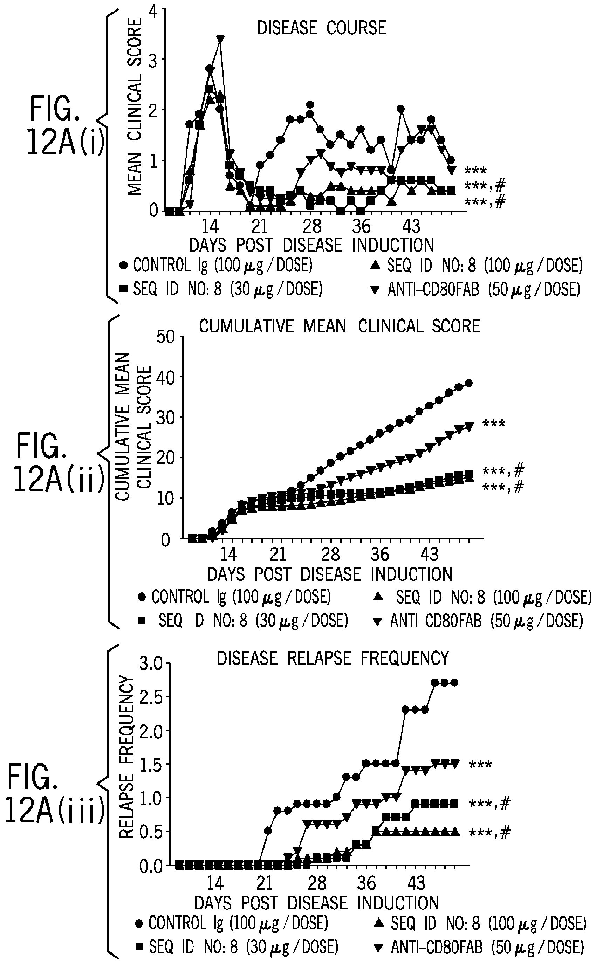

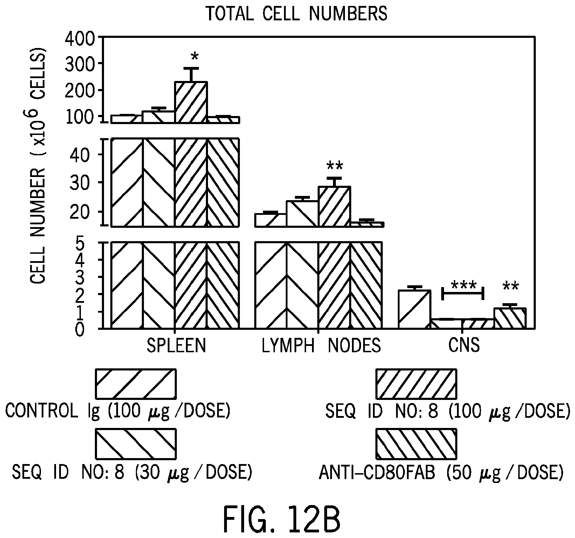

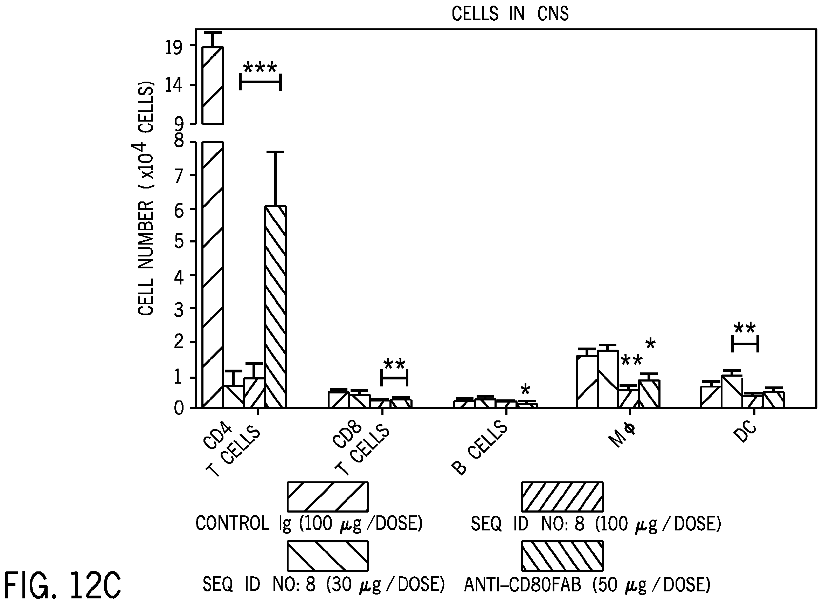

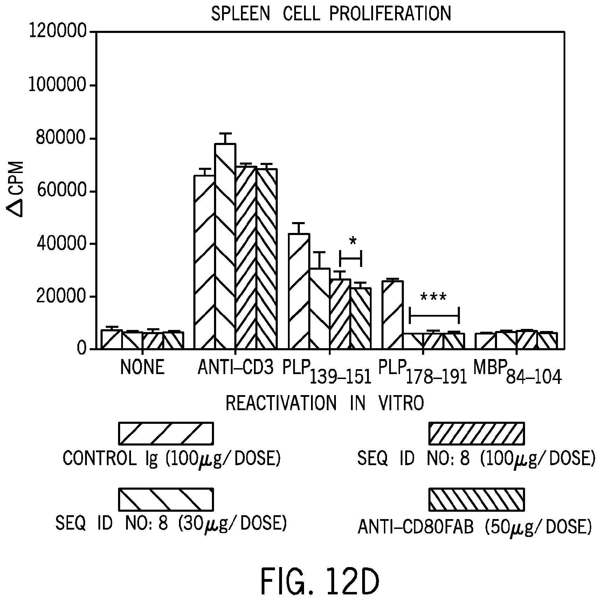

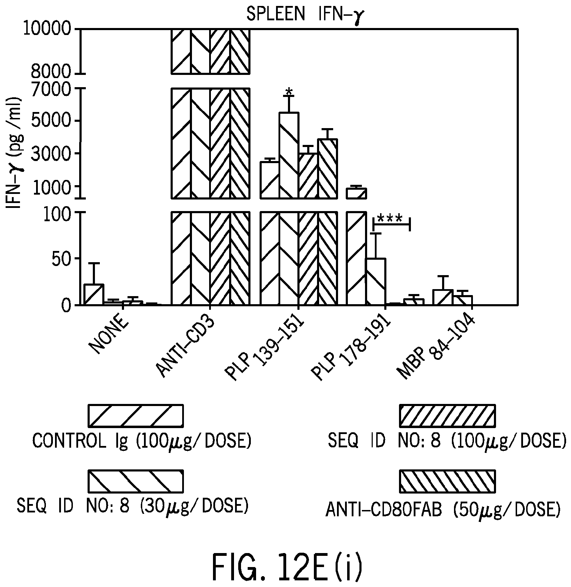

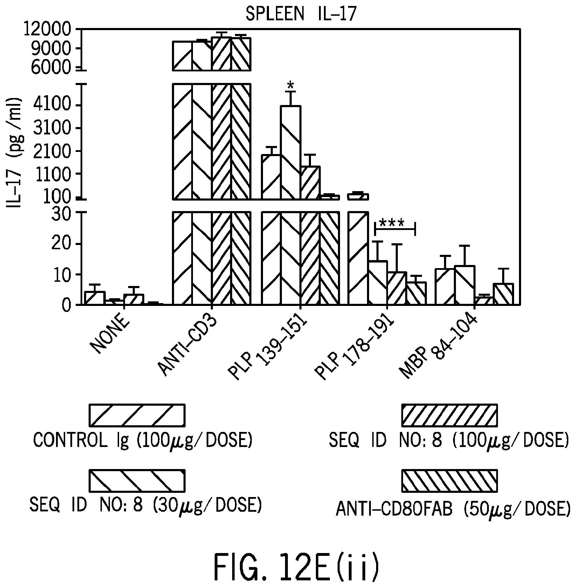

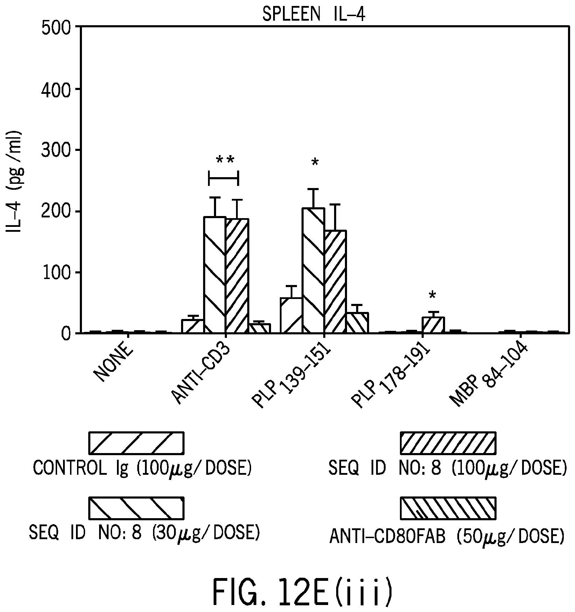

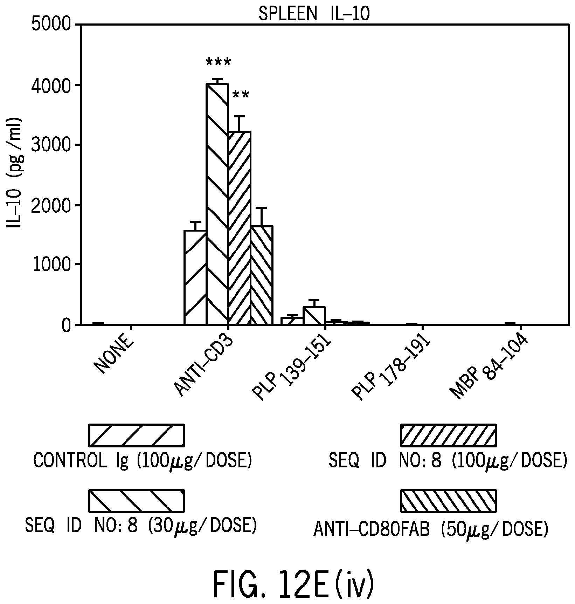

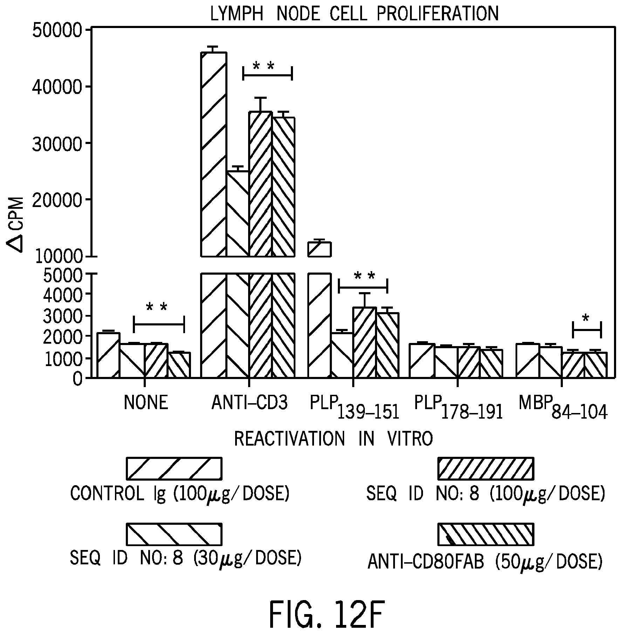

Figure 12 shows the effect of C1ORF32-ECD-mFc (SEQ ID NO:8) administred in a therapeutic mode, i.p, 3 times per week for two weeks in PLP139-151-induced R-EAE in SJL mice. Demonstrated are effects on Mean Clinical Score, Cumulative Clinical Score and Relapse Frequency (Figure 12A); recruitment of immune cells to the spleen, lymph nodes and CNS (Figure 12B); immune cell populations infiltrating the CNS (Figure 12C); recall responses of splenocytes to initiating and spread epitopes via proliferation (Figure 12D); recall responses of splenocytes to initiating and spread epitoes via cytokine secretion (Figure 12E); recall responses of cervical lymph node cells to initiating epitope via proliferation (Figure 12F). In this study the effect of C1ORF32-P8-V1-ECD-mFc(SEQ ID NO:8) was studied in comparison to Ig control (100microg/mouse) and anti-CD80 Fab (50microg/mouse).

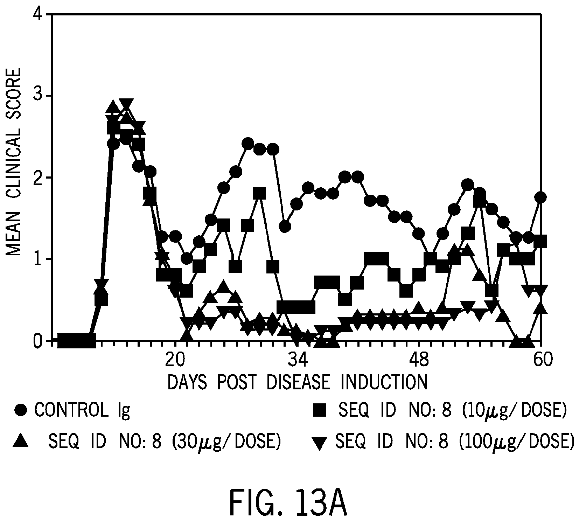

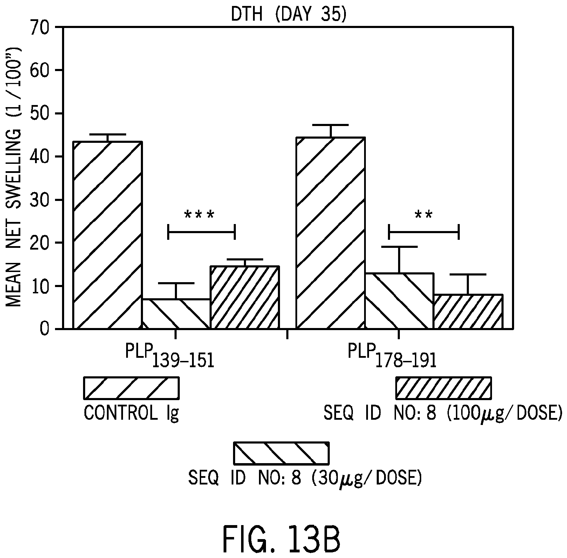

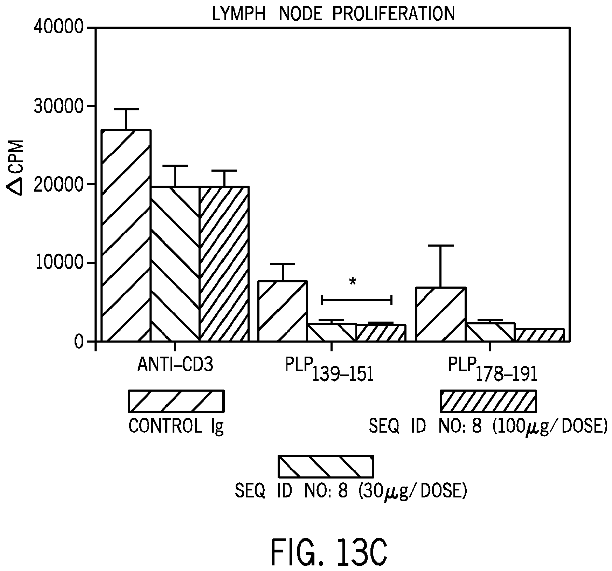

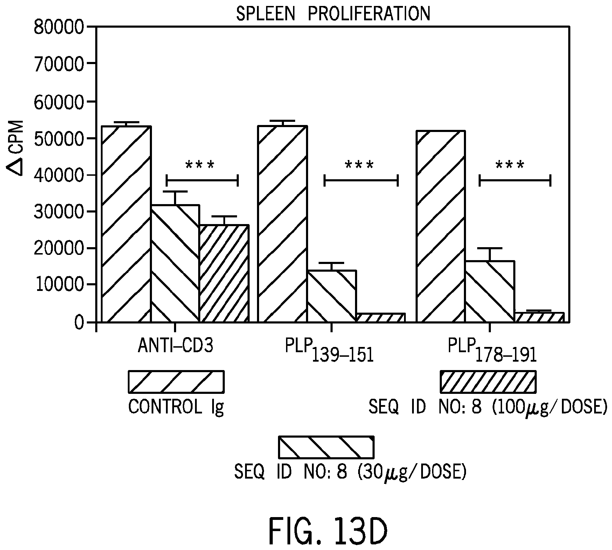

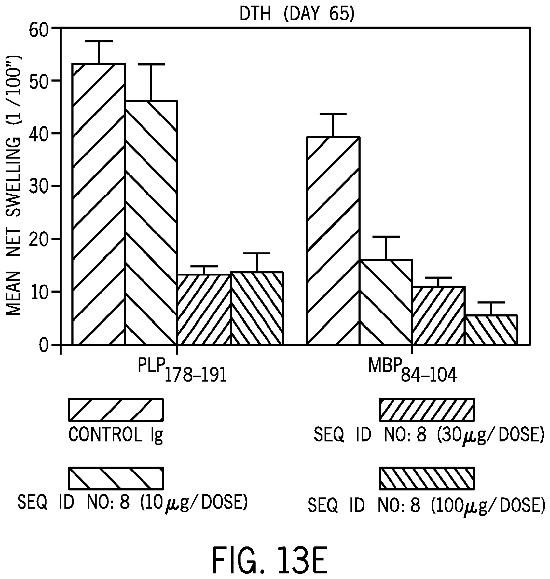

Figure 13A shows a dose response effect of therapeutic treatment of C1OR-F32-ECDmFc (SEQ ID NO:8) at 100, 30 and 10 microg/mouse, i.p, 3 times per week for 2 weeks in PLP139-151-induced R-EAE in SJL mice, in comparison to Ig control. Presented is clinical efficacy manifested as Mean Clinical Score (Figure 13A); Also estimated is the effect of C1ORF32-ECD-mFc (SEQ ID NO:8), administered as above at 100 and 30 microg/mouse, on DTH responses to inducing epitope (PLP139-151) or spread epitope (PLP178-191), carried out on day 35 after R-EAE induction (Figure 13B), on recall responses at day 35 after R-EAE induction, manifested as proliferation of lymph node cells (Figure 13C)or spleen cells (Figure 13D) in response to inducing epitope (PLP139-151), spread epitope (PLP178-191) and anti CD3; on DTH responses to spread epitopes (PLP178-191 and MBP84-104), carried out on day 65 from R-EAE induction (Figure 13E), and on recall responses at day 65 after R-EAE induction, manifested as proliferation of splenocytes in response to anti CD3, OVA 323-339, inducing epitope (PLP139-151) and spread epitopes (PLP178-191 and MBP84-104).
Figure 14 shows the effect of C10RF32-ECD-mFc (SEQ ID NO:8) or control Ig treatment on R-EAE development (Figure 14A) and recruitment of total immune cells (Figure 14B), and outoreactive T cells (Figure 14C) to the to the spleen, lymph nodes and CNS in adoptive transfer model upon administration at time of cell transfer.
Figures 15 and 16 show the effect of C10RF32-ECD-mFc (SEQ ID NO:8) on activation human T cell from various human donors, manifested in cell proliferation and IFNγ secretion. Activation was carried out using beads coated with C1ORF32-ECD-mFc (SEQ ID NO:8), anti CD3 and anti CD28 in the one-step method (Figure 15) or the two-step method (Figure 16).
Figure 17 shows the dose dependency of the effect of C1ORF32-ECD-mFc (SEQ ID NO:8) on human T cell proliferation.
Figure 18 shows the effect of C1ORF32-ECD-mFc (SEQ ID NO:8) and of Control Ig on proliferation of purified human T cells from different donors activated by beads coated with anti-CD3 and anti-CD28 antibodies together with C1ORF32-ECD-mFc (SEQ ID NO:8).
Figure 19 shows the effect of C1ORF32-ECD-mFc (SEQ ID NO:8) and of Control Ig on proliferation of purified human T cells from different donors activated by irradiated autologous PBMCs and anti-CD3 and anti-CD28 antibodies.
Figure 20 shows the therapeutic effect of C10RF32-ECD-mFc (SEQ ID NO:8) administred at 100 or 30 microg/mouse, i.p, 3 times per week for 10 days in collagen induced arthritis (CIA) model of Rhematoid Arthritis. Measured are clinical score (A), paw swelling (B) and number of affected paws (C). Enbrel (TNF-R-Ig, 100microg/mouse) was used as a positive control while control Ig (100microg/mouse) was used as negative control.
Figure 21 shows the effect of C1OR-F32-P8-V1-ECD-mFc (SEQ ID NO:8) at 1.5 and 5 mg/ kg on DTH in the trans vivo assay as manifested as the change (delta) in paw thickness in comparison to isotype control (mIgG2a, 5 mg/kg) or vehicle (PBS) treated mice, as detailed in the figure. FK506 was used as a positive control at doses of 3 and 30 mg/kg. Results obtained from injection of PBMCs pooled from 4 different donors are shown on Figure 21A, individual data obtained upon injection of PBMCs from each donor are presented in Figures 21B and 21C.
Figure 22 shows the effect of C1ORF32-P8-V1-ECD-mFc (SEQ ID NO:8) on IFNγ (A and B), IL-2 (C and D) and IL-4 (E and F) production by splenocytes which were stimulated in the presence of plate-bound anti-CD3 and soluble anti-CD28 for 24 hours. FK506 and B7-H4-Ig were used as positive control. mIgG2a was used as negative control.For each cytokine, the results of two studies are presented, labeled on the drawings as studies 1 and 2.

### DETAILED DESCRIPTION OF THE INVENTION

In order that the present invention may be more readily understood, certain terms are first defined. Additional definitions are set forth throughout the detailed description.

As used herein the term "isolated" refers to a compound of interest (for example a polynucleotide or a polypeptide) that is in an environment different from that in which the compound naturally occurs e.g. separated from its natural milieu such as by concentrating a peptide to a concentration at which it is not found in nature. "Isolated" includes compounds that are within samples that are substantially enriched for the compound of interest and/or in which the compound of interest is partially or substantially purified.

An "immune cell" refers to any cell from the hemopoietic origin including but not limited to T cells, B cells, monocytes, dendritic cells, and macrophages.

As used herein, the term "polypeptide" refers to a chain of amino acids of any length, regardless of modification (e.g., phosphorylation or glycosylation).

As used herein, a "costimulatory polypeptide" or "costimulatory molecule" is a polypeptide that, upon interaction with a cell-surface molecule on T cells, modulates T cell responses.

As used herein, a "costimulatory signaling" is the signaling activity resulting from the interaction between costimulatory polypeptides on antigen presenting cells and their receptors on T cells during antigen-specific T cell responses. Without wishing to be limited by a single hypothesis, the antigen-specific T cell response is believed to be mediated by two signals: 1) engagement of the T cell Receptor (TCR) with antigenic peptide presented in the context of MHC (signal 1), and 2) a second antigen-independent signal delivered by contact between different costimulatory receptor/ligand pairs (signal 2). Without wishing to be limited by a single hypothesis, this "second signal" is critical in determining the type of T cell response (activation vs inhibition) as well as the strength and duration of that response, and is regulated by both positive and negative signals from costimulatory molecules, such as the B7 family of proteins.

As used herein, the term "B7" polypeptide means a member of the B7 family of proteins that costimulate T cells including, but not limited to B7-1, B7-2, B7-DC, B7-H5, B7-H1, B7-H2, B7-H3, B7-H4, B7-H6, B7-S3 and biologically active fragments and/or variants thereof. Representative biologically active fragments include the extracellular domain or fragments of the extracellular domain that costimulate T cells.

As used herein, "inflammatory molecules" refers to molecules that induce inflammatory responses (directly or indirectly) including, but not limited to, cytokines and metalloproteases such as including, but not limited to, IL-1beta, TNF-alpha, TGF-beta, IFN-gamma, IL-17, IL-6, IL-23, IL-22, IL-21, and MMPs.

As used herein, a "vector" is a replicon, such as a plasmid, phage, or cosmid, into which another DNA segment may be inserted so as to bring about the replication of the inserted segment. The vectors described herein can be expression vectors. As used herein, an "expression vector" is a vector that includes one or more expression control sequences

As used herein, an "expression control sequence" is a DNA sequence that controls and regulates the transcription and/or translation of another DNA sequence.

"Operably linked" refers to an arrangement of elements wherein the components so described are configured so as to perform their usual or intended function. Thus, two different polypeptides operably linked together retain their respective biological functions while physically linked together.

As used herein, "valency" refers to the number of binding sites available per molecule.

As used herein, a "variant" polypeptide contains at least one amino acid sequence alteration as compared to the amino acid sequence of the corresponding wild-type polypeptide.

As used herein, "conservative" amino acid substitutions are substitutions wherein the substituted amino acid has similar structural or chemical properties. As used herein, the term "host cell" refers to prokaryotic and eukaryotic cells into which a recombinant vector can be introduced.

As used herein, "transformed" and "transfected" encompass the introduction of a nucleic acid (e.g. a vector) into a cell by a number of techniques known in the art.

As used herein, the terms "immunologic", "immunological" or "immune" response is the development of a beneficial humoral (antibody mediated) and/or a cellular (mediated by antigen-specific T cells or their secretion products) response directed against a peptide in a recipient patient. Such a response can be an active response induced by administration of immunogen or a passive response induced by administration of antibody or primed T-cells. Without wishing to be limited by a single hypothesis, a cellular immune response is elicited by the presentation of polypeptide epitopes in association with Class I or Class II MHC molecules to activate antigen-specific CD4+ T helper cells and/or CD8+ cytotoxic T cells. The response may also involve activation of monocytes, macrophages, NK cells, basophils, dendritic cells, astrocytes, microglia cells, eosinophils, activation or recruitment of neutrophils or other components of innate immunity. The presence of a cell-mediated immunological response can be determined by proliferation assays (CD4+ T cells) or CTL (cytotoxic T lymphocyte) assays. The relative contributions of humoral and cellular responses to the protective or therapeutic effect of an immunogen can be distinguished by separately isolating antibodies and T-cells from an immunized syngeneic animal and measuring protective or therapeutic effect in a second subject.

An "immunogenic agent" or "immunogen" is capable of inducing an immunological response against itself on administration to a mammal, optionally in conjunction with an adjuvant.

As used herein, the term "C1ORF32" refers to the protein encoded by any one of the H19011_1_T8 (SEQ ID NO:1), H19011_1_T9 (SEQ ID NO:2) transcripts reported herein, particularly to proteins as set forth in any one of H19011_1_P8 (SEQ ID NO:4), H19011_1_P8_V1 (SEQ ID NO:5), H19011_1_P9 (SEQ ID NO:6) or H19011_1_P9_V1 (SEQ ID NO:34)

The C1ORF32 ECD refers to any one of the polypeptide sequences below or fragments thereof:
>H19011_1_P8 (SEQ ID NO:4) residues 21 to 186, corresponding to amino acid sequence depicted in SEQ ID NO:14
>H19011_1_P8_V1 (SEQ ID NO:5) residues 21-186, corresponding to amino acid sequence depicted in SEQ ID NO: 35
>H19011_1_P9 (SEQ ID NO:6) residues 21 to 169, corresponding to amino acid sequence depicted in SEQ ID NO:15
>H19011_1_P9_V1 (SEQ ID NO:34) residues 21 to 169, corresponding to amino acid sequence depicted in SEQ ID NO:36
>H19011_1_P8_V1 (SEQ ID NO:5) residues 1 to 184, corresponding to amino acid sequence depicted in SEQ ID NO:19
>H19011_1_P8 (SEQ ID NO:4) residues 1 to 184, corresponding to amino acid sequence depicted in SEQ ID NO:37
>H19011_1_P9 (SEQ ID NO:6) residues 1-169, corresponding to amino acid sequence depicted in SEQ ID NO:28 >H19011_1_P9_V1 (SEQ ID NO:34) residues 1-169, corresponding to amino acid
sequence depicted in SEQ ID NO:30
and variants thereof possessing at least 95, 96, 97, 98 or 99% sequence identity therewith.

### FUSION PROTEINS

C1ORF32 fusion polypeptides have a first fusion partner comprising a part of a C1ORF32 protein fused to a second polypeptide directly or via a linker peptide sequence or a chemical linker useful to connect the two proteins. The C1ORF32 polypeptide may optionally be fused to a second polypeptide to form a fusion protein as described herein. The presence of the second polypeptide can alter the solubility, stability, affinity and/or valency of the C1ORF32 fusion polypeptide. As used herein, "valency" refers to the number of binding sites available per molecule. The second polypeptide is a polypeptide from a different source or different protein.

The second polypeptide contains one or more domains of an immunoglobulin heavy chain constant region, preferably having an amino acid sequence corresponding to the hinge, CH2 and CH3 regions of a human immunoglobulin Cγ1, Cy2, Cy3 or Cy4 chain or to the hinge, CH2 and CH3 regions of a murine immunoglobulin Cy2a chain. SEQ ID NO: 20 provides exemplary sequence for the hinge, CH2 and CH3 regions of a human immunoglobulin Cγ1.

According to at least some embodiments, the fusion protein is a dimeric fusion protein. In an optional dimeric fusion protein, the dimer results from the covalent bonding of Cys residue in the hinge region of two of the Ig heavy chains that are the same Cys residues that are disulfide linked in dimerized normal Ig heavy chains. Such proteins are referred to as C1ORF32 polypeptides, fragments or fusion proteins thereof.

In one embodiment, the immunoglobulin constant domain may contain one or more amino acid insertions, deletions or substitutions that enhance binding to specific cell types, increase the bioavailablity, or increase the stability of the C1ORF32 polypeptides, fusion proteins, or fragments thereof. Suitable amino acid substitutions include conservative and non-conservative substitutions, as described above.

The fusion proteins optionally contain a domain that functions to dimerize or multimerize two or more fusion proteins. The peptide/polypeptide linker domain can either be a separate domain, or alternatively can be contained within one of the other domains (C1ORF32 polypeptide or second polypeptide) of the fusion protein. Similarly, the domain that functions to dimerize or multimerize the fusion proteins can either be a separate domain, or alternatively can be contained within one of the other domains (C1ORF32 polypeptide, second polypeptide or peptide/polypeptide linker domain) of the fusion protein. In one embodiment, the dimerization/multimerization domain and the peptide/polypeptide linker domain are the same. Further specific, illustrative and non-limiting examples of dimerization/multimerization domains and linkers are given below.

Fusion proteins disclosed herein according to at least some embodiments of the present invention are of formula I: N-R1-R2-R3-C wherein "N" represents the N-terminus of the fusion protein, "C" represents the C-terminus of the fusion protein. In the further embodiment, "R1" is a C1ORF32 polypeptide, "R2" is an optional peptide/polypeptide or chemical linker domain, and "R3" is a second polypeptide. Alternatively, R3 may be a C1ORF32 polypeptide and R1 may be a second polypeptide.

Optionally, the fusion protein comprises the C1ORF32 polypeptide fragments as described herein, fused, optionally by a linker peptide of one or more amino acids (e.g. GS) to one or more "half-life extending moieties". A "half-life extending moiety" is any moiety, for example, a polypeptide, small molecule or polymer, that, when appended to protein, extends the in vivo half-life of that protein in the body of a subject (e.g., in the plasma of the subject). For example, a half-life extending moiety is, in an embodiment of the invention, polyethylene glycol (PEG), monomethoxy PEG (mPEG) or an immunoglobulin (Ig). In an embodiment of the invention, PEG is a 5, 10, 12, 20, 30, 40 or 50 kDa moiety or larger or comprises about 12000 ethylene glycol units (PEG12000).

Dimerization or multinierization can occur between or among two or more fusion proteins through dimerization or multimerization domains. Alternatively, dimerization or multimerization of fusion proteins can occur by chemical crosslinking. The dimers or multimers that are formed can be homodimeric/homomultimeric or heterodimeric/ heteromultimeric.The second polypeptide "partner" in the C1ORF32 fusion polypeptides may be comprised of one or more other proteins, protein fragments or peptides as described herein, including but not limited to any immunoglobulin (Ig) protein or portion thereof, preferably the Fc region, or a portion of a biologically or chemically active protein such as the papillomavirus E7 gene product, melanoma-associated antigen p97), and HIV env protein (gp120). The "partner" is optionally selected to provide a soluble dimer/multimer and/or for one or more other biological activities as described herein.

Dimerization or multimerization can occur between or among two or more fusion proteins through dimerization or multimerization domains, including those described above. Alternatively, dimerization or multimerization of fusion proteins can occur by chemical crosslinking. Fusion protein dimers can be homodimers or heterodimers. Fusion protein multimers can be homomultimers or heteromultimers. Fusion protein dimers as disclosed herein are of formula II: N-R1-R2-R3-C N-R4-R5-R6-C or, alternatively, are of formula III: N-R1-R2-R3-C C-R4-R5-R6-N wherein the fusion proteins of the dimer provided by formula II are defined as being in a parallel orientation and the fusion proteins of the dimer provided by formula III are defined as being in an antiparallel orientation. Parallel and antiparallel dimers are also referred to as cis and trans dimers, respectively. "N" and "C" represent the N- and C-termini of the fusion protein, respectively. The fusion protein constituents "R1", "R2" and "R3" are as defined above with respect to formula I. With respect to both formula II and formula III, "R4" is a C1ORF32 polypeptide or a second polypeptide, "R5" is an optional peptide/polypeptide linker domain, and "R6" is a C1ORF32 polypeptide or a second polypeptide, wherein "R6" is a C1ORF32 polypeptide when "R4" is a second polypeptide, and "R6'" is a second polypeptide when "R4" is a C1ORF32 polypeptide. In one embodiment, "R1" is a C1ORF32 polypeptide, "R4" is also a C1ORF32 polypeptide, and "R3" and "R6" are both second polypeptides.

Fusion protein dimers of formula II are defined as homodimers when "R1" = "R4", "R2" = "R5" and "R3" = "R6". Similarly, fusion protein dimers of formula III are defined as homodimers when "R1" = "R6", "R2" = "R5" and "R3" = "R4". Fusion protein dimers are defined as heterodimers when these conditions are not met for any reason. For example, heterodimers may contain domain orientations that meet these conditions (i.e., for a dimer according to formula II, "R1" and "R4" are both C1ORF32 polypeptides, "R2" and "R5" are both peptide/polypeptide linker domains and "R3" and "R6" are both second polypeptides), however the species of one or more of these domains is not identical. For example, although "R3" and "R6" may both be C1OR-F32 polypeptides, one polypeptide may contain a wild-type C1ORF32 amino acid sequence while the other polypeptide may be a variant C1ORF32 polypeptide. An exemplary variant C1ORF32 polypeptide is C1ORF32 polypeptide that has been modified to have increased or decreased binding to a target cell, increased activity on immune cells, increased or decreased half life or stability. Dimers of fusion proteins that contain either a CHI or CL region of an immunoglobulin as part of the polypeptide linker domain preferably form heterodimers wherein one fusion protein of the dimer contains a CHI region and the other fusion protein of the dimer contains a CL region.

Fusion proteins can also be used to form multimers. As with dimers, multimers may be parallel multimers, in which all fusion proteins of the multimer are aligned in the same orientation with respect to their N- and C- termini. Multimers may be antiparallel multimers, in which the fusion proteins of the multimer are alternatively aligned in opposite orientations with respect to their N- and C-termini. Multimers (parallel or antiparallel) can be either homomultimers or heteromultimers. The fusion protein is optionally produced in dimeric form; more preferably, the fusion is performed at the genetic level as described below, by joining polynucleotide sequences corresponding to the two (or more) proteins, portions of proteins and/or peptides, such that a joined or fused protein is produced by a cell according to the joined polynucleotide sequence. A description of preparation for such fusion proteins is described with regard to US Patent No. 5,851,795 to Linsley et al.

The fusion protein may also optionally be prepared by chemical synthetic methods and the "join" effected chemically, either during synthesis or post-synthesis. Cross-linking and other such methods may optionally be used (optionally also with the above described genetic level fusion methods), as described for example in US Patent No. 5,547,853 to Wallner et al. According to the present invention, a fusion protein may be prepared from a C1ORF32 protein by fusion with a portion of an immunoglobulin comprising a constant region of an immunoglobulin. The portion of the immunoglobulin comprises a heavy chain constant region which is optionally and more preferably a human heavy chain constant region. The heavy chain constant region is most preferably an IgG heavy chain constant region, and optionally and most preferably is an Fc chain, most preferably an IgG Fc fragment that comprises the hinge, CH2 and CH3 domains. The Fc chain may optionally be a known or "wild type" Fc chain, or alternatively may be mutated or truncated. The Fc portion of the fusion protein may optionally be varied by isotype or subclass, may be a chimeric or hybrid, and/or may be modified, for example to improve effector functions, control of half-life, tissue accessibility, augment biophysical characteristics such as stability, and improve efficiency of production (and less costly). Many modifications useful in construction of disclosed fusion proteins and methods for making them are known in the art, see for example Mueller, et al, Mol. Immun., 34(6):441-452 (1997), Swann, et al., Cur. Opin. Immun., 20:493-499 (2008), and Presta, Cur. Opin. Immun. 20:460-470 (2008). In some embodiments the Fc region is the native IgG1, IgG2, or IgG4 Fc region. In some embodiments the Fc region is a hybrid, for example a chimeric consisting of IgG2/IgG4 Fc constant regions.

Modications to the Fc region include, but are not limited to, IgG4 modified to prevent binding to Fc gamma receptors and complement, IgG1 modified to improve binding to one or more Fc gamma receptors, IgG1 modified to minimize effector function (amino acid changes), IgG1 with altered/ no glycan (typically by changing expression host), and IgG1 with altered pH- dependent binding to FcRn. The Fc region may include the entire hinge region, or less than the entire hinge region.

In another embodiment, the Fc domain may contain one or more amino acid insertions, deletions or substitutions that reduce binding to the low affinity inhibitory Fc receptor CD32B (FcyRIIB) and retain wild-type levels of binding to or enhance binding to the low affinity activating Fc receptor CD16A (FcyRIIIA).

Another embodiment includes IgG2-4 hybrids and IgG4 mutants that have reduced binding to FcR (Fc receptor) which increase their half life. Representative IgG2-4 hybrids and IgG4 mutants are described in Angal, S. et al., Molecular Immunology, 30(1): 105-108 (1993); Mueller, J. et al., Molecular Immunology, 34(6): 441-452 (1997); and U.S. Patent No. 6,982,323 to Wang et al. In some embodiments the IgG1 and/or IgG2 domain is deleted; for example, Angal et al. describe IgG1 and IgG2 having serine 241 replaced with a proline.

In a further embodiment, the Fc domain contains amino acid insertions, deletions or substitutions that enhance binding to CD16A. A large number of substitutions in the Fc domain of human IgGl that increase binding to CD16A and reduce binding to CD32B are known in the art and are described in Stavenhagen, et al., Cancer Res., 57(18):8882-90 (2007). Exemplary variants of human IgG1 Fc domains with reduced binding to CD32B and/or increased binding to CD16A contain F243L, R929P, Y300L, V305I or P296L substitutions. These amino acid substitutions may be present in a human IgG1 Fc domain in any combination.

In one embodiment, the human IgG1 Fc domain variant contains a F243L, R929P and Y300L substitution. In another embodiment, the human IgG1 Fc domain variant contains a F243L, R929P, Y300L, V3O5I and P296L substitution. In another embodiment, the human IgG1 Fc domain variant contains an N297A/Q substitution, as these mutations abolishFcyR binding. Non-limiting, illustrative, exemplary types of mutations are described in US Patent Application No. 20060034852, published on February 16, 2006. The term "Fc chain" also optionally comprises any type of Fc fragment.

Several of the specific amino acid residues that are important for antibody constant region-mediated activity in the IgG subclass have been identified. Inclusion, substitution or exclusion of these specific amino acids therefore allows for inclusion or exclusion of specific immunoglobulin constant region-mediated activity. Furthermore, specific changes may result in aglycosylation for example and/or other desired changes to the Fc chain. At least some changes may optionally be made to block a function of Fc which is considered to be undesirable, such as an undesirable immune system effect, as described in greater detail below.

Non-limiting, illustrative examples of mutations to Fc which may be made to modulate the activity of the fusion protein include the following changes (given with regard to the Fc sequence nomenclature as given by Kabat, from Kabat EA et al: Sequences of Proteins of Immunological Interest. US Department of Health and Human Services, NIH, 1991): 220C - > S; 233-238 ELLGGP - > EAEGAP; 265D - > A, preferably in combination with 434N -> A; 297N - > A (for example to block N-glycosylation); 318-322 EYKCK - > AYACA; 330-331AP - > SS; or a combination thereof (see for example M. Clark, "Chemical Immunol and Antibody Engineering", pp 1-31 for a description of these mutations and their effect). The construct for the Fc chain which features the above changes optionally and preferably comprises a combination of the hinge region with the CH2 and CH3 domains.

The above mutations may optionally be implemented to enhance desired properties or alternatively to block non-desired properties. For example, aglycosylation of antibodies was shown to maintain the desired binding functionality while blocking depletion of T-cells or triggering cytokine release, which may optionally be undesired functions (see M. Clark, "Chemical Immunol and Antibody Engineering", pp 1-31). Substitution of 331proline for serine may block the ability to activate complement, which may optionally be considered an undesired function (see M. Clark, "Chemical Immunol and Antibody Engineering", pp 1-31). Changing 330alanine to serine in combination with this change may also enhance the desired effect of blocking the ability to activate complement.

Residues 235 and 237 were shown to be involved in antibody-dependent cell-mediated cytotoxicity (ADCC), such that changing the block of residues from 233-238 as described may also block such activity if ADCC is considered to be an undesirable function.

Residue 220 is normally a cysteine for Fc from IgG1, which is the site at which the heavy chain forms a covalent linkage with the light chain. Optionally, this residue may be changed to a serine, to avoid any type of covalent linkage (see M. Clark, "Chemical Immunol and Antibody Engineering", pp 1-31).

The above changes to residues 265 and 434 may optionally be implemented to reduce or block binding to the Fc receptor, which may optionally block undesired functionality of Fc related to its immune system functions (see "Binding site on Human IgG1 for Fc Receptors", Shields et al, Vol 276, pp 6591-6604, 2001).

The above changes are intended as illustrations only of optional changes and are not meant to be limiting in any way. Furthermore, the above explanation is provided for descriptive purposes only, without wishing to be bound by a single hypothesis.

Exemplary fusion proteins are set forth in SEQ ID NOs: 8, 22, 23, 38, 29.

The aforementioned exemplary fusion proteins can incorporate any combination of the variants described herein. In another embodiment the terminal lysine of the aforementioned exemplary fusion proteins is deleted.
The disclosed fusion proteins can be isolated using standard molecular biology techniques. For example, an expression vector containing a DNA sequence encoding a C10RF32 polypeptides, fragments or fusion proteins thereof fusion protein is transfected into 293 cells by calcium phosphate precipitation and cultured in serum-free DMEM. The supernatant is collected at 72 h and the fusion protein is purified by Protein G, or preferably Protein A SEPHAROSE^{®} columns (Pharmacia, Uppsala, Sweden). Optionally, a DNA sequence encoding a C1ORF32 polypeptides, fragments or fusion proteins thereof fusion protein is transfected into GPEx^{®} retrovectors and expressed in CHO-S cells following four rounds of retrovector transduction. The protein is clarified from supernatants using protein A chromatography.

In another embodiment the second polypeptide may have a conjugation domain through which additional molecules can be bound to the C1ORF32 fusion proteins. In one such embodiment, the conjugated molecule is capable of targeting the fusion protein to a particular organ or tissue; further specific, illustrative, non-limiting examples of such targeting domains and/or molecules are given below.

In another such embodiment the conjugated molecule is another immunomodulatory agent that can enhance or augment the effects of the C1ORF32 fusion protein. In another embodiment the conjugated molecule is Polyethylene Glycol (PEG).

### Peptide or polypeptide linker domain

The disclosed C1ORF32 fusion proteins optionally contain a peptide or polypeptide linker domain that separates the C1ORF32 polypeptide from the second polypeptide. In one embodiment, the linker domain contains the hinge region of an immunoglobulin. In a further embodiment, the hinge region is derived from a human immunoglobulin. Suitable human immunoglobulins that the hinge can be derived from include IgG, IgD and IgA. In a further embodiment, the hinge region is derived from human IgG. Amino acid sequences of immunoglobulin hinge regions and other domains are well known in the art. In one embodiment, C1OR-F32 fusion polypeptides contain the hinge, CH2 and CH3 regions of a human immunoglobulin Cγ1 chain having at least 85%, 90%, 95%, 99% or 100% sequence homology to amino acid sequence set forth in SEQ ID NO:20:

The hinge can be further shortened to remove amino acids 1, 2, 3, 4, 5, or combinations thereof of SEQ ID NO: 20. In one embodiment, amino acids 1-5 of SEQ ID NO: 20 are deleted.

In another embodiment, C1OR-F32 fusion polypeptides contain the, CH2 and CH3 regions of a human immunoglobulin Cγ1 chain having at least 85%, 90%, 95%, 99% or 100% sequence homology to amino acid sequence set forth in SEQ ID NO:21:

In another embodiment, the C10RF32 fusion polypeptides contain the hinge, CH2 and CH3 regions of a murine immunoglobulin Cy2a chain at least 85%, 90%, 95%, 99% or 100% sequence homology to amino acid sequence set forth in SEQ ID NO: 31: EPRGPTIKPCPPCKCPAPNLLGGPSVFIFPPKIKDVLMISLSPIVTCVVVDVSEDDPDV QISWFVNNVEVHTAQTQTHREDYNSTLRVVSALPIQHQDWMSGKEFKCKVNNKD LPAPIERTISKPKGSVRAPQVYVLPPPEEEMTKKQVTLTCMVTDFMPEDIYVEWTN NGKTELNYKNTEPVLDSDGSYFMYSKLRVEKKNWVERNSYSCSVVHEGLHNHHT TKSFSRTPGK. In another embodiment, the linker domain contains a hinge region of an immunoglobulin as described above, and further includes one or more additional immunoglobulin domains.

Other suitable peptide/polypeptide linker domains include naturally occurring or non-naturally occurring peptides or polypeptides. Peptide linker sequences are at least 2 amino acids in length. Optionally the peptide or polypeptide domains are flexible peptides or polypeptides. A "flexible linker" herein refers to a peptide or polypeptide containing two or more amino acid residues joined by peptide bond(s) that provides increased rotational freedom for two polypeptides linked thereby than the two linked polypeptides would have in the absence of the flexible linker. Such rotational freedom allows two or more antigen binding sites joined by the flexible linker to each access target antigen(s) more efficiently. Exemplary flexible peptides/polypeptides include, but are not limited to, the amino acid sequences Gly-Ser (SEQ ID NO:24), Gly-Ser-Gly-Ser (SEQ ID NO:25), Ala-Ser (SEQ ID NO:26), Gly-Gly-Gly-Ser (SEQ ID NO:27), Gly4-Ser (SEQ ID NO:39), (Gly4-Ser)2 (SEQ ID NO:40), (Gly4-Ser)3 (SEQ ID NO:32) and (Gly4-Ser)4 (SEQ ID NO: 33). Additional flexible peptide/polypeptide sequences are well known in the art.

### Dimerization, multimerization and targeting domains

The fusion proteins disclosed herein optionally contain a dimerization or multimerization domain that functions to dimerize or multimerize two or more fusion proteins. The domain that functions to dimerize or multimerize the fusion proteins can either be a separate domain, or alternatively can be contained within one of the other domains (C1ORF32 polypeptide, second polypeptide, or peptide/polypeptide linker domain) of the fusion protein.

A "dimerization domain" is formed by the association of at least two amino acid residues or of at least two peptides or polypeptides (which may have the same, or different, amino acid sequences). The peptides or polypeptides may interact with each other through covalent and/or non- covalent associations). Optional dimerization domains contain at least one cysteine that is capable of forming an intermolecular disulfide bond with a cysteine on the partner fusion protein. The dimerization domain can contain one or more cysteine residues such that disulfide bond(s) can form between the partner fusion proteins. In one embodiment, dimerization domains contain one, two or three to about ten cysteine residues. In a further embodiment, the dimerization domain is the hinge region of an immunoglobulin.

Additional exemplary dimerization domains can be any known in the art and include, but not limited to, coiled coils, acid patches, zinc fingers, calcium hands, a C_{H}1-C_{L} pair, an "interface" with an engineered "knob" and/or "protruberance" as described in U.S. Patent No. 5,821,333, leucine zippers (e.g., from jun and/or fos) (U.S. Patent No. 5,932,448), SH2 (src homology 2), SH3 (src Homology 3) (Vidal, et al, Biochemistry, 43, 7336- 44 ((2004)), phosphotyrosine binding (PTB) (Zhou, et al., Nature, 378:584- 592 (1995)), WW (Sudol, Prog, Biochys. MoL Bio., 65:113-132 (1996)), PDZ (Kim, et al., Nature, 378: 85-88 (1995); Komau, et al, Science, 269.1737-1740 (1995)) 14-3-3, WD40 (Hu5 et al., J Biol Chem., 273, 33489- 33494 (1998)) EH, Lim, an isoleucine zipper, a receptor dimer pair (e.g., interleukin-8 receptor (IL-8R); and integrin heterodimers such as LFA-I and GPIIIb/IIIa), or the dimerization region(s) thereof, dimeric ligand polypeptides (e.g. nerve growth factor (NGF), neurotrophin-3 (NT-3), interleukin-8 (IL-8), vascular endothelial growth factor (VEGF), VEGF-C, VEGF-D, PDGF members, and brain-derived neurotrophic factor (BDNF) (Arakawa, et al., J Biol. Chem., 269(45): 27833-27839 (1994) and Radziejewski, et al., Biochem., 32(48): 1350 (1993)) and can also be variants of these domains in which the affinity is altered. The polypeptide pairs can be identified by methods known in the art, including yeast two hybrid screens. Yeast two hybrid screens are described in U.S. Pat. Nos. 5,283,173 and 6,562,576. Affinities between a pair of interacting domains can be determined using methods known in the art, including as described in Katahira, et at, J. Biol Chem, 277, 9242-9246 (2002)). Alternatively, a library of peptide sequences can be screened for heterodimerization, for example, using the methods described in WO 01/00814. Useful methods for protein-protein interactions are also described in U.S. Patent No. 6,790,624.

A "multimerization domain" is a domain that causes three or more peptides or polypeptides to interact with each other through covalent and/or non-covalent association(s). Suitable multimerization domains include, but are not limited to, coiled-coil domains. A coiled-coil is a peptide sequence with a contiguous pattern of mainly hydrophobic residues spaced 3 and 4 residues apart, usually in a sequence of seven amino acids (heptad repeat) or eleven amino acids (undecad repeat), which assembles (folds) to form a multimeric bundle of helices. Coiled-coils with sequences including some irregular distribution of the 3 and 4 residues spacing are also contemplated. Hydrophobic residues are in particular the hydrophobic amino acids VaI, He, Leu, Met, Tyr, Phe and Trp. "Mainly hydrophobic" means that at least 50% of the residues must be selected from the mentioned hydrophobic amino acids.

The coiled coil domain may be derived from laminin. In the extracellular space, the heterotrimeric coiled coil protein laminin plays an important role in the formation of basement membranes. Apparently, the multifunctional oligomeric structure is required for laminin function. Coiled coil domains may also be derived from the thrombospondins in which three (TSP-I and TSP-2) or five (TSP-3, TSP-4 and TSP-5) chains are connected, or from COMP (COMPcc) (Guo, et at., EMBO J, 1998, 17: 5265-5272) which folds into a parallel five-stranded coiled coil (Malashkevich, et al., Science, 274: 761-765 (1996)). Additional coiled-coil domains derived from other proteins, and other domains that mediate polypeptide multimerization are known in the art and are suitable for use in the disclosed fusion proteins.

In another embodiment, C1ORF32 polypeptides, fusion proteins, or fragments thereof can be induced to form multimers by binding to a second multivalent polypeptide, such as an antibody. Antibodies suitable for use to multimerize C1ORF32 polypeptides, fusion proteins, or fragments thereof include, but are not limited to, IgM antibodies and crosslinked, multivalent IgG, IgA, IgD, or IgE complexes.

### Targeting Domains

The C1ORF32 polypeptides and fusion proteins can contain a targeting domain to target the molecule to specific sites in the body. Optional targeting domains target the molecule to areas of inflammation. Exemplary targeting domains are antibodies, or antigen binding fragments thereof that are specific for inflamed tissue or to a proinflammatory cytokine including but not limited to IL17, IL-4, IL-6, IL-12, IL-21, IL-22, and IL-23. In the case of neurological disorders such as Multiple Sclerosis, the targeting domain may target the molecule to the CNS or may bind to VCAM-I on the vascular epithelium. Additional targeting domains can be peptide aptamers specific for a proinflammatory molecule. In other embodiments, the C1ORF32 fusion protein can include a binding partner specific for a polypeptide displayed on the surface of an immune cell, for example a T cell. In still other embodiments, the targeting domain specifically targets activated immune cells. Optional immune cells that are targeted include Th0, Th1, Th 17, Th2 and Th22 T cells, other cells that secrete, or cause other cells to secrete inflammatory molecules including, but not limited to, IL-1beta, TNF-alpha, TGF-beta, IFN-gamma, IL-17, IL-6, IL-23, IL-22, IL-21, and MMPs, and Tregs. For example, a targeting domain for Tregs may bind specifically to CD25.

The terms "individual", "host", "subject", and "patient" are used interchangeably herein, and refer any human or nonhuman animal. The term "nonhuman animal" includes all vertebrates, e.g., mammals and non-mammals, such as nonhuman primates, sheep, dogs, cats, horses, cows chickens, amphibians, reptiles, etc.

The disclosure is further described by the items which follow (not claimed):
1. Use of an isolated polypeptide comprising a soluble C10RF32 polypeptide or fragment or variant thereof, capable of inhibition of T cell activation, for treatment of one or more of benign multiple sclerosis, relapsing remitting multiple sclerosis, secondary progressive multiple sclerosis, primary progressive multiple sclerosis, progressive relapsing multiple sclerosis, chronic progressive multiple sclerosis, transitional/progressive multiple sclerosis, rapidly worsening multiple sclerosis, clinically-definite multiple sclerosis, malignant multiple sclerosis, also known as Marburg's Variant, and acute multiple sclerosis, or conditions relating to multiple sclerosis, selected from the group consistiong of Devic's disease, also known as Neuromyelitis Optica; acute disseminated encephalomyelitis, acute demyelinating optic neuritis, demyelinative transverse myelitis, Miller-Fisher syndrome, encephalomyelradiculoneuropathy, acute demyelinative polyneuropathy, tumefactive multiple sclerosis and Balo's concentric sclerosis; or one or more of gout and pseudo-gout, juvenile idiopathic arthritis, juvenile rheumatoid arthritis, Still's disease, ankylosing spondylitis, rheumatoid vasculitis, or conditions relating to rheumatoid arthritis., selected from the group consisting of osteoarthritis, sarcoidosis, Henoch-Schonlein purpura, Psoriatic arthritis, Reactive arthritis, Spondyloarthropathy, septic arthritis, Haemochromatosis, Hepatitis, vasculitis, Wegener's granulomatosis, Lyme disease, Familial Mediterranean fever, Hyperimmunoglobulinemia D with recurrent fever, TNF receptor associated periodic syndrome, and Enteropathic arthritis associated with inflammatory bowel disease; or one or more of anterior uveitis (or iridocyclitis), intermediate uveitis (pars planitis), posterior uveitis (or chorioretinitis) and the panuveitic form; or one or more of Crohn's disease, ulcerative colitis (UC), Collagenous colitis, Lymphocytic colitis, Ischaemic colitis, Diversion colitis, Behçet's disease, Indeterminate colitis; or one or more of Nonpustular Psoriasis including Psoriasis vulgaris and Psoriatic erythroderma (erythrodermic psoriasis), Pustular psoriasis including Generalized pustular psoriasis (pustular psoriasis of von Zumbusch), Pustulosis palmaris et plantaris (persistent palmoplantar pustulosis, pustular psoriasis of the Barber type, pustular psoriasis of the extremities), Annular pustular psoriasis, Acrodermatitis continua, Impetigo herpetiformis; drug-induced psoriasis, Inverse psoriasis, Napkin psoriasis, Seborrheic-like psoriasis, Guttate psoriasis, Nail psoriasis, Psoriatic arthritis; or one or more of insulin-dependent diabetes mellitus, idiopathic diabetes, juvenile type 1diabetes, maturity onset diabetes of the young, latent autoimmune diabetes in adults, gestational diabetes; neuropathy including polyneuropathy, mononeuropathy, peripheral neuropathy and autonomicneuropathy; glaucoma, cataracts, or retinopathy; or one or more of Primary Sjogren's syndrome, Secondary Sjogren's syndrome, connective tissue disease, such as rheumatoid arthritis, systemic lupus erythematosus, scleroderma, pneumonia, polmunary fibrosis, interstitial nephritis, inflammation of the tissue around the kidney's filters, glomerulonephritis, renal tubular acidosis, carpal tunnel syndrome, peripheral neuropathy, cranial neuropathy, primary biliary cirrhosis (PBC), cirrhosis, Inflammation in the esophagus, stomach, pancreas, and liver (including hepatitis), Polymyositis, Raynaud's phenomenon, Vasculitis, Autoimmune thyroid problems, lymphoma; or one or more of discoid lupus, lupus arthritis, lupus pneumonitis, lupus nephritis, or conditions relating to systemic lupus erythematosus selected from the group consisting of osteoarticular tuberculosis, antiphospholipid antibody syndrome, inflammation of various parts of the heart, such as pericarditis, myocarditis, and endocarditis, Lung and pleura inflammation, pleuritis, pleural effusion, chronic diffuse interstitial lung disease, pulmonary hypertension, pulmonary emboli, pulmonary hemorrhage, and shrinking lung syndrome, lupus headache, Guillain-Barre syndrome, aseptic meningitis, demyelinating syndrome, mononeuropathy, mononeuritis multiplex, myasthenia gravis, myelopathy, cranial neuropathy, polyneuropathy, vasculitis.
2. The use of the protein according to item 1, where the C1ORF32 polypeptide is fused to a heterologous sequence, directly or indirectly via a linker peptide, a polypeptide sequence or a chemical linker.
3. The use of items 1 or 2, wherein the C1OR-F32 polypeptide comprises the extracellular domain of C10RF32, or fragment or variant thereof, or a polypeptide comprising the extracellular domain of H19011_1_P8 (SEQ ID NO:4), H19011_1_P8_V1 (SEQ ID NO:5), H19011_1_P9 (SEQ ID NO:6) or H19011_1_P9_V1 (SEQ ID NO:34), or a fragment or variant or homolog thereof.
4. The use of item 3 wherein the C1OR-F32 polypeptide is selected from the group consisting of polypeptide comprising a sequence of amino acid residues having at least 95% sequence identity with amino acid residues 21-186 of H19011_1_P8 (SEQ ID NO:4), corresponding to amino acid sequence depicted in SEQ ID NO:14, or residues 21-186 of H19011_1_P8_V1 (SEQ ID NO:5), corresponding to amino acid sequence depicted in SEQ ID NO:35, or residues 21-169 of H19011_1_P9 (SEQ ID NO:6), corresponding to amino acid sequence depicted in SEQ ID NO:15, or residues 21-169 of H19011_1_P9_V1 (SEQ ID NO:34), corresponding to amino acid sequence depicted in SEQ ID NO:36, or residues 1-184 of the sequence H19011_1_P8 (SEQ ID NO:4), corresponding to amino acid sequence depicted in SEQ ID NO:37, or residues 1-184 of the sequence H19011_1_P8_V1 (SEQ ID NO:5), corresponding to amino acid sequence depicted in SEQ ID NO:19, or residues 1-169 of H19011_1_P9 (SEQ ID NO:6), corresponding to amino acid sequence depicted in SEQ ID NO:28, or residues 1-169 of H19011_1_P9_V1 (SEQ ID NO:34), corresponding to amino acid sequence depicted in SEQ ID NO:30.
5. The use of item 2, wherein the heterologous sequence comprises at least a portion of an immunoglobulin constant domain.
6. The use of item 5 wherein the fusion protein comprises an immunoglobulin heavy chain constant region corresponding to an antibody isotype selected from the group consisting of an IgG1, IgG2, IgG3, IgG4, IgM, IgE, IgA and IgD.
7. The use of item 6, wherein the immunoglobulin constant domain comprises the hinge, CH2 and CH3 regions of a human IgG immunoglobulin, selected from the group consisting of Cγ1, Cγ2, Cγ3 and Cy4 chain.
8. The use of any of items 1-7, wherein the fusion protein further comprises a domain that mediates dimerization or multimerization of the fusion protein to form homodimers, heterodimers, homomultimers, or heteromultimers.
9. The use of item 8, wherein the domain that mediates dimerization or multimerization is selected from the group consisting of one or more cysteines that are capable of forming an intermolecular disulfide bond with a cysteine on the partner fusion protein, a coiled-coil domain, an acid patch, a zinc finger domain, a calcium hand domain,a CHI region, a CL region, a leucine zipper domain, an SH2 (src homology 2) domain, an SH3 (src Homology 3) domain, a PTB (phosphotyrosine binding) domain, a WW domain, a PDZ domain, a 14-3-3 domain, a WD40 domain, an EH domain, a Lim domain, an isoleucine zipper domain, and a dimerization domain of a receptor dimer pair.
10. The use of any of items 2-9 wherein the fusion protein comprises the polypeptide of any one of SEQ ID NOs: 8, 22, 23, 38, 29.
11. The use of any of the above items, wherein the fusion protein is a dimeric protein comprising a first and a second fusion protein as described in any of items 2-10, wherein the first and the second fusion proteins are bound to one another by covalent or noncovalent bonds to form a dimer.
12. The use of any of items 2-11, wherein the fusion proteins are bound together by disulfide bonds.
13. The use of any of the above items, wherein the protein is administered in the form of a pharmaceutical composition, and a pharmaceutically acceptable diluent or carrier, adapted for treatment of immune related disorder.
14. The use of any or the above items, wherein the protein is attached to a detectable or therapeutic moiety.
15. The use of any of the above items, wherein administering an effective amount of the protein or pharmaceutical composition to the subject inhibits or reduces differentiation of, proliferation of, activity of, and/or cytokine production and/or secretion by an immune cell selected from the group consisting of Th1, Th17, Th22, other cells that secrete, or cells that cause other cells to secrete, inflammatory molecules.
16. The use of item 15, wherein the protein or pharmaceutical composition is administered in an effective amount to inhibit or reduce differentiation of, proliferation of, activity of, and/or cytokine production and/or secretion by Th1, Th17 and/or Th22 cells.
17. The use of items 15 or 16, wherein the protein or pharmaceutical composition is administered in an effective amount to enhance the suppressive or immunomodulatory effect of Tregs and/or Th2 cells on Th1 or Th17 cells.
18. The use of any of items 15-17, wherein the protein or pharmaceutical composition is administered in an effective amount to promote or enhance IL-10 production.
19. The use of any of items 15-18, wherein the protein or pharmaceutical composition is administered in an effective amount to increase cell numbers or increase populations of any of Tregs and/or Th2 cells.
20. The use of any of items 15-19, wherein the protein or pharmaceutical composition is administered in an effective amount to inhibit the Th1 and/or Th17 pathways and to enhance the activity of Tregs and/or Th2 cells on the Th1 and Th17 pathways and/or to promote or enhance IL-10 secretion.
21. The use of any of items 15-20, wherein the protein or pharmaceutical composition is administered in an effective amount for reducing proinflammatory molecule production in a subject.
22. The use of any of the above items, further comprising administering a second therapeutic agent effective for treatment of immune related disorder.
23. The use of any of the above items wherein treatment comprises one or more of preventing, curing, managing, reversing, attenuating, alleviating, minimizing, suppressing, managing, or halting the deleterious effects of the above-described diseases.
24. The use of item 23 wherein the treating comprises treatment of immune related disorder without global immunosuppression.
25. The use of items 23 or 24 wherein the treatment of immune related disorder comprises induction of immune tolerance.
26. The use of any of items 23-25, wherein the treatment comprises inhibition of infiltration of reactive T lymphocytes into the central nervous system.
27. The use of any of items 23-26, wherein the treatment comprises prevention of damage to the myelin coat of neural cells in the central nervous system.
28. The use of any of items 23-27, wherein treatment comprises reducing the severity of the disease, reducing the frequency of episodes of the disease, reducing the duration of such episodes, or reducing the severity of such episodes or a combination thereof.
29. The use of any of items 23-28 wherein the subject did not previously respond to treatment with TNF blockers.

Various aspects of the invention are described in further detail in the following subsections.

### PEPTIDES

The terms "polypeptide," "peptide" and "protein" are used interchangeably herein to refer to a polymer of amino acid residues. The terms apply to amino acid polymers in which one or more amino acid residue is an analog or mimetic of a corresponding naturally occurring amino acid, as well as to naturally occurring amino acid polymers. Polypeptides can be modified, e.g., by the addition of carbohydrate residues to form glycoproteins. The terms "polypeptide," "peptide" and "protein" include glycoproteins, as well as non-glycoproteins.

Polypeptide products can be biochemically synthesized such as by employing standard solid phase techniques. Such methods include exclusive solid phase synthesis, partial solid phase synthesis methods, fragment condensation, classical solution synthesis. These methods are preferably used when the peptide is relatively short (i.e., 10 kDa) and/or when it cannot be produced by recombinant techniques (i.e., not encoded by a nucleic acid sequence) and therefore involves different chemistry.

Solid phase polypeptide synthesis procedures are well known in the art and further described by John Morrow Stewart and Janis Dillaha Young, Solid Phase Peptide Syntheses (2nd Ed., Pierce Chemical Company, 1984).

Synthetic polypeptides can be purified by preparative high performance liquid chromatography [Creighton T. (1983) Proteins, structures and molecular principles. WH Freeman and Co. N.Y.] and the composition of which can be confirmed via amino acid sequencing.

In cases where large amounts of a polypeptide are desired, it can be generated using recombinant techniques such as described by Bitter et al., (1987) Methods in Enzymol. 153:516-544, Studier et al. (1990) Methods in Enzymol. 185:60-89, Brisson et al. (1984) Nature 310:511-514, Takamatsu et al. (1987) EMBO J. 6:307-311, Coruzzi et al. (1984) EMBO J. 3:1671-1680 and Brogli et al., (1984) Science 224:838-843, Gurley et al. (1986) Mol. Cell. Biol. 6:559-565 and Weissbach & Weissbach, 1988, Methods for Plant Molecular Biology, Academic Press, NY, Section VIII, pp 421-463.

It will be appreciated that peptides identified according to the teachings of the present invention may be degradation products, synthetic peptides or recombinant peptides as well as peptidomimetics, typically, synthetic peptides and peptoids and semipeptoids which are peptide analogs, which may have, for example, modifications rendering the peptides more stable while in a body or more capable of penetrating into cells. Such modifications include, but are not limited to N terminus modification, C terminus modification, peptide bond modification, including, but not limited to, CH2-NH, CH2-S, CH2-S=O, O=C-NH, CH2-O, CH2-CH2, S=C-NH, CH=CH or CF=CH, backbone modifications, and residue modification. Methods for preparing peptidomimetic compounds are well known in the art and are specified, for example, in Quantitative Drug Design, C.A. Ramsden Gd., Chapter 17.2, F. Choplin Pergamon Press (1992). Further details in this respect are provided hereinunder.

Peptide bonds (-CO-NH-) within the peptide may be substituted, for example, by N-methylated bonds (-N(CH3)-CO-), ester bonds (-C(R)H-C-O-O-C(R)-N-), ketomethylen bonds (-CO-CH2-), α-aza bonds (-NH-N(R)-CO-), wherein R is any alkyl, e.g., methyl, carba bonds (-CH2-NH-), hydroxyethylene bonds (-CH(OH)-CH2-), thioamide bonds (-CS-NH-), olefinic double bonds (-CH=CH-), retro amide bonds (-NH-CO-), peptide derivatives (-N(R)-CH2-CO-), wherein R is the "normal" side chain, naturally presented on the carbon atom.

These modifications can occur at any of the bonds along the peptide chain and even at several (2-3) at the same time.

Natural aromatic amino acids, Trp, Tyr and Phe, may be substituted by synthetic non-natural acid such as Phenylglycine, TIC, naphthylelanine (Nol), ring-methylated derivatives of Phe, halogenated derivatives of Phe or o-methyl-Tyr.

In addition to the above, the peptides may also include one or more modified amino acids or one or more non-amino acid monomers (e.g. fatty acids, complex carbohydrates etc).

As used herein in the specification and in the claims section below the term "amino acid" or "amino acids" is understood to include the 20 naturally occurring amino acids; those amino acids often modified post-translationally in vivo, including, for example, hydroxyproline, phosphoserine and phosphothreonine; and other unusual amino acids including, but not limited to, 2-aminoadipic acid, hydroxylysine, isodesmosine, nor-valine, nor-leucine and ornithine. Furthermore, the term "amino acid" includes both D- and L-amino acids.

Since the peptides are preferably utilized in therapeutics which require the peptides to be in soluble form, the peptides preferably include one or more non-natural or natural polar amino acids, including but not limited to serine and threonine which are capable of increasing peptide solubility due to their hydroxyl-containing side chain.

In cases where large amounts of the peptides are desired, the peptides can be generated using recombinant techniques such as described by Bitter et al., (1987) Methods in Enzymol. 153:516-544, Studier et al. (1990) Methods in Enzymol. 185:60-89, Brisson et al. (1984) Nature 310:511-514, Takamatsu et al. (1987) EMBO J. 6:307-311, Coruzzi et al. (1984) EMBO J. 3:1671-1680 and Brogli et al., (1984) Science 224:838-843, Gurley et al. (1986) Mol. Cell. Biol. 6:559-565 and Weissbach & Weissbach, 1988, Methods for Plant Molecular Biology, Academic Press, NY, Section VIII, pp 421-463.

### PROTEIN CHEMICAL MODIFICATIONS

In the present invention any part of a protein of the invention may optionally be chemically modified, i.e. changed by addition of functional groups. For example the side amino acid residues appearing in the native sequence may optionally be modified, although as described below alternatively other parts of the protein may optionally be modified, in addition to or in place of the side amino acid residues. The modification may optionally be performed during synthesis of the molecule if a chemical synthetic process is followed, for example by adding a chemically modified amino acid. However, chemical modification of an amino acid when it is already present in the molecule ("in situ" modification) is also possible.

The amino acid of any of the sequence regions of the molecule can optionally be modified according to any one of the following exemplary types of modification (in the peptide conceptually viewed as "chemically modified"). Non-limiting exemplary types of modification include carboxymethylation, acylation, phosphorylation, glycosylation or fatty acylation. Ether bonds can optionally be used to join the serine or threonine hydroxyl to the hydroxyl of a sugar. Amide bonds can optionally be used to join the glutamate or aspartate carboxyl groups to an amino group on a sugar (Garg and Jeanloz, Advances in Carbohydrate Chemistry and Biochemistry, Vol. 43, Academic Press (1985); Kunz, Ang. Chem. Int. Ed. English 26:294-308 (1987)). Acetal and ketal bonds can also optionally be formed between amino acids and carbohydrates. Fatty acid acyl derivatives can optionally be made, for example, by acylation of a free amino group (e.g., lysine) (Toth et al., Peptides: Chemistry, Structure and Biology, Rivier and Marshal, eds., ESCOM Publ., Leiden, 1078-1079 (1990)).

As used herein the term "chemical modification", when referring to a protein or peptide, refers to a protein or peptide where at least one of its amino acid residues is modified either by natural processes, such as processing or other post-translational modifications, or by chemical modification techniques which are well known in the art. Examples of the numerous known modifications typically include, but are not limited to: acetylation, acylation, amidation, ADP-ribosylation, glycosylation, GPI anchor formation, covalent attachment of a lipid or lipid derivative, methylation, myristylation, pegylation, prenylation, phosphorylation, ubiquitination, or any similar process.

Other types of modifications optionally include the addition of a cycloalkane moiety to a biological molecule, such as a protein, as described in PCT Application No. WO 2006/050262. These moieties are designed for use with biomolecules and may optionally be used to impart various properties to proteins.

Furthermore, optionally any point on a protein may be modified. For example, pegylation of a glycosylation moiety on a protein may optionally be performed, as described in PCT Application No. WO 2006/050247. One or more polyethylene glycol (PEG) groups may optionally be added to O-linked and/or N-linked glycosylation. The PEG group may optionally be branched or linear. Optionally any type of water-soluble polymer may be attached to a glycosylation site on a protein through a glycosyl linker.

### ALTERED GLYCOSYLATION PROTEIN MODIFICATION

Proteins may be modified to have an altered glycosylation pattern (i.e., altered from the original or native glycosylation pattern). As used herein, "altered" means having one or more carbohydrate moieties deleted, and/or having at least one glycosylation site added to the original protein.

Glycosylation of proteins is typically either N-linked or O-linked. N-linked refers to the attachment of the carbohydrate moiety to the side chain of an asparagine residue. The tripeptide sequences, asparagine-X-serine and asparagine-X-threonine, where X is any amino acid except proline, are the recognition sequences for enzymatic attachment of the carbohydrate moiety to the asparagine side chain. Thus, the presence of either of these tripeptide sequences in a polypeptide creates a potential glycosylation site. O-linked glycosylation refers to the attachment of one of the sugars N-acetylgalactosamine, galactose, or xylose to a hydroxyamino acid, most commonly serine or threonine, although 5-hydroxyproline or 5-hydroxylysine may also be used.

Addition of glycosylation sites to proteins is conveniently accomplished by altering the amino acid sequence of the protein such that it contains one or more of the above-described tripeptide sequences (for N-linked glycosylation sites). The alteration may also be made by the addition of, or substitution by, one or more serine or threonine residues in the sequence of the original protein (for O-linked glycosylation sites). The protein's amino acid sequence may also be altered by introducing changes at the DNA level.

Another means of increasing the number of carbohydrate moieties on proteins is by chemical or enzymatic coupling of glycosides to the amino acid residues of the protein. Depending on the coupling mode used, the sugars may be attached to (a) arginine and histidine, (b) free carboxyl groups, (c) free sulfhydryl groups such as those of cysteine, (d) free hydroxyl groups such as those of serine, threonine, or hydroxyproline, (e) aromatic residues such as those of phenylalanine, tyrosine, or tryptophan, or (f) the amide group of glutamine. These methods are described in WO 87/05330, and in Aplin and Wriston, CRC Crit. Rev. Biochem., 22: 259-306 (1981).

Removal of any carbohydrate moieties present on proteins may be accomplished chemically, enzymatically or by introducing changes at the DNA level. Chemical deglycosylation requires exposure of the protein to trifluoromethanesulfonic acid, or an equivalent compound. This treatment results in the cleavage of most or all sugars except the linking sugar (N-acetylglucosamine or N-acetylgalactosamine), leaving the amino acid sequence intact.

Chemical deglycosylation is described by Hakimuddin et al., Arch. Biochem. Biophys., 259: 52 (1987); and Edge et al., Anal. Biochem., 118: 131 (1981). Enzymatic cleavage of carbohydrate moieties on proteins can be achieved by the use of a variety of endo- and exoglycosidases as described by Thotakura et al., Meth. Enzymol., 138: 350 (1987).

### METHODS OF TREATMENT

As used herein the term "treating" refers to preventing, delaying the onset of, curing, reversing, attenuating, alleviating, minimizing, suppressing or halting the deleterious effects of the above-described diseases, disorders or conditions. It also includes managing the disease as described above. By "manage" it is meant reducing the severity of the disease, reducing the frequency of episodes of the disease, reducing the duration of such episodes, reducing the severity of such episodes and the like.

It will be appreciated that treatment of the above-described diseases may be combined with other treatment methods known in the art (i.e., combination therapy), as described herein.

Thus, treatment of multiple sclerosis using the agents according to at least some embodiments of the present invention may be combined with, for example, any known therapeutic agent or method for treating multiple sclerosis. Non-limiting examples of such known therapeutic agent or method for treating multiple sclerosis include interferon class, IFN-beta-1a (REBIF^{®}. AVONEX^{®} and CINNOVEX^{®}) and IFN-beta-1b (BETASERON^{®}, EXTAVIA^{®}, BETAFERON^{®}, ZIFERON^{®}); glatiramer acetate (COPAXONE^{®}), a polypeptide; natalizumab (TYSABRI^{®}); and mitoxantrone (NOVANTRONE^{®}), a cytotoxic agent, Fampridine (AMPYRA^{®}). Other drugs include corticosteroids, methotrexate, cyclophosphamide, azathioprine, and intravenous immunoglobulin (IVIG), inosine, Ocrelizumab (R1594), Mylinax (Caldribine), alemtuzumab (Campath), daclizumab (Zenapax), Panaclar/ dimethyl fumarate (BG-12), Teriflunomide (HMR1726), fingolimod (FTY720), laquinimod (ABR216062), as well as Haematopoietic stem cell transplantation, Neurovax, Rituximab (Rituxan) BCG vaccine, low dose naltrexone, helminthic therapy, angioplasty, venous stents, and alternative therapy, such as vitamin D, polyunsaturated fats, medical marijuana.

Thus, treatment of rheumatoid arthritis, using the agents according to at least some embodiments of the present invention may be combined with, for example, any known therapeutic agent or method for treating rheumatoid arthritis. Non-limiting examples of such known therapeutic agents or methods for treating rheumatoid arthritis include glucocorticoids, nonsteroidal anti-inflammatory drug (NSAID) such as salicylates, or cyclooxygenase-2 inhibitors, ibuprofen and naproxen, diclofenac, indomethacin, etodolac Disease-modifying antirheumatic drugs (DMARDs)- Oral DMARDs: Auranofin (Ridaura), Azathioprine (Imuran), Cyclosporine (Sandimmune, Gengraf, Neoral, generic), D-Penicillamine (Cuprimine), Hydroxychloroquine (Plaquenil), IM gold Gold sodium thiomalate (Myochrysine) Aurothioglucose (Solganal), Leflunomide (Arava), Methotrexate (Rheumatrex), Minocycline (Minocin), Staphylococcal protein A immunoadsorption (Prosorba column), Sulfasalazine (Azulfidine). Biologic DMARDs: TNF-α blockers including Adalimumab (Humira), Etanercept (Enbrel), Infliximab (Remicade), golimumab (Simponi), certolizumab pegol (Cimzia), and other Biological DMARDs, such as Anakinra (Kineret), Rituximab (Rituxan), Tocilizumab (Actemra), CD28 inhibitor including Abatacept (Orencia) and Belatacept.

Thus, treatment of IBD, using the agents according to at least some embodiments of the present invention may be combined with, for example, any known therapeutic agent or method for treating IBD. Non-limiting examples of such known therapeutic agents or methods for treating IBD include immunosuppression to control the symptom, such as prednisone, Mesalazine (including Asacol, Pentasa, Lialda, Aspiro), azathioprine (Imuran), methotrexate, or 6-mercaptopurine, steroids, Ondansetron, TNF-α blockers (including infliximab, adalimumab golimumab, certolizumab pegol), Orencia (abatacept), ustekinumab (Stelara^{®}), Briakinumab (ABT-874), Certolizumab pegol (Cimzia^{®}), ITF2357 (givinostat), Natalizumab (Tysabri), Firategrast (SB-683699), Remicade (infliximab), vedolizumab (MLN0002), other drugs including GSK1605786 CCX282-B (Traficet-EN), AJM300, Stelara (ustekinumab), Semapimod (CNI-1493) tasocitinib (CP-690550), LMW Heparin MMX, Budesonide MMX, Simponi (golimumab), MultiStem^{®}, Gardasil HPV vaccine, Epaxal Berna (virosomal hepatitis A vaccine), surgery, such as bowel resection, strictureplasty or a temporary or permanent colostomy or ileostomy; antifungal drugs such as nystatin (a broad spectrum gut antifungal) and either itraconazole (Sporanox) or fluconazole (Diflucan); alternative medicine, prebiotics and probiotics, cannabis, Helminthic therapy or ova of the Trichuris suis helminth.

Thus, treatment of psoriasis, using the agents according to at least some embodiments of the present invention may be combined with, for example, any known therapeutic agent or method for treating psoriasis. Non-limiting examples of such known therapeutics for treating psoriasis include topical agents, typically used for mild disease, phototherapy for moderate disease, and systemic agents for severe disease. Non-limiting examples of topical agents: bath solutions and moisturizers, mineral oil, and petroleum jelly; ointment and creams containing coal tar, dithranol (anthralin), corticosteroids like desoximetasone (Topicort), Betamethasone, fluocinonide, vitamin D3 analogues (for example, calcipotriol), and retinoids. Non-limiting examples of phototherapy: sunlight; wavelengths of 311-313 nm, psoralen and ultraviolet A phototherapy (PUVA). Non-limiting examples of systemic agents: Biologics, such as interleukin antagonists, TNF-α blockers including antibodies such as infliximab (Remicade), adalimumab (Humira), golimumab, certolizumab pegol, and recombinant TNF-α decoy receptor, etanercept (Enbrel); drugs that target T cells, such as efalizumab (Xannelim/Raptiva), alefacept (Ameviv), dendritic cells such Efalizumab; monoclonal antibodies (MAbs) targeting cytokines, including anti- IL-12/IL-23 (ustekinumab (brand name Stelara)) and anti-Interleukin-17; Briakinumab (ABT-874); small molecules, including but not limited to ISA247; Immunosuppressants, such as methotrexate, cyclosporine; vitamin A and retinoids (synthetic forms of vitamin A); and alternative therapy, such as changes in diet and lifestyle, fasting periods, low energy diets and vegetarian diets, diets supplemented with fish oil rich in Vitamin A and Vitamin D (such as cod liver oil), Fish oils rich in the two omega-3 fatty acids eicosapentaenoic acid (EPA) and docosahexaenoic acid (DHA) and contain Vitamin E. Ichthyotherapy, Hypnotherapy, cannabis.

Thus, treatment of type 1 diabetes, using the agents according to at least some embodiments of the present invention may be combined with, for example, any known therapeutic agent or method for treating type 1diabetes. Non-limiting examples of such known therapeutics for treating type 1 diabetes include insulin, insulin analogs, islet transplantation, stem cell therapy including PROCHYMAL^{®}, non-insulin therapies such as il-1beta inhibitors including Anakinra (Kineret^{®}), Abatacept (Orencia^{®}), Diamyd, alefacept (Ameviv^{®}), Otelixizumab, DiaPep277 (Hsp60 derived peptide), Alpha 1-Antitrypsin, Prednisone, azathioprine, Ciclosporin, E1-INT (an injectable islet neogenesis therapy comprising an epidermal growth factor analog and a gastrin analog), statins including Zocor^{®}, Simlup^{®}, Simcard^{®}, Simvacor^{®}, Sitagliptin (dipeptidyl peptidase (DPP-4) inhibitor), Anti-CD3 mAb (e.g., Teplizumab); CTLA4-Ig (abatacept), Anti IL-1Beta (Canakinumab), Anti-CD20 mAb (e.g, rituximab).

Thus, treatment of uveitis, using the agents according to at least some embodiments of the present invention may be combined with, for example, any known therapeutic agent or method for treating uveitis. Non-limiting examples of such known therapeutics for treating uveitis include corticosteroids, topical cycloplegics, such as atropine or homatropine, or injection of PSTTA (posterior subtenon triamcinolone acetate), antimetabolite medications, such as methotrexate, TNF-α blockers (including infliximab, adalimumab, etanercept, golimumab, certolizumab pegol).

Thus, treatment for Sjogren's syndrome, using the agents according to at least some embodiments of the present invention may be combined with, for example, any known therapeutic agent or method for treating for Sjogren's syndrome. Non-limiting examples of such known therapeutics for treating for Sjogren's syndrome include Cyclosporine, pilocarpine (Salagen) and cevimeline (Evoxac), Hydroxychloroquine (Plaquenil), cortisone (prednisone and others) and/or azathioprine (Imuran) or cyclophosphamide (Cytoxan), Dexamethasone, Thalidomide, Dehydroepiandrosterone, NGX267, Rebamipide, FID 114657, Etanercept, Raptiva, Belimumab, MabThera (rituximab); Anakinra, intravenous immune globulin (IVIG), Allogeneic Mesenchymal Stem Cells (AlloMSC), Automatic neuro-electrostimulation by "Saliwell Crown".

Thus, treatment for systemic lupus erythematosus, using the agents according to at least some embodiments of the present invention may be combined with, for example, any known therapeutic agent or method for treating for systemic lupus erythematosus. Non-limiting examples of such known therapeutics for treating for systemic lupus erythematosus include corticosteroids and Disease-modifying antirheumatic drugs (DMARDs), commonly anti-malarial drugs such as plaquenil and immunosuppressants (e.g. methotrexate and azathioprine) Hydroxychloroquine, cytotoxic drugs (e.g., cyclophosphamide and mycophenolate), Hydroxychloroquine (HCQ), Benlysta (belimumab), nonsteroidal antiinflammatory drugs, Prednisone, Cellcept, Prograf, Atacicept, Lupuzor, Intravenous Immunoglobulins (IVIGs), CellCept (mycophenolate mofetil), Orencia, CTLA4-IgG4m (RG2077), rituximab, Ocrelizumab, Epratuzumab, CNTO 136, Sifalimumab (MEDI-545), A-623 (formerly AMG 623), AMG 557, Rontalizumab, paquinimod (ABR-215757), LY2127399, CEP-33457, Dehydroepiandrosterone, Levothyroxine, abetimus sodium (LJP 394), Memantine, Opiates, Rapamycin, Renal transplantation, stem cell transplantation.

For example, MS disease may be treated with C10RF32 fusion proteins in conjunction with, but not limited to, immunosuppressants such as corticosteroids, cyclosporin, prednisone, azathioprine, methotrexate, TNF-alpha blockers or antagonists, or any other biological agent targeting any inflammatory cytokine, nonsteroidal antiinflammatory drugs/Cox-2 inhibitors, hydroxychloroquine, sulphasalazopryine, gold salts, etanercept, infliximab, rapamycin, mycophenolate mofetil, azathioprine, tacrolismus, basiliximab, cytoxan, interferon beta-la, interferon beta-lb, glatiramer acetate, mitoxantrone hydrochloride, anakinra and/or other biologics. The C1ORF32 fusion proteins can also be used in combination with one or more of the following agents to regulate an immune response: soluble gp39 (also known as CD40 ligand (CD40L), CD154, T-BAM, TRAP), soluble CD29, soluble CD40, soluble CD80 (e.g. ATCC 68627), soluble CD86, soluble CD28 (e.g. 68628), soluble CD56, soluble Thy-1, soluble CD3, soluble TCR, soluble VLA-4, soluble VCAM-1, soluble LECAM-1, soluble ELAM-1, soluble CD44, antibodies reactive with gp39 (e.g. ATCC HB-10916, ATCC HB-12055 and ATCC HB-12056), antibodies reactive with CD40 (e.g. ATCC HB-9110), antibodies reactive with B7 (e.g. ATCC HB-253, ATCC CRL-2223, ATCC CRL-2226, ATCC HB-301, ATCC HB-11341, etc), antibodies reactive with CD28 (e.g. ATCC HB-11944 or mAb 9.3), antibodies reactive with LFA-1 (e.g. ATCC HB-9579 and ATCC TIB-213), antibodies reactive with LFA-2, antibodies reactive with IL-2, antibodies reactive with IL-12, antibodies reactive with IFN-gamma, antibodies reactive with CD2, antibodies reactive with CD48, antibodies reactive with any ICAM (e.g., ICAM-1 (ATCC CRL-2252), ICAM-2 and ICAM-3), antibodies reactive with CTLA4 (e.g. ATCC HB-304), antibodies reactive with Thy-1, antibodies reactive with CD56, antibodies reactive with CD3, antibodies reactive with CD29, antibodies reactive with TCR, antibodies reactive with VLA-4, antibodies reactive with VCAM-1, antibodies reactive with LECAM-1, antibodies reactive with ELAM-1, antibodies reactive with CD44. In certain embodiments, monoclonal antibodies are preferred. In other embodiments, antibody fragments are preferred. As persons skilled in the art will readily understand, the combination can include the C1OR-F32 polypeptides, fragments or fusion proteins thereof with one other immunosuppressive agent, with two other immunosuppressive agents, with three other immunosuppressive agents, etc. The determination of the optimal combination and dosages can be determined and optimized using methods well known in the art.

The C1ORF32 fusion proteins can also be used in combination with one or more of the following agents: L104EA29YIg, CD80 monoclonal antibodies (mAbs), CD86 mAbs, gp39 mAbs, CD40 mAbs, CD28 mAbs; anti-LFA1 mAbs, antibodies or other agents targeting mechanisms of the immune system such as CD52 (alemtuzumab), CD25 (daclizumab), VLA-4 (natalizumab), CD20 (rituximab), IL2R (daclizumab) and MS4A1 (ocrelizumab); novel oral immunomodulating agents have shown to prevent lymphocyte recirculation from lymphoid organs such as fingolimod (FTY720) or leading to lymphocyte depletion such as mylinax (oral cladribine) or teriflunomide; and agents that prevent immunoactivation such as panaclar (dimethyl fumarate BG-12) or laquinimod (ABR216062). Other combinations will be readily appreciated and understood by persons skilled in the art.

The C1ORF32 fusion proteins may be administered as the sole active ingredient or together with other drugs in immunomodulating regimens or other anti-inflammatory agents e.g. for the treatment or prevention of allo- or xenograft acute or chronic rejection or inflammatory or autoimmune disorders, or to induce tolerance. For example, it may be used in combination with a calcineurin inhibitor, e.g. cyclosporin A or FK506; an immunosuppressive macrolide, e.g. rapamycine or a derivative thereof; e.g. 40-O-(2-hydroxy)ethyl-rapamycin, a lymphocyte homing agent, e.g. FTY720 or an analog thereof, corticosteroids; cyclophosphamide; azathioprene; methotrexate; leflunomide or an analog thereof; mizoribine; mycophenolic acid; mycophenolate mofetil; 15-deoxyspergualine or an analog thereof; immunosuppressive monoclonal antibodies, e.g., monoclonal antibodies to leukocyte receptors, e.g., MHC, CD2, CD3, CD4, CD 11a/CD18, CD7, CD25, CD 27, B7, CD40, CD45, CD58, CD 137, ICOS, CD150 (SLAM), OX40, 4-1BB or their ligands; or other immunomodulatory compounds, e.g. CTLA4/CD28-Ig, or other adhesion molecule inhibitors, e.g. mAbs or low molecular weight inhibitors including LFA-1 antagonists, Selectin antagonists and VLA-4 antagonists.

Where the C1OR-F32 fusion proteins are administered in conjunction with other immunosuppressive/immunomodulatory or anti-inflammatory therapy, e.g. as hereinabove specified, dosages of the co-administered immunosuppressant, immunomodulatory or antiinflammatory compound will of course vary depending on the type of co-drug employed, e.g. whether it is a steroid or a cyclosporin, on the specific drug employed, on the condition being treated and so forth.

### Methods of Therapeutic Use

The C1ORF32 fusions disclosed herein are useful as therapeutic agents. Immune cells, preferably T cells, can be contacted in vivo or ex vivo with C1ORF32 fusion polypeptides to decrease or inhibit immune responses including, but not limited to inflammation. The T cells contacted with C1OR-F32 fusion polypeptides can be any cell which expresses the T cell receptor, including α/β and γ/δ T cell receptors. T-cells include all cells which express CD3, including T-cell subsets which also express CD4 and CDS. T-cells include both naive and memory cells and effector cells such as CTL. T-cells also include cells such as Th1, Tc1, Th2, Tc2, Th3, Th17, Th22, Treg, and Tr1 cells. T-cells also include NKT-cells and similar unique classes of the T-cell lineage. For example the compositions can be used to modulate Th1, Th17, Th22, or other cells that secrete, or cause other cells to secrete, inflammatory molecules, including, but not limited to, IL-1beta, TNF-alpha, TGF-beta, IFN-gamma, IL-17, IL-6, IL-23, IL-22, IL-21, and MMPs. The compositions can also be used to increase or promote the activity of Tregs, increase the production of cytokines such as IL-10 from Tregs, increase the differentiation of Tregs, increase the number of Tregs, or increase the survival of Tregs. The compositions can also be used to increase or promote the activity of Th2 cells, increase the production of cytokines such as IL-10 or IL-4 from Th2 cells, increase the differentiation of Th2 cells, increase the number of Th2 cells, or increase the survival of Th2 cells.

In some embodiments, the disclosed C1ORF32 fusions are administered in combination with a second therapeutic. Combination therapies may be useful in immune modulation. In some embodiments, C1ORF32 fusions can be used to attenuate or reverse the activity of a pro-inflammatory drug, and/or limit the adverse effects of such drugs. Other immune cells that can be treated with the disclosed C1OR-F32 fusion include T cell precursors, antigen presenting cells such as dendritic cells and monocytes or their precursors, B cells or combinations thereof. The C1OR-F32 compositions can be used to modulate the production of antibodies by B cells by contacting the B cells with an effective amount of the C1OR-F32 composition to inhibit or reduce antibody production by the B cell relative to a control. The C10RF32 compositions can also modulate the production of cytokines by the B cells.

### Methods of treating inflammatory responses

The C1ORF32 fusion proteins inhibit T cell activation, as manifested by T cell proliferation and cytokine secretion. Specifically, the proteins inhibit Th1 and Th17 responses, while promoting Th2 responses.

The C10RF32 fusion proteins are potentially used for therapy of diseases that require down-regulation of costimulatory pathways and or such that require downregulation of Th1 and/or Thl7 responses.

A further embodiment provides methods for treating or alleviating one or more symptoms of inflammation. In a further embodiment, the compositions and methods disclosed are useful for treating chronic and persistent inflammation. Inflammation in general can be treated using the disclosed C1OR-F32 fusions.

An immune response including inflammation can be inhibited or reduced in a subject, preferably a human, by administering an effective amount of C1ORF32 fusion to inhibit or reduce the biological activity of an immune cell or to reduce the amounts of proinflammatory molecules at a site of inflammation. Exemplary proinflammatory molecules include, but are not limited to, IL-1beta, TNF-alpha, TGF-beta, IFN-gamma, IL-17, IL-6, IL-23, IL-22, IL-21, and MMPs. Th1 and Th17 are exemplary T cells that can be targeted for inhibition by C1OR-F32 polypeptides, fusion proteins or fragments thereof to inhibit or reduce inflammation.

Without wishing to be limited by a single hypothesis for this biological mechanism or any other biological mechanism described herein, the C1ORF32 fusion proteins are useful for treating inflammation by any or all of the following: inhibiting or reducing differentiation of Th1, Thl7, Th22, and/or other cells that secrete, or cause other cells to secrete, inflammatory molecules, including, but not limited to, IL-1beta, TNF-alpha, TGF-beta, IFN-gamma, IL-17, IL-6, IL-23, IL-22, IL-21, and MMPs; inhibiting or reducing activity of ThI, Th 17, Th22, and/or other cells that secrete, or cause other cells to secrete, inflammatory molecules, including, but not limited to, IL-1beta, TNF-alpha, TGF-beta, IFN-gamma, IL-17, IL-6, IL-23, IL-22, IL-21, and MMPs; inhibiting or reducing the Th1 and/or Thl7 pathways; inhibiting or reducing cytokine production and/or secretion by Th1, Th17, Th22, and/or other cells that secrete, or cause other cells to secrete, inflammatory molecules, including, but not limited to, IL-1beta, TNF-alpha, TGF-beta, IFN-gamma, IL-17, IL-6 IL-23, IL-22, IL-21, and MMPs; inhibiting or reducing proliferation of Th1, Th17, Th22, and/or other cells that secrete, or cause other cells to secrete, inflammatory molecules, including, but not limited to, IL-1beta, TNF-alpha, TGF-beta, IFN-gamma, IL-17, IL-6, IL-23, IL-22, IL-21, and MMPs.

Additionally, C10RF32 fusion proteins can also enhance Th2 immune responses. C10RF32 fusion proteins can also act directly on Th2 cells to promote or enhance production of IL-4, IL-5 or IL-10, or to increase the number of Th2 cells, resulting in inhibition of Th1 and/or Thl7, and in immune modulation via a Th1/Th2 shift.

Additionally, C1ORF32 fusion proteins can cause Tregs to have an enhanced suppressive effect on an immune response. Tregs can suppress differentiation, proliferation, activity, and/or cytokine production and/or secretion by Th1, Th17, Th22, and/or other cells that secrete, or cause other cells to secrete, inflammatory molecules, including, but not limited to, IL-1beta, TNF-alpha, TGF-beta, IFN-gamma, IL-17, IL-6, IL-23, IL-22, IL-21, and MMPs. For example, C1OR-F32 fusion proteins can cause Tregs to have an enhanced suppressive effect on Th1 and/or Th17 cells to reduce the level of IFN-gamma and IL-17 produced, respectively. C1OR-F32 fusion proteins can also act directly on Tregs to promote or enhance production of IL-10 to suppress the Th1 and/or Th17 pathway, and/or to increase the number of Tregs.

Additionally, C1OR-F32 fusion proteins can cause Th2 to have an enhanced modulatory effect on an immune response. Th2 cells can modulate differentiation, proliferation, activity, and/or cytokine production and/or secretion by Th1, Th17, Th22, and/or other cells that secrete, or cause other cells to secrete, inflammatory molecules, including, but not limited to, IL-1beta, TNF-alpha, TGF-beta, IFN-gamma, IL-17, IL-6, IL-23, IL-22, IL-21, and MMPs. For example, C1ORF32 fusion proteins can cause Th2 cells to have an enhanced modulatory effect on Th1 and/or Th17 cells to reduce the level of IFN-gamma and IL-17 produced, respectively. C1ORF32 fusion proteins can also act directly on Th2 cells to promote or enhance production of IL-10 to suppress the Th1 and/or Th17 pathway, and/or to increase the number of Th2 cells.

Without wishing to be limited by a single hypothesis, it is believed that C1ORF32 fusion proteins act at multiple points in multiple T cell pathways. For example, C1ORF32 fusion proteins can inhibit the differentiation of naive T cells into either Th1 or Th17 cells. Alternatively, C1OR-F32 fusion proteins can interact with Th1 cells or Th17 cells, or both to inhibit or reduce the production of proinflammatory molecules.

Additionally, C1ORF32 fusion proteins may increase the differentiation of and/or promote Th2 responses resulting in an immunomdulatory effect on the Th1 and/or Th17 pathways to reduce the level of INF-gamma and/or IL-17 produced. C1ORF32 fusion proteins enhances the production of IL-10 from cells such as Th2 and/or Tregs, which in turn inhibits the activity of Th1 and/or Th17 cells.

Additionally, C1ORF32 fusion proteins can affect Tregs to have an enhanced suppressive effect on Th1 and/or Th17 pathways to reduce the level of INF-gamma and/or IL-17 produced. Additionally, C1ORF32 fusion proteins can enhance the production of IL-10 which inhibits the activity of Th1 and/or Th17 cells.

### Inhibition of Th1 Responses

### a. Inhibition of Th1 Development

One method for inhibiting or reducing inflammation includes administering an effective amount of a C1ORF32 fusion protein, to inhibit Th1 development in a subject in need thereof. Inflammation can be inhibited or reduced by blocking naive T cells from differentiating into Th1 cells by administering C1ORF32 fusion proteins. In one embodiment, the C1ORF32 fusion protein may inhibit or reduce proliferation of Th1 cells. C1ORF32 fusion proteins may also reduce naive T cells from differentiating into Th1 cells, by blocking antigen presenting cell maturation. Alternatively, C1ORF32 fusion proteins increase the differentiation of Th2 cells and thereby reduce the number of Th1 cells in a subject. By restricting the number of Th1 cells that can develop in the subject, the amount of proinflammatory molecules such as INF-gamma can be reduced or contained. INF-gamma stimulates the production or release of other proinflammatory molecules including IL-1beta, TNF-alpha, and MMPs. Thus, by controlling the number of Th1 cells in a subject, the levels of these other proinflammatory molecules can be controlled, thereby reducing inflammatory responses.

### b. Inhibition of Proinflammatory molecules

Another disclosure provides a method of inhibiting or reducing inflammation in a subject by administering to the subject an effective amount of a C10RF32 polypeptide, fusion protein thereof, or fragment thereof to inhibit or reduce production of proinflammatory molecules by Th1 cells.

Exemplary proinflammatory molecules produced by Th1 cells includes IFN-gamma. In this disclosure the C10RF32 polypeptide, fusion protein thereof, or fragment thereof can interact directly with the Th1 cell and inhibit or reduce IFN-gamma production by the Th1 cells. In this disclosure, the amount of proinflammatory molecules is regulated rather than the population of Th1 cells.

### Inhibition of Thl7 Responses

### a. Inhibition of Thl7 Development

Inflammation can also be inhibited or reduced in a subject by administering an effective amount of a C1ORF32 fusion, to inhibit or block naive T cells from developing into Th17 cells. In one embodiment, the C1OR-F32 fusion protein increases the suppressive activity of Tregs on the differentiation of naive T cells into Th17 cells by an amount sufficient to reduce the number of Th17 cells in a subject. Alternatively, the C1OR-F32 fusion protein inhibits or reduces proliferation of Th17 cells. C1OR-F32 fusion proteins may also reduce naive T cells from differentiating into Th17 cells, by blocking antigen presenting cell maturation. By reducing the population of Th17 cells in a subject, the amount of IL-17 can be reduced, as well as IL-22 and IL-21. IL-17 is a proinflammatory cytokine that causes increases in other proinflammatory molecules such as IL-1beta, TNF-alpha, and MMPs. Thus, by reducing the amount of IL- 17 these other proinflammatory molecules can be reduced, thereby reducing or inhibiting inflammation.

### b. Inhibition of IL-17 Production

Still another disclosure provides a method for treating inflammation in a subject by administering an effective amount of C1OR-F32 polypeptide, fusion protein thereof, or fragments thereof, to inhibit production of IL-17 by Th17 cells, as well as IL-22 and IL-21. In this embodiment, the C1ORF32 polypeptide or fusion protein can act directly on Th17 cells, for example by binding to Th17 cells resulting in inhibition of IL-17 (or IL-22 and IL-21) production by those Th17 cells. As noted above, inhibition or reduction of IL-17 (and IL-22 or IL-21) leads to the reduction of other proinflammatory molecules, thereby reducing or inhibitng inflammation.

### Inhibiting Th1 and Th17 Responses

The disclosed C1OR-F32 fusion proteins can be used to inhibit both the Th1 and Th17 pathways simultaneously. Using one anti-inflammatory agent to inhibit two separate pathways provides more robust inhibition or reduction of the immune response.

### Promoting Th2 Responses and IL-10 production.

Inflammation can also be treated by administering C1OR-F32 fusion proteins to a subject in an amount effective to enhance Th2 responses, and the suppressive activity of IL-10 producing cells, and to enhance suppressive or modulatory activity on the Th1 and/or Th17 pathways. In this disclosure the disclosed C10RF32 fusion proteins cause an increased suppressive effect on IFN-gamma and/or IL-17 production. Another disclosure provides a method for treating inflammation by administering an effective amount of C10RF32 polypeptide, fusion proteins thereof, or fragments thereof to increase production of IL-10 by Th2, Tregs or other immune cells.

Increased production of IL-10 results in the decreased production of IL-17 by Th17 cells and deceased production of IFN-gamma by Th1 cells. In this disclosure, the C10RF32 polypeptides, fusion proteins, and fragments thereof can interact directly with with immune cells to increase IL-10 production.

Still another disclosure provides a method for treating inflammation by administering an effective amount of C10RF32 polypeptides, fusion proteins thereof, and fragments thereof to inhibit or interfere with the Th1 pathway and Th17 pathway, and to enhance the suppressive effect on the Th1 and/or Th17 pathways by Th2 cells.

The C1OR-F32 fusion proteins can also be administered to a subject in an amount effective to increase Th2 cell populations or numbers.

IL-10 production can be increased relative to a control by contacting Th2 cells, Tregs or other immune cells with an effective amount of C1ORF32 fusion proteins. The increase can occur in vitro or in vivo,

### Inflammatory Disease to Be Treated

Immune related diseases and disorders that may be treated using C10RF32 fusion polypeptides are described herein.

C1OR-F32 acts at multiple points in the inflammatory pathway as a master regulator to control the expression and/or activity of effectory cytokines such as IFN-gamma and TNF-alpha. Therefore, the C1OR-F32 compositions described herein are particularly useful for treating patients that do not respond to TNF-alpha blockers such as Enbrel, Remicade, Cimzia and Humira, or where TNF-alpha blockers are not safe or effective. In addition, because of its activity as a master regulator in the inflammatory pathway, the C10RF32 compositions disclosed are particularly useful for treating chronic and persistent inflammation. In a further embodiment, the C1OR-F32 compositions described herein are used to treat relapsing and/or remitting multiple sclerosis.

### Inhibition of Epitope Spreading

Epitope spreading refers to the ability of B and T cell immune response to diversify both at the level of specificity, from a single determinant to many sites on an auto antigen, and at the level of V gene usage (Monneaux, F. et al., Arthritis & Rheumatism, 46(6): 1430-1438 (2002). Epitope spreading is not restricted to systemic autoimmune disease. It has been described in T cell dependent organ specific diseases such as Diabetes mellitus type 1 and multiple sclerosis in humans, and EAE induced experimental animals with a variety of myelin proteins.

Epitope spreading involves the acquired recognition of new epitopes in the same self molecule as well as epitopes residing in proteins that are associated in the same macromolecular complex. Epitope spreading can be assessed by measuring delayed-type hypersensitivity (DTH) responses, methods of which are known in the art.

One disclosure provides a method for inhibiting or reducing epitope spreading in a subject by administering to the subject an effective amount of C1ORF32 polypeptide, fragment or fusion protein thereof. In a further disclosure the C1ORF32 polypeptide, fragment or fusion protein thereof inhibits epitope spreading in individuals with multiple sclerosis. Preferably, the C1ORF32 polypeptide or fusion thereof inhibits or blocks multiple points of the inflammation pathway.

Yet another disclosure provides a method for inhibiting or reducing epitope spreading in subjects with multiple sclerosis by administering to a subject an effective amount of C10RF32 polypeptide, fragment or fusion protein thereof to inhibit or reduce differentiation of, proliferation of, activity of, and/or cytokine production and/or secretion by Th1, Th17, Th22, and/or other cells that secrete, or cause other cells to secrete, inflammatory molecules, including, but not limited to, IL-1beta, TNF-alpha, TGF-beta, IFN-gamma, IL- 17, IL-6, IL-23, IL-22, IL-21, and MMPs. Another embodiment provides a method for treating multiple sclerosis by administering to a subject an effective amount of C1ORF32 fusion protein to interact with Tregs, enhance Treg activity, promote or enhances IL-10 secretion by Tregs, increase the number of Tregs, increase the suppressive capacity of Tregs, or combinations thereof. Another disclosure provides a method for treating multiple sclerosis by administering to a subject an effective amount of C1ORF32 polypeptide, fragment or fusion protein thereof to interact with Th2 cells, enhance Th2 activity, promote or enhance IL-10 secretion by Th2 cells, increase the number of Th2 cells, increase the modulatory capacity of Th2 cells, or combinations thereof.

### Induction of immune tolerance

In one disclosure, the present disclosure provides a method for inducing or re-establishing immune tolerance in a subject by administering to the subject an effective amount of C10RF32 fusion protein. According to the present invention the C10RF32 fusion protein induces tolerance in individuals with immune related diseases. In a specific embodiment the C10RF32 fusion protein induces tolerance in individuals with multiple sclerosis. Preferably, the C10RF32 fusion inhibits or blocks multiple points of the inflammation pathway. In another specific embodiment, the C10RF32 fusion protein induces tolerance in individuals with rheumatoid arthritis. Another disclosure provides a method for treating immune related diseases by administering to a subject an effective amount of C10RF32 polypeptide, fragment or fusion protein thereof to induce immune tolerance by interacting with Tregs, enhancing Treg activity, increasing the number of Tregs, increase the suppressive capacity of Tregs, or combinations thereof. Another disclosure provides a method for treating immune related diseases by administering to a subject an effective amount of C10RF32 polypeptide, fragment or fusion protein thereof to promote or enhance IL-10 secretion by immune cells.

### Combination therapy

C1ORF32 fusion polypeptides can be used alone or in combination with additional therapeutic agents. The additional therapeutic agents include, but are not limited to, immunosuppressive agents (e.g., antibodies against other lymphocyte surface markers (e.g., CD40, alpha-4 integrin) or against cytokines), other fusion proteins (e.g., CTLA-4-Ig (Orencia^{®}), TNFR-Ig (Enbrel^{®})), TNF-alpha blockers such as Enbrel, Remicade, Cimzia and Humira, cyclophosphamide (CTX) (i.e. Endoxan^{®}, Cytoxan^{®}, Neosar^{®}, Procytox^{®}, Revimmune^{™}), methotrexate (MTX) (i.e. Rheumatrex^{®}, Trexall^{®}), belimumab (i.e. Benlysta^{®}), or other immunosuppressive drugs (e.g., cyclosporin A, FK506-like compounds, rapamycin compounds, or steroids), anti-proliferatives, cytotoxic agents, or other compounds that may assist in immunosuppression.

In a further embodiment, the additional therapeutic agent functions to inhibit or reduce T cell activation through a separate pathway. In one such embodiment, the additional therapeutic agent is a CTLA-4 fusion protein, such as CTLA-4-Ig (abatacept). CTLA-4-Ig fusion proteins compete with the co-stimulatory receptor, CD28, on T cells for binding to CD80/CD86 (B7-1/B7-2) on antigen presenting cells, and thus function to inhibit T cell activation. In another embodiment, the additional therapeutic agent is a CTLA-4-Ig fusion protein known as belatacept. Belatacept contains two amino acid substitutions (L104E and A29Y) that markedly increase its avidity to CD86 in vivo. In another embodiment, the additional therapeutic agent is Maxy-4.

In another embodiment, the second therapeutic agent is cyclophosphamide (CTX). Cyclophosphamide (the generic name for Endoxan^{®}, Cytoxan^{®}, Neosar^{®}, Procytox^{®}, Revimmune^{™}), also known as cytophosphane, is a nitrogen mustard alkylating agent from the oxazophorines group. It is used to treat various types of cancer and some autoimmune disorders. In a further embodiment, C1ORF32 polypeptides, fragments or fusion proteins thereof and CTX are coadministered in effective amount to prevent or treat a chronic autoimmune disease or disorder such as Systemic lupus erythematosus (SLE). Cyclophosphamide (CTX) is the primary drug used for diffuse proliferative glomerulonephritis in patients with renal lupus. In some embodiments the combination therapy is administered in an effective amount to reduce the blood or serum levels of antidouble stranded DNA (anti-ds DNA) auto antibodies and/or to reduce proteinuria in a patient in need thereof.

In another embodiment, the second therapeutic agent increases the amount of adenosine in the serum, see, for example, WO 08/147482. In a further embodiment, the second therapeutic is CD73-Ig, recombinant CD73, or another agent (e.g. a cytokine or monoclonal antibody or small moelcule) that increases the expression of CD73, see for example WO 04/084933. In another embodiment the second therapeutic agent is Interferon-beta.

In another embodiment, the second therapeutic is Tysabri or another therapeutic for MS. In a further embodiment, C1ORF32 polypeptides, fragments or fusion proteins thereof is cycled with Tysabri or used during a drug holiday in order to allow less frequent dosing with the second therapeutic and reduce the risk of side effects such as PML and to prevent resistance to the second therapeutic.

In another embodiment, the second therapeutic agent preferentially treats chronic inflammation, whereby the treatment regimen targets both acute and chronic inflammation. In a further embodiment the second therapeutic is a TNF-alpha blocker.

In another embodiment, the second therapeutic agent is a small molecule that inhibits or reduces differentiation, proliferation, activity, and/or cytokine production and/or secretion by Th1, Th17, Th22, and/or other cells that secrete, or cause other cells to secrete, inflammatory molecules, including, but not limited to, IL-1beta, TNF-alpha, TGF-beta, IFN-gamma, IL- 17, IL-6, IL-23, IL-22, IL-21, and MMPs. In another embodiment, the second therapeutic agent is a small molecule that interacts with Tregs, enhances Treg activity, promotes or enhances IL-10 secretion by Tregs, increases the number of Tregs, increases the suppressive capacity of Tregs, or combinations thereof.

Typically useful small molecules are organic molecules, preferably small organic compounds having a molecular weight of more than 100 and less than 2,500 daltons, more preferably between 100 and 2000, more preferably between 100 and 1250, more preferably between 100 and 1000, more preferably between 100 and 750, more preferably between 200 and 500 daltons. Small molecules comprise functional groups necessary for structural interaction with proteins, particularly hydrogen bonding, and typically include at least an amine, carbonyl, hydroxyl or carboxyl group, preferably at least two of the functional chemical groups. The small molecules often comprise cyclical carbon or heterocyclic structures andlor aromatic or polyaromatic structures substituted with one or more of the above functional groups. Small molecules also include biomolecules including peptides, saccharides, fatty acids, steroids, purines, pyrimidines, derivatives, structural analogs or combinations thereof. In one embodiment, the small molecule is retinoic acid or a derivative thereof. The examples below demonstrate that retinoic acid inhibits or reduces differentiation and/or activity of ThI 7 cells. In a further embodiment, the compositions are used in combination or succession with compounds that increase Treg activity or production. Exemplary Treg enhancing agents include but are not limited to glucocorticoid fluticasone, salmeteroal, antibodies to IL- 12, IFN-gamma, and IL-4; vitamin D3, and dexamethasone, and combinations thereof. Antibodies to other proinflammatory molecules can also be used in combination or alternation with the disclosed C10RF32 polypeptides, fusion proteins, or fragments thereof. Preferred antibodies bind to IL-6, IL-23, IL-22 or IL-21.

As used herein the term "rapamycin compound" includes the neutral tricyclic compound rapamycin, rapamycin derivatives, rapamycin analogs, and other macrolide compounds which are thought to have the same mechanism of action as rapamycin (e.g., inhibition of cytokine function). The language "rapamycin compounds" includes compounds with structural similarity to rapamycin, e.g., compounds with a similar macrocyclic structure, which have been modified to enhance their therapeutic effectiveness. Exemplary Rapamycin compounds are known in the art. The language "FK506-Hke compounds" includes FK506, and FK506 derivatives and analogs, e.g., compounds with structural similarity to FK506, e.g., compounds with a similar macrocyclic structure which have been modified to enhance their therapeutic effectiveness. Examples of FK506-like compounds include, for example, those described in WO 00101385. Preferably, the language "rapamycin compound" as used herein does not include FK506-like compounds.

Other suitable therapeutics include, but are not limited to, anti-inflammatory agents. The anti-inflammatory agent can be non-steroidal, steroidal, or a combination thereof. One embodiment provides oral compositions containing about 1% (w/w) to about 5% (w/w), typically about 2.5 % (w/w) or an anti-inflammatory agent. Representative examples of non-steroidal anti-inflammatory agents include, without limitation, oxicams, such as piroxicam, isoxicam, tenoxicam, sudoxicam; salicylates, such as aspirin, disalcid, benorylate, trilisate, safapryn, solprin, diflunisal, and fendosal; acetic acid derivatives, such as diclofenac, fenclofenac, indomethacin, sulindac, tolmetin, isoxepac, furofenac, tiopinac, zidometacin, acematacin, fentiazac, zomepirac, clmdanac, oxepinac, felbmac, and ketorolac; fenamates, such as mefenamic, meclofenamic, flufenamic, niflumic, and tolfenamic acids; propionic acid derivatives, such as ibuprofen, naproxen, benoxaprofen, flurbiprofen, ketoprofen, fenoprofen, fenbufen, indopropfen, pirprofen, carprofen, oxaprozin, pranoprofen, miroprofen, tioxaprofen, suprofen, alminoprofen, and tiaprofenic; pyrazoles, such as phenylbutazone, oxyphenbutazone, feprazone, azapropazone, and trimethazone. Mixtures of these non-steroidal anti-inflammatory agents may also be employed. Representative examples of steroidal anti-inflammatory drugs include, without limitation, corticosteroids such as hydrocortisone, hydroxyl- triamcinolone, alpha-methyl dexamethasone, dexamethasone-phosphate, beclomethasone dipropionates, clobetasol valerate, desonide, desoxymethasone, desoxycorticosterone acetate, dexamethasone, dichlorisone, diflorasone diacetate, diflucortolone valerate, fluadrenolone, fluclorolone acetonide, fludrocortisone, flumethasone pivalate, fiuosinolone acetonide, fluocinonide, flucortine butylesters, fluocortolone, fluprednidene (fluprednylidene) acetate, flurandrenolone, halcinonide, hydrocortisone acetate, hydrocortisone butyrate, methylprednisolone, triamcinolone acetonide, cortisone, cortodoxone, flucetonide, fludrocortisone, difluorosone diacetate, fluradrenolone, fludrocortisone, diflurosone diacetate, fluradrenolone acetonide, medrysone, amcinafel, amcinafide, betamethasone and the balance of its esters, chloroprednisone, chlorprednisone acetate, clocortelone, clescinolone, dichlorisone, diflurprednate, flucloronide, flunisolide, fluoromethalone, fluperolone, fluprednisolone, hydrocortisone valerate, hydrocortisone cyclopentylpropionate, hydrocortamate, meprednisone, paramethasone, prednisolones prednisone, beclomethasone dipropionate, triamcinolone, and mixtures thereof.

### PHARMACEUTICAL COMPOSITIONS

The present invention, in some embodiments, features a pharmaceutical composition for use in the treatment of an immune related disorder, wherein the immune related disorder is selected from multiple sclerosis, rheumatoid arthritis, type I diabetes, psoriasis, systemic lupus erythematosus, inflammatory bowel disease, uveitis, or Sjogren's syndrome, the pharmaceutical composition comprising the fusion protein and a pharmaceutically acceptable diluent or carrier.

As used herein, "pharmaceutically acceptable carrier" includes any and all solvents, dispersion media, coatings, antibacterial and antifungal agents, isotonic and absorption delaying agents, and the like that are physiologically compatible. Preferably, the carrier is suitable for intravenous, intramuscular, subcutaneous, parenteral, spinal or epidermal administration (e.g., by injection or infusion).

A pharmaceutical composition for use according to at least some embodiments of the present invention also may include a pharmaceutically acceptable anti-oxidants. Examples of pharmaceutically acceptable antioxidants include: (1) water soluble antioxidants, such as ascorbic acid, cysteine hydrochloride, sodium bisulfate, sodium metabisulfite, sodium sulfite and the like; (2) oil-soluble antioxidants, such as ascorbyl palmitate, butylated hydroxyanisole (BHA), butylated hydroxytoluene (BHT), lecithin, propyl gallate, alpha-tocopherol, and the like; and (3) metal chelating agents, such as citric acid, ethylenediamine tetraacetic acid (EDTA), sorbitol, tartaric acid, phosphoric acid, and the like. A pharmaceutical composition for use according to at least some embodiments of the present invention also may include additives such as detergents and solubilizing agents (e.g., TWEEN 20 (polysorbate-20), TWEEN 80 (polysorbate-80)) and preservatives (e.g., Thimersol, benzyl alcohol) and bulking substances (e.g., lactose, mannitol).

Examples of suitable aqueous and nonaqueous carriers that may be employed in the pharmaceutical compositions for use according to at least some embodiments of the present invention include water, buffered saline of various buffer content (e.g., Tris-HCl, acetate, phosphate), pH and ionic strength, ethanol, polyols (such as glycerol, propylene glycol, polyethylene glycol, and the like), and suitable mixtures thereof, vegetable oils, such as olive oil, and injectable organic esters, such as ethyl oleate.

Proper fluidity can be maintained, for example, by the use of coating materials, such as lecithin, by the maintenance of the required particle size in the case of dispersions, and by the use of surfactants.

These compositions may also contain adjuvants such as preservatives, wetting agents, emulsifying agents and dispersing agents. Prevention of presence of microorganisms may be ensured both by sterilization procedures, supra, and by the inclusion of various antibacterial and antifungal agents, for example, paraben, chlorobutanol, phenol sorbic acid, and the like. It may also be desirable to include isotonic agents, such as sugars, sodium chloride, and the like into the compositions. In addition, prolonged absorption of the injectable pharmaceutical form may be brought about by the inclusion of agents which delay absorption such as aluminum monostearate and gelatin.

Pharmaceutically acceptable carriers include sterile aqueous solutions or dispersions and sterile powders for the extemporaneous preparation of sterile injectable solutions or dispersion. The use of such media and agents for pharmaceutically active substances is known in the art. Supplementary active compounds can also be incorporated into the compositions.

Therapeutic compositions typically must be sterile and stable under the conditions of manufacture and storage. The composition can be formulated as a solution, microemulsion, liposome, or other ordered structure suitable to high drug concentration. The carrier can be a solvent or dispersion medium containing, for example, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. In many cases, it will be preferable to include isotonic agents, for example, sugars, polyalcohols such as mannitol, sorbitol, or sodium chloride in the composition. Prolonged absorption of the injectable compositions can be brought about by including in the composition an agent that delays absorption, for example, monostearate salts and gelatin. Sterile injectable solutions can be prepared by incorporating the active compound in the required amount in an appropriate solvent with one or a combination of ingredients enumerated above, as required, followed by sterilization microfiltration. Generally, dispersions are prepared by incorporating the active compound into a sterile vehicle that contains a basic dispersion medium and the required other ingredients from those enumerated above. In the case of sterile powders for the preparation of sterile injectable solutions, the preferred methods of preparation are vacuum drying and freeze-drying (lyophilization) that yield a powder of the active ingredient plus any additional desired ingredient from a previously sterile-filtered solution thereof.

Sterile injectable solutions can be prepared by incorporating the active compound in the required amount in an appropriate solvent with one or a combination of ingredients enumerated above, as required, followed by sterilization microfiltration. Generally, dispersions are prepared by incorporating the active compound into a sterile vehicle that contains a basic dispersion medium and the required other ingredients from those enumerated above. In the case of sterile powders for the preparation of sterile injectable solutions, the preferred methods of preparation are vacuum drying and freeze-drying (lyophilization) that yield a powder of the active ingredient plus any additional desired ingredient from a previously sterile-filtered solution thereof.

The amount of active ingredient which can be combined with a carrier material to produce a single dosage form will vary depending upon the subject being treated, and the particular mode of administration. The amount of active ingredient which can be combined with a carrier material to produce a single dosage form will generally be that amount of the composition which produces a therapeutic effect. Generally, out of one hundred per cent, this amount will range from 0.01 per cent to ninety-nine percent of active ingredient, preferably from 0.1 per cent to 70 per cent, most preferably from 1 per cent to 30 per cent of active ingredient in combination with a pharmaceutically acceptable carrier.

Dosage regimens are adjusted to provide the optimum desired response (e.g., a therapeutic response). For example, a single bolus may be administered, several divided doses may be administered over time or the dose may be proportionally reduced or increased as indicated by the exigencies of the therapeutic situation. It is especially advantageous to formulate parenteral compositions in dosage unit form for ease of administration and uniformity of dosage. Dosage unit form as used herein refers to physically discrete units suited as unitary dosages for the subjects to be treated; each unit contains a predetermined quantity of active compound calculated to produce the desired therapeutic effect in association with the required pharmaceutical carrier.

A composition for use of the present invention can be administered via one or more routes of administration using one or more of a variety of methods known in the art. As will be appreciated by the skilled artisan, the route and/or mode of administration will vary depending upon the desired results. Preferred routes of administration include intravascular delivery (e.g. injection or infusion), intravenous, intramuscular, intradermal, intraperitoneal, subcutaneous, spinal, oral, enteral, rectal, pulmonary (e.g. inhalation), nasal, topical (including transdermal, buccal and sublingual), intravesical, intravitreal, intraperitoneal, vaginal, brain delivery (e.g. intra-cerebroventricular, intra-cerebral, and convection enhanced diffusion), CNS delivery (e.g. intrathecal, perispinal, and intra-spinal) or parenteral (including subcutaneous, intramuscular, intraperitoneal, intravenous (IV) and intradermal), transdermal (either passively or using iontophoresis or electroporation), transmucosal (e.g., sublingual administration, nasal, vaginal, rectal, or sublingual), administration or administration via an implant, or other parenteral routes of administration, for example by injection or infusion, or other delivery routes and/or forms of administration known in the art. The phrase "parenteral administration" as used herein means modes of administration other than enteral and topical administration, usually by injection, and includes, without limitation, intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal, epidural and intrasternal injection and infusion or using bioerodible inserts, and can be formulated in dosage forms appropriate for each route of administration. In a specific embodiment, a fusion protein, or a pharmaceutical composition for use according to at least some embodiments of the present invention can be administered intraperitoneally or intravenously.

Compositions for use of the present invention can be delivered to the lungs while inhaling and traverse across the lung epithelial lining to the blood stream when delivered either as an aerosol or spray dried particles having an aerodynamic diameter of less than about 5 microns. A wide range of mechanical devices designed for pulmonary delivery of therapeutic products can be used, including but not limited to nebulizers, metered dose inhalers, and powder inhalers, all of which are familiar to those skilled in the art. Some specific examples of commercially available devices are the Ultravent nebulizer (Mallinckrodt Inc., St. Louis, Mo.); the Acorn II nebulizer (Marquest Medical Products, Englewood, Colo.); the Ventolin metered dose inhaler (Glaxo Inc., Research Triangle Park, N.C.); and the Spinhaler powder inhaler (Fisons Corp., Bedford, Mass.). Nektar, Alkermes and Mannkind all have inhalable insulin powder preparations approved or in clinical trials where the technology could be applied to the formulations described herein.

In some in vivo approaches, the compositions disclosed herein are administered to a subject in a therapeutically effective amount. As used herein the term "effective amount" or "therapeutically effective amount" means a dosage sufficient to treat, inhibit, or alleviate one or more symptoms of the disorder being treated or to otherwise provide a desired pharmacologic and/or physiologic effect. The precise dosage will vary according to a variety of factors such as subject-dependent variables (e.g., age, immune system health, etc.), the disease, and the treatment being effected. For the polypeptide compositions disclosed herein and nucleic acids encoding the same, as further studies are conducted, information will emerge regarding appropriate dosage levels for treatment of various conditions in various patients, and the ordinary skilled worker, considering the therapeutic context, age, and general health of the recipient, will be able to ascertain proper dosing. The selected dosage depends upon the desired therapeutic effect, on the route of administration, and on the duration of the treatment desired. For polypeptide compositions, generally dosage levels of 0.0001 to 100 mg/kg of body weight daily are administered to mammals and more usually 0.001 to 20 mg/kg. For example dosages can be 0.3 mg/kg body weight, 1 mg/kg body weight, 3 mg/kg body weight, 5 mg/kg body weight or 10 mg/kg body weight or within the range of 1-10 mg/kg. An exemplary treatment regime entails administration once per week, once every two weeks, once every three weeks, once every four weeks, once a month, once every 3 months or once every three to 6 months. Generally, for intravenous injection or infusion, dosage may be lower. Dosage regimens are adjusted to provide the optimum desired response (e.g., a therapeutic response). For example, a single bolus may be administered, several divided doses may be administered over time or the dose may be proportionally reduced or increased as indicated by the exigencies of the therapeutic situation. It is especially advantageous to formulate parenteral compositions in dosage unit form for ease of administration and uniformity of dosage. Dosage unit form as used herein refers to physically discrete units suited as unitary dosages for the subjects to be treated; each unit contains a predetermined quantity of active compound calculated to produce the desired therapeutic effect in association with the required pharmaceutical carrier..

Optionally the polypeptide formulation may be administered in an amount between 0.0001 to 100 mg/kg weight of the patient/day, preferably between 0.001 to 20.0 mg/kg/day, according to any suitable timing regimen. A pharamaceutical composition for use according to at least some embodiments of the present invention can be administered, for example, three times a day, twice a day, once a day, three times weekly, twice weekly or once weekly, once every two weeks or 3, 4, 5, 6, 7 or 8 weeks. Moreover, the composition can be administered over a short or long period of time (e.g., 1 week, 1 month, 1 year, 5 years).

Alternatively, therapeutic agent can be administered as a sustained release formulation, in which case less frequent administration is required. Dosage and frequency vary depending on the half-life of the therapeutic agent in the patient. In general, human antibodies show the longest half life, followed by humanized antibodies, chimeric antibodies, and nonhuman antibodies. The half-life for fusion proteins may vary widely. The dosage and frequency of administration can vary depending on whether the treatment is prophylactic or therapeutic. In prophylactic applications, a relatively low dosage is administered at relatively infrequent intervals over a long period of time. Some patients continue to receive treatment for the rest of their lives. In therapeutic applications, a relatively high dosage at relatively short intervals is sometimes required until progression of the disease is reduced or terminated, and preferably until the patient shows partial or complete amelioration of symptoms of disease. Thereafter, the patient can be administered a prophylactic regime.

Actual dosage levels of the active ingredients in the pharmaceutical compositions for use of the present invention may be varied so as to obtain an amount of the active ingredient which is effective to achieve the desired therapeutic response for a particular patient, composition, and mode of administration, without being toxic to the patient. The selected dosage level will depend upon a variety of pharmacokinetic factors including the activity of the particular compositions for use of the present invention employed, the route of administration, the time of administration, the rate of excretion of the particular compound being employed, the duration of the treatment, other drugs, compounds and/or materials used in combination with the particular compositions employed, the age, sex, weight, condition, general health and prior medical history of the patient being treated, and like factors well known in the medical arts.

A "therapeutically effective dosage" of C1ORF32 soluble protein or C1ORF32 ectodomain or C1ORF32 fusion protein containing same, preferably results in a decrease in severity of disease symptoms, an increase in frequency and duration of disease symptom-free periods, an increase in lifepan, disease remission, or a prevention or reduction of impairment or disability due to the disease affliction.

One of ordinary skill in the art would be able to determine a therapeutically effective amount based on such factors as the subject's size, the severity of the subject's symptoms, and the particular composition or route of administration selected.

In certain embodiments, the polypeptide compositions are administered locally, for example by injection directly into a site to be treated. Typically, the injection causes an increased localized concentration of the polypeptide compositions which is greater than that which can be achieved by systemic administration. For example, in the case of a neurological disorder like Multiple Sclerosis, the protein may be administered locally to a site near the CNS. In another example, as in the case of an arthritic disorder like Rheumatoid Arthritis, the protein may be administered locally to the synovium in the affected joint. The polypeptide compositions can be combined with a matrix as described above to assist in creating a increased localized concentration of the polypeptide compositions by reducing the passive diffusion of the polypeptides out of the site to be treated.

The active compounds can be prepared with carriers that will protect the compound against rapid release, such as a controlled release formulation, including implants, transdermal patches, and microencapsulated delivery systems. Biodegradable, biocompatible polymers can be used, such as ethylene vinyl acetate, polyanhydrides, polyglycolic acid, collagen, polyorthoesters, and polylactic acid. Many methods for the preparation of such formulations are patented or generally known to those skilled in the art. See, e.g., Sustained and Controlled Release Drug Delivery Systems, J. R. Robinson, ed., Marcel Dekker, Inc., New York, 1978.

In certain embodiments, C1ORF32 fusion proteins for use according to at least some embodiments of the present invention can be formulated to ensure proper distribution in vivo. For example, the blood-brain barrier (BBB) excludes many highly hydrophilic compounds. To ensure that the fusion proteins for use according to at least some embodiments of the present invention cross the BBB (if desired), they can be formulated, for example, in liposomes. For methods of manufacturing liposomes, see, e.g., U.S. Pat. Nos. 4,522,811; 5,374,548; and 5,399,331. The liposomes may comprise one or more moieties which are selectively transported into specific cells or organs, thus enhance targeted drug delivery (see, e.g., V. V. Ranade (1989) J. Clin. Pharmacol. 29:685). Exemplary targeting moieties include folate or biotin (see, e.g., U.S. Pat. No. 5,416,016 to Low et al.); mannosides (Umezawa et al., (1988) Biochem. Biophys. Res. Commun. 153:1038); antibodies (P. G. Bloeman et al. (1995) FEBS Lett. 357:140; M. Owais et al. (1995) Antimicrob. Agents Chemother. 39:180); surfactant protein A receptor (Briscoe et al. (1995) Am. J Physiol. 1233:134); p120 (Schreier et al. (1994) J. Biol. Chem. 269:9090); see also K. Keinanen; M. L. Laukkanen (1994) FEBS Lett. 346:123; J. J. Killion; I. J. Fidler (1994) Immunomethods 4:273.

### Formulations for parenteral administration

In a further embodiment, compositions disclosed herein, including those containing peptides and polypeptides, are administered in an aqueous solution, by parenteral injection. The formulation may also be in the form of a suspension or emulsion. In general, pharmaceutical compositions are provided including effective amounts of a peptide or polypeptide, and optionally include pharmaceutically acceptable diluents, preservatives, solubilizers, emulsifiers, adjuvants and/or carriers. Such compositions optionally include one or more for the following: diluents, sterile water, buffered saline of various buffer content (e.g., Tris-HCl, acetate, phosphate), pH and ionic strength; and additives such as detergents and solubilizing agents (e.g., TWEEN 20 (polysorbate-20), TWEEN 80 (polysorbate-80)), anti-oxidants (e.g., water soluble antioxidants such as ascorbic acid, sodium metabisulfite, cysteine hydrochloride, sodium bisulfate, sodium metabisulfite, sodium sulfite; oil-soluble antioxidants, such as ascorbyl palmitate, butylated hydroxyanisole (BHA), butylated hydroxytoluene (BHT), lecithin, propyl gallate, alpha-tocopherol; and metal chelating agents, such as citric acid, ethylenediamine tetraacetic acid (EDTA), sorbitol, tartaric acid, phosphoric acid), and preservatives (e.g., Thimersol, benzyl alcohol) and bulking substances (e.g., lactose, mannitol). Examples of non-aqueous solvents or vehicles are ethanol, propylene glycol, polyethylene glycol, vegetable oils, such as olive oil and corn oil, gelatin, and injectable organic esters such as ethyl oleate. The formulations may be freeze dried (lyophilized) or vacuum dried and redissolved/resuspended immediately before use. The formulation may be sterilized by, for example, filtration through a bacteria retaining filter, by incorporating sterilizing agents into the compositions, by irradiating the compositions, or by heating thecompositions.

### Formulations for topical administration

C1ORF32 fusion polypeptides disclosed herein can be applied topically. Topical administration does not work well for most peptide formulations, although it can be effective especially if applied to the lungs, nasal, oral (sublingual, buccal), vaginal, or rectal mucosa.

Compositions can be delivered to the lungs while inhaling and traverse across the lung epithelial lining to the blood stream when delivered either as an aerosol or spray dried particles having an aerodynamic diameter of less than about 5 microns.

A wide range of mechanical devices designed for pulmonary delivery of therapeutic products can be used, including but not limited to nebulizers, metered dose inhalers, and powder inhalers, all of which are familiar to those skilled in the art. Some specific examples of commercially available devices are the Ultravent nebulizer (Mallinckrodt Inc., St. Louis, Mo.); the Acorn II nebulizer (Marquest Medical Products, Englewood, Colo.); the Ventolin metered dose inhaler (Glaxo Inc., Research Triangle Park, N.C.); and the Spinhaler powder inhaler (Fisons Corp., Bedford, Mass.). Nektar, Alkermes and Mannkind all have inhalable insulin powder preparations approved or in clinical trials where the technology could be applied to the formulations described herein.

Formulations for administration to the mucosa will typically be spray dried drug particles, which may be incorporated into a tablet, gel, capsule, suspension or emulsion. Standard pharmaceutical excipients are available from any formulator. Oral formulations may be in the form of chewing gum, gel strips, tablets or lozenges.

Transdermal formulations may also be prepared. These will typically be ointments, lotions, sprays, or patches, all of which can be prepared using standard technology. Transdermal formulations will require the inclusion of penetration enhancers.

### Controlled delivery polymeric matrices

C10RF32 fusion polypeptides disclosed herein may also be administered in controlled release formulations. Controlled release polymeric devices can be made for long term release systemically following implantation of a polymeric device (rod, cylinder, film, disk) or injection (microparticles). The matrix can be in the form of microparticles such as microspheres, where peptides are dispersed within a solid polymeric matrix or microcapsules, where the core is of a different material than the polymeric shell, and the peptide is dispersed or suspended in the core, which may be liquid or solid in nature. Unless specifically defined herein, microparticles, microspheres, and microcapsules are used interchangeably. Alternatively, the polymer may be cast as a thin slab or film, ranging from nanometers to four centimeters, a powder produced by grinding or other standard techniques, or even a gel such as a hydrogel.

Either non-biodegradable or biodegradable matrices can be used for delivery of polypeptides or nucleic acids encoding the polypeptides, although biodegradable matrices are preferred. These may be natural or synthetic polymers, although synthetic polymers are preferred due to the better characterization of degradation and release profiles. The polymer is selected based on the period over which release is desired. In some cases linear release may be most useful, although in others a pulse release or "bulk release" may provide more effective results. The polymer may be in the form of a hydrogel (typically in absorbing up to about 90% by weight of water), and can optionally be crosslinked with multivalent ions or polymers.

The matrices can be formed by solvent evaporation, spray drying, solvent extraction and other methods known to those skilled in the art. Bioerodible microspheres can be prepared using any of the methods developed for making microspheres for drug delivery, for example, as described by Mathiowitz and Langer, J. Controlled Release, 5:13-22 (1987); Mathiowitz, et al., Reactive Polymers, 6:275-283 (1987); and Mathiowitz, et al., J. Appl Polymer ScL, 35:755-774 (1988).

The devices can be formulated for local release to treat the area of implantation or injection
- which will typically deliver a dosage that is much less than the dosage for treatment of an entire body - or systemic delivery. These can be implanted or injected subcutaneously, into the muscle, fat, or swallowed.

### EXAMPLES

### EXAMPLE 1

### DESCRIPTION FOR CLUSTER H19011_1 (H19011)

Cluster H19011_1 (internal ID 76432827) features 2 transcripts and 5 segments of interest, the names for which are given in Tables 1 and 2, respectively. The selected protein variants are given in table 3.

**Table 1 - Transcripts of interest**

| Transcript Name |
|---|
| H19011_1_T8 (SEQ ID NO:1) |
| H19011_1_T9 (SEQ ID NO:2) |

**Table 2 - Segments of interest**

| Segment Name |
|---|
| H19011_1_N13 (SEQ ID NO:9) |
| H19011_1_N8 (SEQ ID NO:10) |
| H19011_1_N10 (SEQ ID NO:11) |
| H19011_1_N11 (SEQ ID NO:12) |
| H19011_1_N12 (SEQ ID NO:13) |

**Table 3 - Proteins of interest**

| Protein Name | Corresponding Transcripts |
|---|---|
| H19011_1_P8 (SEQ ID NO:4) | H19011_1_T8 (SEQ ID NO: 1) |
| H19011_1_P9 (SEQ ID NO:6) | H19011_1_T9 (SEQ ID NO:2) |

These sequences are variants of the known protein hypothetical protein LOC387597 (RefSeq accession identifier NP_955383 (SEQ ID NO: 3), synonims: C1ORF32, NP_955383; LISCH-like;RP4-782G3.2; dJ782G3.1; ILDR2), referred to herein as the previously known protein.

C1ORF32 is a hypothetical protein that was computationally discovered during the annotation of chromosome 1 (Gregory SG et al. 2006, Nature 441 (7091) 315-321). Its closest annotated homolog belongs to the LISCH7 family, a subfamily of the immunoglobulin super family. One of the annotated members of this family is the lipolysis-stimulated lipoprotein receptor which has a probable role in the clearance of triglyceride-rich lipoprotein from blood (Swissprot annotation of accession Q86X29). Another homolog of C1ORF32 is the immunoglobulin-like domain containing receptor 1 (ILDR1), which may function as a multimeric receptor at the cell surface (annotation of NCBI gene id 286676).

C1ORF32 was predicted to be a novel member of the B7 family of costimulatory proteins based on the presence of an IgV domain, in its extracellular domain (ECD) in addition of its being a type I membrane protein, like other known B7 members. Also, there are also two alternatively spliced variants (H19011_1_P8 (SEQ ID NO:4) and H19011_1_P9 (SEQ ID NO:6)), which share only the first 5 exons with the known C1ORF32 (NP_955383), and also have an IgV domain, and transmembrane domain.

As noted above, cluster H19011 features 2 transcripts, which were listed in Table 1 above. These transcripts encode for proteins which are variants of protein hypothetical protein LOC387597 (SEQ ID NO:3). A description of each variant protein is now provided.

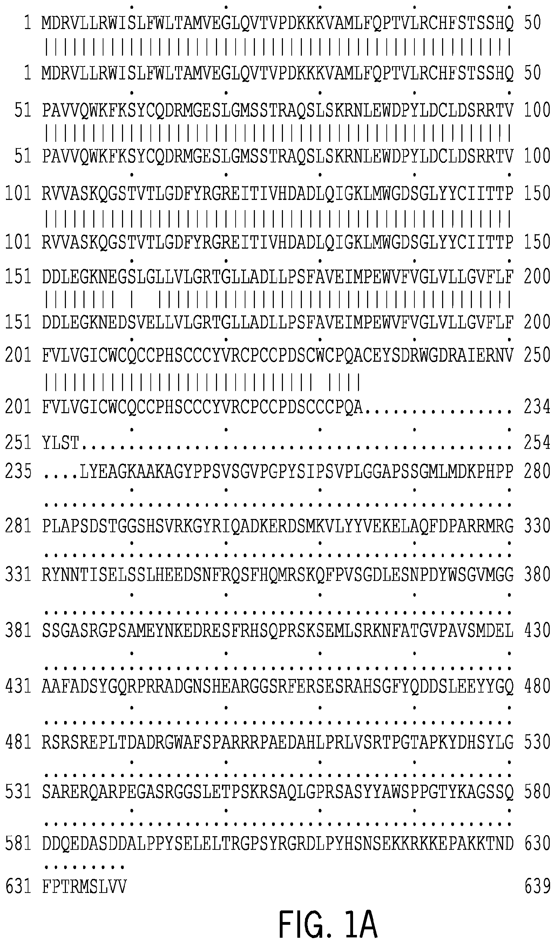

Variant protein H19011_1_P8 (SEQ ID NO:4) has an amino acid sequence as encoded by transcript H19011_1_T8 (SEQ ID NO:1). Alignments to one or more previously published protein sequences are shown in Figure 1A. A brief description of the relationship of the variant protein to each such aligned protein is as follows:
Comparison report between H19011_1_P8 (SEQ ID NO:4) and known proteins Q71H61_HUMAN and NP_955383 (SEQ ID NO: 3) (Figure 1A):
A. An isolated chimeric polypeptide encoding for H19011_1_P8 (SEQ ID NO:4), comprising a first amino acid sequence being at least 90% homologous to MDRVLLRWISLFWLTAMVEGLQVTVPDKKKVAMLFQPTVLRCHFSTSSHQPAVV QWKFKSYCQDRMGESLGMSSTRAQSLSKRNLEWDPYLDCLDSRRTVRVVASKQG STVTLGDFYRGREITIVHDADLQIGKLMWGDSGLYYCIITTPDDLEGKNE corresponding to amino acids 1 - 158 of known proteins Q71H61 HUMAN and NP_955383 (SEQ ID NO: 3), which also corresponds to amino acids 1 - 158 of H19011_1_P8 (SEQ ID NO:4), a bridging amino acid G corresponding to amino acid 159 of H19011_1_P8 (SEQ ID NO:4), a second amino acid sequence being at least 90% homologous to S corresponding to amino acids 160 - 160 of known proteins Q71H61_HUMAN and NP_955383 (SEQ ID NO: 3), which also corresponds to amino acids 160 - 160 of H19011_1_P8 (SEQ ID NO:4), bridging amino acids LG corresponding to amino acid 161 - 162 of H19011_1_P8 (SEQ ID NO:4), a third amino acid sequence being at least 90% homologous to LLVLGRTGLLADLLPSFAVEIMPEWVFVGLVLLGVFLFFVLVGICWCQCCPHSCCC YVRCPCCPDSC corresponding to amino acids 163 - 229 of known proteins Q71H61_HUMAN and NP_955383 (SEQ ID NO: 3), which also corresponds to amino acids 163 - 229 of H19011_1_P8 (SEQ ID NO:4), a bridging amino acid W corresponding to amino acid 230 of H19011_1_P8 (SEQ ID NO:4), a fourth amino acid sequence being at least 90% homologous to CPQA corresponding to amino acids 231 - 234 of known proteins Q71H61_HUMAN and NP_955383 (SEQ ID NO:3), which also corresponds to amino acids 231 - 234 of H19011_1_P8 (SEQ ID NO:4), and a fifth amino acid sequence being at least 70%, optionally at least 80%, preferably at least 85%, more preferably at least 90% and most preferably at least 95, 96, 97, 98 or 99% homologous to a polypeptide having the sequence CEYSDRWGDRAIERNVYLST (SEQ ID NO:18) corresponding to amino acids 235 - 254 of H19011_1_P8 (SEQ ID NO:4), wherein said first amino acid sequence, bridging amino acid, second amino acid sequence, bridging amino acid, third amino acid sequence, bridging amino acid, fourth amino acid sequence and fifth amino acid sequence are contiguous and in a sequential order.
B. An isolated polypeptide encoding for an edge portion of H19011_1_P8 (SEQ ID NO:4), comprising an amino acid sequence being at least 70%, optionally at least about 80%, preferably at least about 85%, more preferably at least about 90% and most preferably at least about 95, 96, 97, 98 or 99% homologous to the sequence CEYSDRWGDRAIERNVYLST (SEQ ID NO:18) of H19011_1_P8 (SEQ ID NO:4).

The localization of the variant protein was determined according to results from a number of different software programs and analyses, including analyses from SignalP and other specialized programs. The variant protein is believed to be located as follows with regard to the cell: membrane.

Variant protein H19011_1_P8 (SEQ ID NO:4) also has the following non-silent SNPs (Single Nucleotide Polymorphisms) as listed in Table 4, (given according to their positions on the amino acid sequence, with the alternative amino acids listed). An example of such a deduced sequence, with alternative amino-acids, that was produced (using part of the SNPs below), is given under the name H19011_1_P8_V1 (SEQ ID NO:5).

**Table 4 - Amino acid mutations**

| SNP positions on amino acid sequence | Alternative amino acids |
|---|---|
| 159 | G->D |
| 161 | L -> V |
| 162 | G -> E |
| 202 | V -> D |
| 202 | V -> G |
| 230 | W -> C |

Variant protein H19011_1_P8 (SEQ ID NO:4) is encoded by the transcript H19011_1_T8 (SEQ ID NO:1), for which the coding portion starts at position 181 and ends at position 942. The transcript also has the following SNPs as listed in Table 5 (given according to their position on the nucleotide sequence, with the alternative nucleic acid listed).

**Table 5 - Nucleic acid SNPs**

| Polymorphism | SNP positions on nucleotide sequence |
|---|---|
| G->A | 656 |
| C->G | 661 |
| G->A | 665 |
| T -> A | 785 |
| T -> G | 785 |
| G -> C | 870 |

The genomic structure of protein H19011_1_P8 (SEQ ID NO:4) (number of exons relevant to the extra-cellular region of the protein, the length of these exons, the frame of the codon in which the introns are inserted and the location of the protein features and domains in the gene structure) is characteristic to the ligands of the B7 /co-stimulatory protein family.

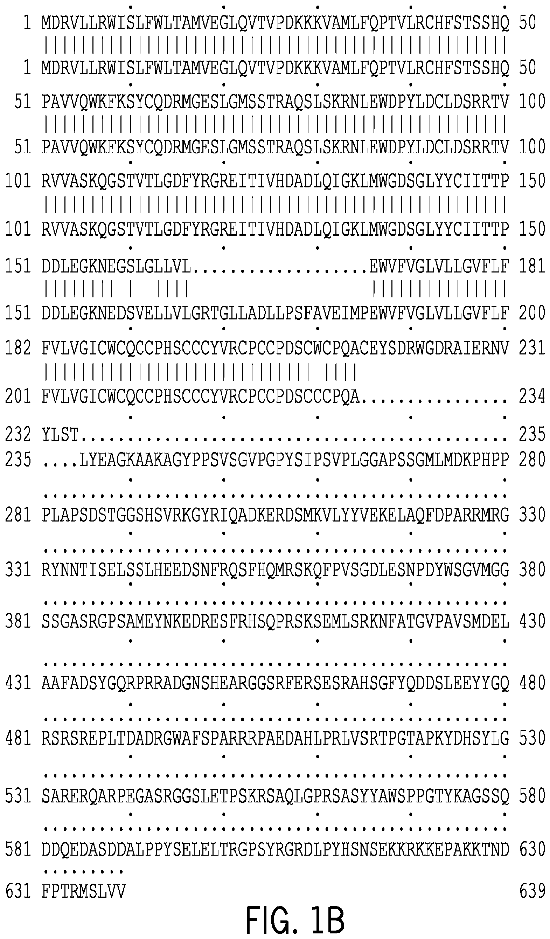

Variant protein H19011_1_P9 (SEQ ID NO:6) has an amino acid sequence as encoded by transcript H19011_1_T9 (SEQ ID NO:2). Alignments to one or more previously published protein sequences are shown in Figure 1B. A brief description of the relationship of the variant protein to each such aligned protein is as follows:
Comparison report between H19011_1_P9 (SEQ ID NO:6) and known proteins Q71H61 HUMAN and NP_955383 (SEQ ID NO:3) (Figure 1B):
A. An isolated chimeric polypeptide encoding for H19011_1_P9 (SEQ ID NO:6), comprising a first amino acid sequence being at least 90% homologous to MDRVLLRWISLFWLTAMVEGLQVTVPDKKKVAMLFQPTVLRCHFSTSSHQPAVV QWKFKSYCQDRMGESLGMSSTRAQSLSKRNLEWDPYLDCLDSRRTVRVVASKQG STVTLGDFYRGREITIVHDADLQIGKLMWGDSGLYYCIITTPDDLEGKNE corresponding to amino acids 1 - 158 of known proteins Q71H61_HUMAN and NP_955383 (SEQ ID NO:3), which also corresponds to amino acids 1 - 158 of H19011_1_P9 (SEQ ID NO:6), a bridging amino acid G corresponding to amino acid 159 of H19011_1_P9 (SEQ ID NO:6), a second amino acid sequence being at least 90% homologous to S corresponding to amino acids 160 - 160 of known proteins Q71H61_HUMAN and NP_955383 (SEQ ID NO:3), which also corresponds to amino acids 160 - 160 of H19011_1_P9 (SEQ ID NO:6), bridging amino acids LG corresponding to amino acid 161 - 162 of H19011_1_P9 (SEQ ID NO:6), a third amino acid sequence being at least 90% homologous to LLVL corresponding to amino acids 163 - 166 of known proteins Q71H61_HUMAN and NP_955383 (SEQ ID NO:3), which also corresponds to amino acids 163 - 166 of H19011_1_P9 (SEQ ID NO:6), a fourth amino acid sequence being at least 90% homologous to EWVFVGLVLLGVFLFFVLVGICWCQCCPHSCCCYVRCPCCPDSC corresponding to amino acids 186 - 229 of known proteins Q71H61_HUMAN and NP_955383 (SEQ ID NO:3), which also corresponds to amino acids 167 - 210 of H19011_1_P9 (SEQ ID NO:6), a bridging amino acid W corresponding to amino acid 211 of H19011_1_P9 (SEQ ID NO:6), a fifth amino acid sequence being at least 90% homologous to CPQA corresponding to amino acids 231 - 234 of known proteins Q71H61_HUMAN and NP_955383 (SEQ ID NO:3), which also corresponds to amino acids 212 - 215 of H19011_1_P9 (SEQ ID NO:6), and a sixth amino acid sequence being at least 70%, optionally at least 80%, preferably at least 85%, more preferably at least 90% and most preferably at least 95, 96, 97, 98 or 99% homologous to a polypeptide having the sequence CEYSDRWGDRAIERNVYLST (SEQ ID NO:18) corresponding to amino acids 216 - 235 of H19011_1P9 (SEQ ID NO:6), wherein said first amino acid sequence, bridging amino acid, second amino acid sequence, bridging amino acid, third amino acid sequence, fourth amino acid sequence, bridging amino acid, fifth amino acid sequence and sixth amino acid sequence are contiguous and in a sequential order.
B. An isolated chimeric polypeptide encoding for an edge portion of H19011_1_P9 (SEQ ID NO:6), comprising a polypeptide having a length "n", wherein n is at least about 10 amino acids in length, optionally at least about 20 amino acids in length, preferably at least about 30 amino acids in length, more preferably at least about 40 amino acids in length and most preferably at least about 50 amino acids in length, wherein at least two amino acids comprise LE, having a structure as follows: a sequence starting from any of amino acid numbers 166-x to 166; and ending at any of amino acid numbers 167 + ((n-2) - x), in which x varies from 0 to n-2.
C. An isolated polypeptide encoding for an edge portion of H19011_1_P9 (SEQ ID NO:6), comprising an amino acid sequence being at least 70%, optionally at least about 80%, preferably at least about 85%, more preferably at least about 90% and most preferably at least about 95, 96, 97, 98 or 99% homologous to the sequence CEYSDRWGDRAIERNVYLST (SEQ ID NO:18) of H19011_1_P9 (SEQ ID NO:6).

The localization of the variant protein was determined according to results from a number of different software programs and analyses, including analyses from SignalP and other specialized programs. The variant protein is believed to be located as follows with regard to the cell: membrane.

Variant protein H19011_1_P9 (SEQ ID NO:6) also has the following non-silent SNPs (Single Nucleotide Polymorphisms) as listed in Table 6, (given according to their positions on the amino acid sequence, with the alternative amino acids listed). An example of such a deduced sequence, with alternative amino-acids, that was produced (using part of the SNPs below), is given under the name H19011_1_P9_V1 (SEQ ID NO:34).

**Table 6 - Amino acid mutations**

| SNP positions on amino acid sequence | Alternative amino acids |
|---|---|
| 159 | G -> D |
| 161 | L -> V |
| 162 | G -> E |
| 183 | V -> D |
| 183 | V -> G |
| 211 | W -> C |

Variant protein H19011_1_P9 (SEQ ID NO:6) is encoded by the transcript H19011_1_T9 (SEQ ID NO:2), for which the coding portion starts at position 181 and ends at position 885. The transcript also has the following SNPs as listed in Table 7 (given according to their position on the nucleotide sequence, with the alternative nucleic acid listed).

**Table 7 - Nucleic acid SNPs**

| Polymorphism | SNP positions on nucleotide sequence |
|---|---|
| G->A | 656 |
| C->G | 661 |
| G->A | 665 |
| T -> A | 728 |
| T -> G | 728 |
| G -> C | 813 |

### EXAMPLE 2

### CLONING AND EXPRESSION OF C1ORF32 EXTRA CELLULAR DOMAIN (ECD) FUSED TO MOUSE FC

The purpose of this analysis was to clone the C1ORF32 ECDs fused via its corresponding C' terminus to mouse Fc (mFc), and to express the fused ECDs in HEK293T cells (ATCC- CRL-11268), in order to be further used for functional assessment of C1ORF32 ECD.

The coordinates of the cloned ECD are described in table 8:

**Table 8:**

| | protein name | Transcript No. | Protein No. | Full length (aa) | ECD Coordinates (aa) | SEQ ID |
|---|---|---|---|---|---|---|
| | C1ORF32 | T8 (SEQ ID NO:1) | P8 (SEQ ID NO:4) | 254 | 1-184 | SEQ ID No. 19 |

**The cloning of the fusion proteins (ECD_mFc)** was done in two steps:
1. Cloning of ECD to pIRESpuro3 (a bicistronic mammalian expression vector, Clontech catalog number: 631619).
2. Subcloning of the mouse Fc IgG2a comprising hinge, CH2 and CH3 regions of murine immunoglobulin Cy2a chain in frame to the C' terminus of the ECD previously cloned into pIRESpuro3, from step1.

### Cloning of ECD to pIRESpuro3

Cloning of the ECD (corresponding to amino acids 1-184) to pIRESpuro3 was carried out by PCR using its full length sequence as a template, and forward primer corresponding to amino acids 1-6 with Nhel sequence site upstream, and a reverse primer corresponding aa 178-184aa with BamHI sequence site downstream, as listed in table 9.

**Table 9: ECD cloning details**

| candidate name | primer ID | primer sequence | Primer orientation | restrictio n site |
|---|---|---|---|---|
| C1ORF32 | 100-746 SEQ ID NO:16 | | Forward | Nhel |
| | 100-851 SEQ ID NO:17 | | Reverse | BamHI |

In the primer sequences shown in Table 9, the bold letters represent the gene specific sequence while the restriction site extensiuons utilized for cloning purposes are Italic and Kozak sequence is underlined.

The PCR products were purified and digested with the appropriate restriction enzymes as described in table 9. PCR products C1ORF32 were ligated into pIRESpuro3. The ligation mixture was transformed into DH5a competent cells. Positive transformants were screened and verified by DNA sequencing.

### Cloning of ECD-mFc pIRESpuro3

Mouse Fc (IgG2a) (Accession- CAA49868 aa 237-469) protein sequence followed by TEV cleavage site sequence was codon optimized to boost protein expression in mammalian system. The optimized sequence was synthesized by GeneArt (Germany) with flanking BamHI restriction site at the N' terminus and NotI restriction site at the C' terminus. The DNA fragment was digested with BamHI/NotI and ligated in frame into ECD_pIRESpuro3 construct previously digested with the same enzymes to give ECD_mFc_pIRESpuro3. The ligation mixture was transformed into DH5a competent cells. Positive transformants were screened and verified by DNA sequencing.

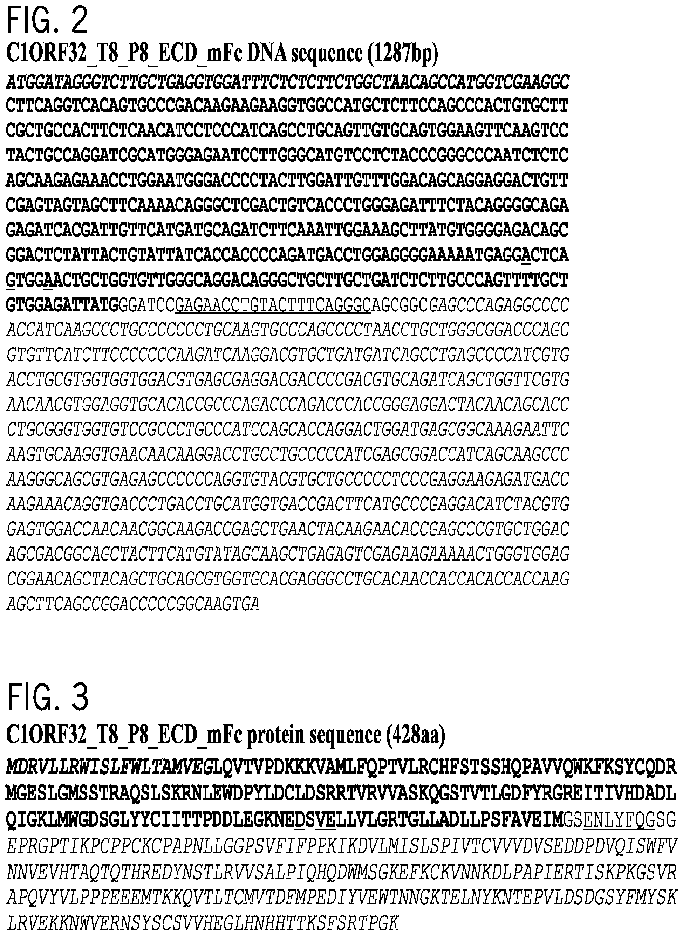

The nucleotide sequences of the resulting ECD_mFc ORFs are shown in Figure 2: gene specific sequence correspond to the ECD sequence is marked in bold faced, TEV cleavage site sequence is underlined, mFc sequence is unbold Italic and signal pepeptide sequence is bold Italic. Figure 2 shows the C1ORF32_P8_V1_ECD_mFc DNA sequence (1287bp) (SEQ ID NO:7).

The sequence of the resulting ECD_mFc fusion proteins are shown in Figure 3; gene specific sequence correspond to the ECD sequence is marked in bold faced, TEV cleavage site sequence is underlined, mFc sequence is unbold Italic and signal pepptide sequence is bold Italic. Figure 3 shows the C1ORF32_P8_V1_ECD_mFc amino acid sequence (428aa) (SEQ ID NO:8).

To generate C1ORF32_P8-V1_ECD_mFc expressing cells, HEK-293T cells were transfected with the above described construct corresponding to C1ORF32 extra cellular domain fused to mouse Fc. Stable pools were generated as follows: 48 hrs post transfection, the cells were trypsinized and transferred to T75 flask containing selection medium (DMEM 10% FCS and 5 µg/ml puromycin) for obtaining stable pool. Media was changed every 3 to 4 days until colonies formation.

To verify the identity of cells, genomic PCR was performed, indicating the correct sequences integrated into the cell genome (data not shown).

### EXAMPLE 3

### PROTEIN PRODUCTION OF C1ORF32 EXTRA CELLULAR DOMAIN (ECD) FUSED TO MOUSE FC (C1ORF32_P8-V1_ECD_mFc)

### Production in HEK293T cells:

To produce C1ORF32 ECD fused to mouse Fc (C1ORF32_P8-VL_ECD_mFc), pool of transfected HEK293T cells stably transfected with the corresponding constructs described herein above, were used. The transfected cells, usually maintained in 10% serum supplemented medium, were transferred into serum free medium (EX-CELL293, SAFC) supplemented with 4 mM glutamine and selection antibiotics (5 ug/ml puromycin), and grown in suspension in shake flasks at 37°C, with agitation. The culture volume was increased by sequential dilutions until a production phase of 3-4 days carried out in 2L spinners flasks. Medium from the spinners was harvested, cleared from cells by centrifugation, filtered through a 0.22 µm filter and kept at -20°C.

The C1ORF32_P8_V1_ECD_mFc protein was purified using nProtein A - affinity chromatography as described below.

Harvests were concentrated approximately 10 fold using PALL ultrafiltration system on two 10 kD cassettes. The concentrate was then adjusted to pH 7.5, by the addition of 5M NaOH and filtrated through 0.2µm Stericup filter.

Purification process was carried out using AKTA Explorer (GE Healthcare). 2 ml of nProtein A Sepharose TM, Fast Flow resin (cat# 17-5280-02) were washed on Poly-prep chromatograohy column under vacumn with 10 column volumes (CV) of 70% ethanol, 10 CV WFI (Sterile Water for Irrigation (TEVA)) followed by 10CV buffer A. 2 ml resin were transffered into two 500 ml tubes (1 ml each) and the concentrated harvest was added. The tube was incubated overnight at 4°C on a roller to allow binding of the protein. Bound resin was then transfered and packed under constant flow into XK16 coulmn (GE Healthcare, cat#18-8773-01). The column was washed with 20CV buffer A (100 Mm Tris pH 7.4) and elution was carried out in one step using 100% buffer B (Citrate/Phosphate pH 3.0). The fractions were titrated with 12.5% (v/v) buffer C (2M Tris pH 8.5) to adjust the pH to ~7.5 and pooled.

The final buffer was exchanged to DPBS (Dulbecco's Phosphate bufferes saline pH 7.4, /o Ca, w/o Mg) pH 7.4 w/o Ca, w/o Mg using a 53 ml HiPrep TM (GE Healthcare, cat# 17-5087-01) desalting column. The protein was filtered through 0.22µm filter, aliquoted under sterile conditions, and stored at -80°C.

The final protein concentration was determined by BCA total protein assay and protein was analyzed by coomassie stained reducing SDS/PAGE (data not shown). Endotoxin level was determined by colorimetric LAL assay (Limulus Amebocyte Lysate, QCL-1000, Cambrex). The identities of the specific proteins were verified by MS (at the Smoler Proteomics Center, Technion, Haifa, data not shown).

The resulting protein analysis is summarized in table 10.

**Table 10**

| Protein | Concentr ation (mg/ml) | Purity (%) | Endotoxins (EU/mg) |
|---|---|---|---|
| C1ORF32-P8-V1-ECD-mFc (SEQ ID NO:8) | 0.9 | >90 | 1.04 |

In addition to the HEK293T produced protein, C1ORF32-P8-V1-ECD-mFc (SEQ ID NO:8) was also produced at Catalent (Middleton, Wisconsin), in CHO cells.

The cDNA sequence of the insert of the previously described cDNA sequence of C1ORF32-P8-V1-ECD-mFc (SEQ ID NO:8) in the vector pIRESpuro3 was verified by Catalent and was used to construct GPEx^{®} retrovectors, followed by four rounds of retrovector transduction into Catalent's CHO-S cell line. A pooled population was produced and expanded and gene copy index was 2.7. Cell culture supernatants were analyzed by Catalent's Fc ELISA assay and relative productivity of the 4x transduced pool was 28µg/ml.

The protein was produced in 5L wave bioreactors, and purified according to their in-house process. Endotoxin levels were tested, and estimated at 0.25-0.5EU/ml. A total of ~400mg were obtained from ~10L of cell pool.

### EXAMPLE 4 - USE OF C1ORF32-P8-V1-ECD-mFc (SEQ ID NO:8) FOR TREATMENT IN PLP139-151-INDUCED EAE (R-EAE), A MODEL OF MULTIPLE SCLEROSIS - MODULATION OF DISEASE INDUCTION AND PROGRESSION

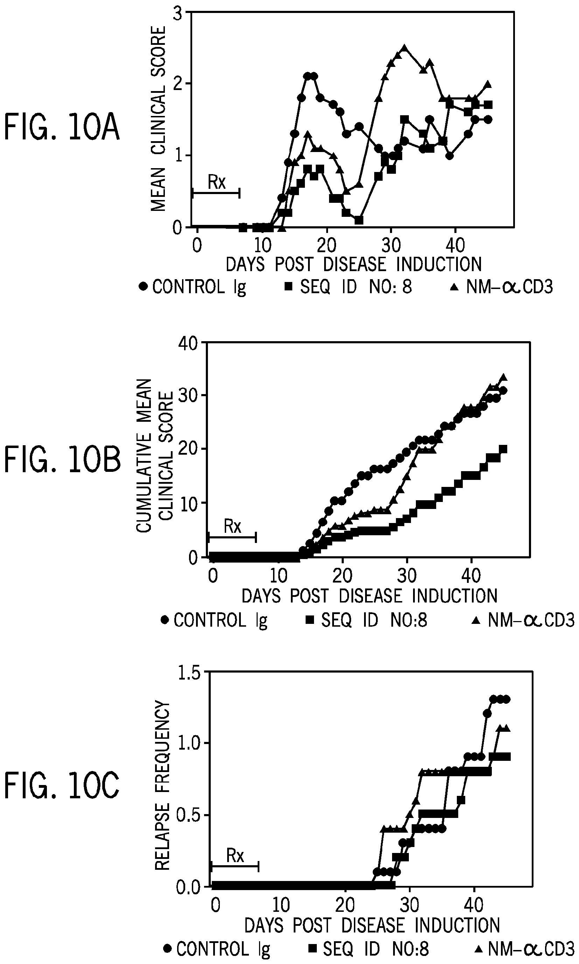

The fusion protein C1ORF32-P8-V1-ECD-mFc (SEQ ID NO:8) produced in CHO cells, as previously described, was tested in R-EAE, an animal model of multiple sclerosis, as well as in related in vitro test models, and was found to be highly effective. This fusion protein, as described above, comprises the extracellular domain (ECD) of C1ORF32-P8_V1 (also referred to herein as H19011_1_P8_V1 (SEQ ID NO:5)), fused to mouse Fc of IgG2a. Two sets of studies were performed: the first set described herein (Figure 5) shows that the fusion protein can effectively treat on going disease in the R-EAE. The second set, described in this Example (Figure 4), showed that preventive treatment with C1ORF32-P8-V1-ECD-mFc (SEQ ID NO:8) administed at priming (i.e. at the time of disease induction, which is before disease onset), reduces the level of disease severity and disease relapse frequency. Similar results were observed using a protein that was produced in HEK-293 cells as described in Example 5 Study D (Figure 10).

This Example shows the efficacy of the CHO produced C1ORF32-P8-V1-ECD-mFc (SEQ ID NO:8) in the R-EAE model [Theien BE, et al., (2003) Blood 102(13):4464-71] upon administration in preventive or therapeutic mode of different doses and frequencies. This model is a relapsing model of multiple sclerosis, based upon the Experimental Autoimmune Encephalomyelitis (EAE) model, which is an inflammatory demyelinating disease of the central nervous system (CNS). Animals in which R-EAE is induced present with a relapsing-remitting disease which may be used to test treatments for relapsing-remitting multiple sclerosis and other sub types of multiple sclerosis.

In this Example, R-EAE was induced through administration of the PLP139-151 peptide to SJL mice. Among the aspects of treatment that were studied include an exemplary, illustrative optimal schedule for C1ORF32-P8-V1-ECD-mFc (SEQ ID NO:8) treatment at the time of disease induction (i.e. preventive administration) and upon onset of disease remission (i.e. therapeutic administration). In addition, the efficacy of C1ORF32-P8-V1-ECD-mFc (SEQ ID NO:8) treatment was studied at 30µg/mouse and 100µg/mouse, and at two dosing frequencies, i.e. 3 times per week for two weeks or daily administration for 6 days.

**Animal methods:** Female SJL mice 6 weeks old were purchased from Harlan and maintained in the CCM central animal care facility (termed herein CCM) for 1 week prior to beginning the experiment. Mice were randomly assigned into groups of 10 animals and primed with 50 micro-g PLP139-151/CFA (Complete Freund's Adjuvant) on day 0. Mice received 6 i.p. injections of Control Ig (mIgG2a), C1ORF32-P8-V1-ECD-mFc (SEQ ID NO:8), anti-CD80 Fab, or non-mitogenic anti-CD3 (via i.v. injection) as listed below. Mice were followed for disease score over a 55 or 100 days period (for preventive and therapeutic administration, respectively) and scored on a 0-5 scale as follows: 0, no abnormality; 1, limp tail; 2, limp tail and hind limb weakness; 3, hind limb paralysis; 4, hind limb paralysis and forelimb weakness; and 5, moribund.

### Groups: (n=10 per group)

In the first set of groups (1-6), treatment began at the time of disease induction (i.e. priming) (day 0):
Group 1: Control Ig (mIgG2a) (100 micro-g /dose; 6 daily consecutive doses)
Group 2: C1ORF32-P8-V1-ECD-mFc (SEQ ID NO:8) (30 micro-g /dose; 6 daily consecutive doses)
Group 3: C1ORF32-P8-V1-ECD-mFc (SEQ ID NO:8) (100 micro-g /dose; 6 daily consecutive doses)
Group 4: C1ORF32-P8-V1-ECD-mFc (SEQ ID NO:8) (30 micro-g /dose; 3 doses per week for 2 weeks)
Group 5: C1ORF32-P8-V1-ECD-mFc (SEQ ID NO:8) (100 micro-g /dose; 3 doses per week for 2 weeks)
Group 6: Non-mitogenic anti-CD3 (50 micro-g / dose; 6 daily consecutive doses)

In the second set of groups (7-12), treatment began at the onset of disease remission (day 20 post disease induction):
Group 7: Control Ig (mIgG2a) (100 micro-g /dose; 6 daily consecutive doses)
Group 8: C1ORF32-P8-V1-ECD- mFc (SEQ ID NO:8) (30 micro-g / dose; 6 daily consecutive doses)
Group 9: C1ORF32-P8-V1-ECD- mFc (SEQ ID NO:8) (100 micro-g / dose; 6 daily consecutive doses)
Group 10: C1ORF32-P8-V1-ECD- mFc (SEQ ID NO:8) (30 micro-g /dose; 3 doses per week for 2 weeks)
Group 11: C10RF32-P8-V1-ECD- mFc (SEQ ID NO:8) (100 micro-g /dose; 3 doses per week for 2 weeks)
Group 12: Anti-CD80 Fab (50 micro-g /dose; 6 daily consecutive doses)

### Results:

Preventive administration beginning on day 0 of C1ORF32-P8-V1-ECD- mFc (SEQ ID NO:8) resulted in potent amelioration of disease state in the acute phase by all regimens used, as demonstrated by the decreased clinical score and delayed disease onset (Figure 4). In addition, a dramatic decrease in relapse rate was observed, manifested as amelioration of the disease symptoms in the relapses, with a most pronounce disease abolishment from day 43, achieved by administration of either 30ug/mouse or 100ug/mouse C1ORF32-P8-ECD- mFc (SEQ ID NO:8) 3 times per week. This beneficial effect was long lasting and persisted throughout the observation period of the study. All groups treated with C1ORF32-P8-ECD- mFc (SEQ ID NO:8) showed better efficacy than the positive control group, treated with non-mitogenic anti CD3.

Figure 4A shows that C1ORF32-P8-V1-ECD-mFc (SEQ ID NO:8) administered in a preventive mode at disease priming, provides potent efficacy in the R-EAE model manifested in amelioration of disease state by all regimens used, as demonstrated by the decrease in clinical scores and delayed disease onset. Shown are Mean Clinical Score (Figure 4A), Cumulative Mean Clinical Score (Figure 4B), and Relapse Frequency (Figure 4C).

In addition, administration of C1ORF32-P8-V1-ECD-mFc (SEQ ID NO:8) in a therapeutic mode, at the onset of disease remission, on day 20, exhibited a dramatic amelioration of the disease symptoms by all regimens of C10RF32-P8-V1-ECD- mFc (SEQ ID NO:8) (Figure 5). This treatment resulted in a complete abolishment of the relapses and elimination of disease signs for up to 100 days, i.e. 66 days after last administration of C10RF32-P8-V1-ECD- mFc (SEQ ID NO:8). The therapeutic efficacy of C1ORF32-P8-V1-ECD- mFc (SEQ ID NO:8) is also superior over that of the positive control, anti-CD80 Fab.

Figure 5 shows that administration of C1ORF32-P8-V1-ECD-mFc (SEQ ID NO:8) in the R-EAE model in a therapeutic mode, at the onset of disease remission (on day 20), exhibited a dramatic and long lasting amelioration of the disease symptoms by all regimens as manifested by reduction in Mean Clinical Score (Figure 5A), Cumulative Mean Clinical Score (Figure 5B), and Relapse Frequency (Figure 5C), throughout the study duration (up to day 100).

Overall, the long lasting beneficial effect of C1ORF32-P8-V1-ECD-mFc (SEQ ID NO:8) indicates that C1ORF32-P8-V1-ECD- mFc (SEQ ID NO:8) may induce tolerance to the myelin epitopes driving disease progression. Further support for tolerance induction is provided in Example 6 Studies A and B.

### EXAMPLE 5 - MODULATION OF T CELL ACTIVITY BY C1ORF32-P8-V1-ECD-mFc (SEQ ID NO:8) AND USE FOR PREVENTION OF MULTIPLE SCLEROSIS

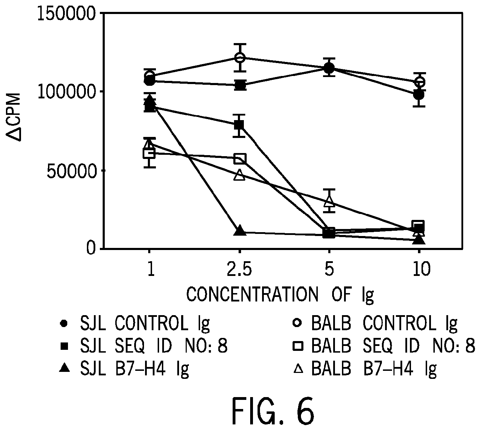

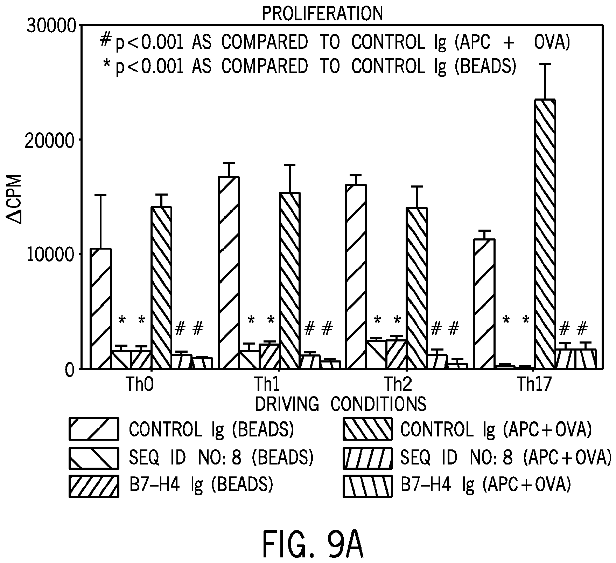

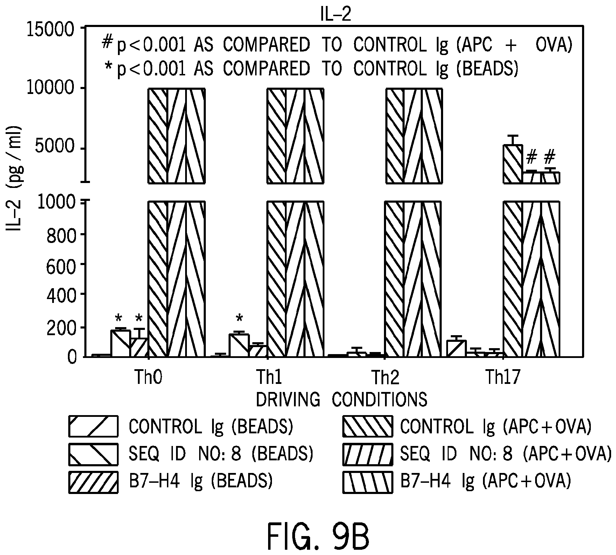

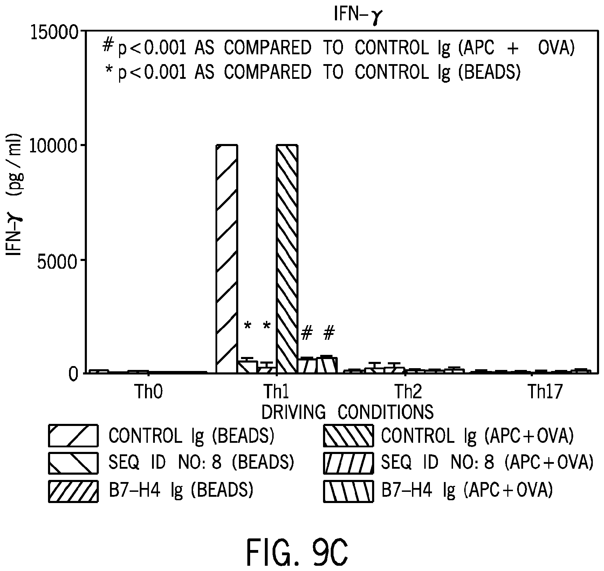

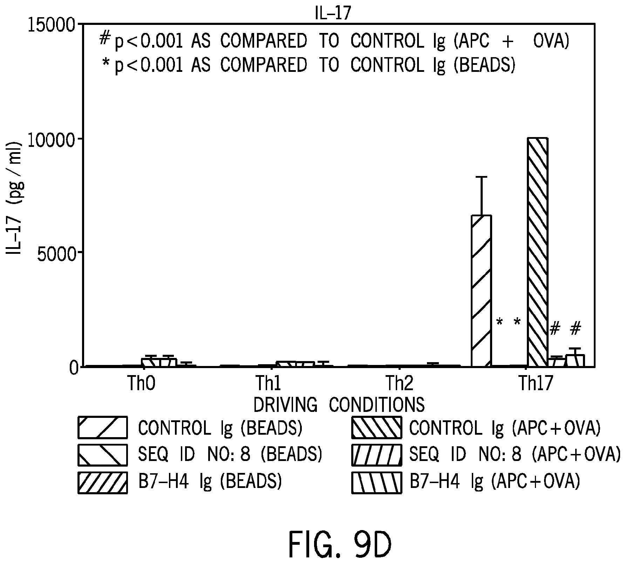

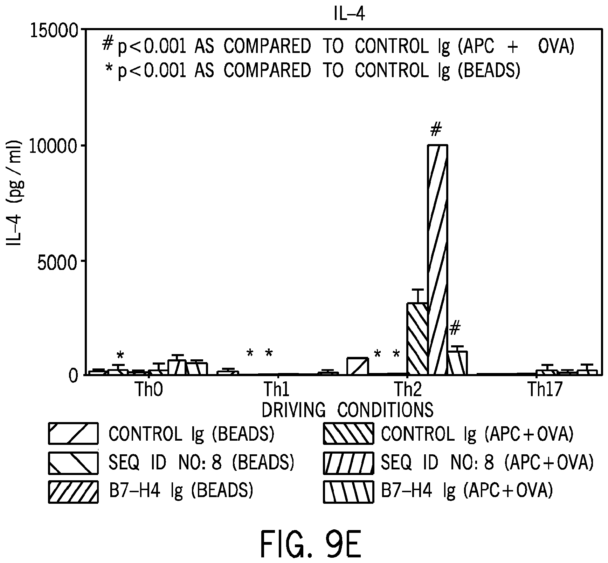

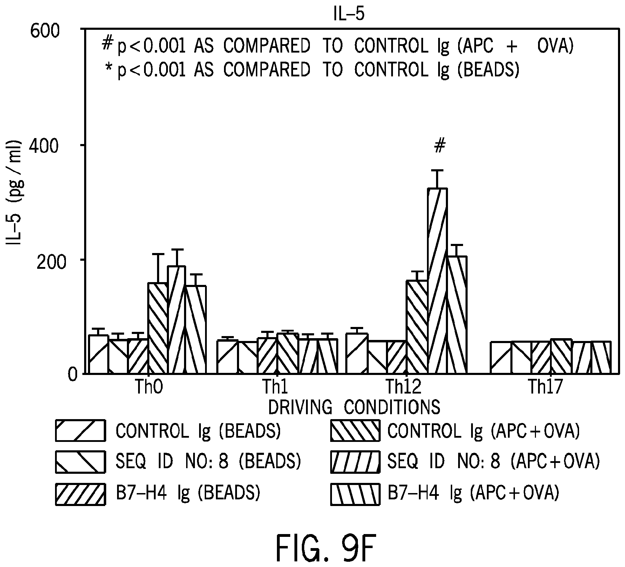

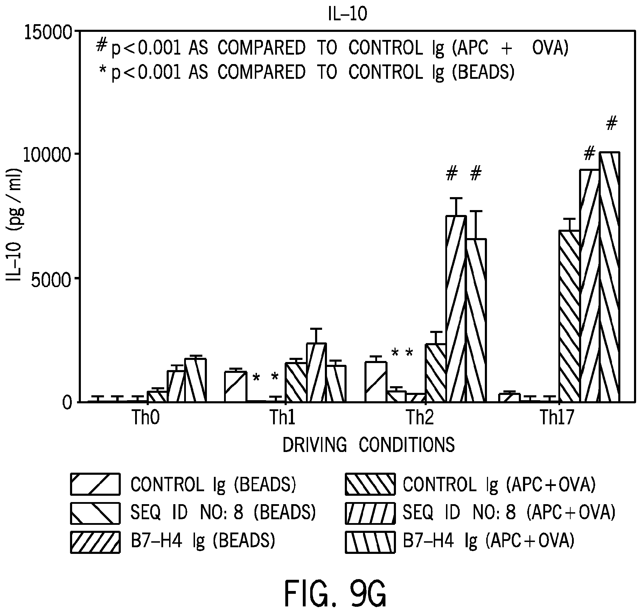

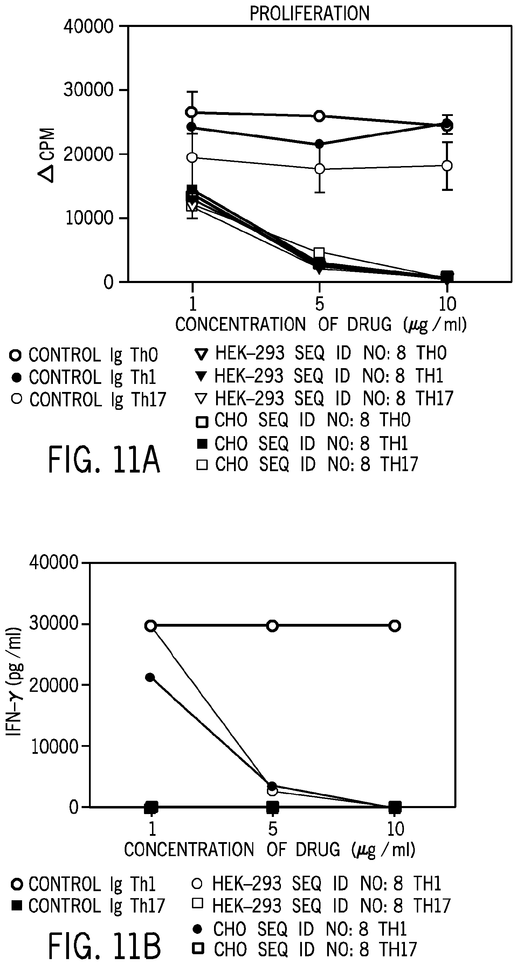

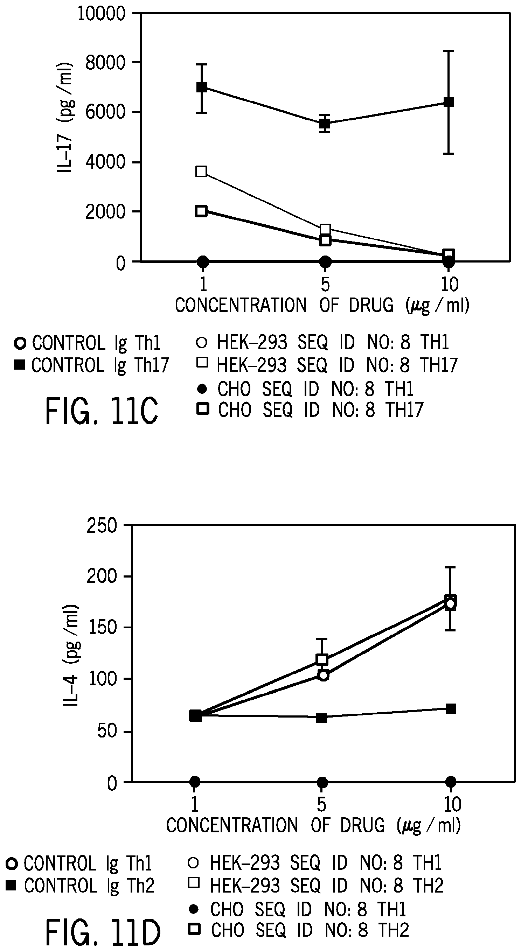

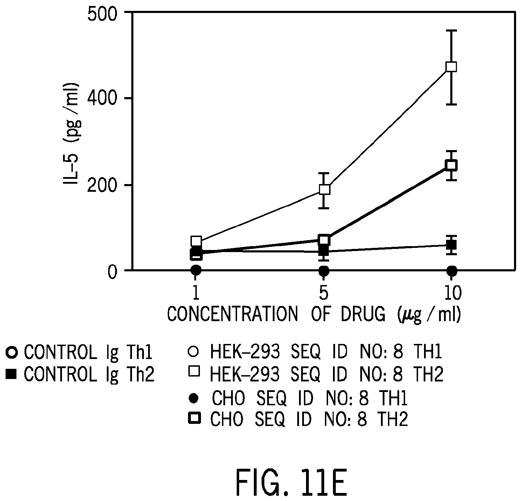

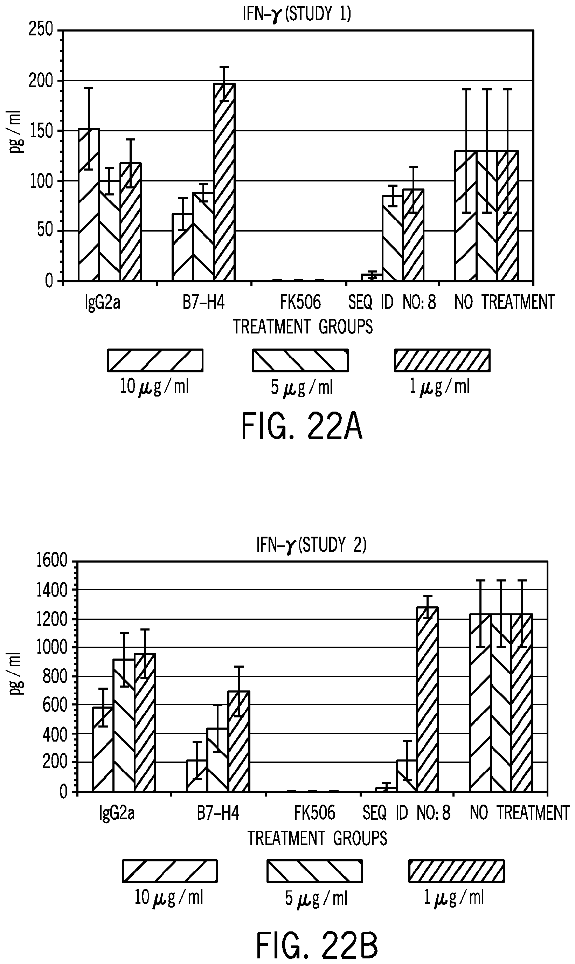

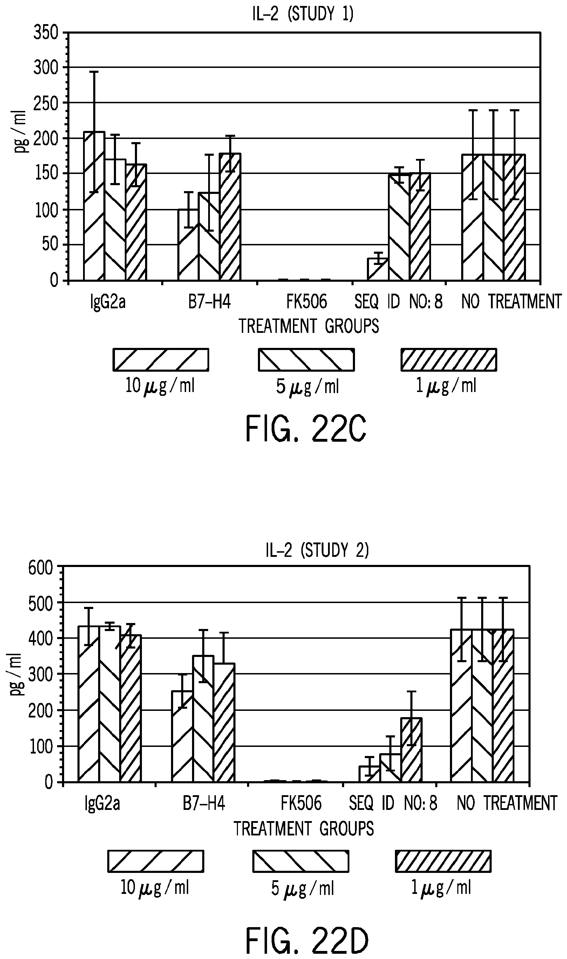

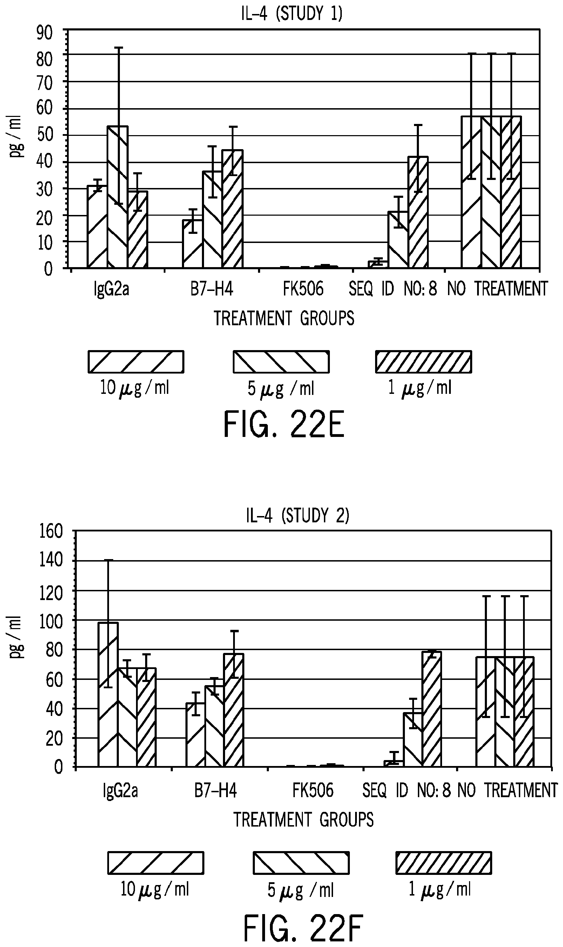

USING HEK-293 or CHO- DERIVED PROTEIN This Example shows the modulatory effect of C1ORF32-P8-V1-ECD-mFc on T cell activity in vitro and that preventive treatment with C1ORF32-P8-V1-ECD-mFc (SEQ ID NO:8) (i.e. at the time of disease induction and before disease onset) reduces the level of disease severity and disease relapse frequency. In vitro, C1ORF32-P8-V1-ECD-mFc (SEQ ID NO:8) produced in HEK-293 cells exhibited a unique ability to inhibit T cell activation manifested in cell proliferation and cytokine secretion (Figures 6-8). In addition, both HEK-293 and CHO-produced C1ORF32-P8-V1-ECD-mFc (SEQ ID NO:8) exhibited the unique ability to inhibit differentiation of pro-inflammatory Th1 and Th17 responses while skewing the immune response towards the regulatory Th2-responses (Figures 9 and 11). The CHO-produced protein also inhibited the activation of mouse splenocytes, exhibited by inhibition of cytokine secretion (Figure 22). Preventive administration of HEK-293- derived C1ORF32-P8-V1-ECD-mFc (SEQ ID NO:8) in mouse PLP-139-151 induced R-EAE, exhibited efficacy manifested as decrease in disease score and relapse frequency (Figure 10). These studies are provided below in six subsections (Studies A-F). The in vitro data, presented in Studies A, B, E, and F indicates that C1ORF32-P8-V1-ECD- mFc (SEQ ID NO:8) produced in either HEK-293 or CHO cells, inhibits the activation of mouse T cells or splenocytes activated in the presence of anti-CD3/anti-CD28 or in the presence of a cognate antigenic peptide. Furthermore, data presented in Study C and in Study E indicates that the addition of C1ORF32-P8-V1-ECD- mFc (SEQ ID NO:8) to naïve CD4+ T cells activated in the presence of a cognate antigenic peptide under Th0 cell- , Th1 cell-, Th2 cell- or Th17 cell-promoting conditions, inhibits Th1 and Th17 responses, and promotes Th2 responses, skewing the cytokine profile towards a Th2 cell phenotype. Study D indicates that treatment of mice at the time of PLP139-151 R-EAE disease induction with C1ORF32-P8-V1-ECD- mFc (SEQ ID NO:8) reduces the level of disease severity as determined by Mean Clinical Score, Cumulative Mean Clinical Score, and Disease Relapse Frequency.

### STUDY A: DETERMINE THE EFFECT AND DOSE RESPONSE OF C1ORF32-P8-V1-ECD- mFc (SEQ ID NO:8) IN VITRO AS DETERMINED BY CELLULAR PROLIFERATION FOLLOWING CD4⁺ T CELL ACTIVATION.

**PURPOSE:** The goal of the present experimental plan was to investigate the ability of C1ORF32-P8-V1-ECD- mFc (SEQ ID NO:8) produced in HEK-293 cells to inhibit CD4⁺ T cell activation and the ideal concentration of C10RF32-P8-V1-ECD- mFc (SEQ ID NO:8) to be used in the future *in vitro* experiments.

**METHODS:** To this end the ability of various doses of C1ORF32-P8-V1-ECD-mFc (SEQ ID NO:8) to inhibit CD4⁺ T cell proliferation was tested. Naive CD4⁺ T cells were isolated from wildtype mice (SJL form Harlan and BALB/c from Jackson) and activated *in vitro* in the presence of anti-CD3/anti-CD28. The rationale for testing both strains of mice was to ensure that there is not a mouse strain-to-strain difference. Naive CD4⁺ T cells were isolated from total lymph node and splenocyte populations, and activated *in vitro* in the presence of anti-CD3/anti-CD28 in the presence of C1ORF32-P8-V1-ECD-mFc (SEQ ID NO:8), Control Ig (mIgG2a, as negative control), or B7-H4 Ig (as positive control). Cellular proliferation was determined via tritiated-thymidine incorporation.

### GROUPS:

BALB/c or SJL T cells + Bead bound Control Ig (mIgG2a) (1, 2.5, 5 and 10µg/ml)
BALB/c or SJL T cells + Bead bound C1ORF32-P8-V1-ECD-mFc (SEQ ID NO:8) (1, 2.5, 5 and 10µg/ml)
BALB/c or SJL T cells + Bead bound B7-H4 Ig (1, 2.5, 5 and 10µg/ml)

**RESULTS:** Results shown in Figure 6 indicate that C1ORF32-P8-V1-ECD-mFc (SEQ ID NO:8) treatment decreased the level of cell proliferation, in T cells derived from SJL or BALB/c mice. The effect was similar to that of the positive control, B7-H4, and 2.5-5µg/ml of C1ORF32-P8-V1-ECD-mFc (SEQ ID NO:8) appears to be optimal concentration of C1ORF32-P8-V1-ECD-mFc (SEQ ID NO:8) in this assay.

### STUDY B: DETERMINE THE EFFECT OF C1ORF32-P8-V1-ECD-MFC (SEQ ID NO:8) ON CD4⁺ T CELL ACTIVATION, PROLIFERATION AND CYTOKINE PRODUCTION IN VITRO.

**PURPOSE:** The goal of the present experiment was to determine the ability of C1ORF32-P8-V1-ECD-mFc (SEQ ID NO:8) produced in HEK-293 cells, when bound to beads, plate bound, or added to cultures in soluble form to inhibit T cell activation, as determined by cell proliferation and cytokine production.

**METHODS:** The ability of C1ORF32-P8-V1-ECD-mFc (SEQ ID NO:8) to inhibit CD4⁺ T cell proliferation and cytokine production in the absence of specific CD4⁺ effector T cell driving conditions was tested. To do so, naive CD4⁺ T cells were isolated from wildtype mice (SJL and BALB/c) and activated *in vitro* in the presence of anti-CD3/anti-CD28. The optimal concentration of C1ORF32-P8-V1-ECD-mFc (SEQ ID NO:8) (5ug/ml) from Study A was used in the present study. Naive CD4⁺ T cells were isolated from total lymph node and splenocyte populations, and activated *in vitro* in the presence of anti-CD3/anti-CD28 in the presence of C1ORF32-P8-V1-ECD-mFc (SEQ ID NO:8), Control Ig (negative control), or B7-H4 Ig (positive control). Two identical sets of cultures were set up, for proliferation and cytokine production. Cellular proliferation was determined via tritiated-thymidine incorporation, and culture supernatants were collected and analyzed via LiquiChip. The level of IL-2, IFN-gamma, IL-17, IL-10, and TFN-alpha produced is shown. The levels of IL-4, IL-5, and IL-12 were also analyzed, but these three cytokines were below the level of detection.

### GROUPS:

T cells + Soluble Control Ig (mIgG2a)
T cells + Plate bound Control Ig (mIgG2a)
T cells + Bead bound Control Ig (mIgG2a)
T cells + Soluble C1ORF32-P8-V1-ECD-mFc (SEQ ID NO:8)
T cells + Plate bound C1ORF32-P8-V1-ECD-mFc (SEQ ID NO:8)
T cells + Bead bound C1ORF32-P8-V1-ECD-mFc (SEQ ID NO:8)
T cells + Soluble B7-H4 Ig
T cells + Plate bound B7-H4 Ig
T cells + Bead bound B7-H4 Ig

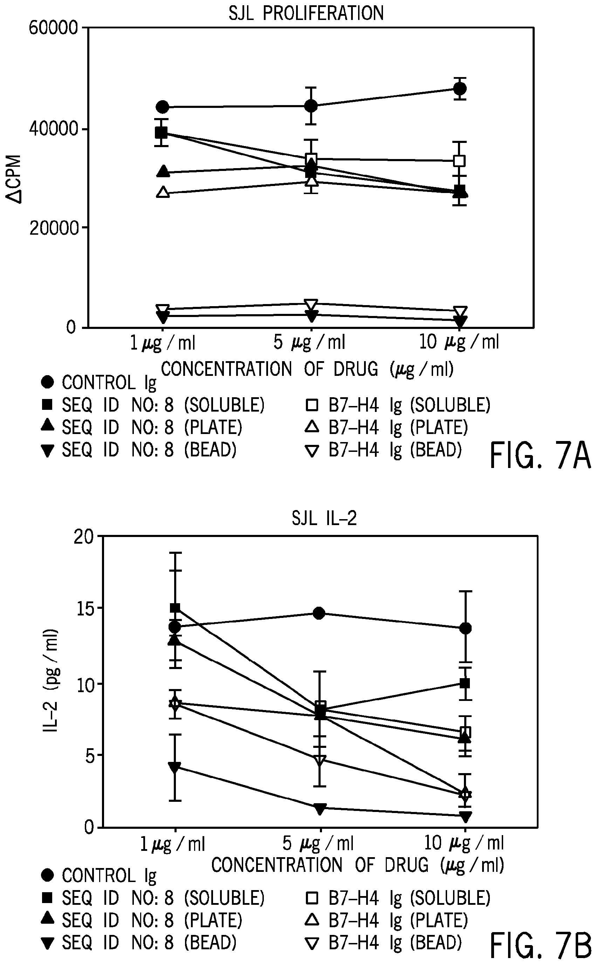

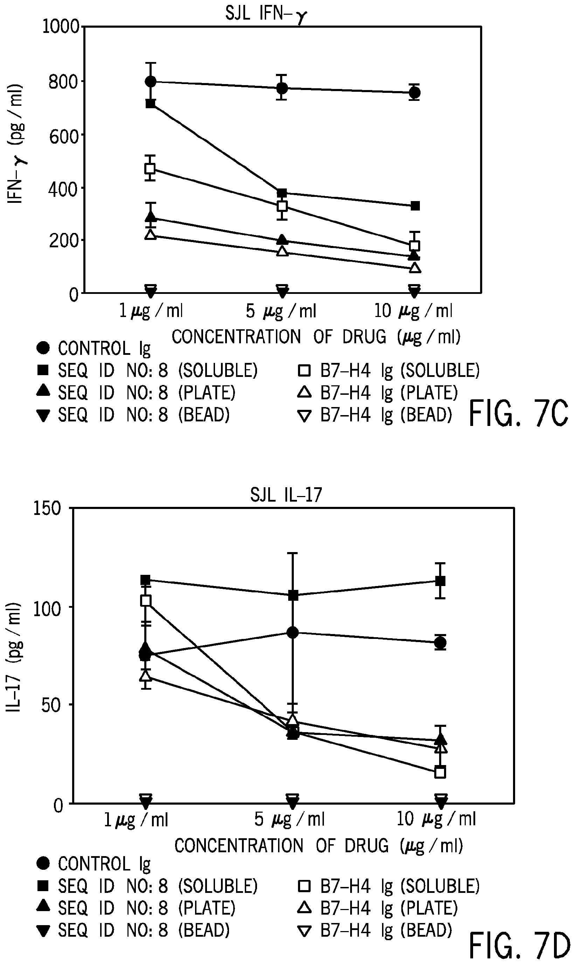

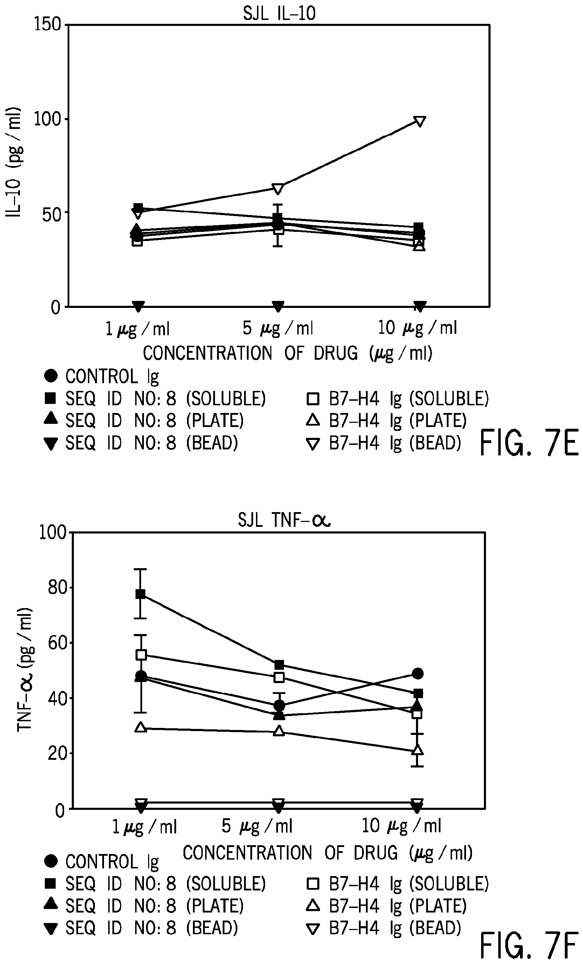

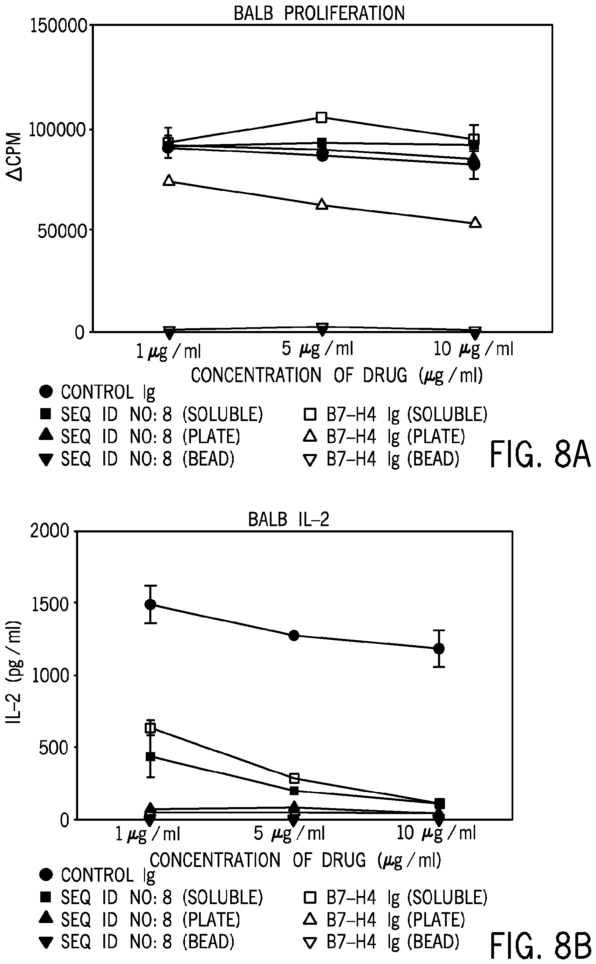

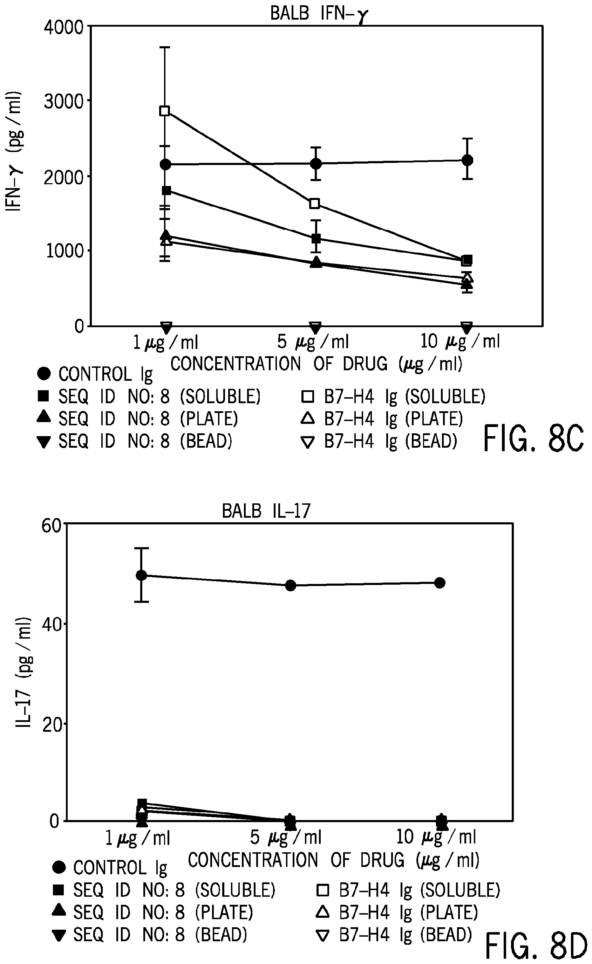

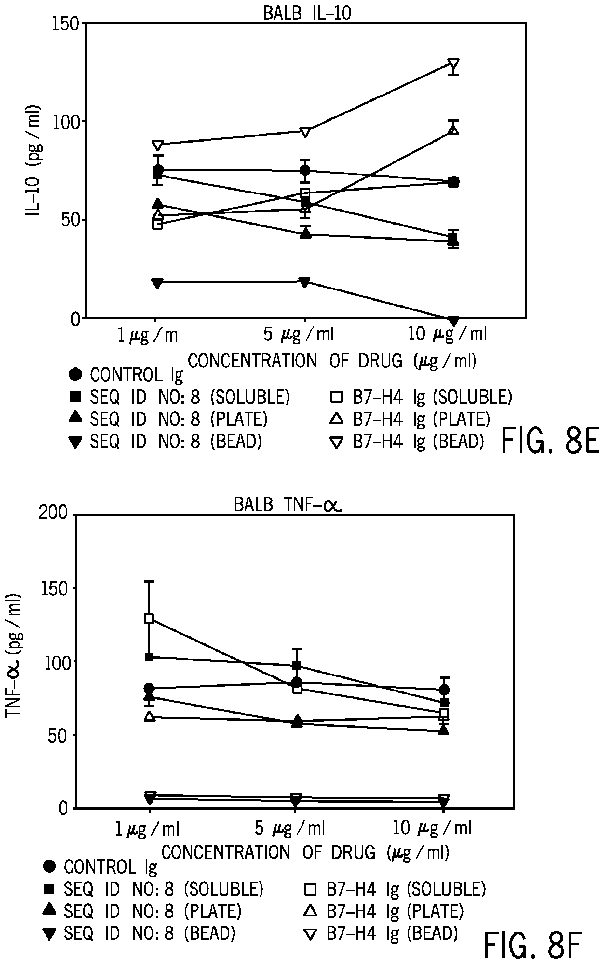

**RESULTS AND CONCLUSIONS:** The results obtained on T cell proliferation and cytokine secretion are presented in Figure 7 (SJL Naive CD4⁺ T cells) and Figure 8 (BALB/c Naive CD4⁺ T cells), showing inhibition of of T cell activation, as manifested by cell proliferation and cytokine secretion, upon treatment with C1ORF32-P8-V1-ECD-mFc (SEQ ID NO:8). Similar results were obtained for T cells from both strains of mice. C1ORF32-P8-V1-ECD-mFc (SEQ ID NO:8) decreases the level of CD4⁺ T cell proliferation in a concentration-dependent manner when it is added in soluble form or as plate-bound or bead-bound forms to the cultures. However, the bead-bound form of C1ORF32-P8-V1-ECD-mFc (SEQ ID NO:8) is much more effective at inhibiting cellular proliferation and cytokine production, and the soluble form of the protein is the least effective. From the present data 5ug/ml of C1ORF32-P8-V1-ECD-mFc (SEQ ID NO:8) coated on a bead is the optimal concentration.

### STUDY C: DETERMINE THE EFFECT OF C1ORF32-P8-V1-ECD-mFc (SEQ ID NO:8) ON CD4⁺ T CELL DIFFERENTIATION AND CYTOKINE PRODUCTION IN VITRO.

**PURPOSE:** The goal of the present experimental plan was to investigate the ability of C1ORF32-P8-V1-ECD-mFc (SEQ ID NO:8) produced in HEK-293 cells to inhibit CD4⁺ T cell activation and differentiation.

**METHODS:** The ability of C1ORF32-P8-V1-ECD-mFc (SEQ ID NO:8) to inhibit CD4⁺ T cell differentiation, cytokine production and proliferation was tested. To do so, naive CD4⁺ T cells were isolated from DO11.10 mice (transgenic mice in which all of the CD4⁺ T cells express a T cell receptor (TCR) that is specific for OVA₃₂₃₋₃₃₉ peptide). The rationale for the use of DO11.10 CD4⁺ T cells is that both polyclonal (anti-CD3/anti-CD28 mAbs) and peptide-specific CD4⁺ T cell activation may be studied on the same population of CD4⁺ T cells. Experimentally, naive CD4⁺ cells were activated in the presence of Th0 cell- (IL-2), Th1 cell- (IL-2 + IL-12), Th2 cell- (IL-2 + IL-4), or Thl7 cell- (TGF-β + IL-6 + IL-23 + anti-IL-2) promoting conditions. To activate the CD4⁺ T cells, the cells were cultured in the presence of anti-CD3/anti-CD28 coated beads or OVA₃₂₃₋₃₃₉ peptide plus irradiated BALB/c splenocytes in the presence of C1ORF32-P8-V1-ECD-mFc (SEQ ID NO:8), Control Ig, or B7-H4 Ig. Two side-by-side culture sets were set up; one culture being pulsed at 24 hours with tritiated-thymidine and harvested at 72 hours while the second plate was harvested at 96 hours for cytokine production. The levels of IL-2, IL-4, IL-5, IL-10, IL-12, IL-17, IFN-γ, and TNF-α were tested via LiquiChip. The level of IL-12 and TNF-α was also analyzed, but these two cytokines were below the level of detection.

### GROUPS:

Group 1: Control Ig (mIgG2a)
   Th0 with anti-CD3/28 plus Bead Bound
   Th1 with anti-CD3/28 plus Bead Bound
   Th2 with anti-CD3/28 plus Bead Bound
   Th17 with anti-CD3/28 plus Bead Bound
   Th0 with OVA+APC plus Soluble
   Th1 with OVA+APC plus Soluble
   Th2 with OVA+APC plus Soluble
   Th17 with OVA+APC plus Soluble
Group 2: C1ORF32-P8-V1-ECD-mFc (SEQ ID NO:8) - Same as for Control Ig
Group 3: B7-H4 Ig - Same as for Control Ig

**RESULTS:** The results shown in Figure 9 indicate that C1ORF32-P8-V1-ECD-mFc (SEQ ID NO:8) decreases the level of CD4⁺ T cell proliferation when it is bound to a bead with anti-CD3/anti-CD28, as well as when the soluble protein is added to irradiated APC+OVA₃₂₃₋₃₃₉ activating conditions. C1ORF32-P8-V1-ECD-mFc (SEQ ID NO:8) decreases the level of IFN-γ and IL-17 produced by CD4⁺ T cells activated in the presence of Th1 cell- and Thl7 cell-promoting conditions, respectively. In addition, C1ORF32-P8-V1-ECD-mFc (SEQ ID NO:8) appears to promote the level of IL-4 and IL-5 produced by CD4⁺ T cells activated in the presence of Th2 cell-promoting conditions. Interestingly, C1ORF32-P8-V1-ECD-mFc (SEQ ID NO:8) appears to also promote the level of the anti-inflammatory cytokine IL-10 produced by CD4⁺ T cells activated in the presence of Th2 or Thl7 cell-promoting conditions.

### STUDY D: TO DETERMINE THE EFFICACY OF C1ORF32-P8-V1-ECD-mFc (SEQ ID NO:8) TREATMENT AT THE TIME OF DISEASE INDUCTION IN PLP₁₃₉₋₁₅₁-INDUCED R-EAE IN SJL MICE.

**PURPOSE:** To determine the efficacy of C1ORF32-P8-V1-ECD-mFc (SEQ ID NO:8) treatment at the time of R-EAE induction in SJL mice with PLP139-151.

**METHODS:** Female SJL mice 6 weeks old were purchased from Harlan and maintained in the CCM facility as previously described for 1 week prior to beginning the experiment. Mice were randomly assigned into groups of 10 animals and primed with 50 µg PLP₁₃₉₋₁₅₁/CFA on day 0. Mice received 6 i.p. injections of either Control Ig or C1ORF32-P8-V1-ECD-mFc (SEQ ID NO:8), or 6 i.v. injections of non-mitogenic anti-CD3 (as a positive control for a decrease in disease induction) beginning on day 0 (day of priming). Mice were followed for disease over a 45 day period.

### GROUPS:

Control Ig (mIgG2a)
C1ORF32-P8-V1-ECD-mFc (SEQ ID NO:8) produced in HEK-293 cells Non-mitogenic anti-CD3

**RESULTS:** The results, presented in Figure 10, show that treatment of mice with C10RF32-P8-V1-ECD-mFc (SEQ ID NO:8) via i.p. daily injection on days 0-5 post disease induction decrease the level of clinical disease, as determined by the Mean Clinical Score and the Cumulative Mean Clinical Score, and this protective effect was more pronounced than that of the positive control. The effect of treatment appears to be lost by or around day +30 post disease induction, and this is shown in the Relapse Frequency data. The loss of disease protection by day +30 post disease induction is to be expected in the actively induced disease model, since there is a bolus of PLP139-151/CFA still on the back of the mice allowing for the activation of new PLP139-151-specific CD4+ T cells over this studied time course.

### STUDY E: COMPARING THE EFFECT OF TWO SOURCES OF C1ORF32-P8-V1-ECD-mFc (SEQ ID NO:8) ON CD4⁺ T CELL PROLIFERATION AND CYTOKINE PRODUCTION IN VITRO.

**PURPOSE:** To investigate the ability of the new C1ORF32-P8-V1-ECD-mFc (SEQ ID NO:8) protein produced in CHO cells to inhibit CD4⁺ T cell activation, and compare it to that of the old C1ORF32-P8-V1-ECD-mFc (SEQ ID NO:8) protein, produced in HEK-293 cells.

**METHODS:** Naive CD4⁺ T cells were activated in the presence of beads coated with anti-CD3 (0.5ug/ml), anti-CD28 (2 µg/ml), and 5ug/ml of Control Ig, C1ORF32-P8-V1-ECD-mFc (SEQ ID NO:8) produced in CHO cells, or C1ORF32-P8-V1-ECD-mFc (SEQ ID NO:8) produced in HEK-293 cells , at a ratio of 1:2 (beads to T cells). Cells were activated in the presence of Th0 cell-, Th1 cell-, Th2 cell-, and Thl7 cell-promoting conditions.

### GROUPS:

SJL T cells + Control Ig (mIgG2a) (lug/ml)
SJL T cells + Control Ig (mIgG2a) (5ug/ml)
SJL T cells + Control Ig (mIgG2a) (10ug/ml)
SJL T cells + new C1ORF32-P8-V1-ECD-mFc (SEQ ID NO:8) (lug/ml) + Control Ig (9ug/ml)
SJL T cells + new C1ORF32-P8-V1-ECD-mFc (SEQ ID NO:8) (5ug/ml) + Control Ig (5ug/ml)
SJL T cells + new C1ORF32-P8-V1-ECD-mFc (SEQ ID NO:8) (10ug/ml) + Control Ig (0ug/ml)
SJL T cells + old C1ORF32-P8-V1-ECD-mFc (SEQ ID NO:8) (lug/ml) + Control Ig (9ug/ml)
SJL T cells + old C1ORF32-P8-V1-ECD-mFc (SEQ ID NO:8) (5ug/ml) + Control Ig (5ug/ml)
SJL T cells + old C1ORF32-P8-V1-ECD-mFc (SEQ ID NO:8) (10ug/ml) + Control Ig (0ug/ml)

**RESULTS:** The results presented in Figure 11 show that C1ORF32-P8-V1-ECD-mFc (SEQ ID NO:8) produced in CHO has similar effects to that produced in HEK-293, and is able to inhibit CD4+ T cell differentiation in the presence of Th1 cell- and Thl7 cell- promoting conditions, while enhancing Th2 cell differentiation.

### STUDY F: EFFECT OF C1ORF32-P8-V1-ECD-mFc (SEQ ID NO:8) in-vitro on mouse splenocytes activation

### Methods

Spleens were harvested from anesthetized mice. Splenocytes were isolated by centrifugation of mashed spleens in Histopaque- (Sigma 1119) and suspended at 1.0 × 10⁶ cells/ml. C1ORF32-P8-V1-ECD-mFc (SEQ ID NO:8) was added to splenocytes as soluble protein to final concentrations of 1, 5 and 10µg/ml. FK506 and B7-H4 Ig were used as positive controls and mIgG2a were used as a negative control at similar concentrations as C1ORF32-P8-V1-ECD-mFc (SEQ ID NO:8). Splenocytes were spiked with anti-CD28 to obtain a final concentration of 1µg/ml in a 100µl/well sample onto anti-CD3 pre-coated wells (1 µg /ml in PBS, overnight at 4°C) in 96 well clear bottom plates. Plates were incubated at 37°C with 5.0% humidity for 24hrs and analyzed for cytokine secretion by standard ELISA.

### Results:

Results of two studies carried out are shown in Figure 22 and indicate that C1ORF32-P8-V1-ECD-mFc (SEQ ID NO:8) treatment decreased the level of IFNy (Figure 22 A and B), IL-2 (Figure 22 C and D) and IL-4 (Figure 22 E and F) production by activated splenocytes in a dose dependent manner. The results of each study are labeled as "study 1" and "study 2", respectively. The effect of C1ORF32-P8-V1-ECD-mFc (SEQ ID NO:8) was roughly similar to that of the positive control, B7-H4 but lower than that of the immunosuppressive FK506. The negative control IgG2a, had essentially no effect. As shown the study was repeated twice with similar results.

### EXAMPLE 6 ― MODE OF ACTION OF THE THERAPEUTIC EFFECT OF C1ORF32-P8-V1-ECD-mFc (SEQ ID NO:8) IN PLP₁₃₉₋₁₅₁-INDUCED R-EAE IN SJL MICE

The relapsing nature of multiple sclerosis results from infiltration of encephalitogenic autoreactive T cells into the CNS which attack endogenous myelin epitopes. This response to newly exposed, relapse-associated myelin epitopes, is known as "epitope spreading". The mode of action underlying the beneficial effect of C1ORF32-P8-V1-ECD-mFc (SEQ ID NO:8) in the R-EAE model was studied by analyzing its effect on immune cell populations in secondary lymphoid organs and the CNS, and by testing recall responses of splenocytes and lymph node cells to spread epitopes during disease.

### Study A- Effect of C1ORF32-P8-V1-ECD-mFc (SEQ ID NO:8) on recall responses to spread epitopes in spleen cells ex vivo, and on cell trafficing within the CNS, spleen, and lymph nodes

**PURPOSE:** To further establish the efficacy of C1ORF32-P8-V1-ECD-mFc (SEQ ID NO:8) treatment during disease remission in PLP139-151-induced R-EAE in SJL mice, and to study the effect of C1ORF32-P8-V1-ECD-mFc (SEQ ID NO:8)treatment on immune cell populations within the CNS and secondary lymphoid organs.

### Method

Female SJL mice 6 weeks old were purchased from Harlan and maintained in the CCM facility for 1 week prior to beginning the experiment. Mice were randomly assigned into 4 groups (10 mice per group), and primed with 50ug PLP₁₃₉₋₁₅₁/CFA on Day 0. Beginning during disease remission (day 18 post disease induction), mice received i.p. injections of either Control Ig, C1ORF32-P8-V1-ECD-mFc (SEQ ID NO:8), or anti-CD80 Fab as indicated in the list of experimental groups listed below. Mice were followed for clinical disease over a 50 day time course. During this time course (day 35 post disease induction) 5 representative mice from each treatment group were assessed for epitope spreading via ex vivo recall responses to spread epitopes via proliferation, and cytokine secretion. In addition,the number and phenotype of cells within the CNS, spleen, and lymph nodes was evaluated For in vitro recall responses, total splenocytes from individual mice were activated in the presence of medium alone, the disease inducing peptide (PLP139-151), spread epitope peptides (PLP178-191 and MBP84-104), and anti-CD3.

### GROUPS: (n=10 mice per group)

Group 1: Control Ig (100ug/dose; 3 doses/week for 2 weeks) (mouse IgG2a; BioXCell Cat. BE0085)
Group 2: C1ORF32-P8-V1-ECD- mFc (SEQ ID NO:8) (30ug/dose; 3 doses/week for 2 weeks)
Group 3: C1ORF32-P8-V1-ECD- mFc (SEQ ID NO:8) (100ug/dose; 3 doses/week for 2 weeks)
Group 4: Anti-CD80 Fab (50mg/dose; 5 consecutive doses) (Fab of Clone 16-10A1; BioXCell Cat. BE0024)

### Results

The present Example shows similar disease modulatory effect of C1ORF32-P8-V1-ECD- mFc (SEQ ID NO:8) treatment in the R-EAE model, for both the lower dose and the higher dose (30 and 100ug/dose, respectively) as previously described (Example 4). As shown in Figure 12A, treatment of SJL mice with PLP139-151-induced R-EAE, using C1ORF32-P8--V1-ECD- mFc (SEQ ID NO:8), at either 30ug/dose or 100ug/dose beginning at onset of disease remission, decreases disease severity to a significant level.

C1ORF32-P8-V1-ECD- mFc (SEQ ID NO:8) treatment affected the total cell number and the composition of immune cell populations in the CNS and secondary lymphoid organs. Most prominent is the robust decrease in the number of infiltrating CD4+ T cells within the CNS, which strongly correlate with the reduction in the level of disease severity (Figure 12B and 12C).

A slight increase in immune cells recruitment to the spleen and lymph node was observed only upon treatment with 100 microg/mouse C1ORF32-P8-V1-ECD-mFc (SEQ ID NO:8).

C1ORF32-P8-V1-ECD- mFc (SEQ ID NO:8) treatment of R-EAE mice also dramatically inhibited recall responses of spleen cells to PLP139-151 (disease inducing epitope) or PLP178-191 (spread epitope), which indicate inhibition of myelin-specific T cell responses by C1ORF32-P8-V1-ECD- mFc (SEQ ID NO:8) (Figure 12D). Specifically, these findings indicate inhibition of epitope spreading and thus support the notion that C1ORF32-P8--V1-ECD- mFc (SEQ ID NO:8) treatment leads to induction of tolerance.

Furthermore, C1ORF32-P8-V1-ECD- mFc (SEQ ID NO:8) treatment inhibited Th1/Th17 responses to PLP178-191, which was accompanied by promotion of Th2 responses and by up-regulation of the anti-inflammatory IL-10 cytokine (Figure 12E). These results are in high correlation with in-vitro effects on Thl/Thl7 and Th2 responses observed upon treatment of T-cells with C1ORF32-P8-ECD- mFc (SEQ ID NO:8) in previous studies (see Example 5).

C1ORF32-P8-V1-ECD- mFc (SEQ ID NO:8) treatment also inhibited lymph node cells recall responses, as manifested in cell proliferation in response to the inducing epitope (PLP139-151) (Figure 12F). The absence of recall responses to spread epitopes by lymph node cells as opposed to splenocytes is to be expected, since splenocytes better reflect the response within the CNS as compared to cervical lymph node cells.

Overall but without wishing to be limited by a single hypothesis, these results indicate that C1ORF32-P8-V1-ECD- mFc (SEQ ID NO:8) exerts its beneficial effects by interfering with the transmigration of encephalitogenic T cells into the CNS, as well as via immune modulation, whereby activation and differentiation of Th1/Th17 cells specific for myelin-associated epitopes is inhibited, while Th2 responses are promoted, limiting the expansion and CNS infiltration of autoreactive Th1 cells. Tolerance induction is suggested by the inhibition of epitope spreading, and is also evident in the long term amelioration of disease symptoms and abolishment of relapses following short term administration of C1ORF32-P8-V1-ECD- mFc (SEQ ID NO:8), as observed in this and in previous studies.

### Study B - Dose response analysis of C1ORF32-P8-V1-ECD- mFc (SEQ ID NO:8) IN R-EAE and its effect on in vivo and ex vivo recall responses to spread epitopes

### Purpose:

To further study the therapeutic effect of C10RF32-P8-V1-ECD- mFc (SEQ ID NO:8) at a wider dose range (10-100 ug/mouse) and to further establish the observed effect of C1ORF32-P8-V1-ECD- mFc (SEQ ID NO:8) on tolerance induction.

In this study, tolerance induction was studied in vivo using DTH (delayed type hypersensitivity) response to the inducing peptide (PLP139-151), and to spread epitope peptides (PLP178-191 and MBP84-104), at the peak of the first relapse (day 35) and 3 weeks after last treatment, a time point in which C1ORF32-P8-V1-ECD- mFc (SEQ ID NO:8) is expected to be cleared and no longer present in the circulation of mice (day 65). Tolerance was also studied ex vivo using recall responses of total splenocytes and lymph node cells to spread epitopes.

### Method:

Female SJL mice 6 weeks old were purchased from Harlan and maintained in the CCM facility for 1 week prior to beginning the experiment. Mice were randomly assigned into groups as described below, and primed with 50µg PLP139-151/CFA on day 0. Mice received 6 i.p. injections, 3 times per week for 2 weeks, of C10RF32-P8-V1-ECD- mFc (SEQ ID NO:8) or mIgG2a isotype control. Mice were treated at the onset of disease remission, every other day and were followed for disease over a period of 65 days.

On day 35 (during relapse peak, when histological damage should be high), 5 mice from groups treated with either C1ORF32-P8-V1-ECD- mFc (SEQ ID NO:8) (30 or 100µg) or isotype control were assayed for a DTH response to the inducing peptide PLP139-151 and the spread epitope peptide PLP178-191 via injection of 10ug of PLP139-151 in one ear and PLP178-191 into the opposite ear. The level of ear swelling was assayed at 24 hours post challenge. After assaying DTH, mice were sacrificed and spleens and cervical lymph nodes collected for ex vivo recall responses to PLP139-151 and PLP178-191, assaying total cellular proliferation.

Three weeks after the day of last treatment, 5 representative mice from each group were chosen for DTH response to the spread epitope peptide PLP178-191 and to the later spread epitope, MBP84-104, via injection of 10ug of PLP178-191 in one ear and MBP84-104 into the opposite ear. The level of ear swelling was assayed at 24 hours post challenge.

**Groups:**

| | | | |
|---|---|---|---|
| Group 1: | Control Ig (mIgG2a) | 100µg/dose | n=15 |
| Group 2: | C1ORF32-P8-V1-ECD- mFc (SEQ ID NO:8) | 10µg/dose | n=10 |
| Group 3: | C1ORF32-P8-V1-ECD- mFc (SEQ ID NO:8) | 30µg/dose | n=15 |
| Group 4: | C1ORF32-P8-V1-ECD- mFc (SEQ ID NO:8) | 100µg/dose | n=15 |

### Results:

The present Example shows a pronounced decrease in disease severity of R-EAE-induced mice upon C1ORF32-P8-V1-ECD- mFc (SEQ ID NO:8) treatment in a therapeutic mode with 30 and 100ug/dose at 3 times per week, while a lower efficacy was observed for the 10 µg/dose as shown in Figure 13A.

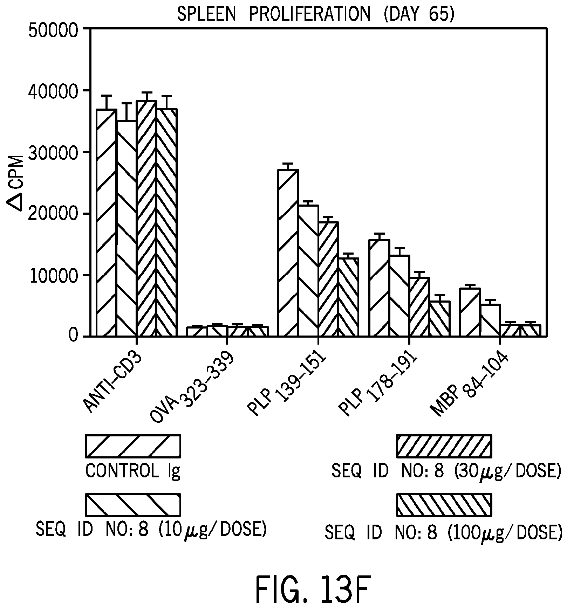

C1ORF32-P8-V1-ECD- mFc (SEQ ID NO:8) treatment of R-EAE mice dramatically inhibited DTH responses to the disease inducing (PLP139-151) and relapse-associated epitopes- PLP178-191 and MBP84-104 at day 35 and 65 (Figures 13B and 13F, respectively).

In addition, C1ORF32-P8-V1-ECD- mFc (SEQ ID NO:8) treatment resulted in reduction of in vitro recall responses on both day 35 and 65. Proliferation of day 35 lymph node cells was inhibited in response to PLP139-151 and to a lesser extent also to anti-CD3 (general activation) and PLP178-191 (Figure 13C). A more pronounced inhibition of proliferation was observed in day 35 splenocytes in response to anti-CD3, PLP139-151 and PLP178-191(Figure 13D). A dose dependent inhibition of proliferation also observed in day 65 splenocytes in response to PLP139-151, PLP178-191 and MBP84-104 (Figure 13F). Altogether, these results showing inhibition of epitope spreading are in corelation with the results described above in Study F and provide further support for induction of tolerance by C1ORF32-P8-V1-ECD- mFc (SEQ ID NO:8) via inhibition of myelin-specific T cell responses.

Additionally, in one study of PLP 139-151 -induced R-EAE in which Treg cells were functionally inactive following anti-CD25 treatment, CGEN-15001 treatment resulted in decrease in disease severity similarly to mice that were not treated with anti-CD25 (data not shown). In one separate EAE study, CGEN-15001 treatment beginning on Day 10 post disease induction with PLP 139-151 did not alter the number of Treg cells or their function as demonstrated by the ability of these cells to proliferate upon co-incubation with effector cells (data not shown). Further studies are carried out in order to fully elucidate the effect of CGEN-15001 on different subtypes of Tregs, and in various animal models.

### EXAMPLE 7 -EFFECT OF C1ORF32-P8-V1-ECD-mFc (SEQ ID NO:8) IN ADOPTIVE TRANSFER R-EAE MODEL

### PURPOSE:

To determine the effect of C10RF32-P8-V1-ECD- mFc (SEQ ID NO:8) treatment on the onset and severity of R-EAE upon transfer of highly activated cells harvested from R-EAE mice to healthy recipient mice, upon administration at the time of cell transfer.

### METHOD:

Donor and recipient female SJL mice (6 weeks old) were purchased from Harlan (Israel) and maintained in the CCM facility for 1 week prior to beginning the experiment. Donor mice were primed with PLP139-151/CFA on Day 0. Draining lymph nodes from donor mice were harvested on day 8 post priming, and total lymph node cells were activated ex vivo in the presence of PLP139-151 for 3 days. After culture cells were stained with PBSE and 10 recipient mice per group received 5×10⁶ blast cells via i.v. injection. At the time of cell transfer, mice were administered i.p. with either Control Ig or C1ORF32-P8-V1-ECD- mFc (SEQ ID NO:8) (100ug/dose, 3 times per week for two weeks, each). On day 14, five mice from each treatment group were scarified and the rest were followed for clinical score until day 30. Spleens, LN and CNS were analyzed for difference in total cell counts and trafficking of transferred cells (PBSE+).

### RESULTS:

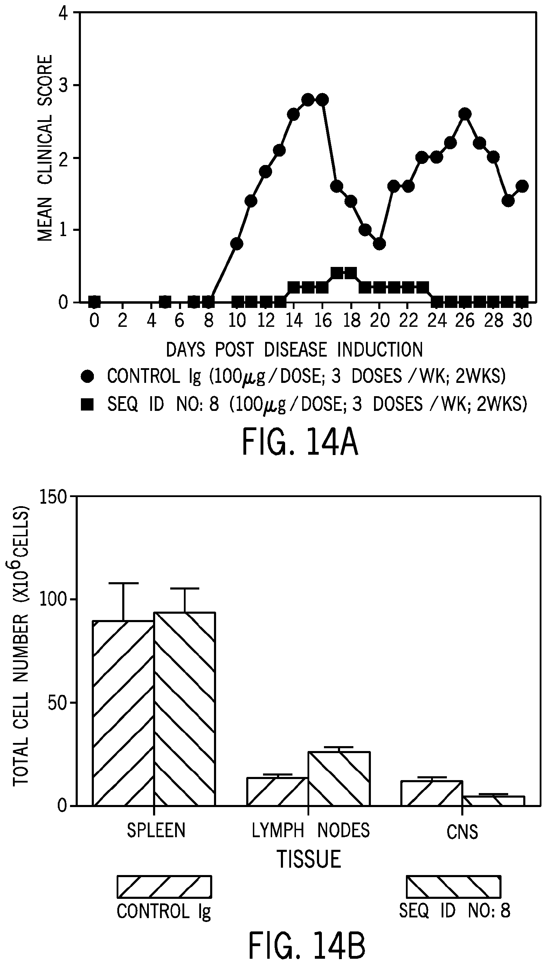

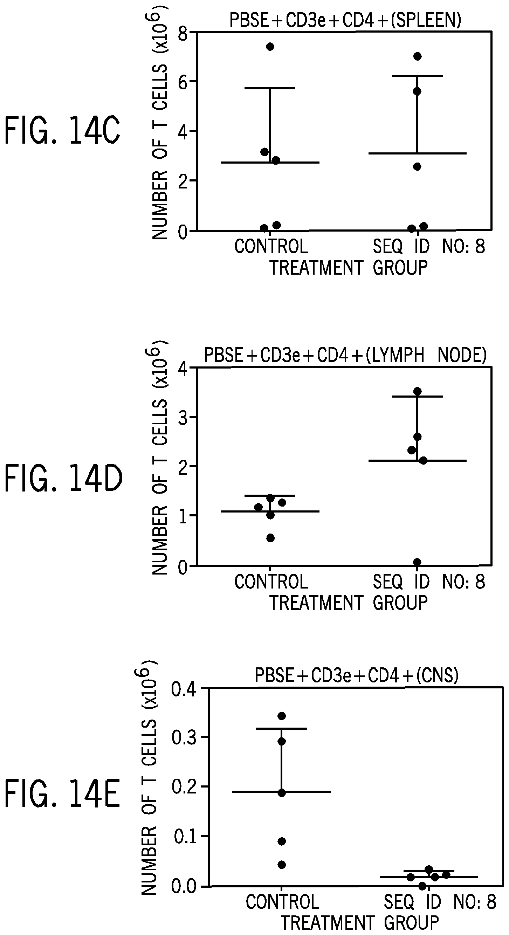

Administration of C1ORF32-P8-V1-ECD- mFc (SEQ ID NO:8) at time of cell transfer resulted in abrogation of disease development. As shown in Figure 14A, mice treated with control Ig developed a severe, relapsing remitting disease manifested by onset on day 10 and reaching pronounced disease score by day 14. C1ORF32-P8-V1-ECD- mFc (SEQ ID NO:8) treated mice were devoid of disease symptoms during the time of observasion (30 days). This abrogation of disease development was accompanied by a decrease in immune cell infiltration into the CNS (Figure 14B) particularily reduced trafficking of transferred autoreactive T cells to the CNS (Figure 14C).

These data are in high correlation with the therapeutic effect afforded by C1ORF32-P8-V1-ECD- mFc (SEQ ID NO:8) which was described in Example 4 and with the inhibition of infiltration of relapse associated encephalitogenic autoreactive T cells into the CNS as described on Example 6, and thus provide further support to the therapeutic capabilities of C1ORF32-P8-V1-ECD- mFc (SEQ ID NO:8) via tolerance induction.

### EXAMPLE 8 - EFFECT OF C1ORF32-P8-V1-ECD-mFc (SEQ ID NO:8) ON HUMAN T-CELL ACTIVATION

Study I- Activation of human T cells with anti-CD3 and anti-CD28-coated beads is inhibited by C1ORF32-P8-V1-ECD-mFc

The effect of C1ORF32-P8-V1-ECD-mFc (SEQ ID NO:8) on human T cell response was tested by two different in vitro assays using purified human T cells. In the first, human T cells were activated by anti-CD3 and anti-CD28 coated beads, and in the other activation was carried out using anti-CD3 and anti-CD28 antibodies in the presence of autologous, irradiated PBMCs. The regulatory activity of C1ORF32-P8-V1-ECD-mFc (SEQ ID NO:8) on human T cell activation, was evaluated by measuring cell proliferation and cytokine release.

### Materials and Methods

T cells were isolated from blood of healthy human donors, and activated *in-vitro* in the presence of beads coated with anti-CD3 and anti-CD28 antibodies. The effect of C1ORF32-P8-V1-ECD-mFc (SEQ ID NO:8) on T cell activation was evaluated by coating the beads in the presence of either C1ORF32-P8-V1-ECD-mFc (SEQ ID NO:8) or control Ig (as negative control). Cell proliferation and IPNγ release were measured after 72hr.

### Detailed Method

Human T cells were purified from whole blood by positive selection using microbeads conjugated to monoclonal anti-human CD3 antibodies (MACS Whole Blood CD3 Microbeads #130-090-874).

In the 'one step' coating method, Dynabeads (M-450 Epoxy Dynabeads, Invitrogen cat. No. 140.11) were coated with anti-CD3 *&* anti CD28 (0.5 & 2ug/ml) in the presence or absence of control Ig or C1ORF32-P8-V1-ECD-mFc (SEQ ID NO:8) (at the concentrations described in the figure). In the 'two step' coating method, Dynalbeads were first coated with anti-CD3 & anti-CD28 (at 0.5 *&* 2ug/ml), and then the Fc fused protein or controls were added.

Purified CD3 T cells were activated with anti-CD3+anti-CD28, coated beads. The cells were seeded at 2×10e5 per well in the presence or absence of CD3+CD28-coated beads, at 2:1 cells to bead ratio.

After 72 hours cell proliferation was measured by H3-thymidine incorporation and supernatants were collected and tested by ELISA for IFNγ levels.

### Results

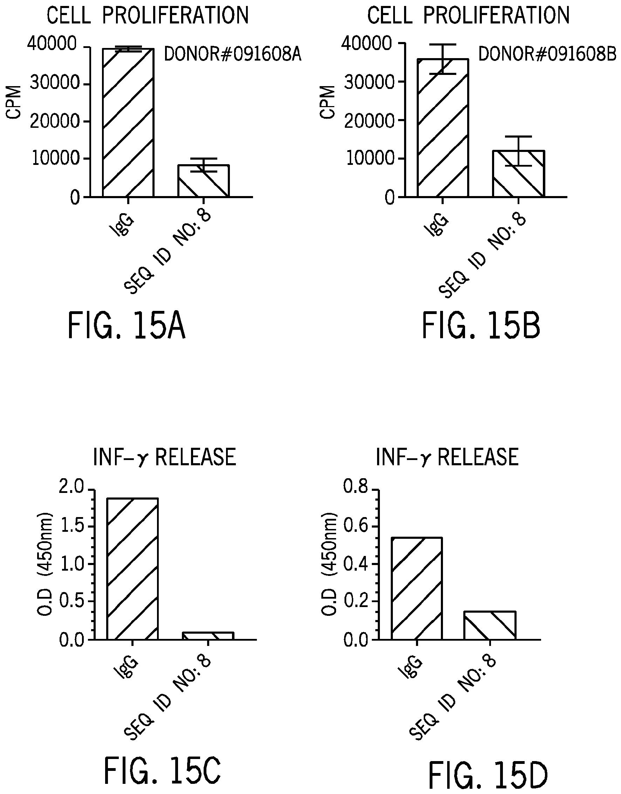

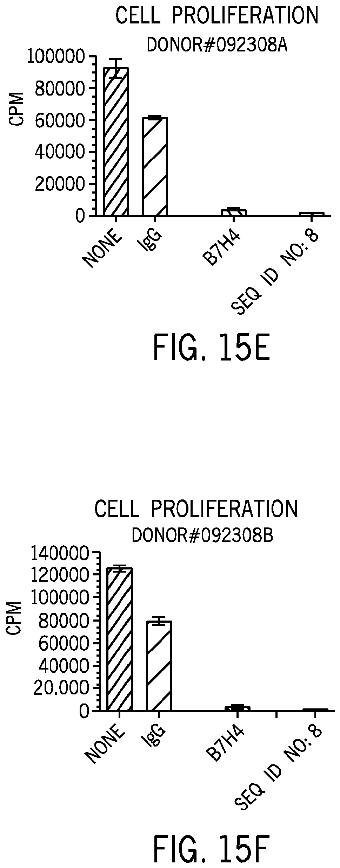

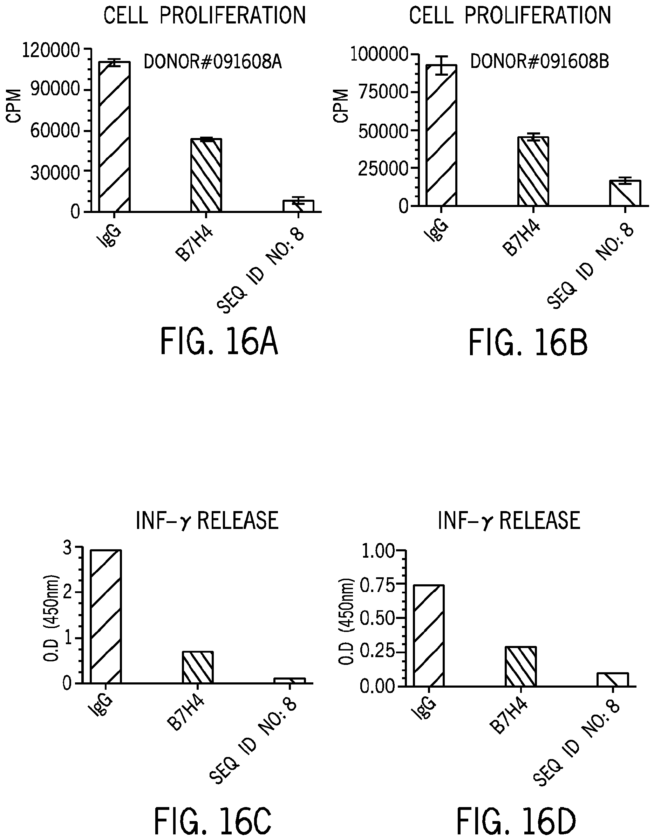

C1ORF32-P8-V1-ECD-mFc (SEQ ID NO:8) displays strong inhibitory effect on T cell activation, manifested in the reduction of cell proliferation and IPNγ secretion in T cells from various human donors, upon coating of beads in the one-step method (Figure 15) or the two-step method (Figure 16). Figures 15A and B show T cell proliferation and IPNγ production (respectively), from donors 091608A and 091608B, while Figure 15C shows T cell proliferation from donors 092308A and 092308B.

Figure 16 shows the results of using the two-step method. The cells were activated with Dynabeads coated in the 'two step' coating method, with anti-CD3 & anti-CD28 (0.5 and 2ug/ml, respectively), followed by either control IgG or C1ORF32-P8-V1-ECD-mFc (SEQ ID NO:8) (10micro-g/ml). As positive control, the known negative costimulator B7H4 was used. Figures 15A and B show T cell proliferation and IPNγ release (respectively) from donors 091608A and 091608B. The extent of this effect was somewhat variable in the different experiments, which might be due to donor variability.

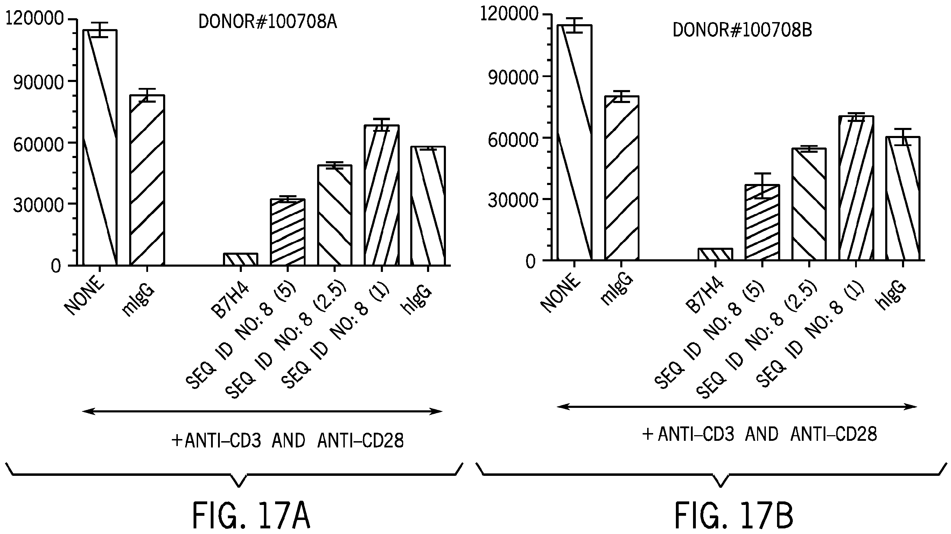

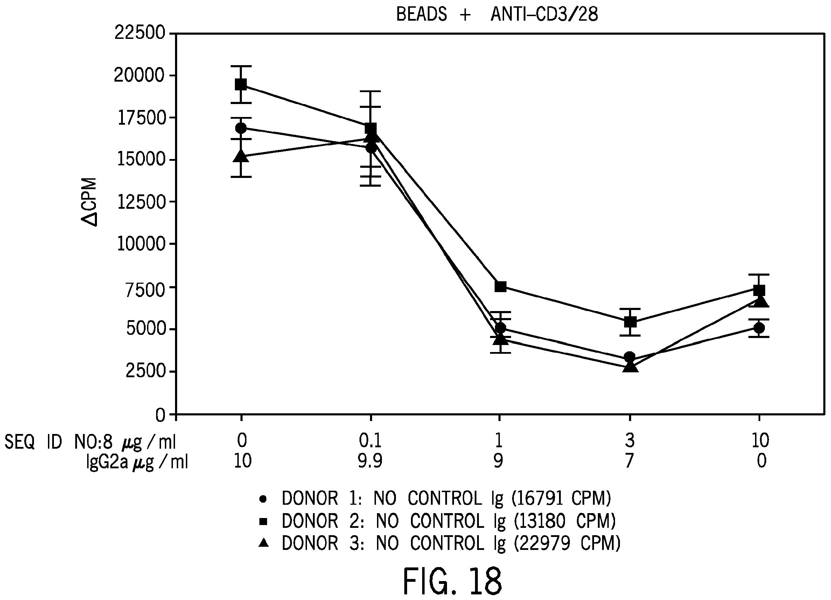

The inhibitory effect on human T cell proliferation was dose dependent as shown in Figure 17. The cells were activated with Dynabeads coated in the 'two step' coating method, with anti-CD3 & anti-CD28 (0.5 and 2ug/ml, respectively), followed by either control IgG or C1ORF32-P8-V1-ECD-mFc (SEQ ID NO:8) (at 1, 2.5 or 5 micro-g/ml). As positive control, known negative costimulator (B7-H4-Ig) was used. Mouse IgG was used as control for the Fc portion. Graphs show T cell proliferation from donors 100708A and 100708B. The effect of C1ORF32-P8-V1-ECD-mFc (SEQ ID NO:8), which appeared to be optimal at 10µg/ml, was comparable to that of B7-H4 a known inhibitory protein of the B7 family of costimulatory molecules. The dose dependent inhibition of T cells proliferation by C1ORF32-P8-V1-ECD- mFc (SEQ ID NO:8) was reproduced in a separate laboratory using similar methodology (Figure 18).

Without wishing to be limited by a single hypothesis, overall, these results, which were reproducible in cells taken from several donors, support the notion that C1ORF32-P8-V1-ECD-mFc (SEQ ID NO:8) is a negative costimulatory protein, and confirm that the inhibitory effects exhibited by C1ORF32-P8-V1-ECD-mFc (SEQ ID NO:8) are transduced via the ECD portion and not via the Fc portion.

### Study II- Activation of human T cells with irradiated autologous PBMCs is inhibited by C1ORF32-P8-V1-ECD-mFc

The effect of C1ORF32-P8-V1-ECD-mFc (SEQ ID NO:8) on human T cell activation was also tested using purified human T cells activated by autologous irradiated PBMCs with anti-CD3 and anti-CD28 antibodies. In this set up, the anti-CD3, anti-CD28 and either C1ORF32-P8-V1-ECD-mFc or control Ig are presented to the T cells by the irradiated PBMCs. The effect on activation was evaluated by measuring T cell proliferation via H³-thymidine incorporation.

### Method

Total PBMC was isolated from fresh blood of healthy human donors using ficoll gradient. 10×10⁶ total PBMCs were resuspended in Ex-Vivo 20 medium, and irradiated at 3000rad. These cells are to be used as total irradiated APCs to activated isolated T cells in vitro. The rest of PBMCs were used for T cells isolation via use of the CD4+ T cell Isolation Kit II from Miltenyi.

For activation, 5×10⁵ isolated T cells were cultured in the presence of 5×10⁵ autologous irradiate PBMCs Anti-CD3 (0.5µg/ml), and anti-CD28 (2 µg/ml) were added in a soluble form. The cultures were pulsed with luCi of triated thymidine at 24hrs, and proliferation was measured at 72 hours.

### Results

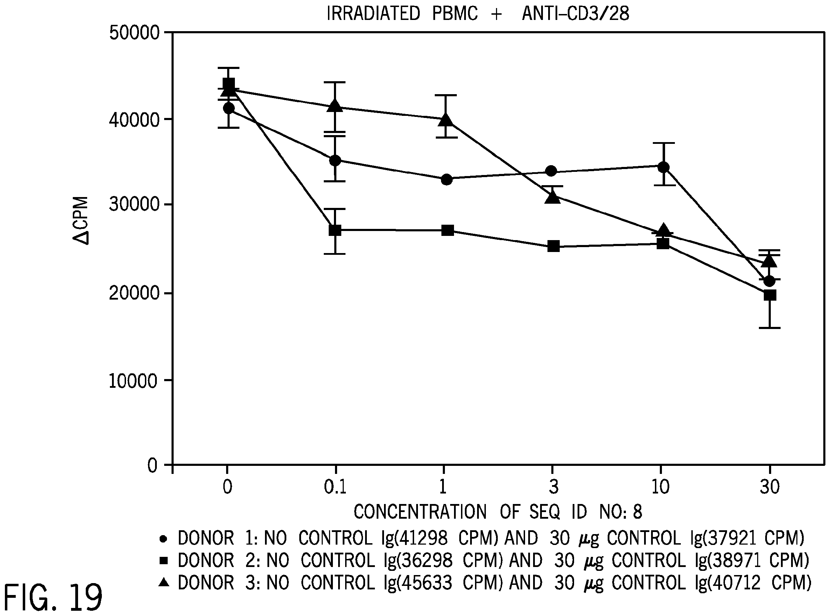

C1ORF32-P8-ECD- mFc (SEQ ID NO:8) inhibited proliferation of human T cells activated with anti-CD3 and anti-CD28 in the presence of autologous irradiated PBMCs (Figure 19) isolated from from various human donors.

The inhibitory effect of C1ORF32-P8-V1-ECD-mFc (SEQ ID NO:8) on T cell activation, manifested in reduction of T cell proliferation and release of the pro-inflammatory cytokine IFNγ, is similar to that of other negative costimulatory B7 proteins. Without wishing to be limited by a single hypothesis, these observations support the possibility that C1ORF32-P8-V1-ECD-mFc (SEQ ID NO:8) belongs to the B7 family. Furthermore, the inhibitory activity of C1ORF32-P8-V1-ECD-mFc (SEQ ID NO:8) supports its potential as an anti-inflammatory agent.

### EXAMPLE 9 - THERAPEUTIC EFFECT OF C1ORF32-P8-V1-ECD-mFc (SEQ ID NO:8) IN COLLAGEN INDUCED ARTHRITIS (CIA) MODEL OF RHEMATOID ARTHRITIS

The therapeutic potential of C1ORF32-P8-V1-ECD- mFc (SEQ ID NO:8) for rheumatoid arthritis was studied using the mouse CIA model.

### Method

Arthritis was induced in male DBA/1 mice by immunisation with type II collagen emulsified in complete Freund's adjuvant. Mice were monitored on a daily basis for signs of arthritis and treatment with C10RF32-P8-V1-ECD- mFc (SEQ ID NO:8) will be initiated on day 1 of arthritis and continued for 10 days. 8-10 mice were included per treatment group as described below in Table 11:

**Table 11 - treatment groups**

| Group | Treatment | Time of treatment |
|---|---|---|
| 1 | C1ORF32-P8-V1-ECD- mFc (SEQ ID NO:8) (100 µg/mouse) | 3 times/week |
| 2 | control IgG2a (100 µg/mouse) | 3 times/week |
| 3 | PBS | 3 times/week |
| 4 | Enbrel (100 µg/mouse) | 3 times/week |

Hind footpad swelling (using microcalipers), as well as the number and degree of joint involvement in all four limbs were routinely measured. Joints were scored as follows: 0-normal joint; 1-slight swelling and/or erythema; 2-pronounced swelling; 3- joint stiffness.

### Results:

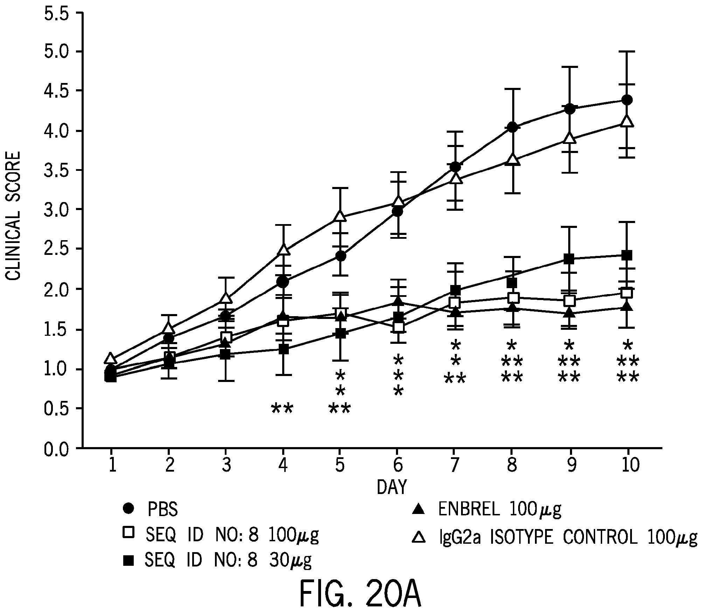

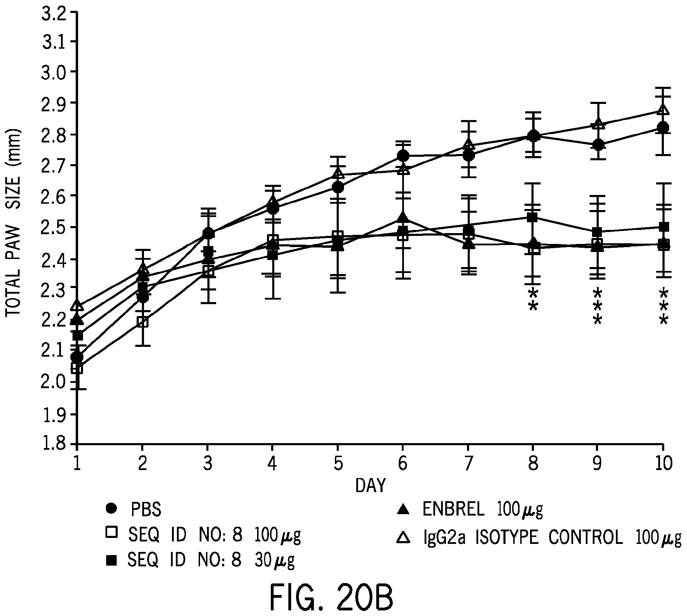

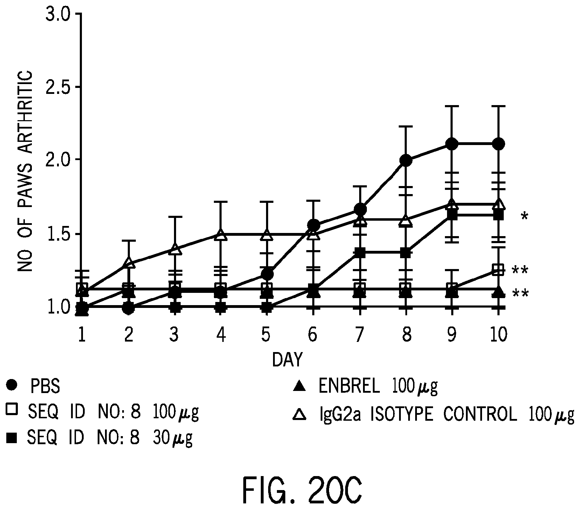

Treatment of mice with established CIA with C1ORF32-P8-V1-ECD- mFc (SEQ ID NO:8) 3 times / week for 10 days resulted in potent reduction of clinical score (Figure 20A) and paw swelling (Figure 20B) at both 100 and 30 ug/doses. Furthermore, C1ORF32-P8-V1-ECD-mFc (SEQ ID NO:8) treatment inhibited spread of disease as manifested in number of paws developing arthritis (Figure 20C). The efficacy of C1ORF32-P8-V1-ECD- mFc (SEQ ID NO:8) was similar to that obtained with Enbrel (TNFalphaR-Ig, etanercept), which served as a positive control in this study (Figure 20).

### EXAMPLE 10-DETERMINE THE EFFECT OF C1ORF32-P8-V1-ECD-MFC (SEQ ID NO:8) ON B CELLS CLASS-SWITCH AND ANTIBODY SECRETION.

Resting B cells are isolated from unprimed C57BL/6 mice and activated in vitro in the presence of anti-CD40 plus either no exogenous cytokine, IL-4, or IFN-γ. The cell cultures receive Control Ig (mIgG2a), anti-CD86 mAb (as a positive control for increased Ig production), or C1ORF32-P8-V1-ECD- mFc (SEQ ID NO:8) at the time of culture set up, and are cultured for 5 days. C1ORF32-P8-V1-ECD- mFc (SEQ ID NO:8) are tested at three concentrations. At the end of culture, supernatants are tested for the presence of IgM, IgG1, and IgG2a via ELISA. mFcIf there appears to be an alteration in the ability of the B cells to class-switch to one isotype of antibody versus another, then the number of B cells that have class switched is determined via ELISPOT. If there is an alteration in the number of antibody producing cells, then it can also be determined if there is an alteration in the level of γ1- and y2a-sterile transcripts versus the mature transcripts for IgG1 and IgG2a. C1ORF32-P8-V1-ECD- mFc (SEQ ID NO:8) is expected to show an inhibitory effect on B cell activation, which could be demonstrated for example by reduction in class switching and/or antibody secretion.

### EXAMPLE 11-

### A- DETERMINE THE EFFECTS OF C1ORF32-P8-V1-ECD-MFC (SEQ ID NO:8) ON DIFFERENTIATION OF HUMAN CD4+ T CELLS USING THE FC-FUSED C1ORF32-P8-ECD- MFC (SEQ ID NO:8)

Naïve CD4+ T cells are isolated from 5 human donors. Naive CD4+ cells are activated in the presence of Th0 cell- (IL-2), Th1 cell- (IL-2 + IL-12), Th2 cell- (IL-2 + IL-4), or Th17 cell- (TGF-Ø + IL-1β + IL-6 + IL-23 + IL-1β + anti-IL-2) promoting conditions. To activate the CD4+ T cells, the cells are cultured in the presence of anti-CD3 mAb/anti-CD28 mAb coated beads in the absence or presence of C1ORF32-P8-V1-ECD-mFc (SEQ ID NO:8), B7-H4-Ig or mIgG2a isotype control. Two side-by-side culture sets are set up; one culture being pulsed at 24 hours with tritiated-thymidine and harvested at 72 hours while the second plate is harvested at 96 hours for cytokine production. IL-2, IL-4, IL-5, IL-10, IL-12, IL-17, IFN-γ, and TNF-α are tested via LiquiChip. C1ORF32-P8-V1-ECD- mFc (SEQ ID NO:8) mFc reduces the responses of the Th1 and Th17 lineages and promotes the Th2 lineage responses, as was observed using murine T cells.

### B- DETERMINE THE EFFECTS OF C10RF32 ON ACTIVATION AND DIFFERENTIATION OF HUMAN CD4+ T CELLS USING T CELL STIMULATOR CELLS EXPRESSING C1ORF32 ON THEIR SURFACE.

For the assessment of the functional role of C10RF32 molecules, 'T cell stimulator cells' are used, expressing high levels of two variants of C1ORF32 ((SEQ ID NO:3) and (SEQ ID NO:5)) molecules or appropriate control molecules.

T cell stimulator cells are based on murine thymoma line cells, Bw5147, which were engineered to express membrane-bound anti-human CD3 antibody fragments. They can trigger the TCR-complex on human T cells thereby generating Signal 1. Upon expression of putative costimulatory or coinhibitory ligands on these cells, their role during human T cell activation can readily be evaluated.

### Method

### 1. The effect of C1ORF32 on human T cell activation

T cell stimulator cells expressing high levels of C1ORF32 molecules are generated by cloning cDNA of C1ORF32 into a retroviral vector. Expression of C1ORF32 is validated using a specific antibody or using a bi-cistronic vector encoding green fluorescent protein (GFP) downstream of these molecules. Control stimulator cells expressing activating costimulatory ligands (e.g. CD80, CD58 or 4-1BBL) or inhibitory costimulatory molecules (e.g B7-H3 or PD-L2) are produced in parallel as well as cells expressing neither activating nor inhibitory human costimulatory molecules (empty vector and/or GFP T cell stimulator cells).

Primary human T cells as well as T cell subsets e.g CD4 or CD8 cells or naive or memory (antigen experienced) T cells, regulatory T cells and MNCs are purified from fresh blood taken from healthy volunteer donors by sorting (MACS).

T cells are activated for different periods of time and analysed for T cell proliferation (³H-Thymidine incorporation and CFSE labelling in conjunction with FACS analysis) and cytokine production. MNCs are analysed by LUMINEX-based multiplex assay focusing on secretion of IFN-gamma, IL-2, IL-4, IL-10, IL-13 and IL-17. C1ORF32-P8-V1-ECD- mFc (SEQ ID NO:8) shows a reduction in IFN-gamma, IL-2, and IL-17 secretion. C1ORF32-P8-V1-ECD- mFc (SEQ ID NO:8) shows an increase in IL-4, IL-13 and IL-10, in accordance with the promotion of IL-10 and Th-2 responses.

In addition, the effects of C10RF32 on T cell activation that receive stronger stimuli provided by exogenous cytokines (e.g. rhIL-2 or IL-15) or costimulatory signals (e.g. upon addition of stimulating anti-CD28 antibodies at different concentrations) are being studied.

### 2. The effect of CIORF32 on human T cell differentiation

Naive human CD4 T cells are stimulated under conditions that promote differentiation towards a Th1, Th2 or Thl7 phenotype. The effects of C1ORF32 molecules are evaluated using T cell stimulator cells expressing C1ORF32 molecules or control stimulator cells to provide Signal 1. The phenotype of the resulting T helper cells is evaluated by cytokine measurement in the culture supernatants and by FACS analysis of permeabilized T cells, and by measuring the expression of lineage specific transcription factors (T-bet, GATA-3, and RORγt-) by qPCR. T cell stimulator cells expressing C1ORF32 molecules induce Th2 related cytokines and transctiption factors and reduce Th1 and/or Th17- related cytokines and transcription factors.

### EXAMPLE 12- DETERMINE THE MECHANISM OF ACTION UNDERLYING THE EFFICACY OF C1ORF32-P8-V1-ECD-MFC (SEQ ID NO:8) IN CIA IN A DOSE DEPENDENT MANNER.

It was previously shown that C1ORF32-P8-V1-ECD-mFc (SEQ ID NO:8) reduces disease symptoms in the CIA model upon treatment with 100 or 30 µg/mouse dose. In this Example the range of therapeutic doses administered is widened (10-100µg/mouse), and the mechanism of action of C1ORF32-P8-V1-ECD-mFc (SEQ ID NO:8) paying particular attention to events within the joints is studied.

Treatment groups (n=8-10)
1. C1ORF32-P8-V1-ECD-mFc (SEQ ID NO:8), 100 µg/mouse
2. C1ORF32-P8-V1-ECD-mFc (SEQ ID NO:8), 30 µg/mouse
3. C1ORF32-P8-V1-ECD-mFc (SEQ ID NO:8), 10 µg/mouse
4. Control IgG2a, 100 µg/mouse

On day 10 post onset, a proportion of the C1ORF32-P8-V1-ECD-mFc (SEQ ID NO:8) treated mice is bled, and the inguinal lymph nodes and affected joints are removed.

Cells from the blood, lymph nodes, and joints are stimulated in vitro for analysis of T cell intracellular cytokine expression by flow cytometry.

The blood, joints and lymph nodes are also examined to ascertain numbers of CD4+ and CD8+ regulatory T cells (FOXP3 expression). Anti-collagen antibody levels are measured by ELISA.

To understand changes in gene expression during therapy with C1ORF32-P8-V1-ECD-mFc (SEQ ID NO:8), joints not used for cell population analysis are homogenised and gene expression changes are measured by qPCR.

Lymph node cells are also cultured in the presence or absence of type II collagen to quantify changes in antigen-stimulated cytokine expression following therapy by ELISA.

Recent research has shown that regulatory T cells are defective in RA and anti-TNF therapy restores their function. After further assessment of the numbers of regulatory cells after treatment (see above), the suppressive effects of these regulatory T cells is assayed. In brief, graded numbers of FoxP3+ cells from C1ORF32-P8-V1-ECD-mFc (SEQ ID NO:8) treated or untreated mice are co-cultured with FoxP3- effector T cells from immunised mice plus APC. The cultures are then stimulated with anti-CD3 or collagen and proliferation responses of the effector cells measured.

Disease symptoms decline upon C1ORF32-P8-V1-ECD-mFc (SEQ ID NO:8) treatment with at least one of the above described doses. Without being limited to a single hypothesis, this effect is accompanied by one or more of reduction in histological damage to bone and/ or cartilage, and decrease in the Th1 related IgG2a anti collagen II antibodies.

### EXAMPLE 13- DETERMINE LONG TERM EFFICACY OF C1ORF32-P8-V1-ECD-MFC (SEQ ID NO:8) IN CHRONIC CIA MODEL

C57BL/6 mice are treated from onset of disease with C1ORF32-P8-V1-ECD-mFc (SEQ ID NO:8), control IgG2a or Enbrel with 3 doses as in previous studies, in groups of 8-10 mice. At day 10, no further treatment is given and the mice are continuously monitored for 20-30 days in order to establish the time taken for the disease to flare again. This assesses the efficacy of C1ORF32-P8-V1-ECD-mFc (SEQ ID NO:8) in the chronic CIA model and the duration of its biological effect mFcin rheumatoid arthritis. Long term efficacy is observed in this model. Without being bound by a single hypothesis, a decrease in disease severity is accompanied by decrease in anti-collagen antibody levels as measured for example by ELISA.

### EXAMPLE 14- EFFECT ON TOLERANCE INDUCTION BY C1ORF32-P8-V1-ECD-MFC (SEQ ID NO:8) IN TRANSFER MODEL OF CIA

To further understand the effect of C1ORF32-P8-V1-ECD- mFc (SEQ ID NO:8) on immune regulation, the ability of C1ORF32-P8-V1-ECD-mFc (SEQ ID NO:8) to induce tolerance in a transfer model of arthritis is analysed.

In brief, spleen and LN cells from arthritic DBA/1 mice treated for 10 days with C1ORF32-P8-V1-ECD- mFc (SEQ ID NO:8) or control Ig2a are removed and injected i.p into T-cell deficient C.B-17 SCID recipients. The mice then receive an injection of 100 µg type II collagen (without CFA), necessary for successful transfer of arthritis. Arthritis is then monitored in the SCID mice; it is determined that the C1ORF32-P8-V1-ECD- mFc (SEQ ID NO:8) treatment confers long-term disease protection. Histology is performed and anti-collagen antibody levels are measured to support this determination.

### EXAMPLE 15- THE EFFECT OF C10RF32-P8-V1-ECD- MFC (SEQ ID NO:8) IN MODULATION OF TYPE 1 DIABETES IN NOD MICE, CD28-KO NOD, AND B7-2-KO NOD

The effect of C1ORF32-P8-V1-ECD- mFc (SEQ ID NO:8) is studied in mouse models of type 1 diabetes using NOD mice as well as two KO mice: CD-28-KO NOD mice - providing CD28-independent model of autoimmune disease and immunity which develop accelerated diabetes, and B7-2KO NOD mice that develop peripheral neuropathy.

These mice are treated with C1ORF32-P8-V1-ECD-mFc (SEQ ID NO:8) before or after disease onset to examine the effects of these compounds on disease pathogenesis and to demonstrate that such treatment reduces disease onset and ameliorates pathogenesis. Upon effect of the tested compounds, the mechanism of disease modification is studied by examination of individual immune cell types (including Tregs, Th cells and CD8 T cells, DCs and B cells); cytokines (Thl and Th2, IL-10 and TGFb) and histology. To study the effect of C1ORF32-P8-V1-ECD-mFc (SEQ ID NO:8) treatment on Insulitis, blood glucose levels are measured 3 times/ week, for up to 25 weeks (Fife et al., J Exp Med. 2006 27;203(12):2737-47).

Mode of action is studied by experimental evaluation of individual immune cell types: Pancreas, pancreatic LN and spleen will be harvested to obtain Tregs, Th cells and CD8 T cells, DCs and B cells. Effect on cytokines secretion from cells isolated from pancreas, pancreatic LN and spleen is analysed. Histologycal analysis is carried out on multiple 5-µm sections from pancreases, stained with H&E. Sections are scored for severity of insulitis as follows:.
- Peri-Insulitis - lymphocytes surrounding, but not infiltrating the architecture of the islets;
- Moderate insulitis-f less than half of the islet architecture is infiltrated with lymphocytes;
- Severe insulitis if more than half of the islet architecture is infiltrated with lymphocytes.

### EXAMPLE 16- THE EFFECT OF C1ORF32-P8-V1-ECD-MFC (SEQ ID NO:8) IN MODULATION OF TYPE 1 DIABETES IN ADOPTIVE TRANSFER MODEL

Diabetes is induced by the transfer of activated CD4+CD62L+CD25- BDC2.5 T cells (transgenic for TCR recognizing islet specific peptide 1040-p31 activated by incubation with 1040-p31) to NOD recipients. Mice are treated with C1ORF32-P8-V1-ECD-mFc (SEQ ID NO:8), control IgG2a or positive control. Treatments begin 1 day following transfer. Mice are followed for glucose levels 10-28 days post transfer (Bour-Jordan et al., J Clin Invest. 2004;114(7):979-87 and Fife et al., J Exp Med. 2006 Nov 27;203(12):2737-47).

Study 1:
The BDC2.5 T cells are labeled with CFSE before transfer for assessment of in vivo proliferation of these cells in the spleen, LN and pancreatic LNs.

Study 2:
Seven days post treatment pancreas, spleen, pancreatic LN and peripheral lymph node cells are extracted and examined for different immune cell populations. In addition, recall responses are measured by testing ex-vivo proliferation and cytokine secretion in response to p31 peptide.

Both studies show that C1ORF32-P8-V1-ECD-mFc prevents or reduces disease onset or the severity thereof.

### EXAMPLE 17- THE EFFECT OF C1ORF32-P8-V1-ECD-MFC (SEQ ID NO:8) in MRL/LPR LUPUS MOUSE MODEL

### Materials and Methods

MRL/lpr mice at 4 weeks of age are used in this experiment. Cyclophosphamide (CTX) is the primary drug used for diffuse proliferative glomerulonephritis in patients with renal lupus, Daikh and Wofsy reported that combination treatment with CTX and CTLA4-Ig was more effective than either agent alone in reducing renal disease and prolonging survival of NZB/NZW F1 lupus mice with advanced nephritis (Daikh and Wofsy, J Immunol, 166(5):2913-6 (2001)). In the proof-of-concept study, treatments with C1ORF32-P8-V1-ECD-mFc (SEQ ID NO:8) and CTX either alone or in combination are tested.

Blood samples are collected from the submandibular vein 3 days before the protein treatment and then every other week during and after treatments for plasma anti-dsDNA autoantibody analysis by ELISA. Glomerulonephritis is evaluated by histological analysis. For this, mouse kidneys are harvested and fixed in 10% formalin. Sections are stained via standard H&E staining. Proteinuria is measured by testing fresh urine samples using urinalysis dipsticks.

C1ORF32-P8-V1-ECD-mFc has a beneficial effect in at least ameliorating renal lupus.

### EXAMPLE 18- THE EFFECT OF C1ORF32-P8-V1-ECD-MFC (SEQ ID NO:8) IN ADOPTIVE TRANSFER MOUSE MODEL OF INFLAMMATORY BOWEL DISEASE

SCID mice are reconstituted by i.p. injection of syngeneic CD45RB^{high}-CD4⁺ T cells either alone or cotransferred with syngeneic CD45RB^{low}-CD4⁺ or CD25⁺CD4⁺ cells (4 × 10⁵/mouse of each cell population). Colitic SCID mice, reconstituted with syngeneic CD45RB^{high}CD4⁺ T cells from spleen of normal mice, are treated i.p. with either C1ORF32-P8-V1-ECD-mFc (SEQ ID NO:8) or Ig isotype control or positive control, twice a week starting at the beginning of T cell transfer up to 8 wk. All mice are monitored weekly for weight, soft stool or diarrhea, and rectal prolapse. All mice are sacrificed 8 wk after T cell transfer or when they exhibite a loss of.20% of original body weight. Colonic tissues are collected for histologic and cytologic examinations (Liu et al., J Immunol. 2001; 167(3): 1830-8). C1ORF32-P8-V1-ECD-mFc has a beneficial effect in at least ameliorating inflammatory bowel disease.

### EXAMPLE 19- THE EFFECT OF C1ORF32-P8-V1-ECD-MFc (SEQ ID NO:8) IN MOUSE MODELS OF PSORIASIS

### STUDY I: ESTABLISHMENT OF PSORIASIS SCID XENOGRAFT MODEL.

Human psoriasis plaques are transplanted on to the SCID mice. Shave biopsies (2.5_2.5 cm) are taken from patients with generalized plaque psoriasis involving 5―10% of the total skin that did not receive any systemic treatment for psoriasis or phototherapy for 6 months and did not receive any topical preparations other than emollients for 6 weeks. The biopsies are obtained from active plaques located on the thigh or arm. Each piece of biopsy is divided into four equal parts of approximately 1 cm2 size. Each piece is transplanted to a separate mouse.

Under general anesthesia, a graft bed of approximately 1 cm2 is created on the shaved area of the back of a 7- to 8-week-old CB17 SCID mouse by removing a full-thickness skin sample, keeping the vessel plexus intact on the fascia covering the underlying back muscles. The partial thickness human skin obtained by shave biopsy is then orthotopically transferred onto the graft bed. Nexaband, a liquid veterinary
bandage (Veterinary Products Laboratories, Phoenix, AZ) is used to attach the human skin to the mouse skin and an antibiotic ointment (bacitracin) is applied. Mice are treated intraperitoneally three times per week for 4 weeks with C1ORF32-P8-V1-ECD-mFc (SEQ ID NO:8), isotype control or CTLA4-Ig (positive control).

Punch biopsies (2 mm) are obtained on day 0 (before treatment) and day 28 (after treatment) of the study period. Biopsies are snap frozen and cryosections for histopathological and immunohistochemical studies. Therapeutic efficacy is determined by comparing pre- and post treatment data: (i) rete peg lengths to determine the effect on epidermal thickness and (ii) the level of lymphomononuclear cell infiltrates to determine the effect on inflammatory cellular infiltrates. (Raychaudhuri et al. 2008, J Invest Dermatol.; 128(8): 1969-76; Boehncke et al., 1999 Arch Dermatol Res 291:104-6).

C1ORF32-P8-V1-ECD-mFc has a beneficial effect in at least ameliorating psoriasis.

### STUDY II: PSORIASIS AND COLITIS MODEL BY ADOPTIVE TRANSFER OF CD45RBHI CD4+ T CELLS IN SCID MICE

Immunocompromised mice are injected intraveneously (i.v.) with 0.3_10⁶ CD4 + CD45RBhi cells. On the day following the adoptive transfer of cells,
mice are injected intraperitoneally (i.p.) with 10 microg of staphylococcal enterotoxin B. Recipient mice are treated with with C1ORF32-P8-V1-ECD-mFc (SEQ ID NO:8), isotype control or CTLA4-Ig (positive control). Mice are evaluated once a week for 8 weeks for weight loss and presence of skin lesions.

Obtained results are similar to those described above.

### EXAMPLE 20-EFFECT OF C1ORF32-P8-V1-ECD-mFc (SEQ ID NO:8) IN TRANS VIVO DELAYED TYPE HYPERSENSITIVITY (DTH) ASSAY

This experiment is performed to determine the effect of C1ORF32-P8-V1-ECD-mFc (SEQ ID NO:8) treatment on T cell responses and transplant rejection. Human allograft acceptance is associated with immune regulation, characterized by donor-antigen-linked suppression of delayed-type hypersensitivity (DTH). The 'trans-vivo' DTH is a mouse model for testing compounds that can affect allograft acceptance using human cells. This model can assess donor-reactive cell-mediated immune responses. The response requires presentation of tetanus toxoid antigen by human antigen presenting cells to human T cells injected into mouse foot pads. The relevance of utilizing a delayed type hypersensitivity protocol is that this reaction is regarded as a hallmark of Th1 mediated autoimmune diseases and transplant rejection.

### Methods:

### Animals

Female C57BL/6 mice were purchased from Harlan Sprague Dawley, Inc. (Indianapolis, Indiana) at 6-8 weeks of age and used within four weeks of arrival.

### Isolation of human peripheral blood mononuclear cells (PBMCs)

Blood was collected by venipuncture from normal human donors that are known to be good tetanus responders. One hundred ml whole blood was drawn into CPT Vacutainer tubes and centrifuged at 1800 RCF for 30 min. The buffy layer containing mononuclear cells and platelets was separated, washed three times, and resuspended in phosphate-buffered saline (PBS) and counted. Platelet contamination was minimized by multiple washes in PBS. No more than a 1:1 ratio of platelets to PBMCs was allowed. The cells were immediately injected into the mouse footpads.

### Tetanus toxoid

Aluminum phosphate-adsorbed Tetanus toxoid (TT-Tetguard, BI-Vetmedica, Inc. St. Joseph, MO) was used at a concentration of 0.25 Lf per injection site (Lf unit is the flocculation value, the amount of toxoid which when mixed with one International Unit of antitoxin produces an optimal flocculating mixture).

### DTH

7-10 X10⁶ PBMCs mixed with 0.25 Lf units of TT in a total volume of 50 micro-liter, was injected into the hind footpads of mice. Footpad thickness was measured prior to injection and 24 hours post-injection, using a dial thickness gauge (Mitutoyo, Aurora, IL). Pre-injection thickness was subtracted from post-injection thickness to obtain the change in paw thickness. All measurements were made in inches.

### Testing of C1ORF32-P8-V1-ECD-mFc (SEQ ID NO:8) Compounds

C1ORF32-P8-V1-ECD-mFc (SEQ ID NO:8) was administered to mice at a dosage of 1.5 and 5 mg per kg by intraperitoneal administration, 2-3 hrs before footpad injections with PBMCs with or without TT. Each dose of compound was tested on PBMCs from four different donors and two mice per treatment per donor were used. FK506 was used as a positive control and mIgG2a was used as a negative control.

### Results:

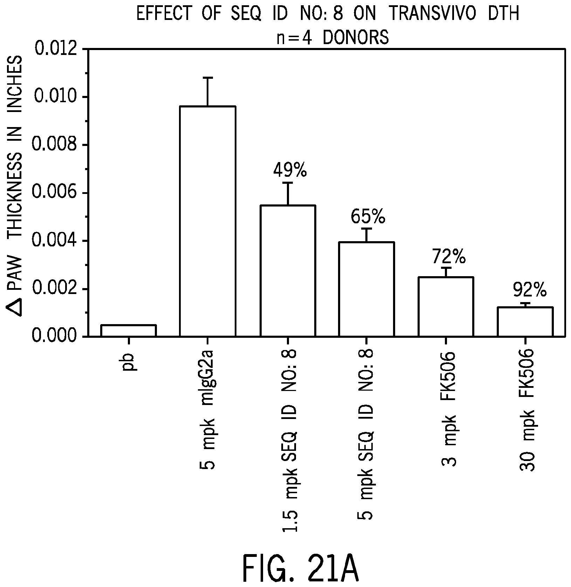

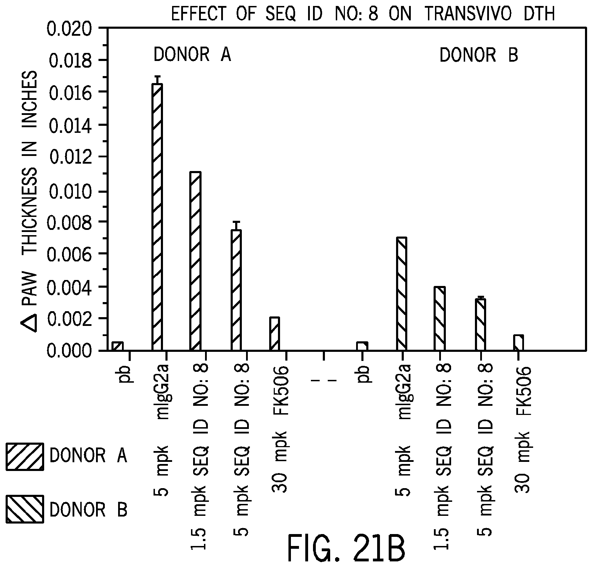

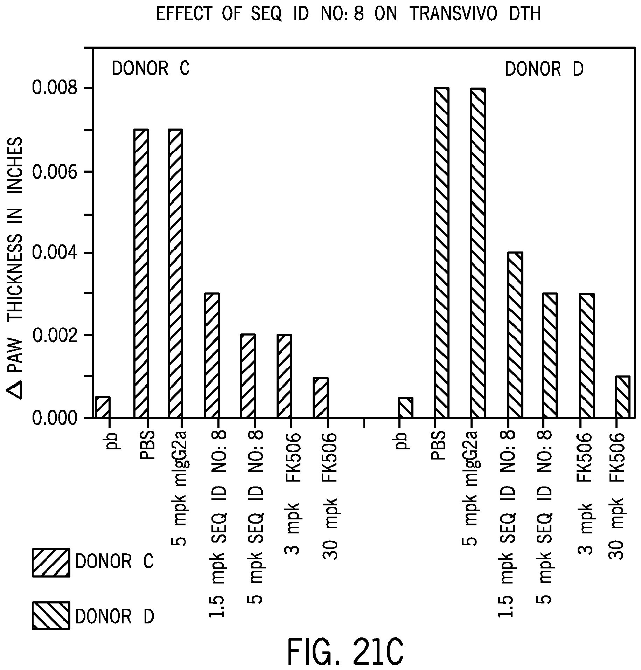

Treatment resulted in a pronounced decrease in DTH response as manifested by the reduction in paw swelling (represented as delta paw thickness) upon treatment of mice with C1ORF32-P8-V1-ECD- mFc (SEQ ID NO:8) prior to injection of human PBMCs into the hind footpads . Paw swelling in response to PBMCs from 4 different human donors was reduced by an average of 49% and 65% upon treatment with 1.5 and 5 mg/kg C1ORF32-P8-V1-ECD-mFc (SEQ ID NO:8), respectively in comparison to mice treated with mIgG2a isotype control at 5mg/kg (Figure 21A, individual donor data are shown in Figures 21B and 21C). Pronounced inhibition was also observed upon treatment with the positive control FK506. mIgG2a did not have any effect on paw swelling as shown when tested in comparison to PBS administration (Figure 21C). These results further support the role of C1ORF32-P8-V1-ECD-mFc (SEQ ID NO:8) in T cell responses and its clinical relevance for treatment of autoimmune diseases and transplantation.

The results presented in these examples, including the efficacy of C1ORF32-P8-V1-ECD-mFc (SEQ ID NO:8) in the R-EAE and CIA animal models, the inhibition of T cell activation, immunomodulation afforded by skewing the immune response from Thl/Thl7 towards Th2, and the indications of induction of tolerance spreading, all support potential therapeutic advantage for C1ORF32-P8-V1-ECD-mFc (SEQ ID NO:8) for treatment of autoimmune diseases with strong Th1 and Thl7 components, such as multiple sclerosis, rheumatoid arthritis, Crohn's disease, psoriasis and type 1 diabetes or any other immune related disorder as described herein.

## Claims

1. A fusion protein for use in the treatment of an immune related disorder, wherein the immune related disorder is selected from multiple sclerosis, rheumatoid arthritis, type I diabetes, psoriasis, systemic lupus erythematosus, inflammatory bowel disease, uveitis, or Sjogren's syndrome, the fusion protein having a first fusion partner and a second fusion partner, wherein
- the first fusion partner comprises a C1ORF32 polypeptide selected from the group consisting of polypeptide comprising a sequence of amino acid residues having at least 95% sequence identity with amino acid residues 21-186 of H19011_1_P8 (SEQ ID NO: 4), corresponding to amino acid sequence depicted in SEQ ID NO: 14, or residues 21-186 of H19011_1_P8_V1 (SEQ ID NO:5), corresponding to amino acid sequence depicted in SEQ ID NO:35, or residues 21-169 of H19011_1_P9 (SEQ ID NO:6), corresponding to amino acid sequence depicted in SEQ ID NO: 15, or residues 21-169 of H19011_1_P9_V1 (SEQ ID NO:34), corresponding to amino acid sequence depicted in SEQ ID NO:36, or residues 1-184 of the sequence H19011_1_P8 (SEQ ID NO:4), corresponding to amino acid sequence depicted in SEQ ID NO:37, or residues 1-184 of the sequence H19011_1_P8_V1 (SEQ ID NO:5), corresponding to amino acid sequence depicted in SEQ ID NO: 19, or residues 1-169 of H19011_1_P9 (SEQ ID NO:6), corresponding to amino acid sequence depicted in SEQ ID NO:28, or residues 1-169 of H19011_1_P9_V1 (SEQ ID NO:34), corresponding to amino acid sequence depicted in SEQ ID NO:30,
- the second fusion partner is composed of a heterologous sequence and contains one or more domains of an immunoglobulin heavy chain constant region, and
- the fusion partners are fused together directly or indirectly via a peptide linker sequence or a chemical linker,
**characterized in that** the fusion protein induces immune tolerance in individuals with the immune related disorder.

2. The fusion protein for use in the treatment of an immune related disorder according to claim 1, wherein the fusion protein induces immune tolerance by interacting with Tregs, enhancing Treg activity, increasing the number of Tregs, increasing the suppressive capacity of Tregs, or combinations thereof.

3. The fusion protein for use in the treatment of an immune related disorder according to at least one of the preceding claims, wherein the C1ORF32 polypeptide is a polypeptide comprising a sequence of amino acid residues having at least 95% sequence identity with amino acid residues 1-184 of the sequence H19011_1_P8 (SEQ ID NO:4), corresponding to amino acid sequence depicted in SEQ ID NO:37, or residues 1-184 of the sequence H19011_1_P8_V1 (SEQ ID NO:5), corresponding to amino acid sequence depicted in SEQ ID NO: 19, or residues 1-169 of H19011_1_P9 (SEQ ID NO:6), corresponding to amino acid sequence depicted in SEQ ID NO:28, or residues 1-169 of H19011_1_P9_V1 (SEQ ID NO:34), corresponding to amino acid sequence depicted in SEQ ID NO:30; or a fragment thereof that does not include the signal peptide sequence.

4. The fusion protein for use in the treatment of an immune related disorder according to at least one of the preceding claims , wherein the one or more domains of an immunoglobulin heavy chain constant region have an amino acid sequence corresponding to the hinge, CH2 and CH3 regions of a human immunoglobulin Cγ1 , Cγ2, Cγ3 or Cγ4 chain or to the hinge, CH2 and CH3 regions of a murine immunoglobulin Cγ2a chain.

5. The fusion protein for use in the treatment of an immune related disorder according to at least one of the preceding claims, wherein the fusion protein contains the CH2 and CH3 regions of a human immunoglobulin Cγ1 chain having amino acid sequence set forth in SEQ ID NO:21.

6. The fusion protein for use in the treatment of an immune related disorder according to at least one the preceding claims, wherein the fusion protein contains the hinge, CH2 and CH3 regions of a human immunoglobulin Cγ1 chain having amino acid sequence set forth in SEQ ID NO:20 or a variant thereof in which the cysteine at position 220 according to the Fc sequence nomenclature as given by Kabat is changed to a serine.

7. The fusion protein for use in the treatment of an immune related disorder according to at least one of the preceding claims, wherein the fusion protein contains a domain that functions to dimerize or multimerize two or more fusion proteins.

8. The fusion protein for use in the treatment of an immune related disorder according to claim 7, wherein the dimerization domain contains at least one cysteine that is capable of forming an intermolecular disulfide bond with a cysteine on a partner fusion protein.

9. The fusion protein for use in the treatment of an immune related disorder according to claim 7 or 8, wherein the dimerization domain is the hinge region of an immunoglobulin.

10. The fusion protein for use in the treatment of an immune related disorder according to at least one of the preceding claims, wherein the fusion protein is dimerized or multimerized to form homodimers, heterodimers, homomultimers or heteromultimers.

11. The fusion protein for use in the treatment of an immune related disorder according to at least one of the preceding claims, wherein the fusion protein is a dimeric fusion protein.

12. The fusion protein for use in the treatment of an immune related disorder according to at least one of the preceding claims, wherein the fusion partners are fused together via a peptide linker that contains the hinge region of an immunoglobulin.

13. Pharmaceutical composition for use in the treatment of an immune related disorder, wherein the immune related disorder is selected from multiple sclerosis, rheumatoid arthritis, type I diabetes, psoriasis, systemic lupus erythematosus, inflammatory bowel disease, uveitis, or Sjogren's syndrome, the pharmaceutical composition comprising the fusion protein of at least one of the preceding claims and a pharmaceutically acceptable diluent or carrier.

## Patentansprüche

1. Fusionsprotein zur Verwendung in der Behandlung einer mit dem Immunsystem in Zusammenhang stehenden Erkrankung, wobei die mit dem Immunsystem in Zusammenhang stehende Erkrankung aus multipler Sklerose, rheumatoider Arthritis, Diabetes Typ I, Psoriasis, systemischem Lupus erythematodes, entzündlicher Darmerkrankung, Uveitis oder Sjögren-Syndrom ausgewählt ist, wobei das Fusionsprotein einen ersten Fusionspartner und einen zweiten Fusionspartner aufweist, wobei
- der erste Fusionspartner ein C10RF32-Polypeptid umfasst, das ausgewählt ist aus der Gruppe, bestehend aus einem Polypeptid umfassend eine Sequenz von Aminosäureresten mit mindestens 95 % Sequenzidentität mit Aminosäureresten 21-186 von H19011_1_P8 (SEQ ID Nr.: 4), entsprechend der in SEQ ID Nr.: 14 gezeigten Aminosäuresequenz, oder Resten 21-186 von H19011_1_P8_V1 (SEQ ID Nr.: 5), entsprechend der in SEQ ID Nr. 35 gezeigten Aminosäuresequenz, oder Resten 21-169 von H19011_1_P9 (SEQ ID Nr.: 6), entsprechend der in SEQ ID Nr.: 15 gezeigten Aminosäuresequenz, oder Resten 21-169 von H19011_1_P9_V1 (SEQ ID Nr.: 34), entsprechend der in SEQ ID Nr.: 36 gezeigten Aminosäuresequenz, oder Resten 1-184 der Sequenz H19011_1_P8 (SEQ ID Nr.: 4), entsprechend der in SEQ ID Nr.: 37 gezeigten Aminosäuresequenz, oder Resten 1-184 der Sequenz H19011_1_P8_V1 (SEQ ID Nr.: 5), entsprechend der in SEQ ID Nr.: 19 gezeigten Aminosäuresequenz, oder Resten 1-169 von H19011_1_P9 (SEQ ID Nr.: 6), entsprechend der in SEQ ID Nr.: 28 gezeigten Aminosäuresequenz, oder Resten 1-169 von H19011_1_P9_V1 (SEQ ID Nr.: 34), entsprechend der in SEQ ID Nr.: 30 gezeigten Aminosäuresequenz,
- der zweite Fusionspartner aus einer heterologen Sequenz besteht und eine oder mehr Domänen einer schweren Kette einer konstanten Immunglobulinregion enthält, und
- die Fusionspartner direkt oder indirekt über eine Peptidlinkersequenz oder einen chemischen Linker miteinander verbunden sind,
**dadurch gekennzeichnet, dass** das Fusionsprotein Immuntoleranz bei Individuen mit der mit dem Immunsystem in Zusammenhang stehenden Erkrankung hervorruft.

2. Fusionsprotein zur Verwendung in der Behandlung einer mit dem Immunsystem in Zusammenhang stehenden Erkrankung gemäß Anspruch 1, wobei das Fusionsprotein Immuntoleranz durch Wechselwirken mit Tregs, Steigern von Treg-Aktivität, Erhöhen der Anzahl von Tregs, Erhöhen des Suppressionsvermögens von Tregs oder Kombinationen davon hervorruft.

3. Fusionsprotein zur Verwendung in der Behandlung einer mit dem Immunsystem in Zusammenhang stehenden Erkrankung gemäß mindestens einem der vorhergehenden Ansprüche, wobei das C10RF32-Polypeptid ein Polypeptid, umfassend eine Sequenz von Aminosäureresten mit mindestens 95 % Sequenzidentität mit Aminosäureresten 1-184 der Sequenz H19011_1_P8 (SEQ ID Nr.: 4), entsprechend der in SEQ ID Nr.: 37 gezeigten Aminosäuresequenz, oder Resten 1-184 der Sequenz H19011_1_P8_V1 (SEQ ID Nr.: 5), entsprechend der in SEQ ID Nr.: 19 gezeigten Aminosäuresequenz, oder Resten 1-169 von H19011_1_P9 (SEQ ID Nr.: 6), entsprechend der in SEQ ID Nr.: 28 gezeigten Aminosäuresequenz, oder Resten 1-169 von H19011_1_P9_V1 (SEQ ID Nr.: 34), entsprechend der in SEQ ID Nr.: 30 gezeigten Aminosäuresequenz; oder ein Fragment davon, welches nicht die Signalpeptidsequenz einschließt, ist.

4. Fusionsprotein zur Verwendung in der Behandlung einer mit dem Immunsystem in Zusammenhang stehenden Erkrankung gemäß mindestens einem der vorhergehenden Ansprüche, wobei die eine oder mehr Domänen einer schweren Kette einer konstanten Immunglobulinregion eine Aminosäuresequenz aufweisen, die den Hinge-, CH2- und CH3-Regionen einer humanen Cγ1-, Cγ2-, Cγ3- oder Cγ4-Immunglobulinkette oder den Hinge-, CH2- und CH3-Regionen einer murinen Cγ2a-Immunglobulinkette entspricht.

5. Fusionsprotein zur Verwendung in der Behandlung einer mit dem Immunsystem in Zusammenhang stehenden Erkrankung gemäß mindestens einem der vorhergehenden Ansprüche, wobei das Fusionsprotein die CH2- und CH3-Regionen einer humanen Cγ1-Immunglobulinkette mit einer in SEQ ID Nr.: 21 dargelegten Aminosäuresequenz enthält.

6. Fusionsprotein zur Verwendung in der Behandlung einer mit dem Immunsystem in Zusammenhang stehenden Erkrankung gemäß mindestens einem der vorhergehenden Ansprüche, wobei das Fusionsprotein die Hinge-, CH2- und CH3-Regionen einer humanen Cγ1-Immunglobulinkette mit einer in SEQ ID Nr.: 20 dargelegten Aminosäuresequenz oder eine Variante davon, in der das Cystein an Position 220 gemäß der Fc-Sequenz-Nomenklatur wie durch Kabat gegeben mit einem Serin ausgetauscht ist, enthält.

7. Fusionsprotein zur Verwendung in der Behandlung einer mit dem Immunsystem in Zusammenhang stehenden Erkrankung gemäß mindestens einem der vorhergehenden Ansprüche, wobei das Fusionsprotein eine Domäne mit einer Funktion von Dimerisieren oder Multimerisieren von zwei oder mehr Fusionsproteinen enthält.

8. Fusionsprotein zur Verwendung in der Behandlung einer mit dem Immunsystem in Zusammenhang stehenden Erkrankung gemäß Anspruch 7, wobei die Dimerisierungsdomäne mindestens ein Cystein, das zum Bilden einer intermolekularen Disulfidbindung mit einem Cystein an einem Partnerfusionsprotein in der Lage ist, enthält.

9. Fusionsprotein zur Verwendung in der Behandlung einer mit dem Immunsystem in Zusammenhang stehenden Erkrankung gemäß Anspruch 7 oder 8, wobei die Dimerisierungsdomäne die Hinge-Region eines Immunglobulins ist.

10. Fusionsprotein zur Verwendung in der Behandlung einer mit dem Immunsystem in Zusammenhang stehenden Erkrankung gemäß mindestens einem der vorhergehenden Ansprüche, wobei das Fusionsprotein dimerisiert oder multimerisiert wird, wobei Homodimere, Heterodimere, Homomultimere oder Heteromultimere gebildet werden.

11. Fusionsprotein zur Verwendung in der Behandlung einer mit dem Immunsystem in Zusammenhang stehenden Erkrankung gemäß mindestens einem der vorhergehenden Ansprüche, wobei das Fusionsprotein ein dimeres Fusionsprotein ist.

12. Fusionsprotein zur Verwendung in der Behandlung einer mit dem Immunsystem in Zusammenhang stehenden Erkrankung gemäß mindestens einem der vorhergehenden Ansprüche, wobei die Fusionspartner zusammen über einen Peptidlinker, der die Hinge-Region eines Immunglobulins enthält, verbunden sind.

13. Pharmazeutische Zusammensetzung zur Verwendung in der Behandlung einer mit dem Immunsystem in Zusammenhang stehenden Erkrankung, wobei die mit dem Immunsystem in Zusammenhang stehende Erkrankung aus multipler Sklerose, rheumatoider Arthritis, Diabetes Typ I, Psoriasis, systemischem Lupus erythematodes, entzündlicher Darmerkrankung, Uveitis oder Sjögren-Syndrom ausgewählt ist, wobei die pharmazeutische Zusammensetzung das Fusionsprotein nach mindestens einem der vorhergehenden Ansprüche und ein pharmazeutisch verträgliches Verdünnungsmittel oder einen pharmazeutisch verträglichen Träger umfasst.

## Revendications

1. Protéine de fusion destinée à être utilisée dans le traitement d'un trouble du système immunitaire, dans laquelle le trouble du système immunitaire est choisi parmi la sclérose en plaques, la polyarthrite rhumatoïde, le diabète de type I, le psoriasis, le lupus érythémateux systémique, la maladie inflammatoire de l'intestin, l'uvéite or le syndrome de Sjögren, la protéine de fusion ayant un premier partenaire de fusion et un second partenaire de fusion, dans laquelle
- le premier partenaire de fusion comprend un polypeptide C1ORF32 choisi dans le groupe consistant de polypeptide comprenant une séquence de résidus d'acides aminés présentant une identité de séquence d'au moins 95% avec les résidus d'acides aminés 21-186 de H19011_1_P8 (SEQ ID No : 4), correspondant à la séquence d'acides aminés décrite par SEQ ID No : 14 ou les résidus 21-186 de H19011_1_P8_V1 (SEQ ID No: 5), correspondant à la séquence d'acides aminés décrite par SEQ ID No : 35, ou les résidus 21-169 de H19011_1_P9 (SEQ ID No: 6), correspondant à la séquence d'acides aminés décrite par SEQ ID No: 15, ou les résidus 21-169 de H19011_1_P9_V1 (SEQ ID No: 34), correspondant à la séquence d'acides aminés décrite par SEQ ID No : 36, ou les résidus 1-184 de H19011_1_P8 (SEQ ID No : 4), correspondant à la séquence d'acides aminés décrite par SEQ ID No : 37, ou les résidus 1-184 de H19011_1_P8_V1 (SEQ ID No: 5), correspondant à la séquence d'acides aminés décrite par SEQ ID No: 19, ou les résidus 1-169 de H19011_1_P9 (SEQ ID No : 6), correspondant à la séquence d'acides aminés décrite par SEQ ID No: 28, ou les résidus 1-169 de H19011_1_P9_V1 (SEQ ID No: 34), correspondant à la séquence d'acides aminés décrite par SEQ ID No : 30,
- le second partenaire de fusion est composé d'une séquence hétérologue et contient un ou plusieurs domaines d'une région constante de chaîne lourde d'immunoglobuline, et
- les partenaires de fusion sont fusionnés l'un à l'autre directement ou indirectement par l'intermédiaire d'une séquence de liaison peptidique ou d'un lieur chimique, **caractérisée en ce que** la protéine de fusion induit une tolérance immunitaire chez les individus atteints du trouble du système immunitaire.

2. Protéine de fusion destinée à être utilisée dans le traitement d'un trouble du système immunitaire selon la revendication 1, dans laquelle la protéine de fusion induit une tolérance immunitaire en interagissant avec les lymphocytes T régulateurs, en stimulant l'activité des lymphocytes T régulateurs, en accroissant le nombre des lymphocytes T régulateurs, en augmentant la capacité suppressive des lymphocytes T régulateurs ou par des combinaisons de ces actions.

3. Protéine de fusion destinée à être utilisée dans le traitement d'un trouble du système immunitaire selon au moins l'une des revendications précédentes, dans laquelle le polypeptide C1ORF32 est un polypeptide comprenant une séquence de résidus d'acides aminés présentant une identité de séquence d'au moins 95 % avec les résidus d'acides aminés 1-184 de H19011_1_P8 (SEQ ID No: 4), correspondant à la séquence d'acides aminés décrite par SEQ ID No: 37, ou les résidus 1-184 de H19011_1_P8_V1 (SEQ ID No: 5), correspondant à la séquence d'acides aminés décrite par SEQ ID No : 19, ou les résidus 1-169 de H19011_1_P9 (SEQ ID No : 6), correspondant à la séquence d'acides aminés décrite par SEQ ID No: 28, ou les résidus 1-169 de H19011_1_P9_V1 (SEQ ID No: 34), correspondant à la séquence d'acides aminés décrite par SEQ ID No : 30; ou fragment de celle-ci ne comprenant pas la séquence de peptide signal.

4. Protéine de fusion destinée à être utilisée dans le traitement d'un trouble du système immunitaire selon au moins l'une des revendications précédentes, dans laquelle le ou les domaines de région constante de chaîne lourde d'immunoglobuline présentent une séquence d'acides aminés correspondant aux régions charnière, CH2 et CH3 d'une chaîne d'immunoglobuline humaine Cγ1, Cγ2, Cy3 ou Cy4 ou aux régions charnière, CH2 et CH3 d'une chaîne d'immunoglobuline murine Cy2a.

5. Protéine de fusion destinée à être utilisée dans le traitement d'un trouble du système immunitaire selon au moins l'une des revendications précédentes, dans laquelle la protéine de fusion contient les régions CH2 et CH3 d'une chaîne d'immunoglobuline humaine Cγ1 présentant une séquence d'acides aminés décrite par SEQ ID No : 21.

6. Protéine de fusion destinée à être utilisée dans le traitement d'un trouble du système immunitaire selon au moins l'une des revendications précédentes, dans laquelle la protéine de fusion contient les régions charnière, CH2 et CH3 d'une chaîne d'immunoglobuline humaine Cγ1 présentant une séquence d'acides aminés décrite par SEQ ID No : 20 ou une variante de celle-ci dans laquelle la cystéine en position 220 selon la nomenclature des séquences Fc donnée par Kabat est remplacée par une sérine.

7. Protéine de fusion destinée à être utilisée dans le traitement d'un trouble du système immunitaire selon au moins l'une des revendications précédentes, dans laquelle la protéine de fusion contient un domaine qui fonctionne de manière à dimériser ou à multimériser deux protéines de fusion ou plus.

8. Protéine de fusion destinée à être utilisée dans le traitement d'un trouble du système immunitaire selon la revendication 7, dans laquelle le domaine de dimérisation contient au moins une cystéine qui est capable de former une liaison disulfure intermoléculaire avec une cystéine sur une protéine de fusion partenaire.

9. Protéine de fusion destinée à être utilisée dans le traitement d'un trouble du système immunitaire selon la revendication 7 ou 8, dans laquelle le domaine de dimérisation est la région charnière d'une immunoglobuline.

10. Protéine de fusion destinée à être utilisée dans le traitement d'un trouble du système immunitaire selon au moins l'une des revendications précédentes, dans laquelle la protéine de fusion est dimérisée ou multimérisée pour former des homodimères, des hétérodimères, des homomultimères ou des hétéromultimères.

11. Protéine de fusion destinée à être utilisée dans le traitement d'un trouble du système immunitaire selon au moins l'une des revendications précédentes, dans laquelle la protéine de fusion est une protéine de fusion dimère.

12. Protéine de fusion destinée à être utilisée dans le traitement d'un trouble du système immunitaire selon au moins l'une des revendications précédentes, dans laquelle les partenaires de fusion sont fusionnés l'une à l'autre par l'intermédiaire d'un lieur peptidique qui contient la région charnière d'une immunoglobuline.

13. Composition pharmaceutique destinée à être utilisée dans le traitement d'un trouble du système immunitaire, dans laquelle le trouble du système immunitaire est choisi parmi sclérose en plaques, la polyarthrite rhumatoïde, le diabète de type I, le psoriasis, le lupus érythémateux systémique, la maladie inflammatoire de l'intestin, l'uvéite or le syndrome de Sjögren, la composition pharmaceutique comprenant la protéine de fusion d'au moins l'une des revendications précédentes et un diluant ou un excipient pharmaceutiquement acceptable.


### SEQUENCE LISTING

<110> Compugen Ltd
<120> POLYPEPTIDES AND USES THEREOF AS A DRUG FOR TREATMENT OF MULTIPLE SCLEROSIS, RHEUMATOID ARTHTRITIS AND OTHER AUTOIMMUNE DISORDERS
<130>
<160> 40
<210> 1
   <211> 1407
   <212> DNA
   <213> Homo Sapiens
<400> 1
   ccggcggcgc gatccagccc ccggccccgc ctgcgcggcc ggcccggcgg gcgctgcgcc     60
   cagggacgcc cggtgcccgc cgctccgccg ccgcccgctg ccgcggggtg acagcgatcc    120
   ttctgttcca gccatttccc actttcctca ctccgtaatt cggctgggaa gttggggaag    180
   atggataggg tcttgctgag gtggatttct ctcttctggc taacagccat ggtcgaaggc    240
   cttcaggtca cagtgcccga caagaagaag gtggccatgc tcttccagcc cactgtgctt    300
   cgctgccact tctcaacatc ctcccatcag cctgcagttg tgcagtggaa gttcaagtcc    360
   tactgccagg atcgcatggg agaatccttg ggcatgtcct ctacccgggc ccaatctctc    420
   agcaagagaa acctggaatg ggacccctac ttggattgtt tggacagcag gaggactgtt    480
   cgagtagtag cttcaaaaca gggctcgact gtcaccctgg gagatttcta caggggcaga    540
   gagatcacga ttgttcatga tgcagatctt caaattggaa agcttatgtg gggagacagc    600
   ggactctatt actgtattat caccacccca gatgacctgg aggggaaaaa tgagggctca    660
   ctgggactgc tggtgttggg caggacaggg ctgcttgctg atctcttgcc cagttttgct    720
   gtggagatta tgccagagtg ggtgtttgtt ggcctggtgc tcctgggcgt cttcctcttc    780
   ttcgtcctgg tggggatctg ctggtgccag tgctgccctc acagctgctg ctgctatgtc    840
   cgctgcccat gctgcccaga ttcctgctgg tgccctcaag cctgtgagta cagtgaccgc    900
   tggggagaca gagcgatcga gagaaatgtc tacctctcta cctgacagct gtgtgcgctg    960
   ggttcctcct ccacctcctg tcctgccacc cccaagattg gtcattccag actcttctcc   1020
   gctgggtgcc cctggcctca gggatgacca ttctcatttg ccttttcacc tacatacacc   1080
   tctccacact tcttatccat atctatcact ccatgcattt ggaattctca tggacactat   1140
   tgataaaatg gaagggcagg tttggcgtgg tgaggttgtg gtgtaagact gttccctctc   1200
   cctggggcat tcaaactaga ggaaaccttc tctggtcgtt cccttcccat gcagagaagt   1260
   tcctttttat atgagaagag tgtgcaaact gtggcctttg ggcacccacc cagccacaga   1320
   tttgttttat ttactcccat gatgacatgg gccacaatag ggcctagttc ttatttgagg   1380
   attcacaatt tttaccttac tggccaa                                       1407
<210> 2
   <211> 1350
   <212> DNA
   <213> Homo Sapiens
<400> 2
   ccggcggcgc gatccagccc ccggccccgc ctgcgcggcc ggcccggcgg gcgctgcgcc     60
   cagggacgcc cggtgcccgc cgctccgccg ccgcccgctg ccgcggggtg acagcgatcc    120
   ttctgttcca gccatttccc actttcctca ctccgtaatt cggctgggaa gttggggaag    180
   atggataggg tcttgctgag gtggatttct ctcttctggc taacagccat ggtcgaaggc    240
   cttcaggtca cagtgcccga caagaagaag gtggccatgc tcttccagcc cactgtgctt    300
   cgctgccact tctcaacatc ctcccatcag cctgcagttg tgcagtggaa gttcaagtcc    360
   tactgccagg atcgcatggg agaatccttg ggcatgtcct ctacccgggc ccaatctctc    420
   agcaagagaa acctggaatg ggacccctac ttggattgtt tggacagcag gaggactgtt    480
   cgagtagtag cttcaaaaca gggctcgact gtcaccctgg gagatttcta caggggcaga    540
   gagatcacga ttgttcatga tgcagatctt caaattggaa agcttatgtg gggagacagc    600
   ggactctatt actgtattat caccacccca gatgacctgg aggggaaaaa tgagggctca    660
   ctgggactgc tggtgttgga gtgggtgttt gttggcctgg tgctcctggg cgtcttcctc    720
   ttcttcgtcc tggtggggat ctgctggtgc cagtgctgcc ctcacagctg ctgctgctat    780
   gtccgctgcc catgctgccc agattcctgc tggtgccctc aagcctgtga gtacagtgac    840
   cgctggggag acagagcgat cgagagaaat gtctacctct ctacctgaca gctgtgtgcg    900
   ctgggttcct cctccacctc ctgtcctgcc acccccaaga ttggtcattc cagactcttc    960
   tccgctgggt gcccctggcc tcagggatga ccattctcat ttgccttttc acctacatac   1020
   acctctccac acttcttatc catatctatc actccatgca tttggaattc tcatggacac   1080
   tattgataaa atggaagggc aggtttggcg tggtgaggtt gtggtgtaag actgttccct   1140
   ctccctgggg cattcaaact agaggaaacc ttctctggtc gttcccttcc catgcagaga   1200
   agttcctttt tatatgagaa gagtgtgcaa actgtggcct ttgggcaccc acccagccac   1260
   agatttgttt tatttactcc catgatgaca tgggccacaa tagggcctag ttcttatttg   1320
   aggattcaca atttttacct tactggccaa 1350
<210> 3
   <211> 639
   <212> PRT
   <213> Homo Sapiens
<400> 3
   Met Asp Arg Val Leu Leu Arg Trp Ile Ser Leu Phe Trp Leu Thr Ala
   1               5                   10                  15
   Met Val Glu Gly Leu Gln Val Thr Val Pro Asp Lys Lys Lys Val Ala
               20                  25                  30
   Met Leu Phe Gln Pro Thr Val Leu Arg Cys His Phe Ser Thr Ser Ser
           35                  40                  45
   His Gln Pro Ala Val Val Gln Trp Lys Phe Lys Ser Tyr Cys Gln Asp
       50                  55                  60
   Arg Met Gly Glu Ser Leu Gly Met Ser Ser Thr Arg Ala Gln Ser Leu
   65                  70                  75                  80
   Ser Lys Arg Asn Leu Glu Trp Asp Pro Tyr Leu Asp Cys Leu Asp Ser
                   85                  90                  95
   Arg Arg Thr Val Arg Val Val Ala Ser Lys Gln Gly Ser Thr Val Thr
               100                 105                 110
   Leu Gly Asp Phe Tyr Arg Gly Arg Glu Ile Thr Ile Val His Asp Ala
           115                 120                 125
   Asp Leu Gln Ile Gly Lys Leu Met Trp Gly Asp Ser Gly Leu Tyr Tyr
       130                 135                 140
   Cys Ile Ile Thr Thr Pro Asp Asp Leu Glu Gly Lys Asn Glu Asp Ser
   145                 150                 155                 160
   Val Glu Leu Leu Val Leu Gly Arg Thr Gly Leu Leu Ala Asp Leu Leu
                   165                 170                 175
   Pro Ser Phe Ala Val Glu Ile Met Pro Glu Trp Val Phe Val Gly Leu
               180                 185                 190
   Val Leu Leu Gly Val Phe Leu Phe Phe Val Leu Val Gly Ile Cys Trp
           195                 200                 205
   Cys Gln Cys Cys Pro His Ser Cys Cys Cys Tyr Val Arg Cys Pro Cys
       210                 215                 220
   Cys Pro Asp Ser Cys Cys Cys Pro Gln Ala Leu Tyr Glu Ala Gly Lys
   225                 230                 235                 240
   Ala Ala Lys Ala Gly Tyr Pro Pro Ser Val Ser Gly Val Pro Gly Pro
                   245                 250                 255
   Tyr Ser Ile Pro Ser Val Pro Leu Gly Gly Ala Pro Ser Ser Gly Met
               260                 265                 270
   Leu Met Asp Lys Pro His Pro Pro Pro Leu Ala Pro Ser Asp Ser Thr
           275                 280                 285
   Gly Gly Ser His Ser Val Arg Lys Gly Tyr Arg Ile Gln Ala Asp Lys
       290                 295                 300
   Glu Arg Asp Ser Met Lys Val Leu Tyr Tyr Val Glu Lys Glu Leu Ala
   305                 310                 315                 320
   Gln Phe Asp Pro Ala Arg Arg Met Arg Gly Arg Tyr Asn Asn Thr Ile
                   325                 330                 335
   Ser Glu Leu Ser Ser Leu His Glu Glu Asp Ser Asn Phe Arg Gln Ser
               340                 345                 350
   Phe His Gln Met Arg Ser Lys Gln Phe Pro Val Ser Gly Asp Leu Glu
           355                 360                 365
   Ser Asn Pro Asp Tyr Trp Ser Gly Val Met Gly Gly Ser Ser Gly Ala
       370                 375                 380
   Ser Arg Gly Pro Ser Ala Met Glu Tyr Asn Lys Glu Asp Arg Glu Ser
   385                 390                 395                 400
   Phe Arg His Ser Gln Pro Arg Ser Lys Ser Glu Met Leu Ser Arg Lys
                   405                 410                 415
   Asn Phe Ala Thr Gly Val Pro Ala Val Ser Met Asp Glu Leu Ala Ala
               420                 425                 430
   Phe Ala Asp Ser Tyr Gly Gln Arg Pro Arg Arg Ala Asp Gly Asn Ser
           435                 440                 445
   His Glu Ala Arg Gly Gly Ser Arg Phe Glu Arg Ser Glu Ser Arg Ala
       450                 455                 460
   His Ser Gly Phe Tyr Gln Asp Asp Ser Leu Glu Glu Tyr Tyr Gly Gln
   465 470 475 480
   Arg Ser Arg Ser Arg Glu Pro Leu Thr Asp Ala Asp Arg Gly Trp Ala
   485 490 495
   Phe Ser Pro Ala Arg Arg Arg Pro Ala Glu Asp Ala His Leu Pro Arg
   500 505 510
   Leu Val Ser Arg Thr Pro Gly Thr Ala Pro Lys Tyr Asp His Ser Tyr
   515 520 525
   Leu Gly Ser Ala Arg Glu Arg Gln Ala Arg Pro Glu Gly Ala Ser Arg
   530 535 540
   Gly Gly Ser Leu Glu Thr Pro Ser Lys Arg Ser Ala Gln Leu Gly Pro
   545 550 555 560
   Arg Ser Ala Ser Tyr Tyr Ala Trp Ser Pro Pro Gly Thr Tyr Lys Ala
   565 570 575
   Gly Ser Ser Gln Asp Asp Gln Glu Asp Ala Ser Asp Asp Ala Leu Pro
   580 585 590
   Pro Tyr Ser Glu Leu Glu Leu Thr Arg Gly Pro Ser Tyr Arg Gly Arg
   595 600 605
   Asp Leu Pro Tyr His Ser Asn Ser Glu Lys Lys Arg Lys Lys Glu Pro
   610 615 620
   Ala Lys Lys Thr Asn Asp Phe Pro Thr Arg Met Ser Leu Val Val
   625 630 635
<210> 4
   <211> 254
   <212> PRT
   <213> Homo Sapiens
<400> 4
   Met Asp Arg Val Leu Leu Arg Trp Ile Ser Leu Phe Trp Leu Thr Ala
   1 5 10 15
   Met Val Glu Gly Leu Gln Val Thr Val Pro Asp Lys Lys Lys Val Ala
   20 25 30
   Met Leu Phe Gln Pro Thr Val Leu Arg Cys His Phe Ser Thr Ser Ser
           35                  40                  45
   His Gln Pro Ala Val Val Gln Trp Lys Phe Lys Ser Tyr Cys Gln Asp
       50                  55                  60
   Arg Met Gly Glu Ser Leu Gly Met Ser Ser Thr Arg Ala Gln Ser Leu
   65                  70                  75                  80
   Ser Lys Arg Asn Leu Glu Trp Asp Pro Tyr Leu Asp Cys Leu Asp Ser
                   85                  90                  95
   Arg Arg Thr Val Arg Val Val Ala Ser Lys Gln Gly Ser Thr Val Thr
               100                 105                 110
   Leu Gly Asp Phe Tyr Arg Gly Arg Glu Ile Thr Ile Val His Asp Ala
           115                 120                 125
   Asp Leu Gln Ile Gly Lys Leu Met Trp Gly Asp Ser Gly Leu Tyr Tyr
       130                 135                 140
   Cys Ile Ile Thr Thr Pro Asp Asp Leu Glu Gly Lys Asn Glu Gly Ser
   145                 150                 155                 160
   Leu Gly Leu Leu Val Leu Gly Arg Thr Gly Leu Leu Ala Asp Leu Leu
                   165                 170                 175
   Pro Ser Phe Ala Val Glu Ile Met Pro Glu Trp Val Phe Val Gly Leu
               180                 185                 190
   Val Leu Leu Gly Val Phe Leu Phe Phe Val Leu Val Gly Ile Cys Trp
           195                 200                 205
   Cys Gln Cys Cys Pro His Ser Cys Cys Cys Tyr Val Arg Cys Pro Cys
       210                 215                 220
   Cys Pro Asp Ser Cys Trp Cys Pro Gln Ala Cys Glu Tyr Ser Asp Arg
   225                 230                 235                 240
   Trp Gly Asp Arg Ala Ile Glu Arg Asn Val Tyr Leu Ser Thr
                   245                 250
<210> 5
   <211> 254
   <212> PRT
   <213> Homo Sapiens
<400> 5
   Met Asp Arg Val Leu Leu Arg Trp Ile Ser Leu Phe Trp Leu Thr Ala
   1               5                   10                  15
   Met Val Glu Gly Leu Gln Val Thr Val Pro Asp Lys Lys Lys Val Ala
               20                  25                  30
   Met Leu Phe Gln Pro Thr Val Leu Arg Cys His Phe Ser Thr Ser Ser
           35                  40                  45
   His Gln Pro Ala Val Val Gln Trp Lys Phe Lys Ser Tyr Cys Gln Asp
       50                  55                  60
   Arg Met Gly Glu Ser Leu Gly Met Ser Ser Thr Arg Ala Gln Ser Leu
   65                  70                  75                  80
   Ser Lys Arg Asn Leu Glu Trp Asp Pro Tyr Leu Asp Cys Leu Asp Ser
                   85                  90                  95
   Arg Arg Thr Val Arg Val Val Ala Ser Lys Gln Gly Ser Thr Val Thr
               100                 105                 110
   Leu Gly Asp Phe Tyr Arg Gly Arg Glu Ile Thr Ile Val His Asp Ala
           115                 120                 125
   Asp Leu Gln Ile Gly Lys Leu Met Trp Gly Asp Ser Gly Leu Tyr Tyr
       130                 135                 140
   Cys Ile Ile Thr Thr Pro Asp Asp Leu Glu Gly Lys Asn Glu Asp Ser
   145                 150                 155                 160
   Val Glu Leu Leu Val Leu Gly Arg Thr Gly Leu Leu Ala Asp Leu Leu
                   165                 170                 175
   Pro Ser Phe Ala Val Glu Ile Met Pro Glu Trp Val Phe Val Gly Leu
               180                 185                 190
   Val Leu Leu Gly Val Phe Leu Phe Phe Val Leu Val Gly Ile Cys Trp
           195                 200                 205
   Cys Gln Cys Cys Pro His Ser Cys Cys Cys Tyr Val Arg Cys Pro Cys
       210                 215                 220
   Cys Pro Asp Ser Cys Cys Cys Pro Gln Ala Cys Glu Tyr Ser Asp Arg
   225                 230                 235                 240
   Trp Gly Asp Arg Ala Ile Glu Arg Asn Val Tyr Leu Ser Thr
                   245                 250
<210> 6
   <211> 235
   <212> PRT
   <213> Homo Sapiens
<400> 6
   Met Asp Arg Val Leu Leu Arg Trp Ile Ser Leu Phe Trp Leu Thr Ala
   1               5                   10                  15
   Met Val Glu Gly Leu Gln Val Thr Val Pro Asp Lys Lys Lys Val Ala
               20                  25                  30
   Met Leu Phe Gln Pro Thr Val Leu Arg Cys His Phe Ser Thr Ser Ser
           35                  40                  45
   His Gln Pro Ala Val Val Gln Trp Lys Phe Lys Ser Tyr Cys Gln Asp
       50                  55                  60
   Arg Met Gly Glu Ser Leu Gly Met Ser Ser Thr Arg Ala Gln Ser Leu
   65                  70                  75                  80
   Ser Lys Arg Asn Leu Glu Trp Asp Pro Tyr Leu Asp Cys Leu Asp Ser
                   85                  90                  95
   Arg Arg Thr Val Arg Val Val Ala Ser Lys Gln Gly Ser Thr Val Thr
               100                 105                 110
   Leu Gly Asp Phe Tyr Arg Gly Arg Glu Ile Thr Ile Val His Asp Ala
           115                 120                 125
   Asp Leu Gln Ile Gly Lys Leu Met Trp Gly Asp Ser Gly Leu Tyr Tyr
       130                 135                 140
   Cys Ile Ile Thr Thr Pro Asp Asp Leu Glu Gly Lys Asn Glu Gly Ser
   145                 150                 155                 160
   Leu Gly Leu Leu Val Leu Glu Trp Val Phe Val Gly Leu Val Leu Leu
                   165                 170                 175
   Gly Val Phe Leu Phe Phe Val Leu Val Gly Ile Cys Trp Cys Gln Cys
               180                 185                 190
   Cys Pro His Ser Cys Cys Cys Tyr Val Arg Cys Pro Cys Cys Pro Asp
   195 200 205
   Ser Cys Trp Cys Pro Gln Ala Cys Glu Tyr Ser Asp Arg Trp Gly Asp
   210 215 220
   Arg Ala Ile Glu Arg Asn Val Tyr Leu Ser Thr
   225 230 235
<210> 7
   <211> 1287
   <212> DNA
   <213> Artificial
<220>
   <223> Synthetic polynucleotide
<400> 7
   atggataggg tcttgctgag gtggatttct ctcttctggc taacagccat ggtcgaaggc 60
   cttcaggtca cagtgcccga caagaagaag gtggccatgc tcttccagcc cactgtgctt 120
   cgctgccact tctcaacatc ctcccatcag cctgcagttg tgcagtggaa gttcaagtcc 180
   tactgccagg atcgcatggg agaatccttg ggcatgtcct ctacccgggc ccaatctctc 240
   agcaagagaa acctggaatg ggacccctac ttggattgtt tggacagcag gaggactgtt 300
   cgagtagtag cttcaaaaca gggctcgact gtcaccctgg gagatttcta caggggcaga 360
   gagatcacga ttgttcatga tgcagatctt caaattggaa agcttatgtg gggagacagc 420
   ggactctatt actgtattat caccacccca gatgacctgg aggggaaaaa tgaggactca 480
   gtggaactgc tggtgttggg caggacaggg ctgcttgctg atctcttgcc cagttttgct 540
   gtggagatta tgggatccga gaacctgtac tttcagggca gcggcgagcc cagaggcccc 600
   accatcaagc cctgcccccc ctgcaagtgc ccagccccta acctgctggg cggacccagc 660
   gtgttcatct tcccccccaa gatcaaggac gtgctgatga tcagcctgag ccccatcgtg 720
   acctgcgtgg tggtggacgt gagcgaggac gaccccgacg tgcagatcag ctggttcgtg 780
   aacaacgtgg aggtgcacac cgcccagacc cagacccacc gggaggacta caacagcacc 840
   ctgcgggtgg tgtccgccct gcccatccag caccaggact ggatgagcgg caaagaattc 900
   aagtgcaagg tgaacaacaa ggacctgcct gcccccatcg agcggaccat cagcaagccc 960
   aagggcagcg tgagagcccc ccaggtgtac gtgctgcccc ctcccgagga agagatgacc 1020
   aagaaacagg tgaccctgac ctgcatggtg accgacttca tgcccgagga catctacgtg 1080
   gagtggacca acaacggcaa gaccgagctg aactacaaga acaccgagcc cgtgctggac 1140
   agcgacggca gctacttcat gtatagcaag ctgagagtcg agaagaaaaa ctgggtggag 1200
   cggaacagct acagctgcag cgtggtgcac gagggcctgc acaaccacca caccaccaag 1260
   agcttcagcc ggacccccgg caagtga 1287
<210> 8
   <211> 428
   <212> PRT
   <213> Artificial
<220>
   <223> Synthetic polypeptide
<400> 8
   Met Asp Arg Val Leu Leu Arg Trp Ile Ser Leu Phe Trp Leu Thr Ala
   1 5 10 15
   Met Val Glu Gly Leu Gln Val Thr Val Pro Asp Lys Lys Lys Val Ala
   20 25 30
   Met Leu Phe Gln Pro Thr Val Leu Arg Cys His Phe Ser Thr Ser Ser
   35 40 45
   His Gln Pro Ala Val Val Gln Trp Lys Phe Lys Ser Tyr Cys Gln Asp
   50 55 60
   Arg Met Gly Glu Ser Leu Gly Met Ser Ser Thr Arg Ala Gln Ser Leu
   65 70 75 80
   Ser Lys Arg Asn Leu Glu Trp Asp Pro Tyr Leu Asp Cys Leu Asp Ser
   85 90 95
   Arg Arg Thr Val Arg Val Val Ala Ser Lys Gln Gly Ser Thr Val Thr
   100 105 110
   Leu Gly Asp Phe Tyr Arg Gly Arg Glu Ile Thr Ile Val His Asp Ala
   115 120 125
   Asp Leu Gln Ile Gly Lys Leu Met Trp Gly Asp Ser Gly Leu Tyr Tyr
   130 135 140
   Cys Ile Ile Thr Thr Pro Asp Asp Leu Glu Gly Lys Asn Glu Asp Ser
   145 150 155 160
   Val Glu Leu Leu Val Leu Gly Arg Thr Gly Leu Leu Ala Asp Leu Leu
   165 170 175
   Pro Ser Phe Ala Val Glu Ile Met Gly Ser Glu Asn Leu Tyr Phe Gln
               180                 185                 190
   Gly Ser Gly Glu Pro Arg Gly Pro Thr Ile Lys Pro Cys Pro Pro Cys
           195                 200                 205
   Lys Cys Pro Ala Pro Asn Leu Leu Gly Gly Pro Ser Val Phe Ile Phe
       210                 215                 220
   Pro Pro Lys Ile Lys Asp Val Leu Met Ile Ser Leu Ser Pro Ile Val
   225                 230                 235                 240
   Thr Cys Val Val Val Asp Val Ser Glu Asp Asp Pro Asp Val Gln Ile
                   245                 250                 255
   Ser Trp Phe Val Asn Asn Val Glu Val His Thr Ala Gln Thr Gln Thr
               260                 265                 270
   His Arg Glu Asp Tyr Asn Ser Thr Leu Arg Val Val Ser Ala Leu Pro
           275                 280                 285
   Ile Gln His Gln Asp Trp Met Ser Gly Lys Glu Phe Lys Cys Lys Val
       290                 295                 300
   Asn Asn Lys Asp Leu Pro Ala Pro Ile Glu Arg Thr Ile Ser Lys Pro
   305                 310                 315                 320
   Lys Gly Ser Val Arg Ala Pro Gln Val Tyr Val Leu Pro Pro Pro Glu
                   325                 330                 335
   Glu Glu Met Thr Lys Lys Gln Val Thr Leu Thr Cys Met Val Thr Asp
               340                 345                 350
   Phe Met Pro Glu Asp Ile Tyr Val Glu Trp Thr Asn Asn Gly Lys Thr
           355                 360                 365
   Glu Leu Asn Tyr Lys Asn Thr Glu Pro Val Leu Asp Ser Asp Gly Ser
       370                 375                 380
   Tyr Phe Met Tyr Ser Lys Leu Arg Val Glu Lys Lys Asn Trp Val Glu
   385                 390                 395                 400
   Arg Asn Ser Tyr Ser Cys Ser Val Val His Glu Gly Leu His Asn His
                   405                 410                 415
   His Thr Thr Lys Ser Phe Ser Arg Thr Pro Gly Lys
               420                 425
<210> 9
   <211> 524
   <212> DNA
   <213> Artificial
<220>
   <223> Synthetic polynucleotide
<400> 9
   gtgagtacag tgaccgctgg ggagacagag cgatcgagag aaatgtctac ctctctacct      60
   gacagctgtg tgcgctgggt tcctcctcca cctcctgtcc tgccaccccc aagattggtc     120
   attccagact cttctccgct gggtgcccct ggcctcaggg atgaccattc tcatttgcct     180
   tttcacctac atacacctct ccacacttct tatccatatc tatcactcca tgcatttgga     240
   attctcatgg acactattga taaaatggaa gggcaggttt ggcgtggtga ggttgtggtg     300
   taagactgtt ccctctccct ggggcattca aactagagga aaccttctct ggtcgttccc     360
   ttcccatgca gagaagttcc tttttatatg agaagagtgt gcaaactgtg gcctttgggc     420
   acccacccag ccacagattt gttttattta ctcccatgat gacatgggcc acaatagggc     480
   ctagttctta tttgaggatt cacaattttt accttactgg ccaa                      524
<210> 10
   <211> 57
   <212> DNA
   <213> Artificial
<220>
   <223> Synthetic polynucleotide
<400> 10
   gcaggacagg gctgcttgct gatctcttgc ccagttttgc tgtggagatt atgccag 57
<210> 11
   <211> 61
   <212> DNA
   <213> Artificial
<220>
   <223> Synthetic polynucleotide
<400> 11
   agtgggtgtt tgttggcctg gtgctcctgg gcgtcttcct cttcttcgtc ctggtgggga      60
   t                                                                      61
<210> 12
   <211> 66
   <212> DNA
   <213> Artificial
<220>
   <223> Synthetic polynucleotide
<400> 12
   ctgctggtgc cagtgctgcc ctcacagctg ctgctgctat gtccgctgcc catgctgccc      60
   agattc                                                                 66
<210> 13
   <211> 20
   <212> DNA
   <213> Artificial
<220>
   <223> Synthetic polynucleotide
<400> 13
   ctgctggtgc cctcaagcct 20
<210> 14
   <211> 166
   <212> PRT
   <213> Artificial
<220>
   <223> Synthetic polypeptide
<400> 14
   Leu Gln Val Thr Val Pro Asp Lys Lys Lys Val Ala Met Leu Phe Gln
   1               5                   10                  15
   Pro Thr Val Leu Arg Cys His Phe Ser Thr Ser Ser His Gln Pro Ala
               20                  25                  30
   Val Val Gln Trp Lys Phe Lys Ser Tyr Cys Gln Asp Arg Met Gly Glu
           35                  40                  45
   Ser Leu Gly Met Ser Ser Thr Arg Ala Gln Ser Leu Ser Lys Arg Asn
       50                  55                  60
   Leu Glu Trp Asp Pro Tyr Leu Asp Cys Leu Asp Ser Arg Arg Thr Val
   65                  70                  75                  80
   Arg Val Val Ala Ser Lys Gln Gly Ser Thr Val Thr Leu Gly Asp Phe
                   85                  90                  95
   Tyr Arg Gly Arg Glu Ile Thr Ile Val His Asp Ala Asp Leu Gln Ile
               100                 105                 110
   Gly Lys Leu Met Trp Gly Asp Ser Gly Leu Tyr Tyr Cys Ile Ile Thr
           115                 120                 125
   Thr Pro Asp Asp Leu Glu Gly Lys Asn Glu Gly Ser Leu Gly Leu Leu
       130                 135                 140
   Val Leu Gly Arg Thr Gly Leu Leu Ala Asp Leu Leu Pro Ser Phe Ala
   145                 150                 155                 160
   Val Glu Ile Met Pro Glu
                   165
<210> 15
   <211> 149
   <212> PRT
   <213> Artificial
<220>
   <223> Synthetic polypeptide
<400> 15
   Leu Gln Val Thr Val Pro Asp Lys Lys Lys Val Ala Met Leu Phe Gln
   1               5                   10                  15
   Pro Thr Val Leu Arg Cys His Phe Ser Thr Ser Ser His Gln Pro Ala
               20                  25                  30
   Val Val Gln Trp Lys Phe Lys Ser Tyr Cys Gln Asp Arg Met Gly Glu
           35                  40                  45
   Ser Leu Gly Met Ser Ser Thr Arg Ala Gln Ser Leu Ser Lys Arg Asn
       50                  55                  60
   Leu Glu Trp Asp Pro Tyr Leu Asp Cys Leu Asp Ser Arg Arg Thr Val
   65                  70                  75                  80
   Arg Val Val Ala Ser Lys Gln Gly Ser Thr Val Thr Leu Gly Asp Phe
                   85                  90                  95
   Tyr Arg Gly Arg Glu Ile Thr Ile Val His Asp Ala Asp Leu Gln Ile
               100                 105                 110
   Gly Lys Leu Met Trp Gly Asp Ser Gly Leu Tyr Tyr Cys Ile Ile Thr
   115 120 125
   Thr Pro Asp Asp Leu Glu Gly Lys Asn Glu Gly Ser Leu Gly Leu Leu
   130 135 140
   Val Leu Glu Trp Val
   145
<210> 16
   <211> 33
   <212> DNA
   <213> Artificial
<220>
   <223> Synthetic polynucleotide
<400> 16
   ctagctagcc accatggata gggtcttgct gag 33
<210> 17
   <211> 29
   <212> DNA
   <213> Artificial
<220>
   <223> Synthetic polynucleotide
<400> 17
   cgcggatccc ataatctcca cagcaaaac 29
<210> 18
   <211> 20
   <212> PRT
   <213> Artificial
<220>
   <223> Synthetic polypeptide
<400> 18
   Cys Glu Tyr Ser Asp Arg Trp Gly Asp Arg Ala Ile Glu Arg Asn Val
   1 5 10 15
   Tyr Leu Ser Thr
   20
<210> 19
   <211> 184
   <212> PRT
   <213> Artificial
<220>
   <223> Synthetic polypeptide
<400> 19
   Met Asp Arg Val Leu Leu Arg Trp Ile Ser Leu Phe Trp Leu Thr Ala
   1               5                   10                  15
   Met Val Glu Gly Leu Gln Val Thr Val Pro Asp Lys Lys Lys Val Ala
               20                  25                  30
   Met Leu Phe Gln Pro Thr Val Leu Arg Cys His Phe Ser Thr Ser Ser
           35                  40                  45
   His Gln Pro Ala Val Val Gln Trp Lys Phe Lys Ser Tyr Cys Gln Asp
       50                  55                  60
   Arg Met Gly Glu Ser Leu Gly Met Ser Ser Thr Arg Ala Gln Ser Leu
   65                  70                  75                  80
   Ser Lys Arg Asn Leu Glu Trp Asp Pro Tyr Leu Asp Cys Leu Asp Ser
                   85                  90                  95
   Arg Arg Thr Val Arg Val Val Ala Ser Lys Gln Gly Ser Thr Val Thr
               100                 105                 110
   Leu Gly Asp Phe Tyr Arg Gly Arg Glu Ile Thr Ile Val His Asp Ala
           115                 120                 125
   Asp Leu Gln Ile Gly Lys Leu Met Trp Gly Asp Ser Gly Leu Tyr Tyr
       130                 135                 140
   Cys Ile Ile Thr Thr Pro Asp Asp Leu Glu Gly Lys Asn Glu Asp Ser
   145                 150                 155                 160
   Val Glu Leu Leu Val Leu Gly Arg Thr Gly Leu Leu Ala Asp Leu Leu
                   165                 170                 175
   Pro Ser Phe Ala Val Glu Ile Met
               180
<210> 20
   <211> 232
   <212> PRT
   <213> Artificial
<220>
   <223> Synthetic polypeptide
<400> 20
   Glu Pro Lys Ser Cys Asp Lys Thr His Thr Cys Pro Pro Cys Pro Ala
   1               5                   10                  15
   Pro Glu Leu Leu Gly Gly Pro Ser Val Phe Leu Phe Pro Pro Lys Pro
               20                  25                  30
   Lys Asp Thr Leu Met Ile Ser Arg Thr Pro Glu Val Thr Cys Val Val
           35                  40                  45
   Val Asp Val Ser His Glu Asp Pro Glu Val Lys Phe Asn Trp Tyr Val
       50                  55                  60
   Asp Gly Val Glu Val His Asn Ala Lys Thr Lys Pro Arg Glu Glu Gln
   65                  70                  75                  80
   Tyr Asn Ser Thr Tyr Arg Val Val Ser Val Leu Thr Val Leu His Gln
                   85                  90                  95
   Asp Trp Leu Asn Gly Lys Glu Tyr Lys Cys Lys Val Ser Asn Lys Ala
               100                 105                 110
   Leu Pro Ala Pro Ile Glu Lys Thr Ile Ser Lys Ala Lys Gly Gln Pro
           115                 120                 125
   Arg Glu Pro Gln Val Tyr Thr Leu Pro Pro Ser Arg Asp Glu Leu Thr
       130                 135                 140
   Lys Asn Gln Val Ser Leu Thr Cys Leu Val Lys Gly Phe Tyr Pro Ser
   145                 150                 155                 160
   Asp Ile Ala Val Glu Trp Glu Ser Asn Gly Gln Pro Glu Asn Asn Tyr
                   165                 170                 175
   Lys Thr Thr Pro Pro Val Leu Asp Ser Asp Gly Ser Phe Phe Leu Tyr
               180                 185                 190
   Ser Lys Leu Thr Val Asp Lys Ser Arg Trp Gln Gln Gly Asn Val Phe
           195                 200                 205
   Ser Cys Ser Val Met His Glu Ala Leu His Asn His Tyr Thr Gln Lys
       210                 215                 220
   Ser Leu Ser Leu Ser Pro Gly Lys
   225                 230
<210> 21
   <211> 217
   <212> PRT
   <213> Artificial
<220>
   <223> Synthetic polypeptide
<400> 21
   Ala Pro Glu Leu Leu Gly Gly Pro Ser Val Phe Leu Phe Pro Pro Lys
   1               5                   10                  15
   Pro Lys Asp Thr Leu Met Ile Ser Arg Thr Pro Glu Val Thr Cys Val
               20                  25                  30
   Val Val Asp Val Ser His Glu Asp Pro Glu Val Lys Phe Asn Trp Tyr
           35                  40                  45
   Val Asp Gly Val Glu Val His Asn Ala Lys Thr Lys Pro Arg Glu Glu
       50                  55                  60
   Gln Tyr Asn Ser Thr Tyr Arg Val Val Ser Val Leu Thr Val Leu His
   65                  70                  75                  80
   Gln Asp Trp Leu Asn Gly Lys Glu Tyr Lys Cys Lys Val Ser Asn Lys
                   85                  90                  95
   Ala Leu Pro Ala Pro Ile Glu Lys Thr Ile Ser Lys Ala Lys Gly Gln
               100                 105                 110
   Pro Arg Glu Pro Gln Val Tyr Thr Leu Pro Pro Ser Arg Asp Glu Leu
           115                 120                 125
   Thr Lys Asn Gln Val Ser Leu Thr Cys Leu Val Lys Gly Phe Tyr Pro
       130                 135                 140
   Ser Asp Ile Ala Val Glu Trp Glu Ser Asn Gly Gln Pro Glu Asn Asn
   145                 150                 155                 160
   Tyr Lys Thr Thr Pro Pro Val Leu Asp Ser Asp Gly Ser Phe Phe Leu
                   165                 170                 175
   Tyr Ser Lys Leu Thr Val Asp Lys Ser Arg Trp Gln Gln Gly Asn Val
               180                 185                 190
   Phe Ser Cys Ser Val Met His Glu Ala Leu His Asn His Tyr Thr Gln
           195                 200                 205
   Lys Ser Leu Ser Leu Ser Pro Gly Lys
   210 215
<210> 22
   <211> 416
   <212> PRT
   <213> Artificial
<220>
   <223> Synthetic polypeptide
<400> 22
   Met Asp Arg Val Leu Leu Arg Trp Ile Ser Leu Phe Trp Leu Thr Ala
   1               5                   10                  15
   Met Val Glu Gly Leu Gln Val Thr Val Pro Asp Lys Lys Lys Val Ala
               20                  25                  30
   Met Leu Phe Gln Pro Thr Val Leu Arg Cys His Phe Ser Thr Ser Ser
           35                  40                  45
   His Gln Pro Ala Val Val Gln Trp Lys Phe Lys Ser Tyr Cys Gln Asp
       50                  55                  60
   Arg Met Gly Glu Ser Leu Gly Met Ser Ser Thr Arg Ala Gln Ser Leu
   65                  70                  75                  80
   Ser Lys Arg Asn Leu Glu Trp Asp Pro Tyr Leu Asp Cys Leu Asp Ser
                   85                  90                  95
   Arg Arg Thr Val Arg Val Val Ala Ser Lys Gln Gly Ser Thr Val Thr
               100                 105                 110
   Leu Gly Asp Phe Tyr Arg Gly Arg Glu Ile Thr Ile Val His Asp Ala
           115                 120                 125
   Asp Leu Gln Ile Gly Lys Leu Met Trp Gly Asp Ser Gly Leu Tyr Tyr
       130                 135                 140
   Cys Ile Ile Thr Thr Pro Asp Asp Leu Glu Gly Lys Asn Glu Asp Ser
   145                 150                 155                 160
   Val Glu Leu Leu Val Leu Gly Arg Thr Gly Leu Leu Ala Asp Leu Leu
                   165                 170                 175
   Pro Ser Phe Ala Val Glu Ile Met Glu Pro Lys Ser Cys Asp Lys Thr
               180                 185                 190
   His Thr Cys Pro Pro Cys Pro Ala Pro Glu Leu Leu Gly Gly Pro Ser
           195                 200                 205
   Val Phe Leu Phe Pro Pro Lys Pro Lys Asp Thr Leu Met Ile Ser Arg
       210                 215                 220
   Thr Pro Glu Val Thr Cys Val Val Val Asp Val Ser His Glu Asp Pro
   225                 230                 235                 240
   Glu Val Lys Phe Asn Trp Tyr Val Asp Gly Val Glu Val His Asn Ala
                   245                 250                 255
   Lys Thr Lys Pro Arg Glu Glu Gln Tyr Asn Ser Thr Tyr Arg Val Val
               260                 265                 270
   Ser Val Leu Thr Val Leu His Gln Asp Trp Leu Asn Gly Lys Glu Tyr
           275                 280                 285
   Lys Cys Lys Val Ser Asn Lys Ala Leu Pro Ala Pro Ile Glu Lys Thr
       290                 295                 300
   Ile Ser Lys Ala Lys Gly Gln Pro Arg Glu Pro Gln Val Tyr Thr Leu
   305                 310                 315                 320
   Pro Pro Ser Arg Asp Glu Leu Thr Lys Asn Gln Val Ser Leu Thr Cys
                   325                 330                 335
   Leu Val Lys Gly Phe Tyr Pro Ser Asp Ile Ala Val Glu Trp Glu Ser
               340                 345                 350
   Asn Gly Gln Pro Glu Asn Asn Tyr Lys Thr Thr Pro Pro Val Leu Asp
           355                 360                 365
   Ser Asp Gly Ser Phe Phe Leu Tyr Ser Lys Leu Thr Val Asp Lys Ser
       370                 375                 380
   Arg Trp Gln Gln Gly Asn Val Phe Ser Cys Ser Val Met His Glu Ala
   385                 390                 395                 400
   Leu His Asn His Tyr Thr Gln Lys Ser Leu Ser Leu Ser Pro Gly Lys
                   405                 410                 415
<210> 23
   <211> 411
   <212> PRT
   <213> Artificial
<220>
   <223> Synthetic polypeptide
<400> 23
   Met Asp Arg Val Leu Leu Arg Trp Ile Ser Leu Phe Trp Leu Thr Ala
   1               5                   10                  15
   Met Val Glu Gly Leu Gln Val Thr Val Pro Asp Lys Lys Lys Val Ala
               20                  25                  30
   Met Leu Phe Gln Pro Thr Val Leu Arg Cys His Phe Ser Thr Ser Ser
           35                  40                  45
   His Gln Pro Ala Val Val Gln Trp Lys Phe Lys Ser Tyr Cys Gln Asp
       50                  55                  60
   Arg Met Gly Glu Ser Leu Gly Met Ser Ser Thr Arg Ala Gln Ser Leu
   65                  70                  75                  80
   Ser Lys Arg Asn Leu Glu Trp Asp Pro Tyr Leu Asp Cys Leu Asp Ser
                   85                  90                  95
   Arg Arg Thr Val Arg Val Val Ala Ser Lys Gln Gly Ser Thr Val Thr
               100                 105                 110
   Leu Gly Asp Phe Tyr Arg Gly Arg Glu Ile Thr Ile Val His Asp Ala
           115                 120                 125
   Asp Leu Gln Ile Gly Lys Leu Met Trp Gly Asp Ser Gly Leu Tyr Tyr
       130                 135                 140
   Cys Ile Ile Thr Thr Pro Asp Asp Leu Glu Gly Lys Asn Glu Asp Ser
   145                 150                 155                 160
   Val Glu Leu Leu Val Leu Gly Arg Thr Gly Leu Leu Ala Asp Leu Leu
                   165                 170                 175
   Pro Ser Phe Ala Val Glu Ile Met Asp Lys Thr His Thr Cys Pro Pro
               180                 185                 190
   Cys Pro Ala Pro Glu Leu Leu Gly Gly Pro Ser Val Phe Leu Phe Pro
           195                 200                 205
   Pro Lys Pro Lys Asp Thr Leu Met Ile Ser Arg Thr Pro Glu Val Thr
       210                 215                 220
   Cys Val Val Val Asp Val Ser His Glu Asp Pro Glu Val Lys Phe Asn
   225                 230                 235                 240
   Trp Tyr Val Asp Gly Val Glu Val His Asn Ala Lys Thr Lys Pro Arg
                   245                 250                 255
   Glu Glu Gln Tyr Asn Ser Thr Tyr Arg Val Val Ser Val Leu Thr Val
               260                 265                 270
   Leu His Gln Asp Trp Leu Asn Gly Lys Glu Tyr Lys Cys Lys Val Ser
           275                 280                 285
   Asn Lys Ala Leu Pro Ala Pro Ile Glu Lys Thr Ile Ser Lys Ala Lys
       290                 295                 300
   Gly Gln Pro Arg Glu Pro Gln Val Tyr Thr Leu Pro Pro Ser Arg Asp
   305                 310                 315                 320
   Glu Leu Thr Lys Asn Gln Val Ser Leu Thr Cys Leu Val Lys Gly Phe
                   325                 330                 335
   Tyr Pro Ser Asp Ile Ala Val Glu Trp Glu Ser Asn Gly Gln Pro Glu
               340                 345                 350
   Asn Asn Tyr Lys Thr Thr Pro Pro Val Leu Asp Ser Asp Gly Ser Phe
           355                 360                 365
   Phe Leu Tyr Ser Lys Leu Thr Val Asp Lys Ser Arg Trp Gln Gln Gly
       370                 375                 380
   Asn Val Phe Ser Cys Ser Val Met His Glu Ala Leu His Asn His Tyr
   385                 390                 395                 400
   Thr Gln Lys Ser Leu Ser Leu Ser Pro Gly Lys
                   405                 410
<210> 24
   <211> 2
   <212> PRT
   <213> Artificial
<220>
   <223> Synthetic polypeptide
<400> 24
   Gly Ser
   1
<210> 25
   <211> 4
   <212> PRT
   <213> Artificial
<220>
   <223> Synthetic polypeptide
<400> 25
   Gly Ser Gly Ser
   1
<210> 26
   <211> 2
   <212> PRT
   <213> Artificial
<220>
   <223> Synthetic polypeptide
<400> 26
   Ala Ser
   1
<210> 27
   <211> 4
   <212> PRT
   <213> Artificial
<220>
   <223> Synthetic polypeptide
<400> 27
   Gly Gly Gly Ser
   1
<210> 28
   <211> 170
   <212> PRT
   <213> Artificial
<220>
   <223> Synthetic polypeptide
<400> 28
<210> 29
   <211> 411
   <212> PRT
   <213> Artificial
<220>
   <223> Synthetic polypeptide
<400> 29
   Met Asp Arg Val Leu Leu Arg Trp Ile Ser Leu Phe Trp Leu Thr Ala
   1               5                   10                  15
   Met Val Glu Gly Leu Gln Val Thr Val Pro Asp Lys Lys Lys Val Ala
               20                  25                  30
   Met Leu Phe Gln Pro Thr Val Leu Arg Cys His Phe Ser Thr Ser Ser
           35                  40                  45
   His Gln Pro Ala Val Val Gln Trp Lys Phe Lys Ser Tyr Cys Gln Asp
       50                  55                  60
   Arg Met Gly Glu Ser Leu Gly Met Ser Ser Thr Arg Ala Gln Ser Leu
   65                  70                  75                  80
   Ser Lys Arg Asn Leu Glu Trp Asp Pro Tyr Leu Asp Cys Leu Asp Ser
                   85                  90                  95
   Arg Arg Thr Val Arg Val Val Ala Ser Lys Gln Gly Ser Thr Val Thr
               100                 105                 110
   Leu Gly Asp Phe Tyr Arg Gly Arg Glu Ile Thr Ile Val His Asp Ala
           115                 120                 125
   Asp Leu Gln Ile Gly Lys Leu Met Trp Gly Asp Ser Gly Leu Tyr Tyr
       130                 135                 140
   Cys Ile Ile Thr Thr Pro Asp Asp Leu Glu Gly Lys Asn Glu Gly Ser
   145                 150                 155                 160
   Leu Gly Leu Leu Val Leu Gly Arg Thr Gly Leu Leu Ala Asp Leu Leu
                   165                 170                 175
   Pro Ser Phe Ala Val Glu Ile Met Asp Lys Thr His Thr Cys Pro Pro
               180                 185                 190
   Cys Pro Ala Pro Glu Leu Leu Gly Gly Pro Ser Val Phe Leu Phe Pro
           195                 200                 205
   Pro Lys Pro Lys Asp Thr Leu Met Ile Ser Arg Thr Pro Glu Val Thr
       210                 215                 220
   Cys Val Val Val Asp Val Ser His Glu Asp Pro Glu Val Lys Phe Asn
   225                 230                 235                 240
   Trp Tyr Val Asp Gly Val Glu Val His Asn Ala Lys Thr Lys Pro Arg
                   245                 250                 255
   Glu Glu Gln Tyr Asn Ser Thr Tyr Arg Val Val Ser Val Leu Thr Val
               260                 265                 270
   Leu His Gln Asp Trp Leu Asn Gly Lys Glu Tyr Lys Cys Lys Val Ser
           275                 280                 285
   Asn Lys Ala Leu Pro Ala Pro Ile Glu Lys Thr Ile Ser Lys Ala Lys
       290                 295                 300
   Gly Gln Pro Arg Glu Pro Gln Val Tyr Thr Leu Pro Pro Ser Arg Asp
   305                 310                 315                 320
   Glu Leu Thr Lys Asn Gln Val Ser Leu Thr Cys Leu Val Lys Gly Phe
   325 330 335
   Tyr Pro Ser Asp Ile Ala Val Glu Trp Glu Ser Asn Gly Gln Pro Glu
   340 345 350
   Asn Asn Tyr Lys Thr Thr Pro Pro Val Leu Asp Ser Asp Gly Ser Phe
   355 360 365
   Phe Leu Tyr Ser Lys Leu Thr Val Asp Lys Ser Arg Trp Gln Gln Gly
   370 375 380
   Asn Val Phe Ser Cys Ser Val Met His Glu Ala Leu His Asn His Tyr
   385 390 395 400
   Thr Gln Lys Ser Leu Ser Leu Ser Pro Gly Lys
   405 410
<210> 30
   <211> 169
   <212> PRT
   <213> Artificial
<220>
   <223> Synthetic polypeptide
<400> 30
   Met Asp Arg Val Leu Leu Arg Trp Ile Ser Leu Phe Trp Leu Thr Ala
   1 5 10 15
   Met Val Glu Gly Leu Gln Val Thr Val Pro Asp Lys Lys Lys Val Ala
   20 25 30
   Met Leu Phe Gln Pro Thr Val Leu Arg Cys His Phe Ser Thr Ser Ser
   35 40 45
   His Gln Pro Ala Val Val Gln Trp Lys Phe Lys Ser Tyr Cys Gln Asp
   50 55 60
   Arg Met Gly Glu Ser Leu Gly Met Ser Ser Thr Arg Ala Gln Ser Leu
   65 70 75 80
   Ser Lys Arg Asn Leu Glu Trp Asp Pro Tyr Leu Asp Cys Leu Asp Ser
   85 90 95
   Arg Arg Thr Val Arg Val Val Ala Ser Lys Gln Gly Ser Thr Val Thr
   100 105 110
   Leu Gly Asp Phe Tyr Arg Gly Arg Glu Ile Thr Ile Val His Asp Ala
           115                 120                 125
   Asp Leu Gln Ile Gly Lys Leu Met Trp Gly Asp Ser Gly Leu Tyr Tyr
       130                 135                 140
   Cys Ile Ile Thr Thr Pro Asp Asp Leu Glu Gly Lys Asn Glu Asp Ser
   145                 150                 155                 160
   Val Glu Leu Leu Val Leu Glu Trp Val
                   165
<210> 31
   <211> 233
   <212> PRT
   <213> Artificial
<220>
   <223> Synthetic polypeptide
<400> 31
   Glu Pro Arg Gly Pro Thr Ile Lys Pro Cys Pro Pro Cys Lys Cys Pro
   1               5                   10                  15
   Ala Pro Asn Leu Leu Gly Gly Pro Ser Val Phe Ile Phe Pro Pro Lys
               20                  25                  30
   Ile Lys Asp Val Leu Met Ile Ser Leu Ser Pro Ile Val Thr Cys Val
           35                  40                  45
   Val Val Asp Val Ser Glu Asp Asp Pro Asp Val Gln Ile Ser Trp Phe
       50                  55                  60
   Val Asn Asn Val Glu Val His Thr Ala Gln Thr Gln Thr His Arg Glu
   65                  70                  75                  80
   Asp Tyr Asn Ser Thr Leu Arg Val Val Ser Ala Leu Pro Ile Gln His
                   85                  90                  95
   Gln Asp Trp Met Ser Gly Lys Glu Phe Lys Cys Lys Val Asn Asn Lys
               100                 105                 110
   Asp Leu Pro Ala Pro Ile Glu Arg Thr Ile Ser Lys Pro Lys Gly Ser
           115                 120                 125
   Val Arg Ala Pro Gln Val Tyr Val Leu Pro Pro Pro Glu Glu Glu Met
       130                 135                 140
   Thr Lys Lys Gln Val Thr Leu Thr Cys Met Val Thr Asp Phe Met Pro
   145 150 155 160
   Glu Asp Ile Tyr Val Glu Trp Thr Asn Asn Gly Lys Thr Glu Leu Asn
   165 170 175
   Tyr Lys Asn Thr Glu Pro Val Leu Asp Ser Asp Gly Ser Tyr Phe Met
   180 185 190
   Tyr Ser Lys Leu Arg Val Glu Lys Lys Asn Trp Val Glu Arg Asn Ser
   195 200 205
   Tyr Ser Cys Ser Val Val His Glu Gly Leu His Asn His His Thr Thr
   210 215 220
   Lys Ser Phe Ser Arg Thr Pro Gly Lys
   225 230
<210> 32
   <211> 15
   <212> PRT
   <213> Artificial
<220>
   <223> Synthetic polypeptide
<400> 32
   Gly Gly Gly Gly Ser Gly Gly Gly Gly Ser Gly Gly Gly Gly Ser
   1 5 10 15
<210> 33
   <211> 20
   <212> PRT
   <213> Artificial
<220>
   <223> Synthetic polypeptide
<400> 33
   Gly Gly Gly Gly Ser Gly Gly Gly Gly Ser Gly Gly Gly Gly Ser Gly
   1 5 10 15
   Gly Gly Gly Ser
   20
<210> 34
   <211> 235
   <212> PRT
   <213> Artificial
<220>
   <223> Synthetic polypeptide
<400> 34
   Met Asp Arg Val Leu Leu Arg Trp Ile Ser Leu Phe Trp Leu Thr Ala
   1               5                   10                  15
   Met Val Glu Gly Leu Gln Val Thr Val Pro Asp Lys Lys Lys Val Ala
               20                  25                  30
   Met Leu Phe Gln Pro Thr Val Leu Arg Cys His Phe Ser Thr Ser Ser
           35                  40                  45
   His Gln Pro Ala Val Val Gln Trp Lys Phe Lys Ser Tyr Cys Gln Asp
       50                  55                  60
   Arg Met Gly Glu Ser Leu Gly Met Ser Ser Thr Arg Ala Gln Ser Leu
   65                  70                  75                  80
   Ser Lys Arg Asn Leu Glu Trp Asp Pro Tyr Leu Asp Cys Leu Asp Ser
                   85                  90                  95
   Arg Arg Thr Val Arg Val Val Ala Ser Lys Gln Gly Ser Thr Val Thr
               100                 105                 110
   Leu Gly Asp Phe Tyr Arg Gly Arg Glu Ile Thr Ile Val His Asp Ala
           115                 120                 125
   Asp Leu Gln Ile Gly Lys Leu Met Trp Gly Asp Ser Gly Leu Tyr Tyr
       130                 135                 140
   Cys Ile Ile Thr Thr Pro Asp Asp Leu Glu Gly Lys Asn Glu Asp Ser
   145                 150                 155                 160
   Val Glu Leu Leu Val Leu Glu Trp Val Phe Val Gly Leu Val Leu Leu
                   165                 170                 175
   Gly Val Phe Leu Phe Phe Val Leu Val Gly Ile Cys Trp Cys Gln Cys
               180                 185                 190
   Cys Pro His Ser Cys Cys Cys Tyr Val Arg Cys Pro Cys Cys Pro Asp
           195                 200                 205
   Ser Cys Cys Cys Pro Gln Ala Cys Glu Tyr Ser Asp Arg Trp Gly Asp
       210                 215                 220
   Arg Ala Ile Glu Arg Asn Val Tyr Leu Ser Thr
   225 230 235
<210> 35
   <211> 166
   <212> PRT
   <213> Artificial
<220>
   <223> Synthetic polypeptide
<400> 35
   Leu Gln Val Thr Val Pro Asp Lys Lys Lys Val Ala Met Leu Phe Gln
   1               5                   10                  15
   Pro Thr Val Leu Arg Cys His Phe Ser Thr Ser Ser His Gln Pro Ala
               20                  25                  30
   Val Val Gln Trp Lys Phe Lys Ser Tyr Cys Gln Asp Arg Met Gly Glu
           35                  40                  45
   Ser Leu Gly Met Ser Ser Thr Arg Ala Gln Ser Leu Ser Lys Arg Asn
       50                  55                  60
   Leu Glu Trp Asp Pro Tyr Leu Asp Cys Leu Asp Ser Arg Arg Thr Val
   65                  70                  75                  80
   Arg Val Val Ala Ser Lys Gln Gly Ser Thr Val Thr Leu Gly Asp Phe
                   85                  90                  95
   Tyr Arg Gly Arg Glu Ile Thr Ile Val His Asp Ala Asp Leu Gln Ile
               100                 105                 110
   Gly Lys Leu Met Trp Gly Asp Ser Gly Leu Tyr Tyr Cys Ile Ile Thr
           115                 120                 125
   Thr Pro Asp Asp Leu Glu Gly Lys Asn Glu Asp Ser Val Glu Leu Leu
       130                 135                 140
   Val Leu Gly Arg Thr Gly Leu Leu Ala Asp Leu Leu Pro Ser Phe Ala
   145                 150                 155                 160
   Val Glu Ile Met Pro Glu
                   165
<210> 36
   <211> 149
   <212> PRT
   <213> Artificial
<220>
   <223> Synthetic polypeptide
<400> 36
   Leu Gln Val Thr Val Pro Asp Lys Lys Lys Val Ala Met Leu Phe Gln
   1               5                   10                  15
   Pro Thr Val Leu Arg Cys His Phe Ser Thr Ser Ser His Gln Pro Ala
               20                  25                  30
   Val Val Gln Trp Lys Phe Lys Ser Tyr Cys Gln Asp Arg Met Gly Glu
           35                  40                  45
   Ser Leu Gly Met Ser Ser Thr Arg Ala Gln Ser Leu Ser Lys Arg Asn
       50                  55                  60
   Leu Glu Trp Asp Pro Tyr Leu Asp Cys Leu Asp Ser Arg Arg Thr Val
   65                  70                  75                  80
   Arg Val Val Ala Ser Lys Gln Gly Ser Thr Val Thr Leu Gly Asp Phe
                   85                  90                  95
   Tyr Arg Gly Arg Glu Ile Thr Ile Val His Asp Ala Asp Leu Gln Ile
               100                 105                 110
   Gly Lys Leu Met Trp Gly Asp Ser Gly Leu Tyr Tyr Cys Ile Ile Thr
           115                 120                 125
   Thr Pro Asp Asp Leu Glu Gly Lys Asn Glu Asp Ser Val Glu Leu Leu
       130                 135                 140
   Val Leu Glu Trp Val
   145
<210> 37
   <211> 184
   <212> PRT
   <213> Artificial
<220>
   <223> Synthetic polypeptide
<400> 37
   Met Asp Arg Val Leu Leu Arg Trp Ile Ser Leu Phe Trp Leu Thr Ala
   1               5                   10                  15
   Met Val Glu Gly Leu Gln Val Thr Val Pro Asp Lys Lys Lys Val Ala
               20                  25                  30
   Met Leu Phe Gln Pro Thr Val Leu Arg Cys His Phe Ser Thr Ser Ser
           35                  40                  45
   His Gln Pro Ala Val Val Gln Trp Lys Phe Lys Ser Tyr Cys Gln Asp
       50                  55                  60
   Arg Met Gly Glu Ser Leu Gly Met Ser Ser Thr Arg Ala Gln Ser Leu
   65                  70                  75                  80
   Ser Lys Arg Asn Leu Glu Trp Asp Pro Tyr Leu Asp Cys Leu Asp Ser
                   85                  90                  95
   Arg Arg Thr Val Arg Val Val Ala Ser Lys Gln Gly Ser Thr Val Thr
               100                 105                 110
   Leu Gly Asp Phe Tyr Arg Gly Arg Glu Ile Thr Ile Val His Asp Ala
           115                 120                 125
   Asp Leu Gln Ile Gly Lys Leu Met Trp Gly Asp Ser Gly Leu Tyr Tyr
       130                 135                 140
   Cys Ile Ile Thr Thr Pro Asp Asp Leu Glu Gly Lys Asn Glu Gly Ser
   145                 150                 155                 160
   Leu Gly Leu Leu Val Leu Gly Arg Thr Gly Leu Leu Ala Asp Leu Leu
                   165                 170                 175
   Pro Ser Phe Ala Val Glu Ile Met
               180
<210> 38
   <211> 416
   <212> PRT
   <213> Artificial
<220>
   <223> Synthetic polypeptide
<400> 38
   Met Asp Arg Val Leu Leu Arg Trp Ile Ser Leu Phe Trp Leu Thr Ala
   1               5                   10                  15
   Met Val Glu Gly Leu Gln Val Thr Val Pro Asp Lys Lys Lys Val Ala
               20                  25                  30
   Met Leu Phe Gln Pro Thr Val Leu Arg Cys His Phe Ser Thr Ser Ser
           35                  40                  45
   His Gln Pro Ala Val Val Gln Trp Lys Phe Lys Ser Tyr Cys Gln Asp
   50 55 60
   Arg Met Gly Glu Ser Leu Gly Met Ser Ser Thr Arg Ala Gln Ser Leu
   65 70 75 80
   Ser Lys Arg Asn Leu Glu Trp Asp Pro Tyr Leu Asp Cys Leu Asp Ser
   85 90 95
   Arg Arg Thr Val Arg Val Val Ala Ser Lys Gln Gly Ser Thr Val Thr
   100 105 110
   Leu Gly Asp Phe Tyr Arg Gly Arg Glu Ile Thr Ile Val His Asp Ala
   115 120 125
   Asp Leu Gln Ile Gly Lys Leu Met Trp Gly Asp Ser Gly Leu Tyr Tyr
   130 135 140
   Cys Ile Ile Thr Thr Pro Asp Asp Leu Glu Gly Lys Asn Glu Gly Ser
   145 150 155 160
   Leu Gly Leu Leu Val Leu Gly Arg Thr Gly Leu Leu Ala Asp Leu Leu
   165 170 175
   Pro Ser Phe Ala Val Glu Ile Met Glu Pro Lys Ser Cys Asp Lys Thr
   180 185 190
   His Thr Cys Pro Pro Cys Pro Ala Pro Glu Leu Leu Gly Gly Pro Ser
   195 200 205
   Val Phe Leu Phe Pro Pro Lys Pro Lys Asp Thr Leu Met Ile Ser Arg
   210 215 220
   Thr Pro Glu Val Thr Cys Val Val Val Asp Val Ser His Glu Asp Pro
   225 230 235 240
   Glu Val Lys Phe Asn Trp Tyr Val Asp Gly Val Glu Val His Asn Ala
   245 250 255
   Lys Thr Lys Pro Arg Glu Glu Gln Tyr Asn Ser Thr Tyr Arg Val Val
   260 265 270
   Ser Val Leu Thr Val Leu His Gln Asp Trp Leu Asn Gly Lys Glu Tyr
   275 280 285
   Lys Cys Lys Val Ser Asn Lys Ala Leu Pro Ala Pro Ile Glu Lys Thr
   290 295 300
   Ile Ser Lys Ala Lys Gly Gln Pro Arg Glu Pro Gln Val Tyr Thr Leu
   305 310 315 320
   Pro Pro Ser Arg Asp Glu Leu Thr Lys Asn Gln Val Ser Leu Thr Cys
   325 330 335
   Leu Val Lys Gly Phe Tyr Pro Ser Asp Ile Ala Val Glu Trp Glu Ser
   340 345 350
   Asn Gly Gln Pro Glu Asn Asn Tyr Lys Thr Thr Pro Pro Val Leu Asp
   355 360 365
   Ser Asp Gly Ser Phe Phe Leu Tyr Ser Lys Leu Thr Val Asp Lys Ser
   370 375 380
   Arg Trp Gln Gln Gly Asn Val Phe Ser Cys Ser Val Met His Glu Ala
   385 390 395 400
   Leu His Asn His Tyr Thr Gln Lys Ser Leu Ser Leu Ser Pro Gly Lys
   405 410 415
<210> 39
   <211> 5
   <212> PRT
   <213> Artificial
<220>
   <223> Synthetic polypeptide
<400> 39
   Gly Gly Gly Gly Ser
   1 5
<210> 40
   <211> 10
   <212> PRT
   <213> Artificial
<220>
   <223> Synthetic polypeptide
<400> 40
   Gly Gly Gly Gly Ser Gly Gly Gly Gly Ser
   1 5 10